# EUROPEAN PATENT APPLICATION

(11) **EP 4 831 167 A1**
(43) Date of publication of application: **28.10.2026**
(21) Application number: 24906497.3
(22) Date of filing: 20.12.2024
(51) Int. Cl.: C07D 401/04, C07D 401/14, C07D 405/04, C07D 405/14, C07D 413/04, C07D 413/14, C07D 409/14, C07D 417/04, C07D 471/04, C07D 417/14, C07D 471/14, A61K 31/4709, A61P 9/12

(54) **AROMATIC HETEROCYCLIC COMPOUND, AND PREPARATION METHOD THEREFOR AND USE THEREOF**

(30) Priority: 22.12.2023 CN 202311781771; 28.06.2024 CN 202410850958
(71) Applicant: Shanghai Innovstone Therapeutics Co., Ltd., Shanghai, 201318 (CN)
(72) Inventor: LIU, Tianqi, Shanghai, 201318 (CN); SONG, Yunlong, Shanghai, 201318 (CN); DUAN, Yunxia, Shanghai, 201318 (CN); WANG, Disha, Shanghai, 201318 (CN); KOU, Hongyan, Shanghai, 201318 (CN); YU, Ning, Shanghai, 201318 (CN); WANG, Yue, Shanghai, 201318 (CN); MIAO, Xinyuan, Shanghai, 201318 (CN); MU, Yongzhao, Shanghai, 201318 (CN); LU, Kai, Shanghai, 201318 (CN)
(74) Representative: Barrow, Nicholas Martin
(86) International application number: PCT/CN2024/140826
(87) International publication number: WO 2025/131022

(57) **Abstract**

Provided are a compound represented by formula (I), and a stereoisomer, tautomer or pharmaceutically acceptable salt thereof. The compound represented by formula (I) serves as a CYP11B2 inhibitor. The compound and a pharmaceutical composition containing same are useful for the preparation of a drug for treating and/or preventing CYP11B2-mediated diseases.

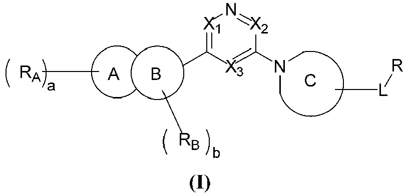

## Description

### Cross Reference to Related Application

The present application claims priority and benefits of Chinese Patent Application No. 202311781771.4 filed with the China National Intellectual Property Administration on December 22, 2023, and Chinese Patent Application No. 202410850958.3 filed with the China National Intellectual Property Administration on June 28, 2024, the entire contents of which are incorporated herein by reference.

### Technical Field

The present application relates to the technical field of medicines, and specifically relates to a class of aromatic heterocyclic compounds, and a preparation method therefor and the use thereof.

### Background Art

Hypertension is a global chronic non-communicable disease, and ranks among the major threats to human health. It stands as the leading cause of disease burden worldwide, and also poses a critical public health problem in China. The prevalence of hypertension in China has been increasing year by year, rising from 5.1% in 1959 to 23.2% in 2015 and further to 27.5% in 2018, which brings enormous challenges to hypertension prevention and treatment efforts.

Antihypertensive drugs refer to a class of medications that can control blood pressure and treat hypertension. They can prevent damages to target organs such as the heart, brain and kidneys caused by long-term elevated blood pressure, and reduce the risk of major cardio-cerebrovascular diseases such as stroke. Based on their mechanism of action, antihypertensive drugs can be classified into diuretics, alpha-blockers, beta-blockers, calcium channel blockers (CCBs), angiotensin-converting enzyme inhibitors (ACEIs), angiotensin receptor blockers (ARBs), and compound preparations.

In hypertension patients receiving long-term treatment with ACEIs or ARBs, the "aldosterone escape" phenomenon leads to a rebound in aldosterone levels after an initial decline, which is associated with end-organ injury. Mineralocorticoid receptor antagonists (MRAs) suppress plasma aldosterone levels, which triggers a compensatory rise in aldosterone and thus limits their therapeutic efficacy. In addition, the adverse reactions of steroidal MRAs are mostly associated with renal impairment or hyperkalaemia, which are particularly unfavourable for patients with kidney injury, resulting in restricted clinical application.

As a component of the renin-angiotensin-aldosterone system (RAAS), aldosterone not only regulates fluid and electrolyte homeostasis, but also serves as an important factor in end-organ injury in various cardiovascular and renal diseases. Aldosterone excess leads to water and sodium retention and increased blood volume, and is one of the major causes of hypertension. Furthermore, aldosterone excess can further lead to cardiovascular and renal events such as inflammation, organ injury and fibrosis. Accordingly, reducing aldosterone levels may be an effective strategy for the management of refractory hypertension and diseases caused by aldosterone excess.

Aldosterone synthase (CYP11B2) is a mitochondrial cytochrome P450 monooxygenase that uses molecular oxygen and electrons derived from NADPH through adrenodoxin reductase (AdR) and adrenodoxin (Adx) to catalyse a three-step oxidation sequence, converting 11-deoxycorticosterone (11-DOC) into aldosterone via the intermediate products corticosterone and 18-hydroxycorticosterone. CYP11B2 is the key enzyme in the final three steps of aldosterone biosynthesis. Inhibition of CYP11B2 can prevent the formation of aldosterone and reduce its circulating plasma levels, thereby representing a promising effective strategy for the treatment of diseases caused by aldosterone excess. Therefore, in recent years, blocking aldosterone biosynthesis through aldosterone synthase inhibitors has been explored as an alternative approach. The development of aldosterone synthase inhibitors is of great significance in the treatment of hypertension.

Currently, the development of CYP11B2 inhibitors has progressed to the clinical stage. For example, baxdrostat, lorundrostat, BI-690517, etc. have completed Phase II clinical trials, but no CYP11B2 inhibitor has yet been approved for marketing. Therefore, discovering effective and safe CYP11B2 inhibitors remains highly necessary.

### Summary of the Invention

The present application provides a class of compounds having novel structures, and a pharmaceutical composition comprising same, a preparation method therefor, and the use thereof as an inhibitor of aldosterone synthase (CYP11B2).

In a first aspect, the present application provides a compound as represented by formula (I), or a stereoisomer, tautomer, isotopic derivative (preferably deuterated derivative) or pharmaceutically acceptable salt thereof: 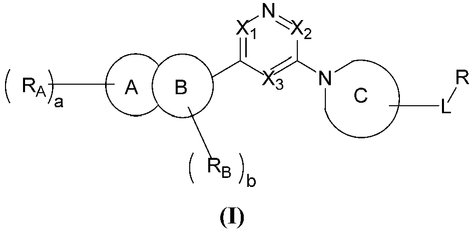 wherein,
ring A is 5- to 6-membered heterocyclyl, 5- to 6-membered heteroaryl or phenyl;
ring B is phenyl, 5- to 6-membered heterocyclyl or 5- to 6-membered heteroaryl;
each R_{A} at each occurrence is independently selected from deuterium, halogen, oxo, -CN, -NH₂, or the following optionally substituted groups: C₁-₄ alkyl, C₁-₄ deuterioalkyl (the "deuterio" means being substituted with one or more (such as 2, 3, 4, 5, 6, 7 or 8) deuterium atom(s); preferably C₁-₃ deuterioalkyl, e.g., -CD₃), C₃-₄ cycloalkyl, C₁-₄ alkoxy, C₁-₄ alkylthio, C₁-₄ haloalkyl and C₁-₄ haloalkoxy; or, when two R_{A} groups are attached to the same carbon atom of the 5- to 6-membered heterocyclyl, and the two R_{A} groups are each independently selected from C₁-₄ alkyl, C₁-₄ alkoxy and C₁-₄ alkylthio, the two R_{A} groups together with the carbon atom to which they are attached form C₃-₆ cycloalkyl or 3- to 7-membered heterocyclyl; wherein, the optionally substituted means being unsubstituted or substituted with one or more (such as 2, 3, 4, 5 or 6) substituents independently selected from deuterium, halogen (such as fluoro, chloro, or bromo), C₁₋₃ alkyl (such as methyl, ethyl, propyl, or isopropyl), C₁₋₃ alkoxy (such as methoxy, ethoxy, propoxy, or isopropoxy), hydroxy, and oxo;
each R_{B} at each occurrence is independently selected from deuterium, halogen, oxo, -CN, -NH₂, or the following optionally substituted groups: C₁-₄ alkyl, C₃-₄ cycloalkyl, C₁-₄ alkoxy, C₁-₄ alkylthio, C₁-₄ haloalkyl and C₁-₄ haloalkoxy; or, when two R_{B} groups are attached to the same carbon atom of the 5- to 6-membered heterocyclyl, and the two R_{B} groups are each independently selected from C₁-₄ alkyl, C₁-₄ alkoxy and C₁-₄ alkylthio, the two R_{B} groups together with the carbon atom to which they are attached form C₃-₆ cycloalkyl or 3-to 7-membered heterocyclyl; wherein, the optionally substituted means being unsubstituted or substituted with one or more (such as 2, 3, 4, 5 or 6) substituents independently selected from deuterium, halogen (such as fluoro, chloro, or bromo), C₁₋₃ alkyl (such as methyl, ethyl, propyl, or isopropyl), C₁₋₃ alkoxy (such as methoxy, ethoxy, propoxy, or isopropoxy), hydroxy, and oxo;
ring C is optionally substituted 4- to 8-membered heterocyclyl; wherein the optionally substituted means being unsubstituted or substituted with one or more (such as 2, 3, 4, 5, 6, 7 or 8) substituents, and the substituents are each independently selected from deuterium, halogen, oxo, -CN, -NH₂, -OH, C₁-₄ alkyl, C₁-₄ alkoxy, C₁-₄ alkylthio, C₁-₄ haloalkyl and C₁-₄ haloalkoxy; the heterocyclyl contains one N atom, and optionally, further contains 1, 2 or 3 heteroatoms, wherein the heteroatoms are independently selected from O, N and S;
X₁ is selected from N and CR_{X1};
X₂ is selected from N and CR_{X2};
X₃ is selected from N and CR_{X3};
provided that X₁, X₂ and X₃ are not N simultaneously;
R_{X1}, R_{X2}, and R_{X3} are each independently selected from hydrogen, deuterium, halogen, -OH, -CN, -NH₂, C₁-₃ alkyl, C₁-₃ alkoxy, C₁-₃ alkylthio, C₁-₃ haloalkyl and C₁-₃ haloalkoxy;
L is a bond, -O-, -S-, or the following optionally substituted group: -C₀₋₄ alkylene-N(R₁)-, -C₀₋₄ alkylene-C(O)-, -C₀₋₄ alkylene-N(R₁)-C(O)-, -C₀₋₄ alkylene-N(R₁)-C(O)-N(R₁)-, -C₀₋₄ alkylene-N(R₁)-S(O)₂-, -C₀₋₄ alkylene-C(O)-N(R₁)-S(O)₂-, -C₀₋₄ alkylene-C(O)-N(R₁)-, -C₀₋₄ alkylene-S(O)₂-N(R₁)-, -C₁₋₄ alkylene-, -C₃-₆ cycloalkyl-C₀₋₄ alkylene-C(O)-N(R₁)-, or 3- to 10-membered heterocyclyl-C₀₋₄ alkylene-C(O)-N(R₁)-; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl, C₁-₄ alkoxy and C₁-₄ alkylthio;
R₁ is selected from hydrogen, deuterium, or the following optionally substituted groups: C₁-₃ alkyl and C₃-₆ cycloalkyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
R is selected from -N(R₂)R₃ and -S(O)₂R₄, or is the following group optionally substituted with one or more (such as 2, 3, 4, 5, 6, 7 or 8) R_{w1}: C₁-₆ alkyl, C₁-₆ alkoxy, C₁-₆ alkylthio, C₃-₁₀ cycloalkyl, 3- to 10-membered heterocyclyl, 5- to 12-membered heteroaryl, or C₆₋₁₂ aryl; wherein, R_{w1} is selected from deuterium, halogen, oxo, -CN, -OH, -N(R₂)R₃, -N(R₅)-C(O)-R₆, -S(O)₂R₄, -C(O)OR₂, -C(O)-N(R₂)R₃, or the following groups optionally substituted with one or more (such as 2, 3, 4, 5, 6, 7 or 8) R_{w2}: C₁-₄ alkyl, C₁-₄ alkoxy, C₁-₄ alkylthio, C₃-₁₀ cycloalkyl, 3- to 10-membered heterocyclyl, C₆₋₁₀ aryl, and 5- to 12-membered heteroaryl; wherein, R_{w2} is selected from deuterium, halogen, oxo, -CN, -OH, -N(R₂)R₃, -S(O)₂R₄, -C(O)N(R₇)R₈, -C(O)OR₉, or the following groups optionally substituted with one or more (such as 2, 3, 4, 5, 6, 7 or 8) R_{w3}: C₁-₄ alkyl, C₁-₄ alkoxy, C₁-₄ alkylthio, C₆₋₁₀ aryl, and 5- to 12-membered heteroaryl; wherein, R_{w3} is selected from deuterium, halogen, oxo, -CN, -OH, -N(R₂)R₃, C₁-₄ alkyl, C₁-₄ alkoxy, C₁-₄ alkylthio, C₃-₆ cycloalkyl, 3- to 6-membered heterocyclyl, 5- to 6-membered heteroaryl, phenyl, C₁-₄ haloalkyl and C₁-₄ haloalkoxy;
R₂ and R₃ are each independently selected from hydrogen, deuterium, or the following optionally substituted groups: C₁-₆ alkyl, C₃-₆ cycloalkyl and 4- to 6-membered heterocyclyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
R₄ is selected from hydrogen, deuterium, or the following optionally substituted groups: C₁-₃ alkyl and C₃-₆ cycloalkyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
R₅ is selected from hydrogen, deuterium, or the following optionally substituted groups: C₁-₃ alkyl and C₃-₆ cycloalkyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
R₆ is selected from hydrogen, deuterium, or the following optionally substituted groups: C₁-₃ alkyl, C₃-₆ cycloalkyl and C₄-₆ heterocyclyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
R₇ and R₈ are each independently selected from hydrogen, deuterium, or the following optionally substituted groups: C₁-₃ alkyl, C₃-₆ cycloalkyl and C₄-₆ heterocyclyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
R₉ is selected from hydrogen, deuterium, or the following optionally substituted groups: C₁-₆ alkyl and C₃-₆ cycloalkyl; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl (preferably C₁₋₃ alkyl, such as methyl, ethyl, n-propyl, n-butyl, isopropyl, or tert-butyl), C₁-₄ alkoxy (preferably C₁₋₃ alkoxy, such as methoxy or ethoxy) and C₁-₄ alkylthio (preferably C₁₋₃ alkylthio, such as methylthio or ethylthio);
a is selected from 0, 1, 2, 3 and 4;
b is selected from 0, 1, 2, 3 and 4;
unless otherwise specified, the heteroatom(s) in the above-described heterocyclyl or heteroaryl are independently selected from O, N and S, and the number of heteroatoms is 1, 2, 3 or 4;
provided that a combination of definitions of the above-described variables results in a stable chemical structure.

In some embodiments,
each R_{A} at each occurrence is independently selected from deuterium, halogen, oxo, -CN, -NH₂, C₁-₄ alkyl, C₃-₄ cycloalkyl, C₁-₄ alkoxy, C₁-₄ alkylthio, C₁-₄ haloalkyl and C₁-₄ haloalkoxy; or, when two R_{A} groups are attached to the same carbon atom of the 5- to 6-membered heterocyclyl, and the two R_{A} groups are each independently selected from C₁-₄ alkyl, the two R_{A} groups together with the carbon atom to which they are attached form C₃-₆ cycloalkyl;
each R_{B} at each occurrence is independently selected from deuterium, halogen, oxo, -CN, -NH₂, C₁-₄ alkyl, C₃-₄ cycloalkyl, C₁-₄ alkoxy, C₁-₄ alkylthio, C₁-₄ haloalkyl and C₁-₄ haloalkoxy; or, when two R_{B} groups are attached to the same carbon atom of the 5- to 6-membered heterocyclyl, and the two R_{B} groups are each independently selected from C₁-₄ alkyl, the two R_{B} groups together with the carbon atom to which they are attached form C₃-₆ cycloalkyl;
L is a bond, -O-, -S-, or the following optionally substituted group: -C₀₋₄ alkylene-N(R₁)-, -C₀₋₄ alkylene-C(O)-, -C₀₋₄ alkylene-N(R₁)-C(O)-, -C₀₋₄ alkylene-N(R₁)-C(O)-N(R₁)-, -C₀₋₄ alkylene-N(R₁)-S(O)₂-, -C₀₋₄ alkylene-C(O)-N(R₁)-, -C₀₋₄ alkylene-S(O)₂-N(R₁)-, -C₁₋₄ alkylene-, -C₃-₆ cycloalkyl-C₀₋₄ alkylene-C(O)-N(R₁)-, or 3- to 10-membered heterocyclyl-C₀₋₄ alkylene-C(O)-N(R₁)-; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl, C₁-₄ alkoxy and C₁-₄ alkylthio.

In some embodiments,
ring A is 5- to 6-membered heterocyclyl, 5- to 6-membered heteroaryl or phenyl;
ring B is phenyl, 5- to 6-membered heterocyclyl or 5- to 6-membered heteroaryl;
each R_{A} at each occurrence is independently selected from deuterium, halogen, oxo, -CN, -NH₂, C₁-₄ alkyl, C₃-₄ cycloalkyl, C₁-₄ alkoxy, C₁-₄ alkylthio, C₁-₄ haloalkyl and C₁-₄ haloalkoxy; or, when two R_{A} groups are attached to the same carbon atom of the 5- to 6-membered heterocyclyl, and the two R_{A} groups are each independently selected from C₁-₄ alkyl, the two R_{A} groups together with the carbon atom to which they are attached form C₃-₆ cycloalkyl;
each R_{B} at each occurrence is independently selected from deuterium, halogen, oxo, -CN, -NH₂, C₁-₄ alkyl, C₃-₄ cycloalkyl, C₁-₄ alkoxy, C₁-₄ alkylthio, C₁-₄ haloalkyl and C₁-₄ haloalkoxy; or, when two R_{B} groups are attached to the same carbon atom of the 5- to 6-membered heterocyclyl, and the two R_{B} groups are each independently selected from C₁-₄ alkyl, the two R_{B} groups together with the carbon atom to which they are attached form C₃-₆ cycloalkyl;
ring C is optionally substituted 4- to 8-membered heterocyclyl; wherein the optionally substituted means being unsubstituted or substituted with one or more (such as 2, 3, 4, 5, 6, 7 or 8) substituents, and the substituents are each independently selected from deuterium, halogen, oxo, -CN, -NH₂, -OH, C₁-₄ alkyl, C₁-₄ alkoxy, C₁-₄ alkylthio, C₁-₄ haloalkyl and C₁-₄ haloalkoxy; the heterocyclyl contains one N atom, and optionally, further contains 1, 2 or 3 heteroatoms, wherein the heteroatoms are independently selected from O, N and S;
X₁ is selected from N and CRx₁;
X₂ is selected from N and CR_{X2};
X₃ is selected from N and CR_{X3};
provided that X₁, X₂ and X₃ are not N simultaneously;
R_{X1}, R_{X2}, and R_{X3} are each independently selected from hydrogen, deuterium, halogen, -OH, -CN, -NH₂, C₁-₃ alkyl, C₁-₃ alkoxy, C₁-₃ alkylthio, C₁-₃ haloalkyl and C₁-₃ haloalkoxy;
L is a bond, -O-, -S-, or the following optionally substituted group: -C₀₋₄ alkylene-N(R₁)-, -C₀₋₄ alkylene-C(O)-, -C₀₋₄ alkylene-N(R₁)-C(O)-, -C₀₋₄ alkylene-N(R₁)-C(O)-N(R₁)-, -C₀₋₄ alkylene-N(R₁)-S(O)₂-, -C₀₋₄ alkylene-C(O)-N(R₁)-, -C₀₋₄ alkylene-S(O)₂-N(R₁)-; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl, C₁-₄ alkoxy and C₁-₄ alkylthio;
R₁ is selected from hydrogen, deuterium, C₁-₃ alkyl and C₃-₆ cycloalkyl;
R is selected from -N(R₂)R₃ and -S(O)₂R₄, or is the following group optionally substituted with one or more (such as 2, 3, 4, 5, 6, 7 or 8) R_{w1}: C₁-₆ alkyl, C₁-₆ alkoxy, C₁-₆ alkylthio, C₃-₁₀ cycloalkyl, 3- to 10-membered heterocyclyl, 5- to 12-membered heteroaryl, or C₆₋₁₂ aryl; wherein, R_{w1} is selected from deuterium, halogen, oxo, -CN, -OH, -N(R₂)R₃, -N(R₅)-C(O)-R₆, -S(O)₂R₄, -C(O)OR₂, or the following groups optionally substituted with one or more (such as 2, 3, 4, 5, 6, 7 or 8) R_{w2}: C₁-₄ alkyl, C₁-₄ alkoxy, C₁-₄ alkylthio, C₃-₁₀ cycloalkyl, 3- to 10-membered heterocyclyl, C₆₋₁₀ aryl, and 5- to 12-membered heteroaryl; wherein, R_{w2} is selected from deuterium, halogen, oxo, -CN, -OH, -N(R₂)R₃, -S(O)₂R₄, -C(O)N(R₇)R₈, -C(O)OR₉, or the following groups optionally substituted with one or more (such as 2, 3, 4, 5, 6, 7 or 8) R_{w3}: C₁-₄ alkyl, C₁-₄ alkoxy, C₁-₄ alkylthio, C₆₋₁₀ aryl, and 5- to 12-membered heteroaryl; wherein, R_{w3} is selected from deuterium, halogen, oxo, -CN, -OH, -N(R₂)R₃, C₁-₄ alkyl, C₁-₄ alkoxy, C₁-₄ alkylthio, C₃-₆ cycloalkyl, 3- to 6-membered heterocyclyl, 5- to 6-membered heteroaryl, phenyl, C₁-₄ haloalkyl and C₁-₄ haloalkoxy;
R₂ and R₃ are each independently selected from hydrogen, deuterium, C₁-₆ alkyl, C₃-₆ cycloalkyl and 4- to 6-membered heterocyclyl;
R₄ is selected from hydrogen, deuterium, C₁-₃ alkyl and C₃-₆ cycloalkyl;
R₅ is selected from hydrogen, deuterium, C₁-₃ alkyl and C₃-₆ cycloalkyl;
R₆ is selected from hydrogen, deuterium, C₁-₃ alkyl, C₃-₆ cycloalkyl and C₄-₆ heterocyclyl;
R₇ and R₈ are each independently selected from hydrogen, deuterium, C₁-₃ alkyl, C₃-₆ cycloalkyl and C₄-₆ heterocyclyl;
R₉ is selected from hydrogen, deuterium, C₁-₆ alkyl and C₃-₆ cycloalkyl;
a is selected from 0, 1, 2, 3 and 4;
b is selected from 0, 1, 2, 3 and 4;
unless otherwise specified, the heteroatom(s) in the above-described heterocyclyl, heterocycloalkyl or heteroaryl are independently selected from O, N and S, and the number of heteroatoms is 1, 2, 3 or 4;
provided that a combination of definitions of the above-described variables results in a stable chemical structure.

In some embodiments, ring A and ring B are fused (also referred to as annulated, i.e., ring A and ring B share two adjacent atoms), bridged (i.e., ring A and ring B share two non-adjacent atoms), or spiro (i.e., ring A and ring B share one atom); preferably, ring A and ring B are fused.

In some embodiments, provided is the compound represented by formula (I), wherein, when a bicyclic system formed by ring A and ring B is 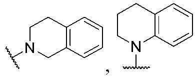 or 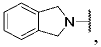 b is not 0, and 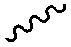 represents the point of attachment to 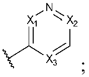
and/or, ring A and ring B are not simultaneously a 6-membered aromatic ring system, i.e., when ring A is phenyl or pyridyl, ring B is not phenyl or pyridyl;
and/or, the compound represented by formula (I) is not:

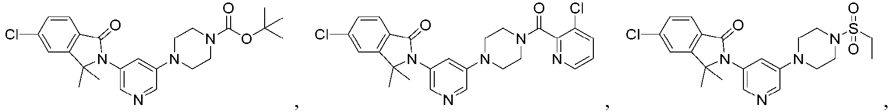

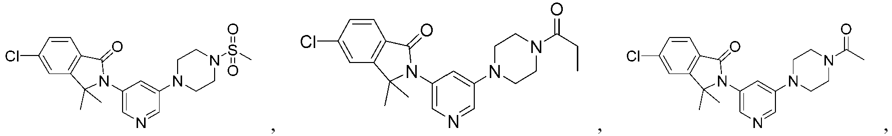

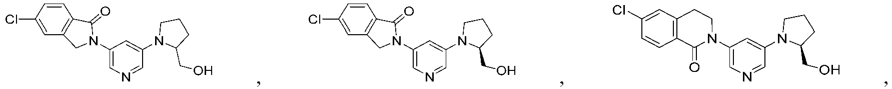

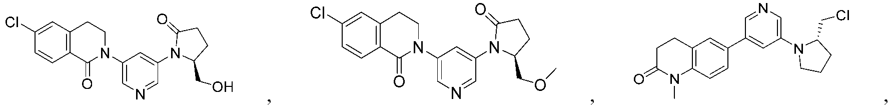

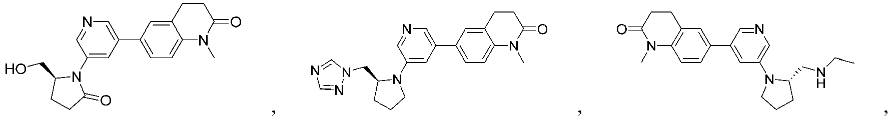

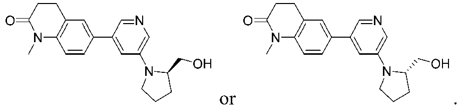

In some embodiments, provided is the compound represented by formula (I), wherein, ring A and ring B are not simultaneously a 6-membered aromatic ring system, i.e., when ring A is phenyl or 6-membered heteroaryl, ring B is not phenyl or 6-membered heteroaryl.

In some embodiments, ring A is 5-membered heterocyclyl, 6-membered heterocyclyl or phenyl.

In some embodiments, ring A is 6-membered heteroaryl; preferably pyridyl.

In some embodiments, ring A is 6-membered heterocyclyl or phenyl;
preferably, ring A is 6-membered heterocyclyl or phenyl, wherein, the heteroatom(s) in the heterocyclyl are independently selected from N and O, and the number of heteroatoms is 1, 2 or 3;
preferably, ring A is 6-membered heterocyclyl or phenyl, wherein, the heteroatom(s) in the heterocyclyl are independently selected from N, and the number of heteroatoms is 1 or 2;
preferably, ring A is piperidyl or phenyl.

In some embodiments, ring A is 6-membered heterocyclyl or phenyl, wherein, the heteroatom(s) in the heterocyclyl are independently selected from N and O, and the number of heteroatoms is 1 or 2.

In some embodiments, ring A is piperidyl, 1,3-oxazinanyl, tetrahydropyrimidinyl, morpholinyl, 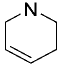 or phenyl.

In some embodiments, ring A is 5-membered heterocyclyl, wherein, the heteroatom(s) in the heterocyclyl are independently selected from N and S, and the number of heteroatoms is 1 or 2.

In some embodiments, ring A is pyrrolidyl or tetrahydrothiazolyl.

In some embodiments, ring B is phenyl or 5- to 6-membered heterocyclyl;
preferably, ring B is 5- to 6-membered heterocyclyl or phenyl, wherein, the heteroatom(s) in the heterocyclyl are independently selected from N and O, and the number of heteroatoms is 1, 2 or 3;
preferably, ring B is 5- to 6-membered heterocyclyl or phenyl, wherein, the heteroatom(s) in the heterocyclyl are independently selected from N, and the number of heteroatoms is 1 or 2;
preferably, ring B is phenyl, piperidyl or tetrahydropyrrolyl.

In some embodiments, ring B is phenyl.

In some embodiments, ring B is 5- to 6-membered heteroaryl, wherein, the heteroatom(s) in the heteroaryl are independently selected from N and S, and the number of heteroatoms is 1 or 2.

In some embodiments, ring B is pyrazolyl, imidazolyl, thienyl, thiazolyl, pyrimidinyl or pyridyl.

In some embodiments, ring A is piperidyl, 1,3-oxazinanyl, tetrahydropyrimidinyl, morpholinyl, pyrrolidyl or tetrahydrothiazolyl, and ring B is phenyl. In some embodiments, ring A is piperidyl, 1,3-oxazinanyl or pyrrolidyl, and ring B is phenyl. In some embodiments, ring A is phenyl, and ring B is piperidyl or tetrahydropyrrolyl.

In some embodiments, the bicyclic system formed by ring A and ring B is selected from: 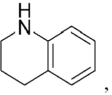 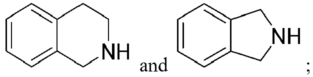 preferably, the bicyclic system formed by ring A and ring B is selected from: 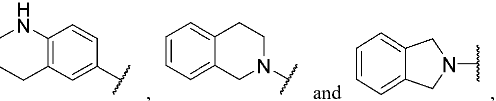 wherein, 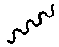 represents the point of attachment to ring

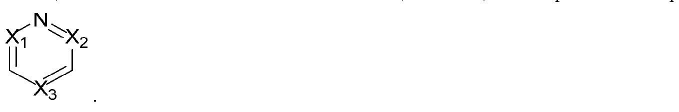

In some embodiments, the bicyclic system formed by ring A and ring B is selected from 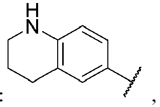 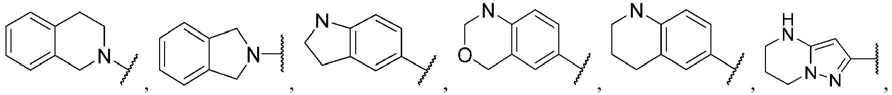 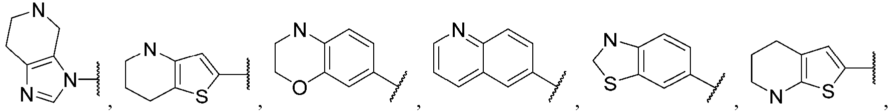 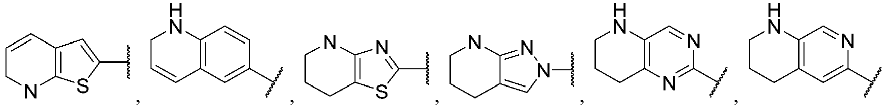 and 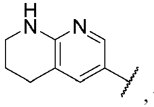 wherein, 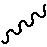 represents the point of attachment to ring

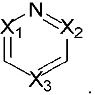

In some embodiments, each R_{A} at each occurrence is independently selected from deuterium, halogen, oxo, -CN, -NH₂, or the following optionally substituted groups: C₁-₃ alkyl (such as methyl, ethyl, propyl, or isopropyl), C₁-₃ deuterioalkyl (such as -CD₃), C₃-₄ cycloalkyl (such as cyclopropyl or cyclobutyl), C₁-₃ alkoxy (such as methoxy, ethoxy, propoxy, or isopropoxy), C₁-₃ haloalkyl (such as trifluoromethyl) and C₁-₃ haloalkoxy; or, two R_{A} together with the respective atom to which they are attached form C₃₋₆ cycloalkyl (preferably C₃₋₅ cycloalkyl, such as cyclopropyl, cyclobutyl, or cyclopentyl), 3- to 7-membered heterocyclyl (preferably 4- to 6-membered heterocyclyl, such as 4, 5- or 6-membered heterocyclyl), 5- to 6-membered heteroaryl or phenyl; wherein, the optionally substituted means being unsubstituted or substituted with one or more (such as 2, 3, 4, 5 or 6) substituents independently selected from deuterium, halogen (such as fluoro or chloro), C₁₋₃ alkyl (such as methyl, ethyl, propyl, or isopropyl), C₁₋₃ alkoxy (such as methoxy, ethoxy, propoxy, or isopropoxy), hydroxy, and oxo;
or, each R_{A} at each occurrence is independently selected from deuterium, halogen, oxo, -CN, -NH₂, C₁-₃ alkyl, C₁-₃ deuterioalkyl, C₃-₄ cycloalkyl, C₁-₃ alkoxy, C₁-₃ haloalkyl and C₁-₃ haloalkoxy; or, when two R_{A} groups are attached to the same carbon atom of the 5- to 6-membered heterocyclyl, and the two R_{A} groups are each independently selected from C₁-₃ alkyl, the two R_{A} groups together with the carbon atom to which they are attached form C₃-₆ cycloalkyl (preferably C₃-₅ cycloalkyl; further preferably C₃-₄ cycloalkyl).

In some embodiments, each R_{A} at each occurrence is independently selected from deuterium, halogen, oxo, -CN, -NH₂, C₁-₃ alkyl, C₃-₄ cycloalkyl, C₁-₃ alkoxy, C₁-₃ haloalkyl and C₁-₃ haloalkoxy; or, when two R_{A} groups are attached to the same carbon atom of the 5- to 6-membered heterocyclyl, and the two R_{A} groups are each independently selected from C₁-₃ alkyl, the two R_{A} groups together with the carbon atom to which they are attached form C₃-₆ cycloalkyl;
preferably, each R_{A} at each occurrence is independently selected from deuterium, halogen, oxo, -CN, -NH₂, C₁-₃ alkyl, C₁-₃ alkoxy, C₁-₃ haloalkyl and C₁-₃ haloalkoxy; or, when two R_{A} groups are attached to the same carbon atom of the 5- to 6-membered heterocyclyl, and the two R_{A} groups are each independently selected from C₁-₃ alkyl, the two R_{A} groups together with the carbon atom to which they are attached form C₃-₄ cycloalkyl;
preferably, each R_{A} at each occurrence is independently selected from deuterium, F, Cl, -CN, methyl, methoxy, cyclopropyl and oxo; or, when two R_{A} groups are attached to the same carbon atom of the 5- to 6-membered heterocyclyl, and the two R_{A} groups are each independently selected from C₁-₃ alkyl, the two R_{A} groups together with the carbon atom to which they are attached form cyclopropyl;
preferably, each R_{A} at each occurrence is independently selected from deuterium, F, Cl, -CN, methyl, methoxy, cyclopropyl and oxo;
preferably, each R_{A} at each occurrence is independently selected from deuterium, F, Cl, -CN, methyl, cyclopropyl and oxo; or, when two R_{A} groups are attached to the same carbon atom of the 5- to 6-membered heterocyclyl, and the two R_{A} groups are each independently selected from C₁-₃ alkyl, the two R_{A} groups together with the carbon atom to which they are attached form cyclopropyl;
preferably, each R_{A} at each occurrence is independently selected from deuterium, F, Cl, -CN, methyl, cyclopropyl and oxo.

In some embodiments, each R_{A} at each occurrence is independently selected from deuterium, halogen, oxo, -CN, -NH₂, C₁-₃ alkyl, C₁-₃ deuterioalkyl, C₃-₄ cycloalkyl, C₁-₃ alkoxy, C₁-₃ haloalkyl and C₁-₃ haloalkoxy; or, when two R_{A} groups are attached to the same carbon atom of the 5- to 6-membered heterocyclyl, the two R_{A} groups together with the carbon atom to which they are attached form C₃-₆ cycloalkyl.

In some embodiments, each R_{A} at each occurrence is independently selected from halogen, oxo, -CN, C₁-₃ alkyl, C₁-₃ deuterioalkyl, C₃-₄ cycloalkyl and C₁-₃ alkoxy; or, when two R_{A} groups are attached to the same carbon atom of the 5- to 6-membered heterocyclyl, the two R_{A} groups together with the carbon atom to which they are attached form C₃-₄ cycloalkyl.

In some embodiments, each R_{A} at each occurrence is independently selected from F, Cl, -CN, methyl, deuteriomethyl (i.e., -CD₃), methoxy, cyclopropyl and oxo, or when two R_{A} groups are attached to the same carbon atom of the 5- to 6-membered heterocyclyl, the two R_{A} groups together with the carbon atom to which they are attached form cyclopropyl.

In some embodiments, each R_{B} at each occurrence is independently selected from deuterium, halogen, oxo, -CN, -NH₂, C₁-₃ alkyl, C₃-₄ cycloalkyl, C₁-₃ haloalkyl and C₁-₃ haloalkoxy; or, when two R_{B} groups are attached to the same carbon atom of the 5- to 6-membered heterocyclyl, and the two R_{B} groups are each independently selected from C₁-₃ alkyl, the two R_{B} groups together with the carbon atom to which they are attached form C₃-₄ cycloalkyl;
preferably, each R_{B} at each occurrence is independently selected from deuterium, F, Cl, -CN, methyl, cyclopropyl and oxo; or, when two R_{B} groups are attached to the same carbon atom, and the two R_{B} groups on the same carbon atom of the 5- to 6-membered heterocyclyl are each independently selected from C₁-₃ alkyl, the two R_{B} groups together with the carbon atom to which they are attached form C₃-₄ cycloalkyl (preferably cyclopropyl).

In some embodiments, each R_{B} at each occurrence is independently selected from halogen, oxo or C₁-₃ alkyl; or, when two R_{B} groups are attached to the same carbon atom of the 5- to 6-membered heterocyclyl, the two R_{B} groups together with the carbon atom to which they are attached form C₃-₄ cycloalkyl.

In some embodiments, each R_{B} at each occurrence is independently selected from F, Cl, methyl and oxo; or, when two R_{B} groups are attached to the same carbon atom of the 5- to 6-membered heterocyclyl, the two R_{B} groups together with the carbon atom to which they are attached form cyclopropyl.

In some embodiments, 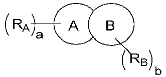 is selected from: 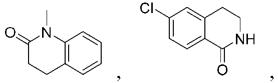 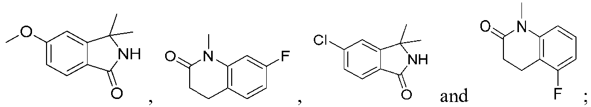 preferably, 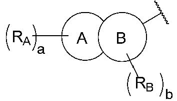 is selected from:

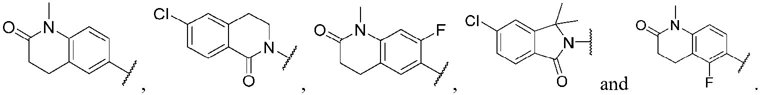

In some embodiments, 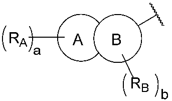 is selected from:

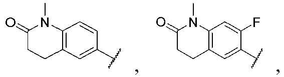

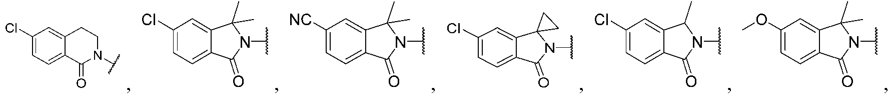

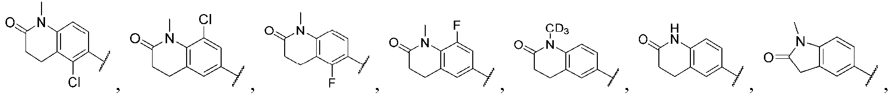

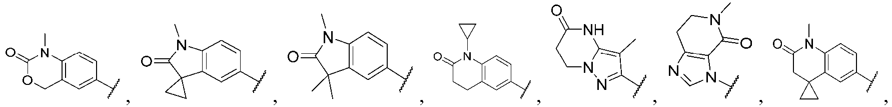

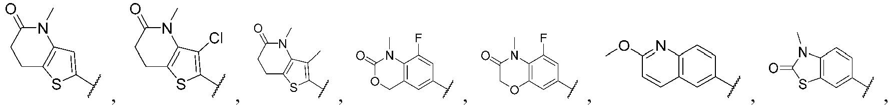

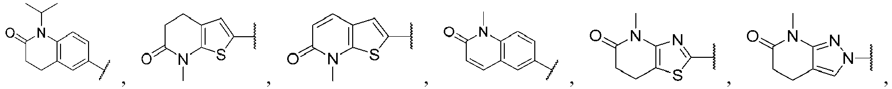

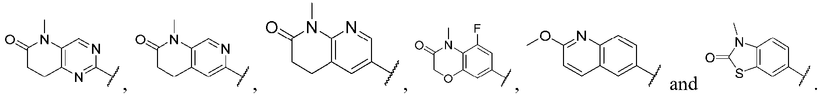

In some embodiments, 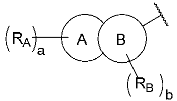 is selected from:

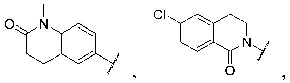

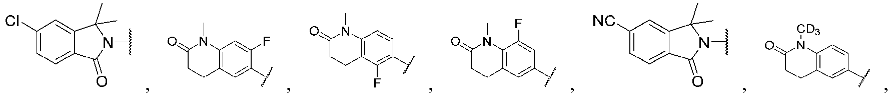

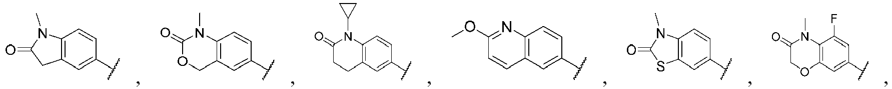

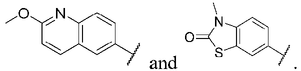

In some embodiments, 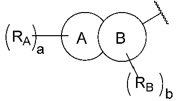 is selected from:

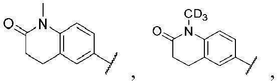

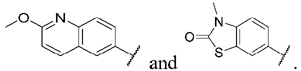

In some embodiments, ring C is optionally substituted 5- to 8-membered (such as 5-membered, 6-membered, 7-membered, or 8-membered) heterocyclyl; preferably, ring C is selected from the following optionally substituted groups: 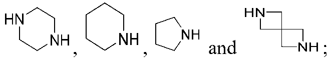 preferably, ring C is selected from the following optionally substituted groups: 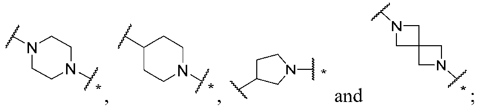 preferably, ring C is selected from the following optionally substituted groups: 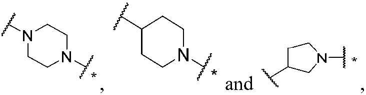 wherein, 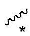 represents the point of attachment to ring 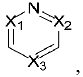 and 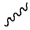 represents the point of attachment to L;
wherein, the optionally substituted means being unsubstituted or substituted with one or more substituents which are each independently selected from deuterium, halogen, oxo, -CN, -NH₂, -OH, C₁-₄ alkyl, C₁-₄ alkoxy, C₁-₄ alkylthio, C₁-₄ haloalkyl and C₁-₄ haloalkoxy.

In some embodiments, ring C is selected from: 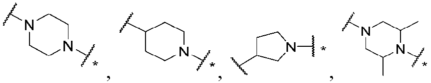 and 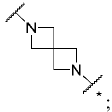 in some embodiments, ring C is selected from: 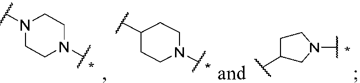 in some embodiments, ring C is 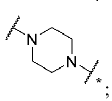 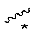 wherein, represents the point of attachment to ring 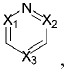 and 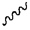 represents the point of attachment to L.

In some embodiments, ring C is optionally substituted 5- to 6-membered heterocyclyl;
wherein, the optionally substituted means being unsubstituted or substituted with one or more substituents which are each independently selected from deuterium, halogen, oxo, -CN, -NH₂, -OH, C₁-₄ alkyl, C₁-₄ alkoxy, C₁-₄ alkylthio, C₁-₄ haloalkyl and C₁-₄ haloalkoxy; the heterocyclyl contains one N atom, and optionally, further contains 1, 2 or 3 heteroatoms, wherein the heteroatoms are independently selected from O, N and S; preferably, the optionally substituted means being unsubstituted or substituted with one or more substituents selected from deuterium, halogen, oxo, -CN, -NH₂, -OH, methyl, methoxy, methylthio, halogenated methyl (such as monofluoromethyl, difluoromethyl, trifluoromethyl, monochloromethyl, dichloromethyl, or trichloromethyl), and halogenated methoxy (such as monofluoromethoxy, difluoromethoxy, trifluoromethoxy, monochloromethoxy, dichloromethoxy, or trichloromethoxy); wherein, the heterocyclyl contains one N atom, and optionally, further contains 1, 2 or 3 heteroatoms, wherein the heteroatoms are independently selected from O, N and S; preferably, the heterocyclyl contains 1 or 2 N atoms;
preferably, ring C is piperidyl, piperazinyl or tetrahydropyrrolyl.

In some embodiments, ring C is 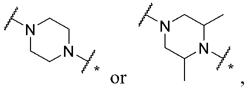 wherein, 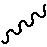 represents the point of attachment to L, and 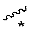 represents the point of attachment to ring

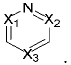

In some embodiments, X₁ is CRx₁; X₂ is CR_{X2}; X₃ is CR_{X3}.

In some embodiments, X₁ is CRx₁; X₂ is N; X₃ is CR_{X3}.

In some embodiments, X₁ is CRx₁; X₂ is CR_{X2}; X₃ is N.

In some embodiments, X₁ is CRx₁; X₂ is N; X₃ is N.

In some embodiments, R_{X1}, R_{X2}, and R_{X3} are each independently selected from hydrogen, deuterium, F, Cl, Br, -CN and -NH₂; preferably, R_{X1}, R_{X2}, and R_{X3} are each independently selected from hydrogen.

In some embodiments, structural fragment 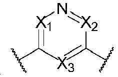 is 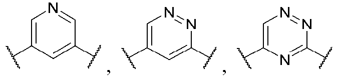 or 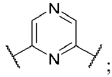 preferably, 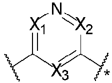 is 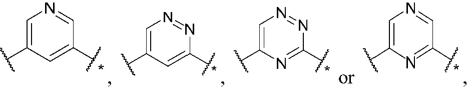 wherein, 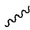 represents the point of attachment to ring B, and 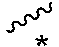 represents the point of attachment to ring C.

In some embodiments, structural fragment 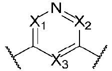 is 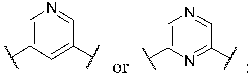 preferably, 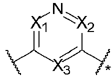 is 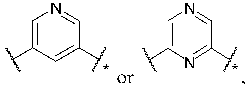 wherein, 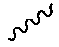 represents the point of attachment to ring B, and 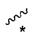 represents the point of attachment to ring C.

In some embodiments, L is a bond, -O-, or the following optionally substituted group: -C₀₋₄ alkylene-N(R₁)-, -C₀₋₄ alkylene-C(O)-, -C₀₋₄ alkylene-N(R₁)-C(O)-, -C₀₋₄ alkylene-N(R₁)-C(O)-N(R₁)-, -C₀₋₄ alkylene-C(O)-N(R₁)-S(O)₂-, -C₀₋₄ alkylene-C(O)-N(R₁)-, -C₁₋₄ alkylene-, or -C₃-₆ cycloalkyl-C₀₋₄ alkylene-C(O)-N(R₁)-; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl, C₁-₄ alkoxy and C₁-₄ alkylthio.

In some embodiments, L is a bond, -O-, or the following optionally substituted groups: -C₀₋₂ alkylene-N(R₁)-, -C₀₋₂ alkylene-C(O)-, -C₀₋₂ alkylene-N(R₁)-C(O)-, -C₀₋₂ alkylene-N(R₁)-C(O)-N(R₁)-, -C₀₋₂ alkylene-C(O)-N(R₁)-S(O)₂-, -C₀₋₂ alkylene-C(O)-N(R₁)-, -C₁₋₄ alkylene-, or -C₃-₄ cycloalkyl-C₀₋₂ alkylene-C(O)-N(R₁)-; wherein, the optionally substituted means being unsubstituted or substituted with one or more (such as 2, 3, or 4) substituents, and the substituents are each independently selected from deuterium, halogen, oxo, and C₁-₃ alkyl.

In some embodiments, L is a bond, -O-, -C(O)-, -N(R₁)-C(O)-, -C(O)-N(R₁)-, -methylene-C(O)-N(R₁)-, -C₁₋₃ alkylene-, -N(R₁)-C(O)-N(R₁)-, -methylene-C(O)-N(R₁)-S(O)₂-, -methylene-C(O)-, -ethylene-C(O)-N(R₁)-, - cyclopropyl-C(O)-N(R₁)- or -ethylene-N(R₁)-C(O)-N(R₁)-, wherein, each R₁ is independently H or methyl, and the methylene and ethylene are each independently optionally substituted with 1 or 2 deuterium or methyl.

In some embodiments, L is a bond, -O-, or the following optionally substituted groups: -C₀₋₄ alkylene-C(O)-, -C₀₋₄ alkylene-N(R₁)-C(O)-, -C₀₋₄ alkylene-C(O)-N(R₁)-, -C₀₋₄ alkylene-N(R₁)-C(O)-N(R₁)-, -C₀₋₄ alkylene-N(R₁)-S(O)₂-, or -C₀₋₄ alkylene-S(O)₂-N(R₁)-; wherein, the optionally substituted means being unsubstituted or substituted with one or more substituents, which are each independently selected from deuterium, halogen, oxo, - CN, -NH₂, C₁-₄ alkyl, C₁-₄ alkoxy and C₁-₄ alkylthio;
preferably, L is a bond, -O-, or the following optionally substituted groups: -C₀₋₂ alkylene-C(O)-, -C₀₋₂ alkylene-N(R₁)-C(O)-, -C₀₋₂ alkylene-C(O)-N(R₁)-, -C₀₋₂ alkylene-N(R₁)-C(O)-N(R₁)-, -C₀₋₂ alkylene-N(R₁)-S(O)₂-, and -C₀₋₂ alkylene-S(O)₂-N(R₁)-; wherein, the optionally substituted means being unsubstituted or substituted with one or more substituents which are each independently selected from deuterium, halogen, oxo, - CN, -NH₂, C₁-₃ alkyl and C₁-₃ alkoxy;
preferably, L is a bond, -O-, -C(O)-, -N(R₁)-C(O)-, -C(O)-N(R₁)- or the following optionally substituted groups: -methylene-C(O)-, -ethylene-C(O)-, -methylene-N(R₁)-C(O)-, -ethylene-N(R₁)-C(O)-, -methylene-C(O)-N(R₁)-, -ethylene-C(O)-N(R₁)-, -N(R₁)-C(O)-N(R₁)-, -N(R₁)-S(O)₂-, -methylene-N(R₁)-C(O)-N(R₁)-, - methylene-N(R₁)-S(O)₂-, -methylene-S(O)₂-N(R₁)-, and -ethylene-S(O)₂-N(R₁)-; wherein, the optionally substituted means being unsubstituted or substituted with one or more substituents which are each independently selected from deuterium, halogen, oxo, -CN, -NH₂, C₁-₃ alkyl (such as methyl or ethyl) and C₁-₃ alkoxy (such as methoxy).

In some embodiments, L is the following optionally substituted group: -C₁₋₃ alkylene-, -C₃-₆ cycloalkyl-C₀₋₄ alkylene-C(O)-N(R₁)-, or 3- to 10-membered heterocyclyl-C₀₋₄ alkylene-C(O)-N(R₁)-; preferably, L is the following optionally substituted group: -C₁₋₃ alkylene-, -C₃-₆ cycloalkyl-C₀₋₂ alkylene-C(O)-N(R₁)-, or 3- to 8-membered heterocyclyl-C₀₋₂ alkylene-C(O)-N(R₁)-; further preferably, L is the following optionally substituted group: -C₁₋₃ alkylene-, -C₃-₄ cycloalkyl-C(O)-N(R₁)-, -C₃-₄ cycloalkyl-methylene-C(O)-N(R₁)-, 4- to 8-membered heterocyclyl-C(O)-N(R₁)-, or 4- to 8-membered heterocyclyl-methylene-C(O)-N(R₁)-; further preferably, L is the following optionally substituted group: -methylene-;
wherein, the optionally substituted means being unsubstituted or substituted with one or more substituents, which are each independently selected from deuterium, halogen, oxo, -CN, -NH₂, C₁-₄ alkyl, C₁-₄ alkoxy and C₁-₄ alkylthio; preferably, the optionally substituted means being unsubstituted or substituted with one or more substituents which are each independently selected from deuterium, halogen, oxo, -CN, -NH₂, C₁-₃ alkyl (such as methyl or ethyl) and C₁-₃ alkoxy (such as methoxy).

In some embodiments, R₁ is selected from hydrogen, deuterium and C₁-₃ alkyl;
preferably, R₁ is selected from hydrogen, deuterium, methyl and ethyl;
further preferably, R₁ is selected from hydrogen.

In some embodiments, L is a bond, -O-, 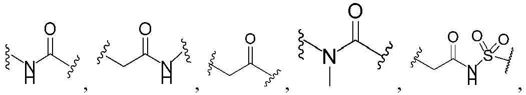 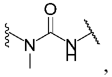 methylene, ethylene, propylene, 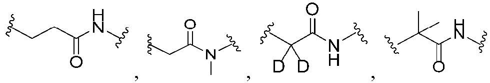 or 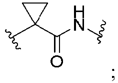 preferably, the left side of the groups is linked to ring C.

In some embodiments, L is a bond, -O-,

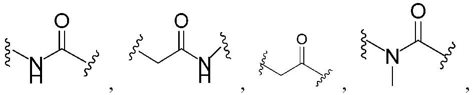

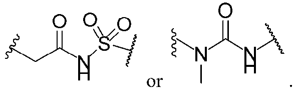

In some embodiments, L is a bond, -O-, 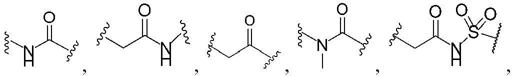 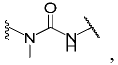 methylene, ethylene, propylene, 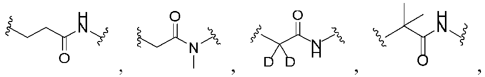 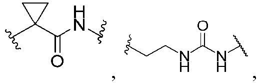 -C(O)- or -S(O)₂-; preferably, the left side of the groups is linked to ring C.

In some embodiments, L is a bond, -O-, 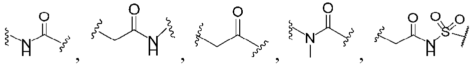 or

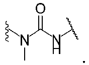

In some embodiments, L is a bond, 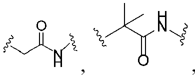 methylene, propylene, 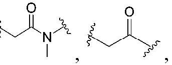 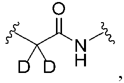 -S(O)₂-, -C(O)-,

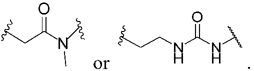

In some embodiments, L is a bond,

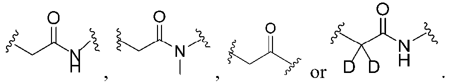

In some embodiments, R is -N(R₂)R₃, -S(O)₂R₄, or the following group optionally substituted with one or more R_{w1}: C₁-₆ alkyl, C₁-₆ alkoxy, C₃-₁₀ cycloalkyl, 3- to 10-membered heterocyclyl, 5- to 10-membered heteroaryl, or 6- to 12-membered aryl; wherein, R_{w1} is selected from deuterium, halogen, oxo, -CN, -OH, -NR₂R₃, -N(R₅)-C(O)-R₆, -S(O)₂R₄, -C(O)OR₂, or the following groups optionally substituted with one or more R_{w2}: C₁-₄ alkyl, C₁-₄ alkoxy, C₃-₁₀ cycloalkyl, 3- to 10-membered heterocyclyl, C₆₋₁₀ aryl, and 5- to 12-membered heteroaryl; wherein, R_{w2} is selected from deuterium, halogen, oxo, -CN, -OH, -NR₂R₃, -S(O)₂R₄, -C(O)NR₇R₈, -C(O)OR₉, or the following groups optionally substituted with one or more R_{w3}: C₁-₄ alkyl, C₁-₄ alkoxy, C₁-₄ alkylthio, C₆₋₁₀ aryl, and 5- to 12-membered heteroaryl; R_{w3} is selected from deuterium, halogen, oxo, -CN, -OH, -NR₂R₃, C₁-₄ alkyl, C₁-₄ alkoxy, C₁-₄ alkylthio, C₁-₄ haloalkyl and C₁-₄ haloalkoxy.

In some embodiments, R is -N(R₂)R₃, -S(O)₂R₄, or the following group optionally substituted with one or more R_{w1}: C₁-₄ alkyl, C₃-₈ cycloalkyl (such as 3-membered, 4-membered, 5-membered, 6-membered, 7-membered, or 8-membered), 4- to 10-membered heterocyclyl (such as 4-membered, 5-membered, 6-membered, 7-membered, 8-membered, 9-membered, or 10-membered), 5- to 10-membered heteroaryl (such as 5-membered, 6-membered, 7-membered, 8-membered, 9-membered, or 10-membered); wherein R_{w1} is selected from deuterium, halogen (such as F or Cl), oxo, -OH, -N(R₂)R₃, -N(R₅)-C(O)-R₆, -S(O)₂R₄, -C(O)OR₂, or the following groups optionally substituted with one or more (such as 1, 2, 3, or 4) R_{w2}: C₁-₄ alkyl, C₁-₄ alkoxy (preferably C₁₋₃ alkoxy, such as methoxy or ethoxy), 3- to 8-membered heterocyclyl (preferably 4- to 7-membered heterocyclyl, further preferably 4- to 6-membered heterocyclyl, further preferably 5- to 6-membered heterocyclyl), phenyl, and 5- to 6-membered heteroaryl; R_{w2} is selected from deuterium, halogen (such as F or Cl), oxo, -N(R₂)R₃, -S(O)₂R₄, -C(O)N(R₇)R₈, - C(O)OR₉, or the following groups optionally substituted with one or more R_{w3}: C₁-₃ alkyl (such as methyl or ethyl), C₁-₃ alkoxy (such as methoxy or ethoxy), and 5- to 6-membered heteroaryl; R_{w3} is selected from deuterium, halogen, oxo, -CN, -OH, -N(R₂)R₃ and C₁-₃ alkyl (such as methyl or ethyl).

In some embodiments, R₂ and R₃ are each independently selected from hydrogen, deuterium and C₁-₄ alkyl.

In some embodiments, R₄ is selected from hydrogen, deuterium, C₁-₃ alkyl and C₃-₄ cycloalkyl.

In some embodiments, R₅ is selected from hydrogen, deuterium and C₁-₃ alkyl.

In some embodiments, each R₆ is independently selected from hydrogen, deuterium, C₁-₃ alkyl and C₃-₆ cycloalkyl.

In some embodiments, R₇ and R₈ are each independently selected from hydrogen, deuterium, C₁-₃ alkyl and C₃-₆ cycloalkyl.

In some embodiments, R₉ is selected from hydrogen, deuterium and C₁-₄ alkyl.

In some embodiments, R₂ and R₃ are each independently selected from hydrogen, deuterium and C₁-₃ alkyl;
R₄ is selected from hydrogen, deuterium, C₁-₃ alkyl and C₃-₄ cycloalkyl;
R₅ is selected from hydrogen, deuterium and C₁-₃ alkyl;
each R₆ is independently selected from hydrogen, deuterium, C₁-₃ alkyl and C₃-₆ cycloalkyl;
R₇ and R₈ are each independently selected from hydrogen, deuterium, C₁-₃ alkyl and C₃-₆ cycloalkyl;
R₉ is selected from hydrogen, deuterium and C₁-₄ alkyl.

In some embodiments, R is -N(R₂)R₃, -S(O)₂R₄, or the following group optionally substituted with one or more R_{w1}: C₁-₄ alkyl, C₃-₁₂ cycloalkyl, 3- to 10-membered heterocyclyl, or 5- to 10-membered heteroaryl; wherein R_{w1} is selected from deuterium, halogen, oxo, -OH, -N(R₂)R₃, -N(R₅)-C(O)-R₆, -S(O)₂R₄, -C(O)OR₂, or the following groups optionally substituted with one or more R_{w2}: C₁-₄ alkyl, C₁-₄ alkoxy, 3- to 10-membered heterocyclyl, C₆₋₁₀ aryl, and 5- to 10-membered heteroaryl; R_{w2} is selected from deuterium, halogen, oxo, -N(R₂)R₃, -S(O)₂R₄, -C(O)N(R₇)R₈, -C(O)OR₉, or the following groups optionally substituted with one or more R_{w3}: C₁-₄ alkyl, C₁-₄ alkoxy, phenyl, and 5- to 6-membered heteroaryl; R_{w3} is selected from deuterium, halogen, oxo, -CN, -OH, -N(R₂)R₃, C₁-₄ alkyl, C₁-₄ alkoxy and C₁-₄ alkylthio;
R₂ and R₃ are each independently selected from hydrogen, deuterium and C₁-₃ alkyl;
R₄ is selected from hydrogen, deuterium, C₁-₃ alkyl and C₃-₄ cycloalkyl;
R₅ is selected from hydrogen, deuterium and C₁-₃ alkyl;
each R₆ is independently selected from hydrogen, deuterium, C₁-₃ alkyl and C₃-₆ cycloalkyl;
R₇ and R₈ are each independently selected from hydrogen, deuterium, C₁-₃ alkyl and C₃-₆ cycloalkyl;
R₉ is selected from hydrogen, deuterium and C₁-₄ alkyl.

In some embodiments, R is -N(R₂)R₃, -S(O)₂R₄, or the following group optionally substituted with one or more R_{w1}: C₁-₄ alkyl, C₃-₈ cycloalkyl, 3- to 10-membered heterocyclyl, or 5- to 10-membered heteroaryl; wherein R_{w1} is selected from deuterium, halogen, oxo, -OH, -N(R₂)R₃, -N(R₅)-C(O)-R₆, -S(O)₂R₄, -C(O)OR₂, or the following groups optionally substituted with one or more R_{w2}: C₁-₄ alkyl, C₁-₄ alkoxy, 3- to 8-membered heterocyclyl, phenyl, and 5- to 6-membered heteroaryl; R_{w2} is selected from deuterium, halogen, oxo, -N(R₂)R₃, -S(O)₂R₄, -C(O)N(R₇)R₈, -C(O)OR₉, or the following groups optionally substituted with one or more R_{w3}: C₁-₃ alkyl, C₁-₃ alkoxy, and 5- to 6-membered heteroaryl; R_{w3} is selected from deuterium, halogen, oxo, -CN, -OH, - N(R₂)R₃ and C₁-₃ alkyl;
R₂ and R₃ are each independently selected from hydrogen, deuterium, methyl, ethyl and isopropyl;
R₄ is selected from hydrogen, deuterium, methyl, ethyl, isopropyl and cyclopropyl;
R₅ is selected from hydrogen, deuterium, methyl and ethyl;
each R₆ is independently selected from hydrogen, deuterium, methyl and ethyl;
R₇ and R₈ are each independently selected from hydrogen, deuterium, methyl and ethyl;
R₉ is selected from hydrogen, deuterium, methyl, ethyl, isopropyl and tert-butyl.

In some embodiments, R is -N(R₂)R₃, -S(O)₂C₁₋₃ alkyl, or the following group optionally substituted with one or more (such as 2 or 3) R_{w1}: cyclohexyl, pyridyl, piperidyl, n-propyl, isopropyl, pyrazolyl, pyrrolidyl, methyl, 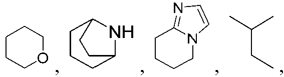 piperazinyl, 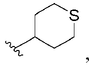 azetidinyl, 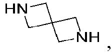 cyclobutanyl, 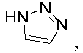 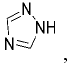 cyclopropyl, 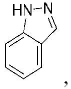 morpholinyl, oxetanyl, 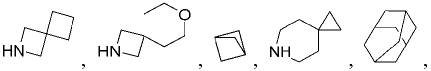 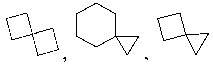 and tert-butyl; wherein R_{w1} is selected from the following groups optionally substituted with one or more (such as 2 or 3) R_{w2}: -NH-C(O)-CH₃, -OH, methyl, -N(CH₃)₂, oxo, morpholinyl, -S(O)₂CH₃, piperidyl, -NH-C(O)-CH (CH₃)₂, methoxy, 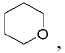 ethyl, pyrimidinyl, -NH-C(O)-cyclopropyl, piperazinyl, methoxy, fluoro, -C(O)OC(CH₃)₃, -S(O)₂ cyclopropane, -S(O)₂C₂H₅ and -S(O)₂CH₃; R_{w2} is selected from fluoro, oxo, methyl, and methoxy.

In some embodiments, R is

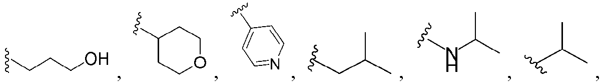

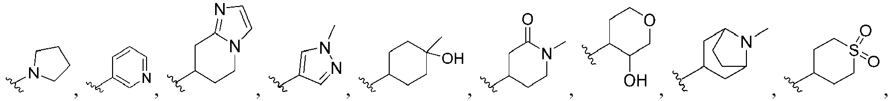

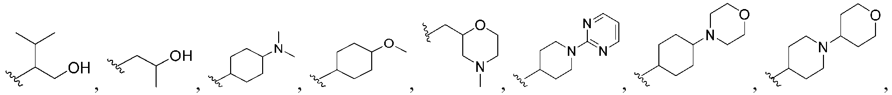

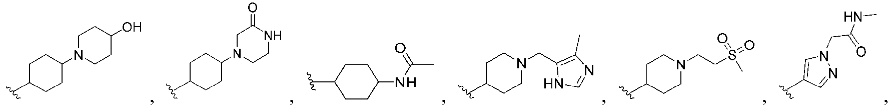

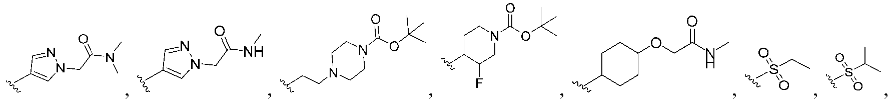

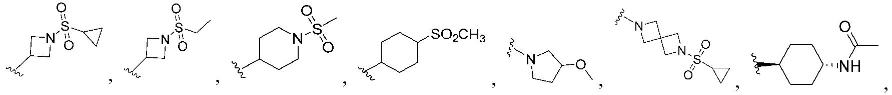

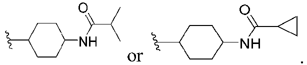

In some embodiments, R is

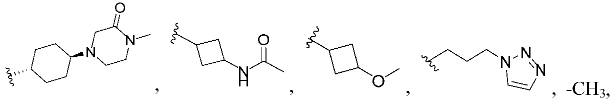

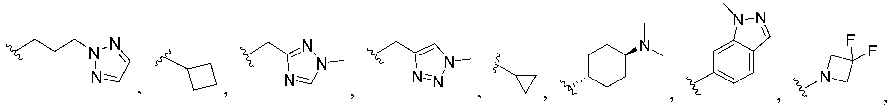

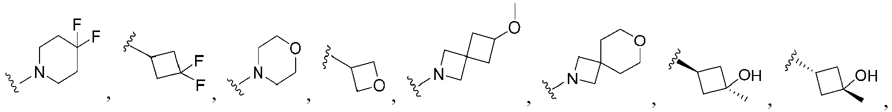

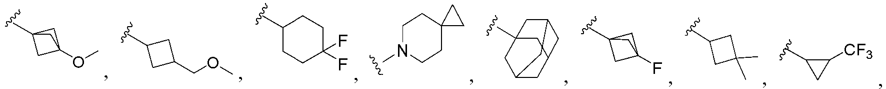

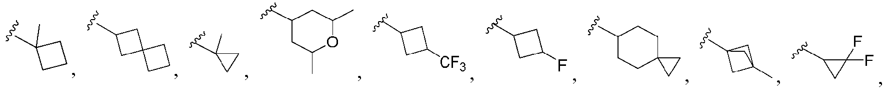

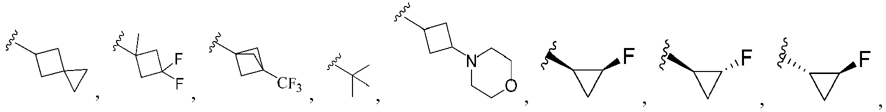

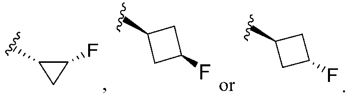

In some embodiments, R is

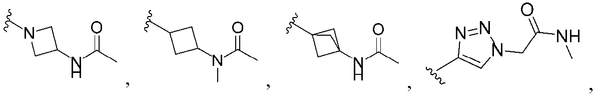

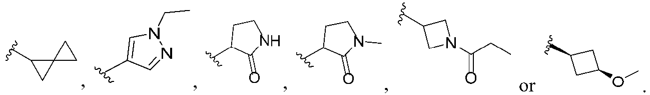

In some embodiments, R is

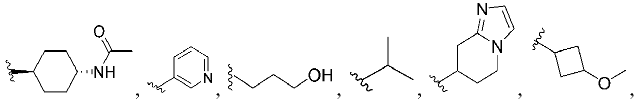

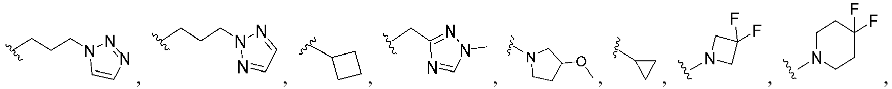

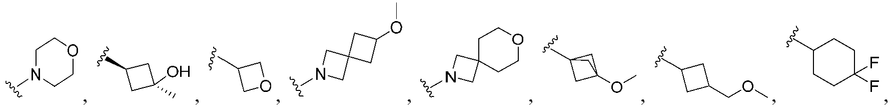

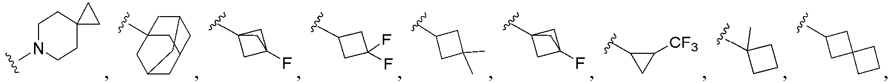

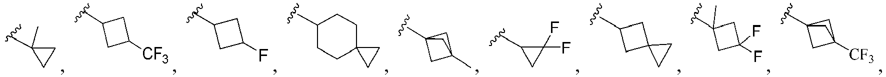

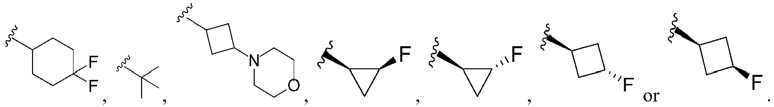

In some embodiments, R is

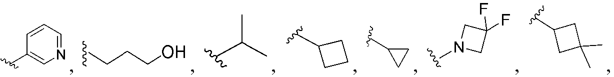

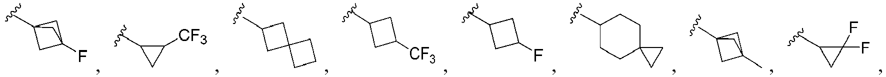

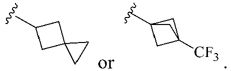

In some embodiments, R is

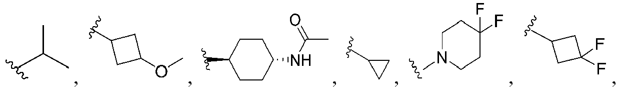

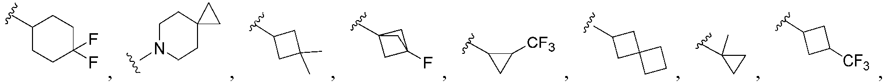

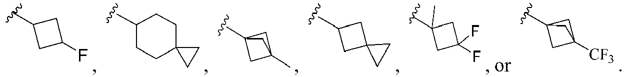

In some embodiments, a is selected from 0, 1 and 2; preferably, a is 2.

In some embodiments, b is selected from 0, 1, 2 and 3; preferably, b is selected from 0 and 3.

In some embodiments, provided is the compound represented by formula (I), which is a compound represented by formula (II-1) or formula (II-2): 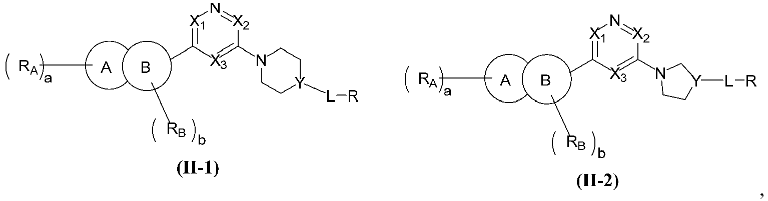
wherein, ring A, ring B, R_{A}, R_{B}, X₁, X₂, X₃, L, R, a, and b are as described in the compound represented by formula (I);
Y is N or CR_{Y};
R_{Y} is selected from hydrogen, deuterium, halogen, -OH, -CN, -NH₂, C₁-₃ alkyl, C₁-₃ alkoxy, C₁-₃ haloalkyl and C₁-₃ haloalkoxy.

In some embodiments, R_{Y} is selected from hydrogen and deuterium; preferably, R_{Y} is hydrogen.

In some embodiments, Y is N.

In some embodiments, Y is CH.

In some embodiments, provided is the compound represented by formula (I), which is a compound represented by formula (II-3) or formula (II-4): 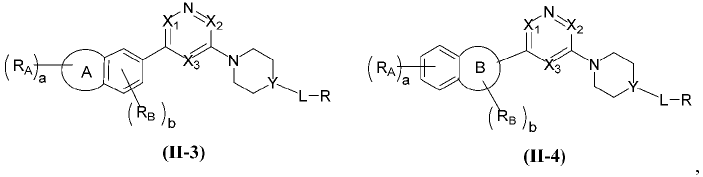
wherein, ring A, ring B, R_{A}, R_{B}, X₁, X₂, X₃, L, R, a, and b are as described in the compound represented by formula (I);
Y is N or CR_{Y};
R_{Y} is selected from hydrogen, deuterium, halogen, -OH, -CN, -NH₂, C₁-₃ alkyl, C₁-₃ alkoxy, C₁-₃ haloalkyl and C₁-₃ haloalkoxy.

In some embodiments, ring A is 5- to 6-membered heterocyclyl, wherein the heteroatom(s) in the heterocyclyl are independently selected from O, N and S, and the number of heteroatoms is 1, 2 or 3; preferably, ring A is 5- to 6-membered heterocycloalkyl, wherein the heteroatom(s) in the heterocycloalkyl are independently selected from O and N, and the number of heteroatoms is 1 or 2; further preferably, ring A is piperidyl, 1,3-oxazinanyl or pyrrolidyl; further preferably, ring A is 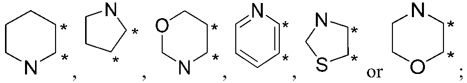 further preferably, 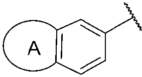 is 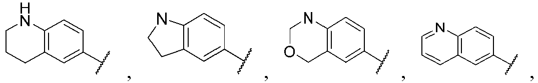 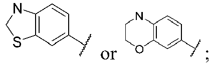 further preferably, 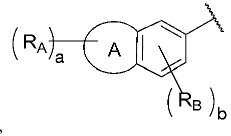 is

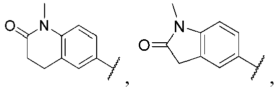

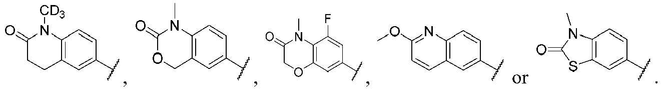

In some embodiments, ring B is 5- to 6-membered heterocyclyl, wherein the heteroatom(s) in the heterocyclyl are independently selected from O, N and S, and the number of heteroatoms is 1, 2 or 3; preferably, ring B is 5-to 6-membered heterocycloalkyl, wherein the heteroatom(s) in the heterocycloalkyl are independently selected from O and N, and the number of heteroatoms is 1 or 2; further preferably, ring B is pyrrolidyl; further preferably, ring B is 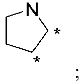 further preferably, 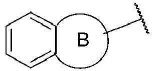 is 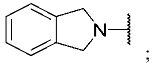 further preferably, 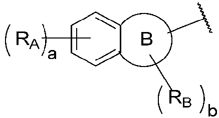 is

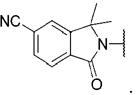

In some embodiments, provided is the compound represented by formula (I), which is a compound represented by formula (III-1), formula (III-2), formula (III-3) or formula (III-4): 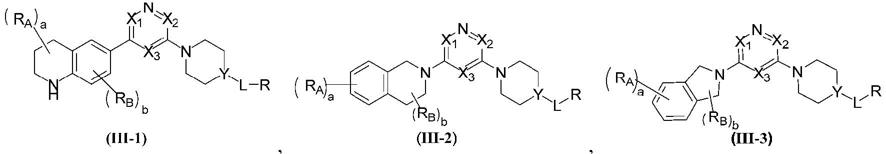 or 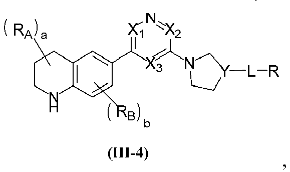 wherein, R_{A}, R_{B}, X₁, X₂, X₃, L, R, a, b, and Y are as described in the compound as represented by formula (I), formula (II-1) or formula (II-2).

In some embodiments, provided is the compound represented by formula (I),which is a compound represented by formula (IV-A), formula (IV-B) or formula (IV-C): 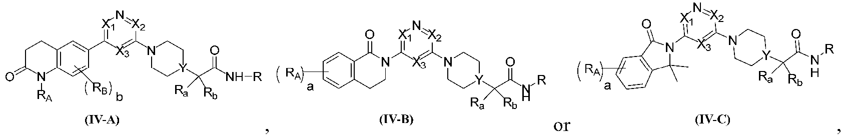
wherein, R_{A}, R_{B}, X₁, X₂, X₃, Y, R, a, and b are as described in the compound represented by formula (I), formula (II-1) or formula (II-2);
Rₐ and R_{b} are each independently selected from hydrogen, deuterium and C₁-₄ alkyl.

In some embodiments, provided is the compound represented by formula (I), which is a compound represented by formula (IV-1), formula (IV-2), or formula (IV-3): 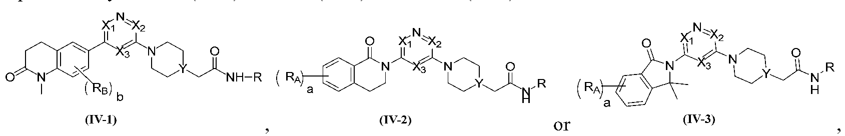 wherein, R_{A}, R_{B}, X₁, X₂, X₃, Y, R, a, and b are as described in the compound represented by formula (I), formula (II-1) or formula (II-2).

In some embodiments, R is

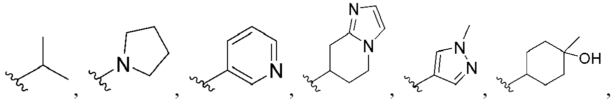

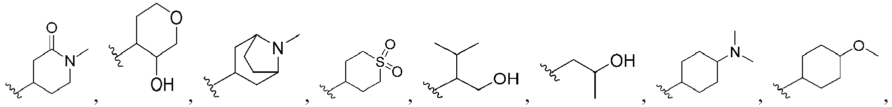

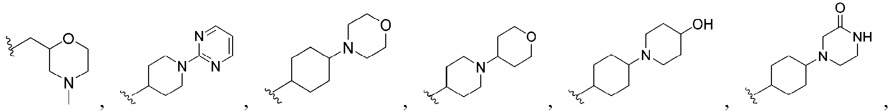

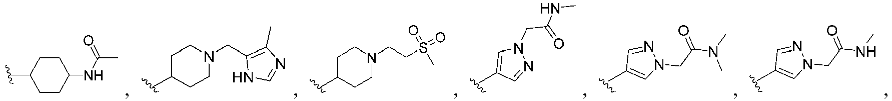

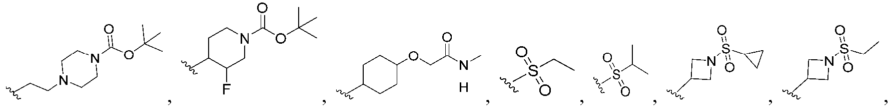

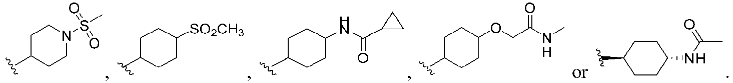

In some embodiments, R is

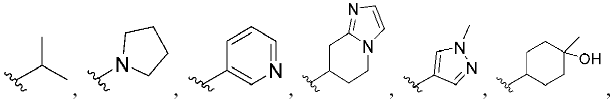

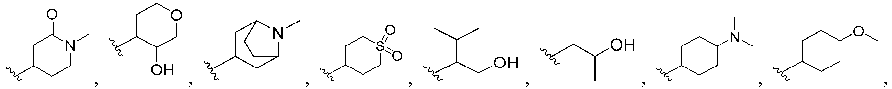

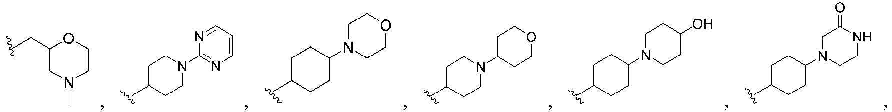

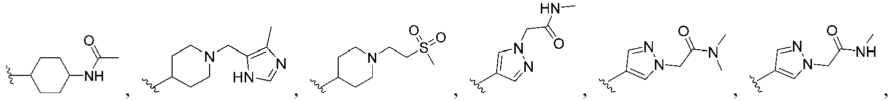

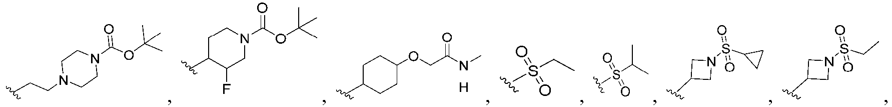

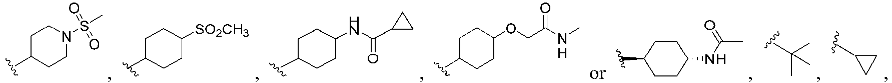

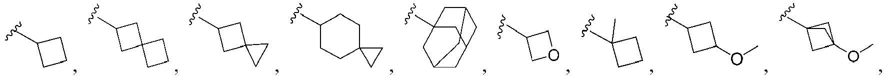

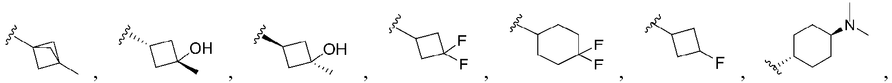

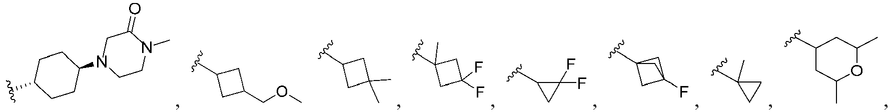

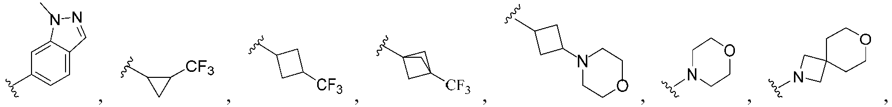

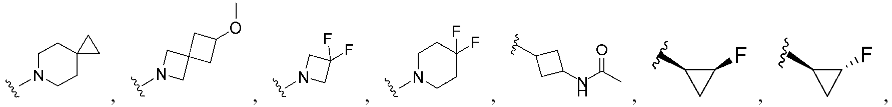

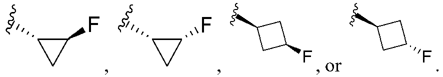

In some embodiments, R is

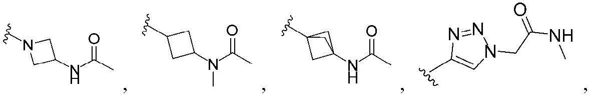

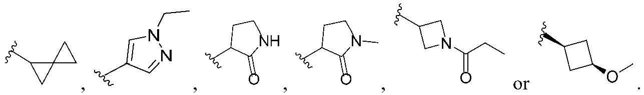

In some embodiments, provided is the compound represented by formula (I), which is a compound represented by formula (V): 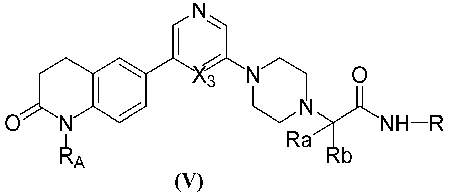
wherein, X₃ and R are as described in the compound represented by formula (I), formula (II-1) or formula (II-2);
Rₐ and R_{b} are each independently selected from hydrogen, deuterium and C₁-₄ alkyl;
R_{A} is selected from hydrogen, C₁-₃ alkyl (such as methyl), deuteriomethyl (-CD₃), and cyclopropyl.

In some embodiments, provided is the compound represented by formula (I), which is a compound represented by formula (A): 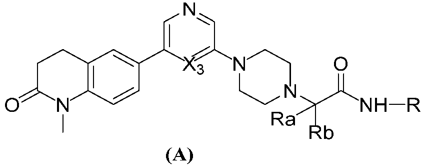
wherein, X₃ and R are as described in the compound represented by formula (I), formula (II-1) or formula (II-2);
Rₐ and R_{b} are each independently selected from hydrogen, deuterium and C₁-₄ alkyl.

In some embodiments, 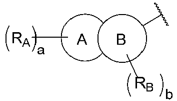 is selected from: 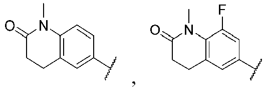 and 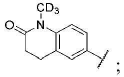 ring C is 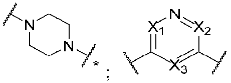 is 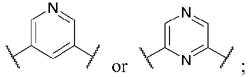 L is 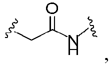 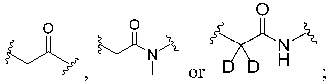 R is

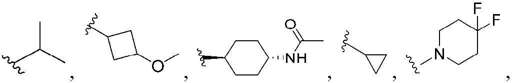

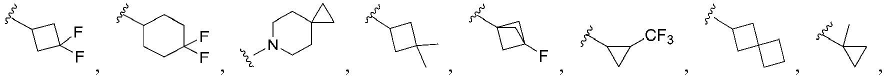

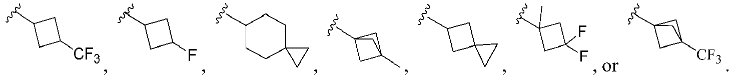

In a second aspect, the present application provides the following compounds, or the stereoisomers, tautomers, isotopic derivatives (preferably deuterated derivatives) or pharmaceutically acceptable salts thereof, wherein the compounds are selected from:

| No. | Structure | No. | Structure |
|---|---|---|---|
| 1 | 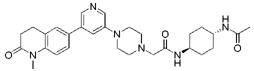 | 2 | 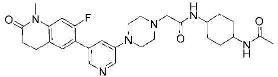 |
| 3 | 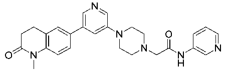 | 4 | 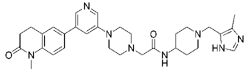 |
| 5 | 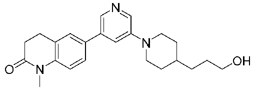 | 6 | 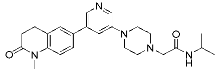 |
| 7 | 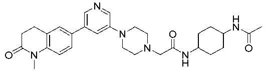 | 8 | 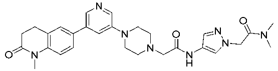 |
| 9 | 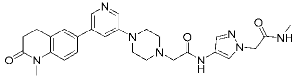 | 10 | 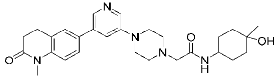 |
| 11 | 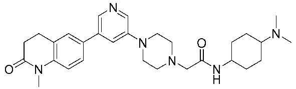 | 12 | 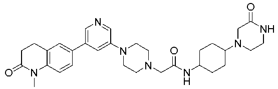 |
| 13 | 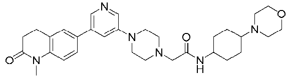 | 14 | 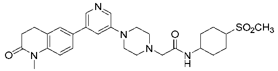 |
| 15 | 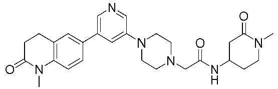 | 16 | 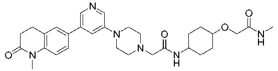 |
| 17 | 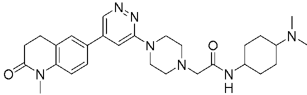 | 18 | 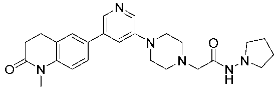 |
| 19 | 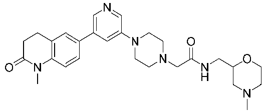 | 20 | 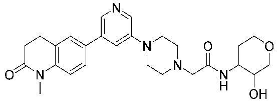 |
| 21 | 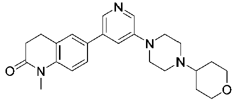 | 22 | 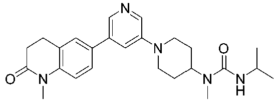 |
| 23 | 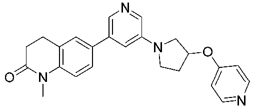 | 24 | 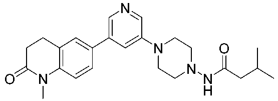 |
| 25 | 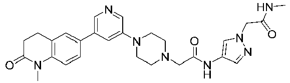 | 26 | 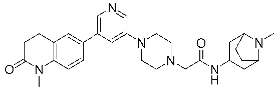 |
| 27 | 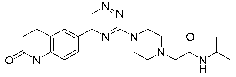 | 28 | 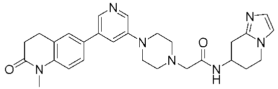 |
| 29 | 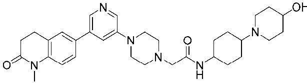 | 30 | 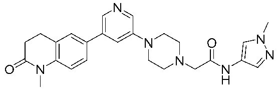 |
| 31 | 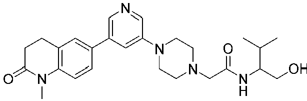 | 32 | 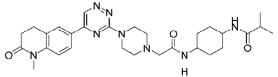 |
| 33 | 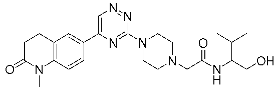 | 34 | 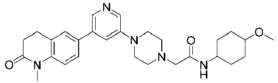 |
| 35 | 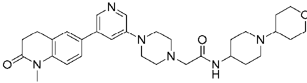 | 36 | 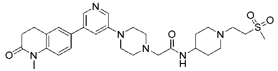 |
| 37 | 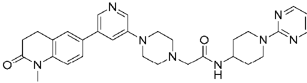 | 38 | 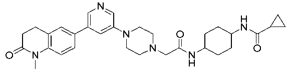 |
| 39 | 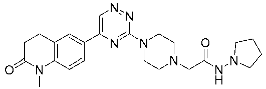 | 40 | 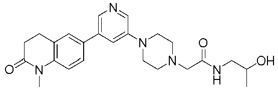 |
| 41 | 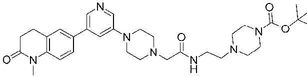 | 42 | 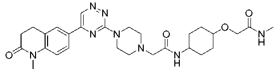 |
| 43 | 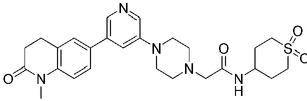 | 44 | 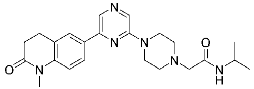 |
| 45 | 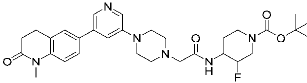 | 46 | 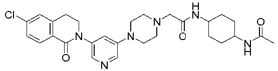 |
| 47 | 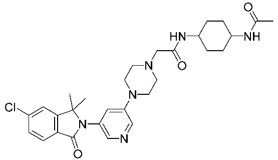 | 48 | 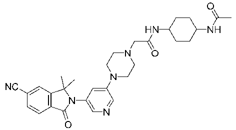 |
| 49 | 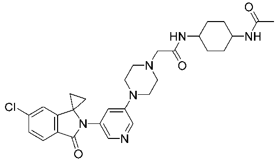 | 50 | 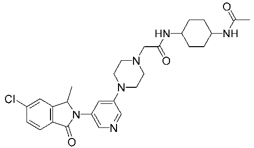 |
| 51 | 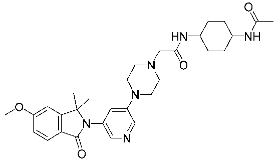 | 52 | 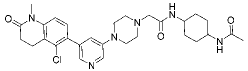 |
| 53 | 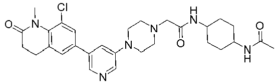 | 54 | 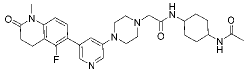 |
| 55 | 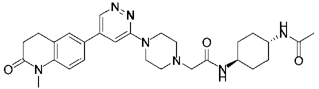 | 56 | 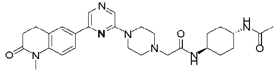 |
| 57 | 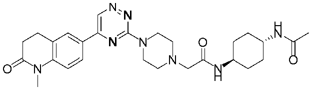 | 58 | 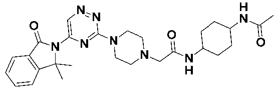 |
| 59 | 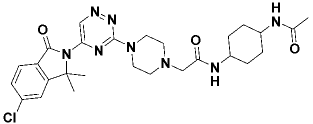 | 60 | 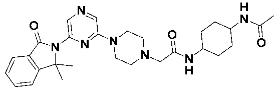 |
| 61 | 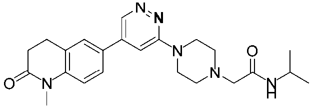 | 62 | 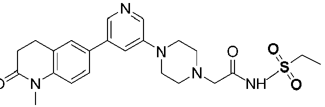 |
| 63 | 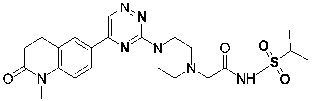 | 64 | 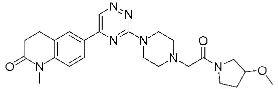 |
| 65 | 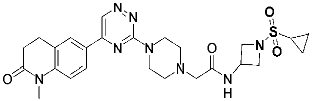 | 66 | 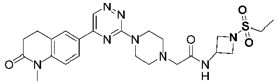 |
| 67 | 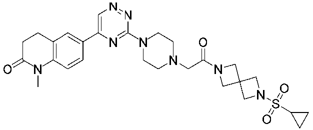 | 68 | 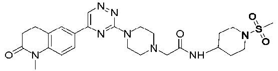 |
| 69 | 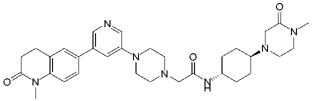 | 70 | 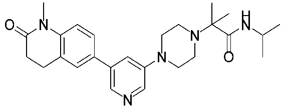 |
| 71 | 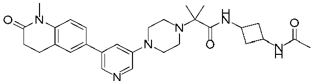 | 72 | 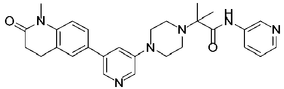 |
| 73 | 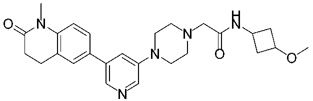 | 74 | 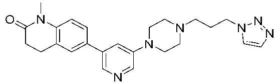 |
| 75 | 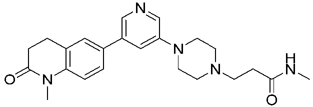 | 76 | 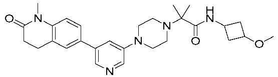 |
| 77 | 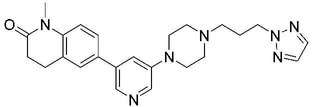 | 78 | 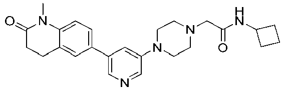 |
| 79 | 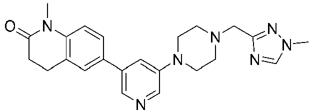 | 80 | 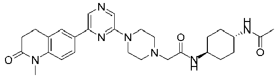 |
| 81 | 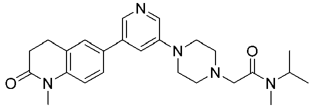 | 82 | 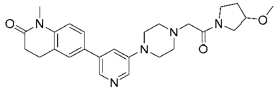 |
| 83 | 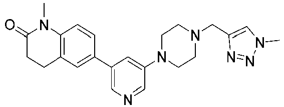 | 84 | 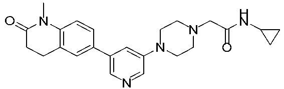 |
| 85 | 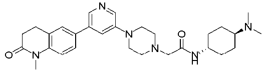 | 86 | 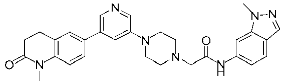 |
| 87 | 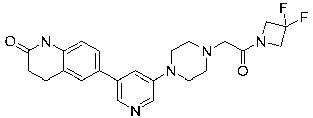 | 88 | 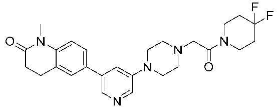 |
| 89 | 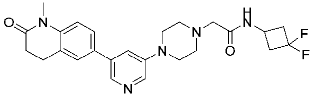 | 90 | 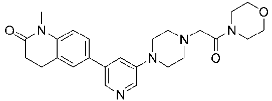 |
| 91 | 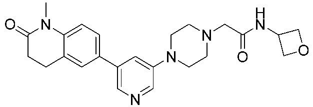 | 92 | 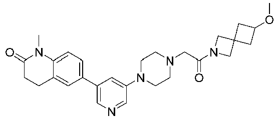 |
| 93 | 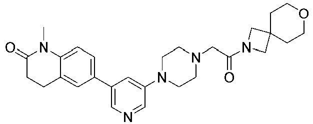 | 94 | 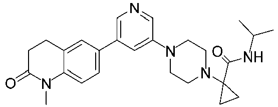 |
| 95 | 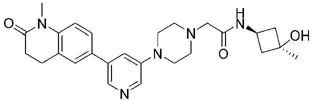 | 96 | 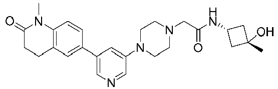 |
| 97 | 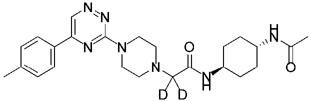 | 98 | 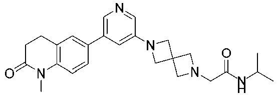 |
| 99 | 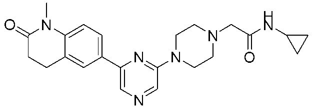 | 100 | 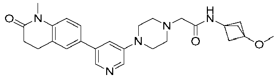 |
| 101 | 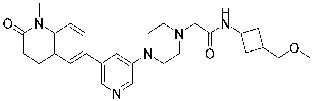 | 102 | 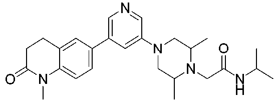 |
| 103 | 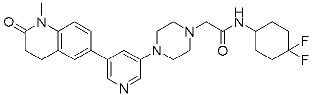 | 104 | 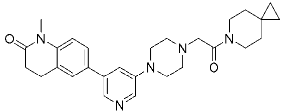 |
| 105 | 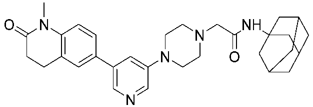 | 106 | 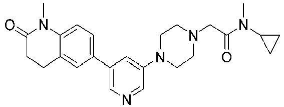 |
| 107 | 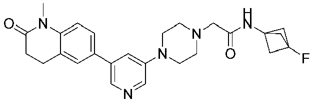 | 108 | 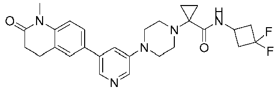 |
| 109 | 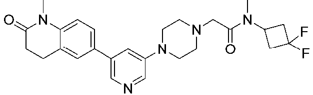 | 110 | 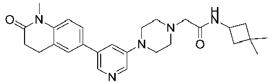 |
| 111 | 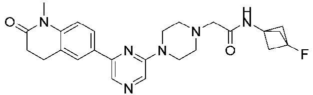 | 112 | 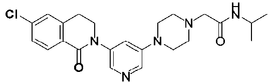 |
| 113 | 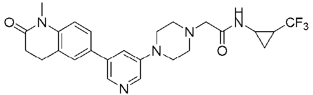 | 114 | 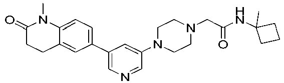 |
| 115 | 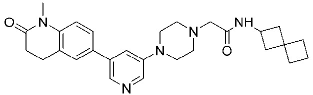 | 116 | 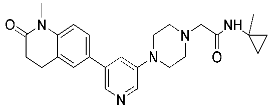 |
| 117 | 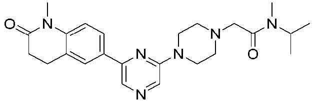 | 118 | 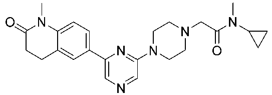 |
| 119 | 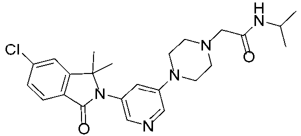 | 120 | 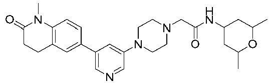 |
| 121 | 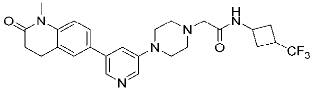 | 122 | 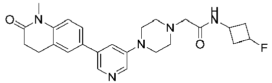 |
| 123 | 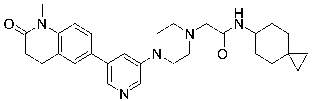 | 124 | 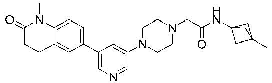 |
| 125 | 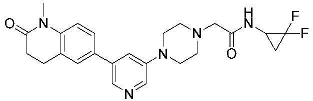 | 126 | 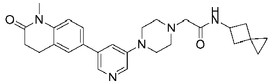 |
| 127 | 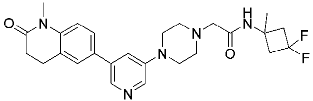 | 128 | 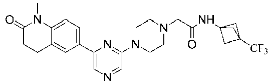 |
| 129 | 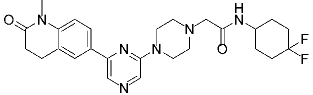 | 130 | 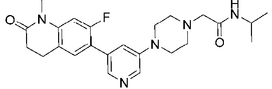 |
| 131 | 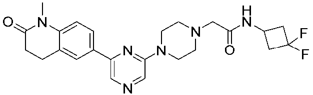 | 132 | 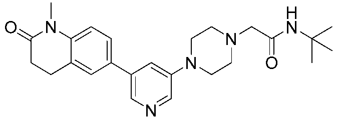 |
| 133 | 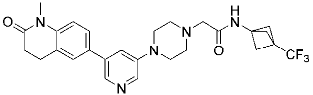 | 134 | 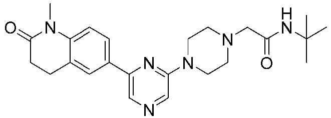 |
| 135 | 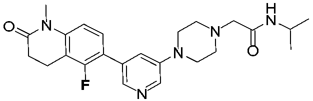 | 136 | 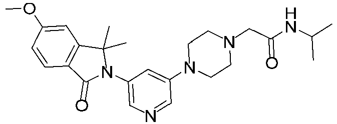 |
| 137 | 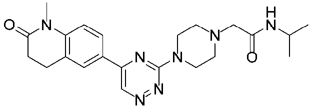 | 138 | 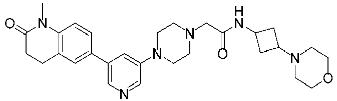 |
| 139 | 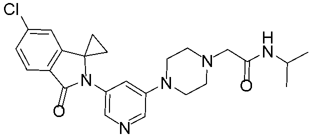 | 140 | 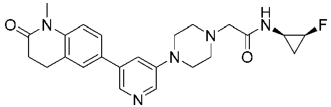 |
| 141 | 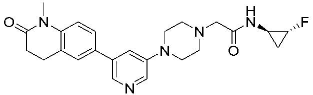 | 142 | 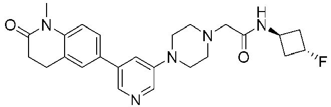 |
| 143 | 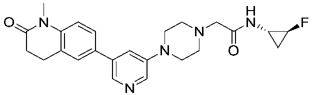 | 144 | 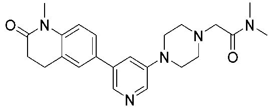 |
| 145 | 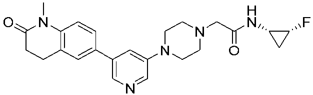 | 146 | 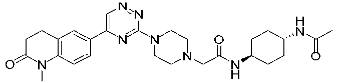 |
| 147 | 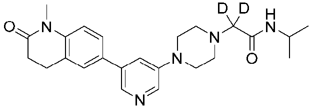 | 148 | 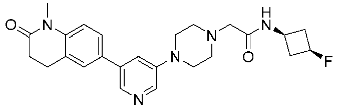 |
| 149 | 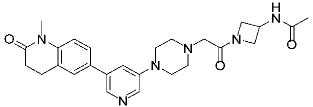 | 150 | 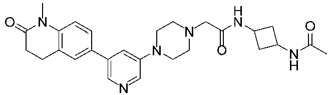 |
| 151 | 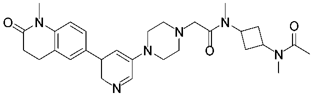 | 152 | 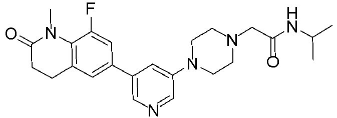 |
| 153 | 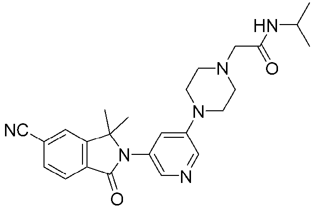 | 154 | 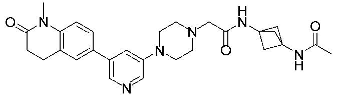 |
| 155 | 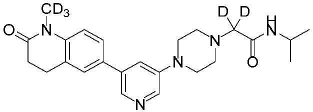 | 156 | 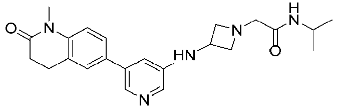 |
| 157 | 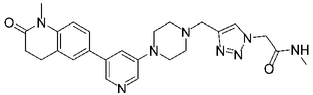 | 158 | 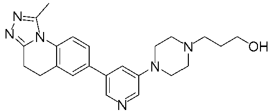 |
| 159 | 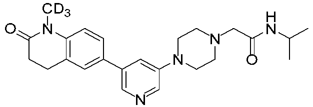 | 160 | 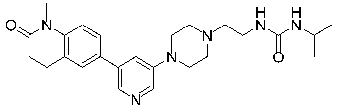 |
| 161 | 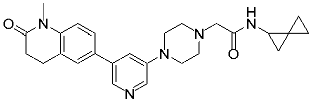 | 162 | 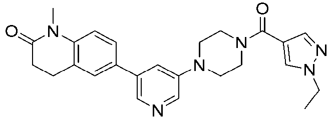 |
| 163 | 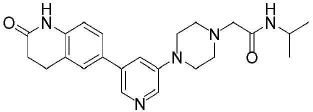 | 164 | 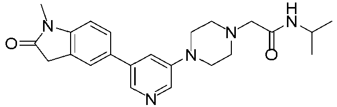 |
| 165 | 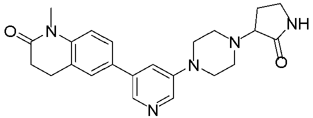 | 166 | 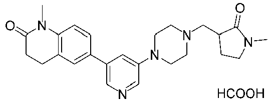 |
| 167 | 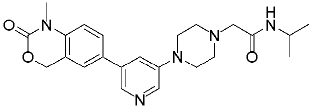 | 168 | 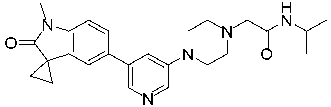 |
| 169 | 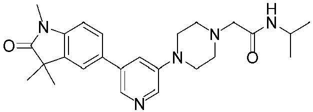 | 170 | 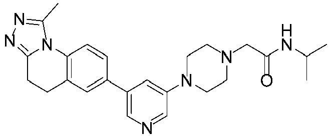 |
| 171 | 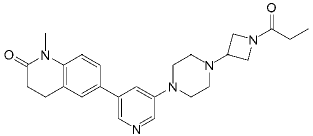 | 172 | 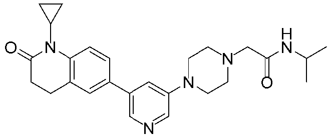 |
| 173 | 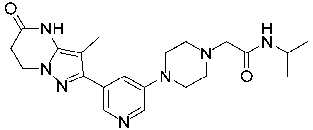 | 174 | 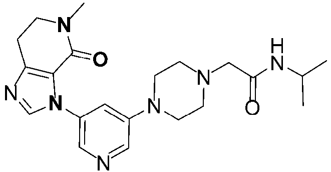 |
| 175 | 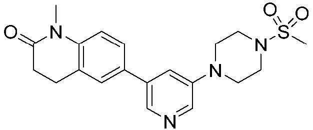 | 176 | 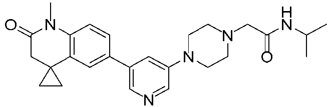 |
| 177 | 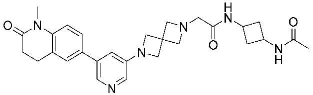 | 178 | 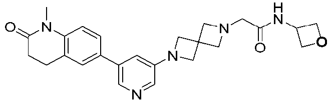 |
| 179 | 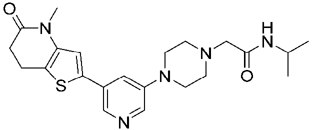 | 180 | 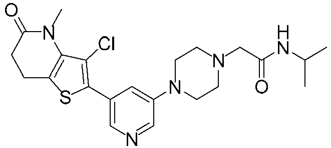 |
| 181 | 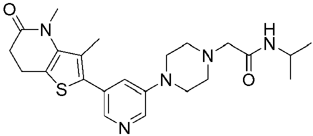 | 182 | 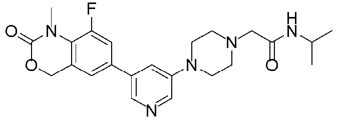 |
| 183 | 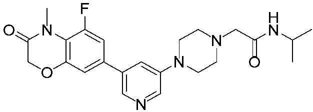 | 184 | 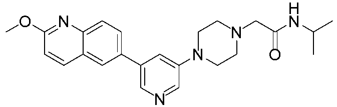 |
| 185 | 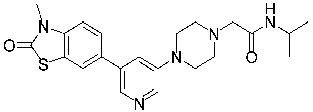 | 186 | 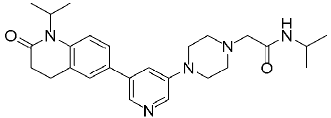 |
| 187 | 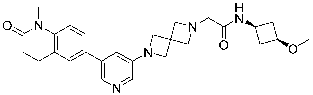 | 188 | 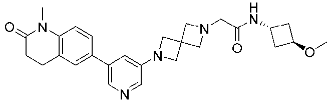 |
| 189 | 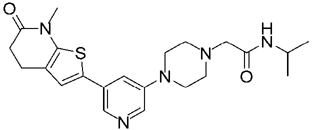 | 190 | 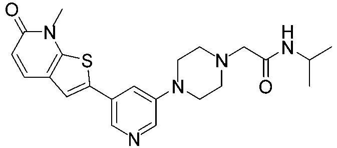 |
| 191 | 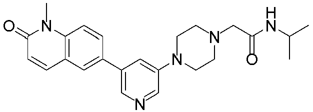 | 192 | 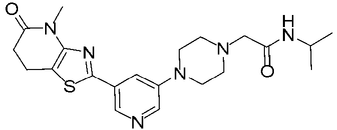 |
| 193 | 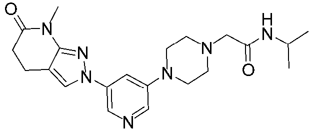 | 194 | 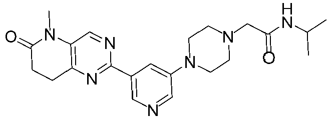 |
| 195 | 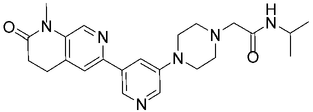 | 196 | 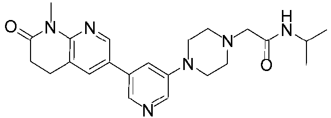 |

In a third aspect, the present application provides a method for preparing the compound represented by formula (I),
including but not limited to the following methods:
general preparation method 1: 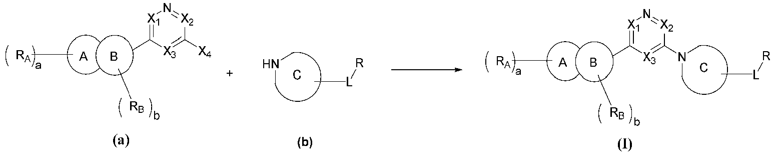
subjecting (a) and (b) to a substitution reaction or a Buchwald-Hartwig coupling reaction to obtain a compound represented by formula (I), wherein X₄ is halogen, preferably Cl or Br, and the definitions of other substituents are as defined for the compound as represented by formula (I), formula (II-1) or formula (II-2);
general preparation method 2: 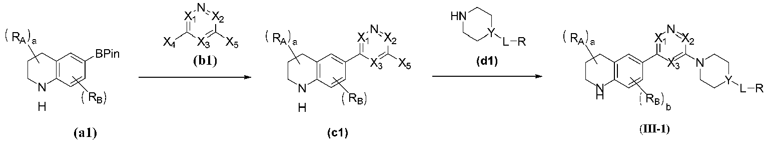
subjecting (a1) and (b1) to a Suzuki reaction to obtain intermediate (c1), wherein X₄ and X₅ are halogen, preferably Cl or Br; subjecting (c1) and (d1) to a substitution reaction or a Buchwald-Hartwig coupling reaction to obtain a compound represented by formula (III-1), wherein the definitions of other substituents are as defined for the compound represented by formula (I), formula (II-1) or formula (II-2);
general preparation method 3: 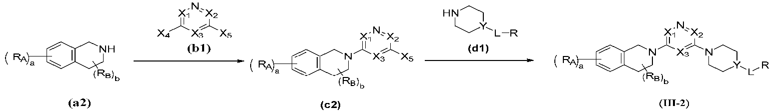
subjecting (a2) and (b1) to a substitution reaction or a metal-catalysed coupling reaction to obtain intermediate (c2), wherein X₄ is halogen or OSO₂CF₃, and X₅ is halogen, preferably Cl or Br; subjecting (c2) and (d1) to a substitution reaction or a Buchwald-Hartwig coupling reaction to obtain a compound represented by formula (III-2), wherein the definitions of other substituents are as described above.

The compound represented by formula (I) described in the present application may be prepared by referring to the general preparation methods described above, and/or by combining similar preparation methods described in the literature (e.g., Liu, Yongfu et al., Journal of Medicinal Chemistry (2020), 63(13), 6876-6897., WO 2013079452 A1**,** etc.).

In a fourth aspect, the present application provides a pharmaceutical composition comprising the compound according to the first aspect of the present application (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof. Preferably, the pharmaceutical composition further comprises a pharmaceutically acceptable carrier.

In a fifth aspect, the present application provides the use of the compound according to the first aspect of the present application (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof, or the pharmaceutical composition according to the fourth aspect, as a medicament or in the manufacture of a medicament; preferably, the medicament is used as a CYP11B2 inhibitor; further preferably, the medicament is used for the prevention and/or treatment of a CYP11B2-mediated disease; further preferably, the medicament is used for the treatment and/or prevention of a disease or condition caused by aldosterone excess; further preferably, the medicament is used for the treatment and/or prevention of a cardio-cerebrovascular disease or a kidney disease; further preferably, the medicament is used for the treatment and/or prevention of hypertension, hyperaldosteronism, Cushing's disease, heart failure or chronic kidney disease; further preferably, the medicament is used for the treatment and/or prevention of refractory hypertension, hypertension due to primary aldosteronism, primary hyperaldosteronism, heart failure or chronic kidney disease.

In some embodiments, provided is a use of the compound according to the first aspect (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof, or the pharmaceutical composition according to the fourth aspect, in the manufacture of a medicament for the treatment and/or prevention of a cardio-cerebrovascular disease or a kidney disease; further preferably, the medicament is used for the treatment and/or prevention of hypertension, hyperaldosteronism, Cushing's disease, heart failure or chronic kidney disease; further preferably, the medicament is used for the treatment and/or prevention of refractory hypertension, hypertension due to primary aldosteronism, primary hyperaldosteronism, Cushing's disease, heart failure or chronic kidney disease.

In a sixth aspect, the present application provides the compound according to the first aspect (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof, or the pharmaceutical composition according to the fourth aspect, for use in the prevention and/or treatment of a disease; preferably, the disease is a CYP11B2-mediated disease; preferably, the disease is a cardio-cerebrovascular disease or a kidney disease; further preferably, the disease is hypertension, hyperaldosteronism, Cushing's disease, heart failure or chronic kidney disease; further preferably, the disease is refractory hypertension, hypertension due to primary aldosteronism, Cushing's disease, primary hyperaldosteronism, heart failure or chronic kidney disease.

In some embodiments, provided is the compound according to the first aspect (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof, or the pharmaceutical composition according to the fourth aspect, for use in the prevention and/or treatment of a disease; preferably, the disease is a cardio-cerebrovascular disease or a kidney disease; further preferably, the disease is hypertension, hyperaldosteronism, Cushing's disease, heart failure or chronic kidney disease; further preferably, the disease is refractory hypertension, hypertension due to primary aldosteronism, Cushing's disease, primary hyperaldosteronism, heart failure or chronic kidney disease.

In a seventh aspect, the present application provides a method for treating a disease, wherein the method comprises administering to a subject in need thereof a therapeutically effective amount of the compound according to the first aspect (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof, or the pharmaceutical composition according to the fourth aspect; preferably, the disease is a CYP11B2-mediated disease; further preferably, the disease is a cardio-cerebrovascular disease or a kidney disease; further preferably, the disease is hypertension, hyperaldosteronism, Cushing's disease, heart failure or chronic kidney disease; further preferably, the disease is refractory hypertension, hypertension due to primary aldosteronism, Cushing's disease, primary hyperaldosteronism, heart failure or chronic kidney disease.

In some embodiments, the present application provides a method for treating a disease, wherein the method comprises administering to a subject in need thereof a therapeutically effective amount of the compound according to the first aspect (the compound represented by formula (I), formula (II-1), formula (II-2), formula (II-3), formula (II-4), formula (III-1), formula (III-2), formula (III-3), formula (III-4), formula (IV-A), formula (IV-B), formula (IV-C), formula (IV-1), formula (IV-2), formula (IV-3), formula (V), or formula (A), or compounds 1-196) or the compound according to the second aspect, or the tautomer, stereoisomer, pharmaceutically acceptable salt and isotopic derivative (preferably deuterated derivative) thereof, or the pharmaceutical composition according to the fourth aspect; preferably, the disease is a cardio-cerebrovascular disease or a kidney disease; further preferably, the disease is hypertension, hyperaldosteronism, Cushing's disease, heart failure or chronic kidney disease; further preferably, the disease is refractory hypertension, hypertension due to primary aldosteronism, Cushing's disease, primary hyperaldosteronism, heart failure or chronic kidney disease.

### Definitions

Unless otherwise specified, the following terms used in the present application have the meanings set forth below. A term that is not specifically defined should not be considered uncertain or unclear, but should be understood according to its ordinary meaning in the art. When a trade name appears herein, it is intended to refer to the corresponding commodity or an active ingredient thereof.

As used herein, "Cₘ₋ₙ" means that the moiety has an integer number of carbon atoms, wherein the integer falls within a specified range. For example, "C₁₋₆" means that the group may have 1, 2, 3, 4, 5, or 6 carbon atoms.

Where any variable (e.g., R_{A}) appears more than once in the composition or structure of a compound, its definition in each case is independent. Thus, for example, if a group, a position, or an atom is substituted with two R_{A}, each R_{A} is independently selected.

As used herein, -N(R₁)- refers to 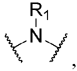 wherein 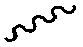 refers to the point of attachment, and -N(R₂)R₃ refers to 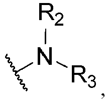 wherein 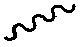 refers to the point of attachment.

As used herein, -C(O)- refers to 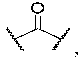 wherein 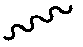 refers to the point of attachment. As used herein, - C(O)N(R₇)R₈ refers to 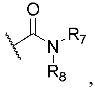 wherein 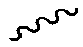 refers to the point of attachment. As used herein, -C(O)OR₉ refers to 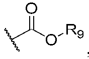 wherein 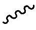 refers to the point of attachment. As used herein, -S(O)- refers to 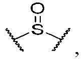 wherein 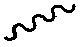 refers to the point of attachment. As used herein, -S(O)₂- refers to 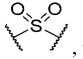 wherein 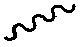 refers to the point of attachment. As used herein, -S(O)₂R₄ refers to 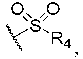 wherein 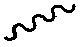 refers to the point of attachment.

Unless otherwise specified, the term "alkyl" refers to a monovalent saturated aliphatic hydrocarbon group, which is a straight or branched chain group containing 1 to 20 carbon atoms, preferably containing 1 to 10 carbon atoms (i.e., C₁₋₁₀ alkyl), further preferably containing 1 to 8 carbon atoms (C₁₋₈ alkyl), more preferably containing 1 to 6 carbon atoms (i.e., C₁₋₆ alkyl), more preferably containing 1 to 4 carbon atoms (i.e., C₁₋₄ alkyl), and more preferably containing 1 to 3 carbon atoms (i.e., C₁₋₃ alkyl). For example, "C₁₋₆ alkyl" means that the group is an alkyl group having from 1 to 6 carbon atoms (specifically, 1, 2, 3, 4, 5, or 6 carbon atoms) on the carbon chain. Examples include, but are not limited to methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, etc.

Unless otherwise specified, the term "halogen" or "halo" refers to F, Cl, Br and I; preferably F, Cl, and Br; more preferably F and Cl.

The term "haloalkyl" refers to an alkyl group as defined above in which one, two, or more hydrogen atoms (e.g., 3, 4, 5, 6, 7, or 8 hydrogen atoms) or all hydrogen atoms are substituted with halogen. For example, when C₁₋₆ alkyl is substituted with halogen, it refers to C₁₋₆ haloalkyl, such as: CCl₃, CH₂F, CHF₂, CF₃, CHCl₂, CH₂Cl, CH₂Br, CH₂I, CH₂CF₃, or CF₂CF₃.

The term "haloalkoxy" refers to an alkoxy group as defined above in which one, two, or more hydrogen atoms (e.g., 3, 4, 5, 6, 7, or 8 hydrogen atoms) or all hydrogen atoms are substituted with halogen. As defined herein for each individual part, haloalkoxy may be optionally substituted.

Unless otherwise specified, the term "cycloalkyl" refers to a monocyclic saturated aliphatic hydrocarbon group having a specified number of carbon atoms, preferably containing 3 to 12 carbon atoms (i.e., C₃₋₁₂ cycloalkyl, or 3- to 12-membered cycloalkyl), more preferably containing 3 to 10 carbon atoms (C₃₋₁₀ cycloalkyl), further preferably 3 to 6 carbon atoms (C₃₋₆ cycloalkyl), 4 to 6 carbon atoms (C₄₋₆ cycloalkyl), or 5 to 6 carbon atoms (C₅₋₆ cycloalkyl). Examples include, but are not limited to cyclopropyl, cyclobutyl, cyclopentyl, cyclohexyl, methylcyclopropyl, 2-ethyl-cyclopentyl, dimethylcyclobutyl, etc.

Unless otherwise specified, the term "alkoxy" refers to an alkyl residue in which one or more carbons (and associated hydrogens thereof) are substituted with oxygen, for example, "alkoxy". Examples include methoxy, ethoxy, propoxy, etc.

Unless otherwise specified, the term "alkylthio" refers to the above-described "alkoxy" in which the oxygen atom is replaced by a sulphur or nitrogen atom.

Unless otherwise specified, the term "heterocyclyl" refers to a saturated or partially unsaturated monocyclic, bicyclic, or polycyclic hydrocarbon substituent having a non-aromatic structure and containing 3 to 20 ring atoms, wherein 1, 2, 3, or more (e.g., 4, 5, 6, 7, or 8) ring atoms are selected from N, O, or S, and the remaining ring atoms are C. Preferably, it contains 3 to 14 ring atoms, 3 to 12 ring atoms, further preferably 3 to 10 ring atoms, or 3 to 8 ring atoms, or 3 to 6 ring atoms, or 4 to 6 ring atoms, or 5 to 6 ring atoms. The number of heteroatoms is preferably 1 to 4, more preferably 1 to 3 (i.e., 1, 2 or 3). Examples of monocyclic heterocyclyl include pyrrolidyl, imidazolidinyl, tetrahydrofuryl, dihydropyrrolyl, piperidyl, piperazinyl, pyranyl, etc. Bicyclic or polycyclic heterocyclyl include spiro, fused (also referred to as annulated), and bridged heterocyclyl. The heterocyclyl may be linked (e.g., bridged, spiro, or fused (or annulated)) to other cyclic groups (including cycloalkyl, cycloalkenyl, heterocycloalkenyl, aryl, or heteroaryl), provided that the point of attachment to the parent moiety is on a carbon atom or a heteroatom of the heterocyclyl. As defined herein for each individual part, each heterocyclyl or other cyclic groups may be optionally substituted.

The term "heterocycloalkyl" refers to a saturated "heterocyclyl" group as defined above, containing 3 to 20 ring atoms, wherein 1, 2, 3, or more (e.g., 4, 5, 6, 7, or 8) ring atoms are selected from N, O, or S, and the remaining ring atoms are C. Preferably, it contains 3 to 14 ring atoms, 3 to 12 ring atoms, further preferably 3 to 10 ring atoms, or 3 to 8 ring atoms, or 3 to 6 ring atoms, or 4 to 6 ring atoms, or 5 to 6 ring atoms. The number of heteroatoms is preferably 1 to 4, more preferably 1 to 3 (i.e., 1, 2 or 3). Bicyclic or polycyclic heterocycloalkyl includes spiro, fused (also referred to as annulated), and bridged heterocycloalkyl. The heterocycloalkyl may be linked (e.g., bridged, spiro, or fused (or annulated)) to other cyclic groups (including cycloalkyl, cycloalkenyl, heterocycloalkenyl, aryl, or heteroaryl), provided that the point of attachment to the parent moiety is on a carbon atom or a heteroatom of the heterocycloalkyl. As defined herein for each individual part, each heterocycloalkyl or other cyclic groups may be optionally substituted.

Unless otherwise specified, the term "aryl" refers to a monocyclic, bicyclic, or tricyclic aromatic carbocyclic ring system containing 6 to 16 carbon atoms (6- to 16-membered aryl), or 6 to 14 carbon atoms, or 6 to 12 carbon atoms, or 6 to 10 carbon atoms, preferably 6 to 10 carbon atoms. The term "aryl" can be used interchangeably with the term "aromatic ring". Examples of aryl groups may include, but are not limited to phenyl, naphthyl, anthryl, phenanthryl, pyrenyl, etc. "Aryl" also includes ring systems in which the aryl as defined above is fused or annulated with one or more cycloalkyl, heterocyclyl or heteroaryl, provided that the point of attachment to the parent moiety is on a carbon atom of the aryl. As defined herein for each individual part, each aryl or other cyclic groups may be optionally substituted.

Unless otherwise specified, the term "heteroaryl" refers to an aromatic monocyclic, bicyclic, or polycyclic ring system having a 5- to 16-membered structure, a 5- to 14-membered structure, a 5- to 12-membered structure, a 5- to 10-membered structure, a 5- to 8-membered structure, or a 5- to 6-membered structure, wherein 1, 2, 3, or more (e.g., 4, 5, 6, 7, or 8) ring atoms are heteroatoms and the remaining atoms are carbon atoms, the heteroatoms are independently selected from O, N, or S, and the number of heteroatoms is preferably 1, 2, 3, or 4; more preferably, the number of heteroatoms is 1, 2 or 3. Examples of heteroaryl may include, but are not limited to furyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiodiazolyl, triazinyl, phthalazinyl, quinolyl, isoquinolyl, pteridinyl, purinyl, indolyl, isoindolyl, indazolyl, benzofuryl, benzothienyl, benzopyridyl, benzopyrimidinyl, benzopyrazinyl, benzoimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridyl, etc. "Heteroaryl" also includes ring systems in which the heteroaryl as defined above is fused or annulated with one or more cycloalkyl, heterocyclyl or aryl, provided that the point of attachment to the parent moiety is on an atom of the heteroaryl. As defined herein for each individual part, each aryl or other cyclic groups may be optionally substituted.

Unless otherwise specified, the term "pharmaceutically acceptable salt" or "pharmaceutical salt" refers to a salt that, within the scope of sound medical judgment, is suitable for contact with the tissues of mammals, particularly humans, without undue toxicity, irritation, allergic reaction, and the like, and is commensurate with a reasonable benefit/risk ratio. For example, pharmaceutically acceptable salts of amines, carboxylic acids, and other types of compounds are well known in the art. Such salts may be prepared in situ during the final separation and purification of the compounds of the present application, or separately by reacting the free base or free acid with an appropriate reagent.

Unless otherwise specified, the term "stereoisomer" refers to compounds that have the same chemical constitution but differ in the spatial arrangement of their atoms or groups. Stereoisomers include optical isomers, enantiomers, diastereomers, conformational isomers (rotamers), geometric isomers (also referred to as cis/trans isomers), atropisomers, etc. Any resulting mixture of stereoisomers can be separated into pure or substantially pure optical isomers, geometric isomers, enantiomers, or diastereomers based on differences in the physicochemical properties of the components, for example, by chromatography and/or fractional crystallisation.

Unless otherwise specified, the term "tautomer" refers to structural isomers that have different energies and can interconvert via a low energy barrier. When tautomerism is possible (e.g., in solution), a chemical equilibrium of tautomers can be achieved. For example, prototropic tautomers (also known as proton-transfer tautomers) include interconversions via proton migration, such as keto-enol tautomerism and imine-enamine tautomerism. Valence tautomers include interconversions via the reorganisation of some bonding electrons.

Unless otherwise indicated, the structural formulas described in the present application include all isomeric forms (e.g., enantiomers, diastereomers, and geometric isomers (or conformational isomers)), such as R and S configurations containing asymmetric centres, (Z) and (E) isomers for double bonds, and (Z) and (E) conformational isomers. Accordingly, individual stereochemical isomers and mixtures of enantiomers, diastereomers, or geometric isomers (or conformational isomers) of the compounds of the present application fall within the scope of the present application.

The compounds of the present disclosure also include isotopic derivatives thereof. Unless otherwise specified, the term "isotopic derivative" means that the compounds of the present disclosure may exist in isotopically labelled or enriched form, containing one or more atoms whose atomic weight or mass number differs from the atomic weight or mass number of the atom most abundantly found in nature. Isotopes can be radioactive or nonradioactive. The isotopes commonly used for isotopic labelling are: hydrogen isotopes ²H and ³H; carbon isotopes ¹³C and ¹⁴C; chlorine isotopes ³⁵Cl and ³⁷Cl; fluorine isotope ¹⁸F; iodine isotopes ¹²³I and ¹²⁵I; nitrogen isotopes ¹³N and ¹⁵N; oxygen isotopes ¹⁵O, ¹⁷O and ¹⁸O, and sulphur isotope ³⁵S. These isotopically labelled compounds can be used to study the distribution of pharmaceutical molecules in tissues. Particularly, ³H and ¹³C are more widely used because they are easy to label and convenient to detect. Substitution with certain heavy isotopes, such as deuterium (²H), can enhance metabolic stability and prolong half-life, thereby achieving the goal of reducing dosage and providing therapeutic advantages. Isotopically labelled compounds are generally synthesised from labelled starting materials using known synthetic techniques, in the same manner as the synthesis of non-isotopically labelled compounds. The isotopic derivative is preferably a deuterated derivative.

Unless otherwise specified, the term "optionally substituted" means that the hydrogen at a substitutable position of the group is unsubstituted, or is substituted with one or more substituents, wherein the substituents are preferably selected from the group of halogen, hydroxy, mercapto, cyano, nitro, amino, azido, oxo (=O), carboxyl, C₂₋₆ alkenyl, C₂₋₆ alkynyl, C₁₋₆ alkyl, C₁₋₆ alkoxy, C₃₋₁₀ cycloalkyl, C₃₋₁₀ cycloalkylsulphonyl, 3- to 10-membered heterocyclyl, C₆₋₁₄ aryl or 5- to 10-membered heteroaryl, wherein, the C₂₋₆ alkenyl, C₂₋₆ alkynyl, C₁₋₆ alkyl, C₁₋₆ alkoxy, C₃₋₁₀ cycloalkyl, C₃₋₁₀ cycloalkylsulphonyl, 3- to 10-membered heterocycloalkyl, C₆₋₁₄ aryl or 5- to 10-membered heteroaryl may be optionally substituted with one or more substituents selected from halogen, hydroxy, amino, cyano, C₁₋₆ alkyl or C₁₋₆ alkoxy, and the oxo means that two H at the same substitution position are replaced by the same O to form a double bond; preferably, the substituents are preferably selected from the group of halogen, hydroxy, mercapto, cyano, nitro, amino, azido, oxo (=O), carboxyl, C₂₋₄ alkenyl, C₂₋₄ alkynyl, C₁₋₄ alkyl, C₁₋₄ alkoxy, C₃₋₇ cycloalkyl, C₃₋₇ cycloalkylsulphonyl, 3- to 7-membered heterocyclyl, C₆₋₁₀ aryl or 5- to 10-membered heteroaryl, wherein, the C₂₋₄ alkenyl, C₂₋₄ alkynyl, C₁₋₄ alkyl, C₁₋₄ alkoxy, C₃₋₇ cycloalkyl, C₃₋₇ cycloalkylsulphonyl, 3- to 7-membered heterocyclyl, C₆₋₁₀ aryl or 5- to 10-membered heteroaryl may be optionally substituted with one or more substituents selected from halogen, hydroxy, amino, cyano, C₁₋₄ alkyl or C₁₋₄ alkoxy; preferably, the substituents are preferably selected from the group of halogen, hydroxy, mercapto, cyano, nitro, amino, azido, oxo (=O), carboxyl, C₂₋₄ alkenyl, C₂₋₄ alkynyl, C₁₋₃ alkyl, C₁₋₃ alkoxy, C₃₋₆ cycloalkyl, C₃₋₆ cycloalkylsulphonyl, 4- to 6-membered heterocyclyl, phenyl or 5- to 6-membered heteroaryl, wherein, the C₂₋₄ alkenyl, C₂₋₄ alkynyl, C₁₋₃ alkyl, C₁₋₃ alkoxy, C₃₋₆ cycloalkyl, C₃₋₆ cycloalkylsulphonyl, 4- to 6-membered heterocyclyl, phenyl or 5- to 6-membered heteroaryl may be optionally substituted with one or more substituents selected from halogen, hydroxy, amino, cyano, C₁₋₃ alkyl or C₁₋₃ alkoxy.

The term "optionally" or "optional" means that the subsequently described event or circumstance may but not necessarily occur, and this description includes instances where the event or circumstance occurs and instances where it does not occur.

The word "comprise" or "comprising" and its English variants such as "comprises" or "comprising" should be understood in an open, non-exclusive sense, i.e., "including but not limited to".

The term "subject", which may also be referred to as "individual" or "participant", refers to a cell or an animal, wherein the animal includes, but is not limited to, mammals such as laboratory animals or humans.

"Effective amount" refers to an amount of a compound or pharmaceutical composition described herein sufficient to achieve the intended application, including but not limited to the treatment of a disease or the alleviation of a symptom. In some embodiments, for example, the amount is a dose that can induce a specific response in a cell or can exert a therapeutic effect on a disease in a model animal. It will vary depending on, for example, the specific compound selected, the species of the subject and their age/current health status, the dosing regimen followed, the severity of the disease, whether it is administered in combination with other agents, the timing of administration, the tissue of administration, and the physical delivery system carrying it.

Some compounds of the present application are optically active. The compounds of the present application may be in the form of a racemate, an optical isomer, or a mixture thereof. The optical isomers of the compounds of the present application may be synthesised either by using starting materials of the optical isomers or by separation from racemates.

Unless otherwise specified, the term "pharmaceutically acceptable carrier", also referred to as "pharmaceutically acceptable adjuvant" or "pharmaceutically acceptable excipient", refers to a medium generally recognised in the field of delivering bioactive agents to animals (particularly mammals). Pharmaceutically acceptable carriers are formulated according to many factors that are well within the purview of those of ordinary skill in the art. These include, but are not limited to, the type and nature of the active agent being formulated, the subject to which the composition containing the agent is to be administered, the intended route of administration of the composition, and the targeted therapeutic indication. Pharmaceutically acceptable carriers include aqueous and non-aqueous liquid media, as well as various solid and semi-solid dosage forms. The preparation of the pharmaceutical composition described herein includes, but is not limited to, for example, mixing the compound, or the tautomer, stereoisomer, or pharmaceutically acceptable salt thereof according to the first aspect or the second aspect, with a pharmaceutically acceptable carrier.

The above embodiments represent exemplary embodiments of the present application, but the present application is not limited to the above embodiments. Furthermore, the various technical features in the above embodiments of the present application may be combined with one another to form one or more new technical solutions, and such new technical solutions also fall within the scope of the present application, provided that such new technical solutions are technically feasible.

### Detailed Description of Embodiments

The present application is further described below in conjunction with specific examples. It should be understood that these examples are merely used for describing the present application, rather than limiting the scope of the present application. In the following examples, the experimental methods for which specific conditions are not indicated are generally carried out under conventional conditions or under conditions recommended by the manufacturers. Unless otherwise defined, all professional and scientific terms used herein have the same meanings as those commonly understood by a person skilled in the art. In addition, any methods and materials similar or equivalent to those described herein can all be applied in the method of the present application. The preferred embodiments and materials described herein are meant for exemplary purposes only.

The structures of the compounds of the present application are determined by nuclear magnetic resonance (NMR) and/or liquid chromatography-mass spectrometry (LC-MS) and/or high performance liquid chromatography (HPLC). NMR measurements are performed using a Bruker 400 MHz and/or Varian 400 MHz instrument. LC-MS measurements are performed using an Agilent 1260 Infinity II-6120/6125MSD instrument. HPLC measurements are performed using a Waters Acquity UPLC_2 and/or Shimadzu LC2030 and/or Agilent 1260 Infinity II instrument. Chiral compound resolution is performed using an SFC-150 (Waters) instrument with a DAICEL CHIRALCEL^{®}OD chiral column (column volume: 20×250 mm; packed with 10 µm particle size).

The starting materials in the examples of the present application are known and commercially available, or can be synthesised using or according to methods known in the art.

The present application provides a method for preparing the compound. The compound can be prepared by the following steps.

The meanings of the abbreviations used in the present application are as follows:
PE: petroleum ether; EA: ethyl acetate; DCM: dichloromethane; MeOH: methanol; FA: formic acid; MeCN: acetonitrile; THF: tetrahydrofuran; TFA: trifluoroacetic acid.

### Beneficial effects of the present application:

The compounds of the present application achieve one or more of the following beneficial effects:
(1) the compounds of the present application exhibit strong inhibitory effects on CYP11B2;
(2) the compounds of the present application exhibit high selectivity for CYP11B2/CYP11B1;
(3) the compounds of the present application exhibit favourable oral absorption properties in monkeys.

### Preparation example 1

### Preparation of 1-methyl-6-(4,4,5,5-tetramethyl-1,3,2-dioxaborolan-2-yl)-3,4-dihydroquinolin-2(1H)-one:

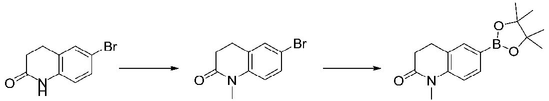

### Step 1: Synthesis of 6-bromo-1-methyl-3,4-dihydroquinolin-2(1H)-one:

6-Bromo-3,4-dihydroquinolin-2(1H)-one (10 g, 1.0 eq) was dissolved in N,N-dimethylformamide (180 mL). At 0°C, 60% sodium hydride (2.54 g) was added in batches. The mixture was slowly warmed to room temperature. After 30 minutes, to the reaction solution was added iodomethane (9.42 g). At room temperature, the mixture was stirred for another 2 hours. The reaction solution was quenched by pouring into ice water (600 mL), and extracted with ethyl acetate (200 mL*3). The organic phases were combined, dried over anhydrous sodium sulphate and filtered, and the filtrate was concentrated under reduced pressure. The residue was purified by flash chromatography (silica gel, petroleum ether (PE) : ethyl acetate (EA) = 10 : 3, v/v) to afford the product (10.5 g). LCMS (ESI) [M+H]⁺ = 239.8.

### Step 2:

To a solution of the product obtained in step 1 (4.5 g, 1.0 eq), bis(pinacolato)diboron (5.71 g), potassium acetate (5.52 g) in 1,4-dioxane (100 mL), was added Pd(dppf)Cl₂ (687.58 mg). The mixture was stirred at 90°C for 12 hours under a nitrogen atmosphere. The reaction solvent was concentrated, and the residue was purified by flash chromatography (silica gel, PE : EA = 10 : 3) to afford the product (5.3 g). LCMS (ESI) [M+H]⁺ = 287.9.

### Preparation example 2

### Preparation of 6-(5-bromopyridin-3-yl)-1-methyl-3,4-dihydroquinolin-2(1H)-one:

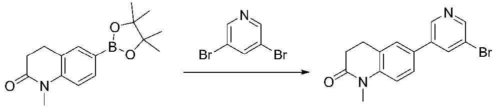

3,5-Dibromopyridine (7.93 g, 1.0 eq) was added to N,N-dimethylformamide (80 mL). The sample of preparation example 1 (10.57 g), aqueous sodium sulphate solution (1 N, 69 mL) and bis(triphenylphosphine)dichloropalladium (2.35 g) were added, and the mixture was stirred at 110°C for 12 hours. The reaction solution was diluted with ethyl acetate (100 mL), and then filtered through a pad of Celite. The filtrate was treated with saturated aqueous sodium bicarbonate solution (500 mL) and extracted with ethyl acetate (500 mL*3). The combined organic phases were dried over anhydrous sodium sulphate and filtered. The filtrate was concentrated under reduced pressure. The residue was separated and purified by flash chromatography (silica gel, PE : EA = 9 : 1) to give the product (1.30 g). LCMS (ESI) [M+H]⁺ = 319.2.

### Preparation example 3

### Preparation of 2-(4-(5-(1-methyl-2-oxo-1,2,3,4-tetrahydroquinolin-6-yl)pyridin-3-yl)piperazin-1-yl)acetic acid:

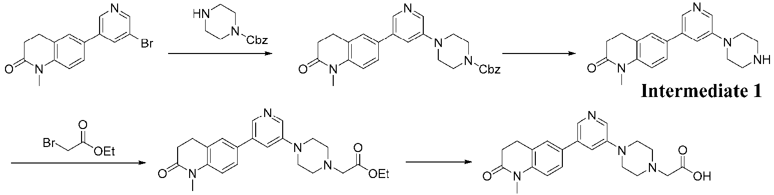

### Step 1: Synthesis of benzyl 4-(5-(1-methyl-2-oxo-1,2,3,4-tetrahydroquinolin-6-yl)pyridin-3-yl)piperazine-1-carboxylate:

The product obtained in of preparation example 2 (1.87 g, 1.0 eq) was dissolved in 1,4-dioxane (13 mL). Benzyl piperazine-1-carboxylate (1.30 g), caesium carbonate (5.77 g) and chloro(2-dicyclohexylphosphino-2',6'-diisopropoxy-1,1'-biphenyl)(2-amino-1,1'-biphenyl-2-yl)palladium(II) (0.23 g) were added sequentially, and the mixture was stirred at 80°C for 12 hours. The reaction solution was filtered, and the filtrate was diluted with water (200 mL) and extracted with ethyl acetate (200 mL*3). The combined organic phases were dried over anhydrous sodium sulphate and filtered. The filtrate was concentrated under reduced pressure. The residue was separated and purified by flash chromatography (silica gel, PE : EA = 1 : 1) to obtain the product (1.48 g). LCMS (ESI) [M+H]⁺ = 457.2.

### Step 2: Synthesis of 1-methyl-6-(5-(piperazin-1-yl)pyridin-3-yl)-3,4-dihydroquinolin-2(1H)-one (intermediate 1):

The product obtained in step 1 (1.00 g, 1.0 eq) was dissolved in isopropanol (40 mL), 10% palladium on carbon (0.1 g) was added. The mixture was stirred at 25°C for 48 hours under a hydrogen atmosphere. The reaction solution was filtered through a pad of Celite, and the filtrate was concentrated to afford the product (0.74 g, crude). LCMS (ESI) [M+H]⁺ = 323.2. The crude was directly used in the next step without further purification.

### Step 3: Synthesis of ethyl 2-(4-(5-(1-methyl-2-oxo-1,2,3,4-tetrahydroquinolin-6-yl)pyridin-3-yl)piperazin-1-yl)acetate:

The sample of intermediate 1 (0.74 g, crude) was dissolved in N,N-dimethylformamide (10 mL). Ethyl bromoacetate (0.42 g) and triethylamine (0.58 g) were added, and the mixture was stirred at 25°C for 12 hours. The reaction solution was diluted with water (200 mL) and extracted with ethyl acetate (200 mL*3). The combined organic phases were dried over anhydrous sodium sulphate and filtered. The filtrate was concentrated under reduced pressure. The residue was separated and purified by flash chromatography (silica gel, DCM : MeOH = 10 : 1) to obtain the product (0.50 g). LCMS (ESI) [M+H]⁺ = 409.2.

### Step 4:

The product obtained in step 3 (0.50 g, 1.0 eq) was added to a mixed solution of ethanol (2 mL) and water (2 mL). Sodium hydroxide (0.42 g) was added, and the mixture was stirred at 25°C for 2 hours. The reaction mixture was acidified to weakly acidic with dilute hydrochloric acid (10 mL), and then concentrated to dryness under reduced pressure to give the product (1.06 g). LCMS (ESI) [M+H]⁺ = 381.2; ¹H NMR (400 MHz, DMSO-*d*₆) δ 8.42 (d, J = 1.6 Hz, 1H), 8.35 (d, J = 2.4 Hz, 1H), 7.79 (s, 1H), 7.73 - 7.66 (m, 2H), 7.22 - 7.15 (m, 1H), 4.10 (s, 2H), 3.70 (s, 6H), 3.29 (d, J = 5.2 Hz, 6H), 2.96 - 2.92 (m, 2H), 2.60 - 2.56 (m, 2H).

### Preparation example 4

### Synthesis of 2-(4-(5-bromopyridin-3-yl)piperazin-1-yl)-N-isopropylacetamide

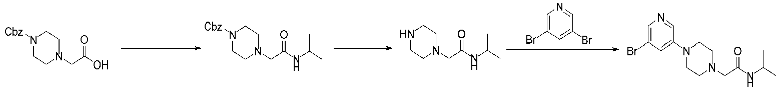

### Step 1: Synthesis of benzyl 4-(2-(isopropylamino)-2-oxoethyl)piperazine-1-carboxylate:

2-(4-((Benzyloxy)carbonyl)piperazin-1-yl)acetic acid (1.2 g, 1 eq), isopropylamine hydrochloride (535.71 mg), 2-(7-azabenzotriazol-1-yl)-N,N,N',N'-tetramethyluronium hexafluorophosphate (HATU, 2.46 g) and N,N-diisopropylethylamine (1.67 g) were added to N,N-dimethylformamide (20 mL), and the reaction was stirred at room temperature for 3 hours. The reaction solution was poured into water (200 mL), and the mixture was extracted with ethyl acetate (50 mL*3). The combined organic phases were dried over anhydrous sodium sulphate and filtered. The filtrate was concentrated under reduced pressure. The residue was purified by flash chromatography (silica gel, petroleum ether : ethyl acetate = 1 : 4) to obtain the product (900 mg, crude). LCMS (ESI) [M+H]⁺ = 320.0.

### Step 2: Synthesis of N-isopropyl-2-(piperazin-1-yl)acetamide:

The product of step 1 (900 mg, 1 eq) was dissolved in methanol (20 mL), and 5% palladium on carbon (300 mg, wet) was added. The reaction was stirred at room temperature overnight under a hydrogen atmosphere. The mixture was filtered through a pad of Celite, and the filter cake was washed with methanol. The filtrate was directly concentrated to afford the product (600 mg, crude).

### Step 3:

3,5-Dibromopyridine (500 mg, 1 eq), the product of step 2 (312.84 mg), sodium tert-butoxide (243.4 mg), 4,5-bis(diphenylphosphino)-9,9-dimethylxanthene (XantPhos, 61.06 mg) and tris(dibenzylideneacetone)dipalladium (38.66 mg) were added to 1,4-dioxane (15 mL). Under a nitrogen atmosphere, the reaction was stirred at 100°C for 5 hours. The reaction solution was concentrated, and the residue was separated and purified by flash chromatography (silica gel, dichloromethane : methanol = 10 : 1) to obtain the target compound (400 mg). LCMS (ESI) [M+H]⁺ = 341.18. ¹HNMR (400 MHz, DMSO-*d₆*) δ 8.29 (d, *J =* 2.6 Hz, 1H), 8.03 (d, *J =* 1.8 Hz, 1H), 7.57-7.48 (m, 2H), 3.95-3.84 (m, 1H), 3.30-3.26 (m, 4H), 2.94 (s, 2H), 2.58-2.54 (m, 4H), 1.07 (d, *J =* 6.6 Hz, 6H).

### Example 1

### Preparation of N-trans-(4-acetamidocyclohexyl)-2-(4-(5-(1-methyl-2-oxo-1,2,3,4-tetrahydroquinolin-6-yl)pyridin-3-yl)piperazin-1-yl)acetamide (compound 1):

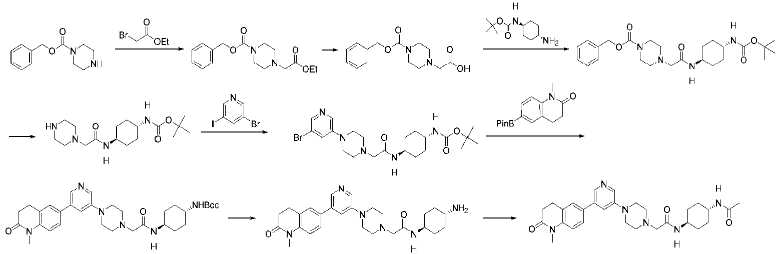

### Step 1: Synthesis of benzyl 4-(2-ethoxy-2-oxoethyl)piperazine-1-carboxylate:

With reference to step 3 of preparation example 3, using benzyl piperazine-1-carboxylate (10.00 g, 1.0 eq) and ethyl bromoacetate (8.30 g) as starting materials and using tetrahydrofuran (100 mL) as the solvent, the reaction was stirred at 25°C for 3 hours. The residue was separated and purified by flash chromatography (silica gel, PE : EA = 5 : 1, v/v) to obtain the product (1.30 g). LCMS (ESI) [M+H]⁺ = 307.0.

### Step 2: Synthesis of 2-(4-((benzyloxy)carbonyl)piperazin-1-yl)acetic acid:

With reference to step 4 of preparation example 3, using benzyl 4-(2-ethoxy-2-oxoethyl)piperazine-1-carboxylate (15.00 g, 1.0 eq) as the starting material, the reaction was stirred at 25°C for 2 hours. The intermediate crude (12.80 g) was obtained. LCMS (ESI) [M+H]⁺ = 279.2. The crude was directly used in the next step without further purification.

### Step 3: Synthesis of benzyl 4-(2-(((trans-4-((tert-butoxycarbonyl)amino)cyclohexyl)amino)-2-oxoethyl)piperazine-1-carboxylate

The product obtained in step 2 (1.00 g, crude) was dissolved in N,N-dimethylformamide (10 mL). Tert-butyl (1R,4R)-4-aminocyclohexyl)carbamate (0.80 g), N,N-diisopropylethylamine (1.40 g) and 2-(7-azabenzotriazol-1-yl)-N,N,N',N'-tetramethyluronium hexafluorophosphate (1.60 g) were added, and the mixture was stirred at 25°C for 3 hours. To the reaction system was added water (100 mL), and the mixture was extracted with ethyl acetate (300 mL*3). The organic phases were combined, dried over anhydrous sodium sulphate and filtered, and the filtrate was concentrated. The residue was purified by flash chromatography (silica gel, PE : EA = 10 : 1) to obtain the intermediate compound (1.33 g). LCMS (ESI) [M+H]⁺ = 475.2.

### Step 4: Synthesis of tert-butyl (trans-4-(2-(piperazin-1-yl)acetamido)cyclohexyl)carbamate:

With reference to step 2 of the preparation example, using the product obtained in step 3 (5.00 g, 1.0 eq) as the starting material, the reaction was stirred at 25°C under a hydrogen atmosphere for 12 hours to obtain the intermediate compound (3.51 g, crude). LCMS (ESI) [M+H]⁺ = 341.2. The crude was directly used in the next step without further purification.

### Step 5: Synthesis of tert-butyl (trans-4-(2-(4-(5-bromopyridin-3-yl)piperazin-1-yl)acetamido)cyclohexyl)carbamate:

The product obtained in step 4 (1.00 g, 1.0 eq, crude) was added to N,N-dimethylformamide (10 mL). 5-Bromo-3-iodopyridine (0.80 g), tris(dibenzylideneacetone)palladium (0.10 g), 2-dicyclohexylphosphino-2',4',6'-triisopropylbiphenyl (0.30 g) and caesium carbonate (2.90 g) were added, and the mixture was stirred at 100°C for 12 hours. To the reaction system was added water (100 mL), and the mixture was extracted with ethyl acetate (300 mL*3). The organic phases were combined, dried over anhydrous sodium sulphate and filtered, and the filtrate was concentrated. The residue was separated and purified by flash chromatography (silica gel, DCM : MeOH = 1 : 1) to obtain the intermediate compound (0.39 g). LCMS (ESI) [M+H]⁺ = 496.2.

### Step 6: Synthesis of tert-butyl (trans-4-(2-(4-(5-(1-methyl-2-oxo-1,2,3,4-tetrahydroquinolin-6-yl)pyridin-3-yl)piperazin-1-yl)acetamido)cyclohexyl)carbamate:

The product obtained in step 5 (387 mg, 1.0 eq) was added to a mixed solution of water (0.5 mL) and dioxane (2.5 mL). The sample of preparation example 1 (269 mg), 1,1-bis(diphenylphosphino)ferrocene dichloropalladium (57 mg) and caesium carbonate (762 mg) were added, and the mixture was stirred at 100°C for 12 hours. To the reaction system was added water (100 mL), and the mixture was extracted with ethyl acetate (100 mL*3). The organic phases were combined, dried over anhydrous sodium sulphate and filtered, and the filtrate was concentrated. The residue was separated and purified by flash chromatography (silica gel, DCM : MeOH = 8 : 1) to obtain the intermediate compound (273 mg). LCMS (ESI) [M+H]⁺ = 577.4.

### Step 7: Synthesis of N-(trans-4-aminocyclohexyl)-2-(4-(5-(1-methyl-2-oxo-1,2,3,4-tetrahydroquinolin-6-yl)pyridin-3-yl)piperazin-1-yl)acetamide:

The product obtained in step 6 (238 mg, 1.0 eq) was added to hydrogen chloride-dioxane (4 M, 5 mL), and the mixture was stirred at 25°C for 2 hours. The reaction solution was concentrated to obtain the product crude (120 mg). LCMS (ESI) [M+H]⁺ = 477.4. The crude was directly used in the next step without further purification.

### Step 8:

The product obtained in step 7 (238 mg, 1.0 eq, crude) was added to dichloromethane (3 mL). Triethylamine (202 mg) and acetic anhydride (153 mg) were added, and the mixture was stirred at 25°C for 12 hours. The reaction solution was directly concentrated and filtered, and the filtrate was separated and purified by prep-HPLC (C18, 0.1% FA in water, MeCN) to obtain compound 1 (43 mg). LCMS (ESI) [M+H]⁺ = 519.3; ¹H NMR (400 MHz, DMSO-d₆) δ 8.28 (dd, J = 11.2, 2.4 Hz, 2H), 7.71 (d, J = 7.6 Hz, 1H), 7.63 (dd, J = 4.4, 2.4 Hz, 2H), 7.59 - 7.47 (m, 2H), 7.19 - 7.15 (m, 1H), 3.61 - 3.43 (m, 2H), 3.33 (s, 2H), 3.29 (d, J = 2.4 Hz, 5H), 2.99 - 2.92 (m, 4H), 2.63 - 2.56 (m, 6H), 1.76 (s, 7H), 1.35 - 1.14 (m, 4H).

### Example 2

### Preparation of 2-(4-(5-(1-methyl-2-oxo-1,2,3,4-tetrahydroquinolin-6-yl)pyridin-3-yl)piperazin-1-yl)-N-(pyridin-3-yl)acetamide (compound 3):

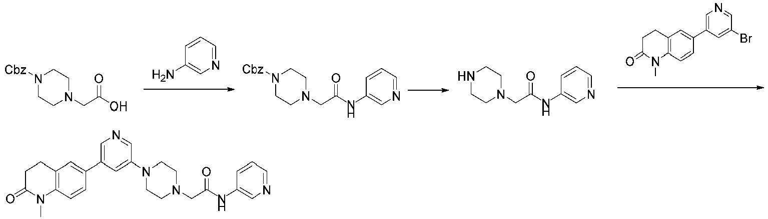

### Step 1: Synthesis of benzyl 4-(2-oxo-2-(pyridin-3-ylamino)ethyl)piperazine-1-carboxylate:

With reference to step 3 of example 1, using 2-(4-((benzyloxy)carbonyl)piperazin-1-yl)acetic acid (1 g, 1.0 eq) as the starting material, the mixture was stirred at room temperature for 15 minutes, and pyridin-3-amine (405.79 mg) was then added. The mixture was further stirred for 2 hours. The residue was separated and purified by flash chromatography (silica gel, PE : EA = 10 : 7) to obtain the product (700 mg). LCMS (ESI) [M+H]⁺ = 354.9.

### Step 2: Synthesis of 2-(piperazin-1-yl)-N-(pyridin-3-yl)acetamide:

The product obtained in step 1 (400 mg, 1.0 eq) was dissolved in methanol (15 mL), and 5% palladium on carbon (100 mg, wet) was added. Under a hydrogen atmosphere, the mixture was stirred at room temperature for 12 hours. The reaction solution was filtered through a pad of Celite, and the filtrate was concentrated to obtain the product (200 mg, crude). LCMS (ESI) [M+H]⁺ = 220.9.

### Step 3:

With reference to step 1 of preparation example 3, using 6-(5-bromopyridin-3-yl)-1-methyl-3,4-dihydroquinolin-2(1H)-one (460.79 mg, 1 eq) and the product obtained in step 2 (320 mg, crude) as starting materials, the reaction was stirred at 110°C under a nitrogen atmosphere for 12 hours. The reaction solvent was concentrated and the residue was separated and purified by flash chromatography (silica gel, DCM : MeOH = 10 : 1). The resulting crude was dissolved in acetonitrile, and separated and purified by prep-HPLC to obtain compound 3 (76.02 mg). LCMS (ESI) [M+H]⁺ = 457.0; ¹H NMR (400 MHz, DMSO-*d₆*) δ 10.00 (s, 1H), 8.80 (d, J = 2.1 Hz, 1H), 8.33 - 8.25 (m, 3H), 8.09 (ddd, J = 8.4, 2.6, 1.5 Hz, 1H), 7.63 (dq, J = 4.5, 2.3 Hz, 2H), 7.52 (t, J = 2.4 Hz, 1H), 7.35 (ddd, J = 8.3, 4.8, 0.8 Hz, 1H), 7.18 (d, J = 9.1 Hz, 1H), 3.38 (t, J = 5.1 Hz, 4H), 3.29 (s, 3H), 3.26 (s, 2H), 2.95 (t, J = 7.4 Hz, 2H), 2.72 (t, J = 5.0 Hz, 4H), 2.63 - 2.55 (m, 2H).

### Example 3

### Preparation of N-(1-((4-methyl-1H-imidazol-5-yl)methyl)piperidin-4-yl)-2-(4-(5-(1-methyl-2-oxo-1,2,3,4-tetrahydroquinolin-6-yl)pyridin-3-yl)piperazin-1-yl)acetamide (compound 4):

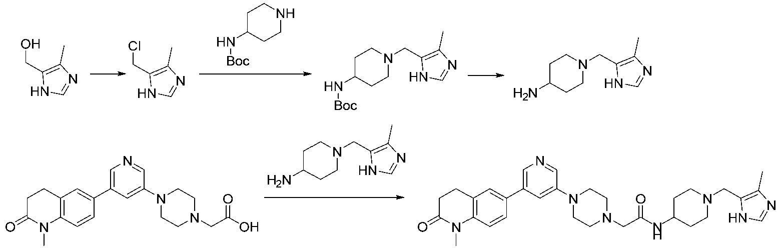

### Step 1: Synthesis of 5-(chloromethyl)-4-methyl-1H-imidazole:

(4-Methyl-1H-imidazol-5-yl)methanol (500 mg, 1.0 eq) was added to dichloromethane (5 mL). Thionyl chloride (5.31 g) was added, and the mixture was stirred at 25°C for 16 hours. After the reaction was completed, the reaction solution was concentrated to obtain the product (500 mg, crude). The crude was directly used in the next step without further purification.

### Step 2: Synthesis of tert-butyl (1-((4-methyl-1H-imidazol-5-yl)methyl)piperidin-4-yl)carbamate:

The product obtained in step 1 (500 mg, crude) was added to dichloromethane (5 mL). Triethylamine (1.17 g) and tert-butyl piperidin-4-yl carbamate (700 mg, 1.0 eq) were added at 25°C. The reaction mixture was stirred at 25°C for 16 hours. The reaction was concentrated, and the residue was purified by flash chromatography (silica gel, DCM : MeOH = 15 : 1) to obtain the product (300 mg). LCMS (ESI) [M+H]⁺ = 295.2.

### Step 3: Synthesis of 1-((4-methyl-1H-imidazol-5-yl)methyl)piperidin-4-amine:

The product obtained in step 2 (150 mg, 1.0 eq) was added to dichloromethane (3 mL). A solution of hydrogen chloride in dioxane (4 N in MeOH, 0.6 mL) was added, and the mixture was stirred at 25°C for 16 hours. The reaction mixture was directly concentrated to dryness by rotary evaporation to afford the product (120 mg, crude). The crude was directly used in the next step without further purification. LCMS (ESI) [M+H]⁺ = 195.2.

### Step 4:

With reference to step 3 of example 1, using the product obtained in step 3 (100 mg, crude) and the sample of preparation example 3 (195 mg, 1.0 eq) as starting materials, the reaction was stirred at 25°C for 16 hours. The reaction mixture was directly concentrated to dryness by rotary evaporation, and dissolved in methanol. The residue was purified by reversed-phase chromatography (15%-25% ACN in water) to obtain compound 4 (23.13 mg). LCMS (ESI) [M+H]⁺ = 557.4; ¹H NMR (400 MHz, CD₃OD) δ 8.70 (s, 1H), 8.50 (d, *J =* 1.4 Hz, 1H), 8.39 (d, *J* = 2.8 Hz, 1H), 8.17 (s, 1H), 7.75 - 7.66 (m, 2H), 7.30 (d, *J =* 8.4 Hz, 1H), 4.41 (s, 2H), 4.04 (s, 1H), 3.93 (s, 2H), 3.80 (s, 4H), 3.57 (d, *J =* 12.4 Hz, 2H), 3.48 (s, 4H), 3.41 (s, 3H), 3.19 (t, *J =* 10.4 Hz, 2H), 3.08 - 3.01 (m, 2H), 2.72 - 2.66 (m, 2H), 2.42 (s, 3H), 2.19 (d, *J =* 13.8 Hz, 2H), 1.92 - 1.83 (m, 2H).

### Example 4

### Preparation of 6-(5-(4-(3-hydroxypropyl)piperidin-1-yl)pyridin-3-yl)-1-methyl-3,4-dihydroquinolin-2(1H)-one (compound 5):

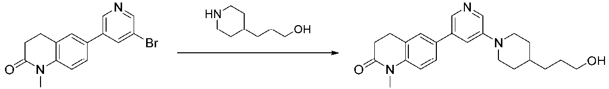

The sample of preparation example 2 (100 mg, 1.0 eq) was added to 1,4-dioxane/water (v/v=3 : 1) (4.0 mL), followed by the addition of 3-(piperidin-4-yl)propan-1-ol (46 mg), tris(dibenzylideneacetone)dipalladium (30 mg), 2-dicyclohexylphosphino-2',6'-diisopropoxy-1,1'-biphenyl (20 mg) and caesium carbonate (315 mg). The mixture was stirred at 90°C for 2 hours. The reaction mixture was filtered, and extracted with ethyl acetate (30 mL*3). The organic phases were combined, dried over anhydrous sodium sulphate and filtered, and the filtrate was concentrated. The residue was purified by prep-HPLC (C18, 0.05% aqueous ammonia in acetonitrile) to obtain compound 5 (8.56 mg). LCMS (ESI) [M+H]⁺ = 378.1; ¹H NMR (400 MHz, CDCl₃) δ 8.28 (d, *J =* 2.8 Hz, 1H), 8.25 (d, *J =* 1.8 Hz, 1H), 7.46 - 7.42 (m, 1H), 7.37 (d, *J =* 1.8 Hz, 1H), 7.32 - 7.29 (m, 1H), 7.06 (d, *J =* 8.4 Hz, 1H), 3.77 (d, *J =* 12.4 Hz, 2H), 3.67 (t, *J =* 6.6 Hz, 2H), 3.40 (s, 3H), 3.01 - 2.96 (m, 2H), 2.80 (s, 2H), 2.70 - 2.67(m, 2H), 1.85 (d, *J =* 12.0 Hz, 2H), 1.66 - 1.60 (m, 3H), 1.43 - 1.35 (m, 4H).

### Example 5

### Preparation of N-isopropyl-2-(4-(5-(1-methyl-2-oxo-1,2,3,4-tetrahydroquinolin-6-yl)pyridin-3-yl)piperazin-1-yl)acetamide (compound 6):

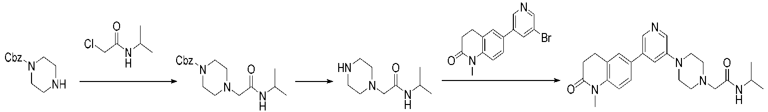

### Step 1: Synthesis of benzyl 4-(2-(isopropylamino)-2-oxoethyl)piperazine-1-carboxylate:

Benzyl piperazine-1-carboxylate (500 mg, 1.0 eq), 2-chloro-N-isopropylacetamide (615.56 mg) and sodium carbonate (481.19 mg) were added to acetonitrile (20 mL). The mixture was stirred at 50°C under a nitrogen atmosphere for 12 hours. The reaction mixture was directly concentrated to dryness by rotary evaporation, and the residue was purified by flash chromatography (silica gel, PE : EA = 1 : 1) to obtain the product (1 g, crude). LCMS (ESI) [M+H]⁺ = 320.0.

### Step 2: Synthesis of N-isopropyl-2-(piperazin-1-yl)acetamide:

The product obtained in step 1 (500 mg, 1 eq) was dissolved in methanol (20 mL), and 5% palladium on carbon (100 mg, wet) was added. The mixture was stirred at room temperature under a hydrogen atmosphere for 12 hours. The reaction mixture was filtered through a pad of Celite, and the filtrate was concentrated to obtain the product (280 mg). LCMS (ESI) [M+H]⁺ = 186.1.

### Step 3:

With reference to step 3 of example 2, using the product obtained in preparation example 2 (428 mg, 1.0 eq) and the product obtained in step 2 (250 mg) as starting materials, the reaction was stirred at 110°C under a nitrogen atmosphere for 12 hours. With reference to the workup and purification method of step 3 in example 2, compound 6 (169.63 mg) was afford. LCMS (ESI) [M+H]⁺ = 422.0; ¹H NMR (400 MHz, DMSO-*d₆*) δ 8.29 (d, J = 1.8 Hz, 1H), 8.27 (d, J = 2.7 Hz, 1H), 7.63 (dq, J = 5.0, 2.3 Hz, 2H),7.55 - 7.49 (m, 2H), 7.17 (d, J = 9.0 Hz, 1H),3.96 - 3.85 (m, 1H), 3.34 (s, 4H), 3.29 (s, 3H), 2.98 - 2.90 (m, 4H), 2.59 (dt, J = 9.2, 6.9 Hz, 6H), 1.09 (s, 3H), 1.07 (s, 3H).

### Example 6

### Preparation of N-methyl-2-(4-(2-(4-(5-(1-methyl-2-oxo-1,2,3,4-tetrahydroquinolin-6-yl)pyridin-3-yl)piperazin-1-yl)acetamido)-1H-pyrazol-1-yl)acetamide (compound 9):

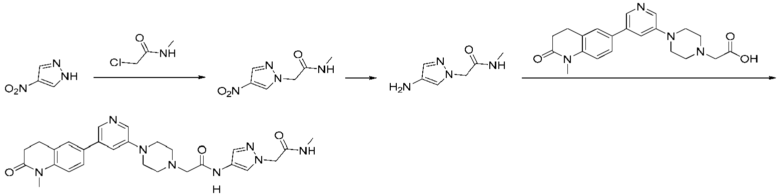

### Step 1: Synthesis of N-methyl-2-(4-nitro-1H-pyrazol-1-yl)acetamide:

4-Nitro-1H-pyrazole (500 mg, 1.0 eq) was dissolved in acetonitrile (10 mL), followed by the addition of 2-chloro-N-methylacetamide (375 mg) and potassium carbonate (1.8 g) to the system. Under a nitrogen atmosphere, the mixture was stirred at 80°C for 2 hours. The reaction solution was cooled to room temperature, and extracted with ethyl acetate (30 mL*3). The organic phases were combined, and directly concentrated to obtain the product (355 mg, crude). The crude was directly used in the next step without further purification. LCMS (ESI) [M+H]⁺ = 185.2.

### Step 2: Synthesis of 2-(4-amino-1H-pyrazol-1-yl)-N-methylacetamide:

The product obtained in step 1 (200 mg, crude) was dissolved in ethanol/water (5 : 1) (4.0 mL). Iron powder (300 mg) and ammonium chloride (580 mg) were added, and the mixture was stirred overnight at 90°C. The reaction solution was cooled to room temperature and filtered, and the filtrate was concentrated to obtain the product (200 mg, crude). The crude was directly used in the next step without further purification. LCMS (ESI) [M+H]⁺ = 155.2.

### Step 3:

With reference to step 3 of example 1, using the product obtained in step 2 (100 mg, crude) and the sample of preparation example 3 (205 mg) as starting materials, the reaction was stirred at 25°C for 2 hours. The residue was purified by prep-HPLC (C18, 0.05% NH₃·H₂O in water, MeCN) to obtain compound 9 (22.7 mg). LCMS (ESI) [M+H]⁺ = 517.4; ¹H NMR (400 MHz, CDCl₃) δ 8.90 (s, 1H), 8.35 - 8.27 (m, 2H), 8.01 (s, 1H), 7.61 (s, 1H), 7.45 - 7.40(m, 1H), 7.34(d, *J* = 18.0 Hz, 2H), 7.06 (d, *J =* 8.4 Hz, 1H), 6.13 (s, 1H), 4.75 (s, 2H), 3.39 (s, 3H), 3.38 - 3.34 (m, 4H), 3.24 (s, 2H), 3.00 - 2.95 (m, 2H), 2.83 - 2.79 (m, 4H), 2.77 (d, *J* = 4.8 Hz, 3H), 2.71 - 2.66 (m, 2H).

### Examples 7-8

With reference to step 3 of example 1, Compounds 15 and 28 were prepared respectively by using the sample from Preparation Example 3 (50 mg, 1.0 eq) and the corresponding starting materials shown in the table below.

| Starting material | Compound | Structural confirmation data |
|---|---|---|
| 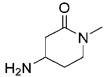 | 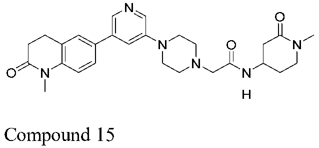 | LCMS (ESI) [M+H] ⁺ = 491.2; ¹H NMR(400 MHz, DMSO-*d₆*) δ 8.32 - 8.24 (m, 2H), 7.85 (d, *J =* 7.8 Hz, 1H), 7.66 - 7.60 (m, 2H), 7.52 - 7.48 (m, 1H), 7.20 - 7.15 (m, 1H), 4.10 - 4.00 (m, 1H), 3.35 - 3.32 (m, 4H), 3.29 (s, 3H), 3.28 - 3.24 (m, 2H), 3.01 (s, 2H), 2.97 - 2.91 (m, 2H), 2.80 (s, 3H), 2.64 - 2.56 (m, 6H), 2.46 - 2.39 (m, 1H), 2.31 - 2.20 (m, 1H), 1.98 - 1.86 (m, 1H), 1.83 - 1.70 (m, 1H). |
| 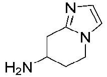 | 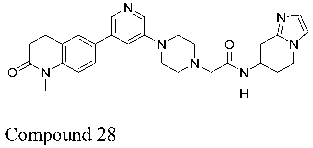 | LCMS (ESI) [M+H] ⁺ = 499.9; ¹H NMR(400 MHz, DMSO-*d₆*) δ 8.33 - 8.23 (m, 2H), 7.94 (d, *J =* 7.6 Hz, 1H), 7.66 - 7.61 (m, 2H), 7.50 (s, 1H), 7.21 - 7.15 (m, 1H), 7.03 - 6.98 (m, 1H), 6.85 - 6.79 (m, 1H), 4.26 - 4.15 (m, 1H), 4.09 - 4.02 (m, 1H), 4.00 - 3.90 (m, 1H), 3.32 - 3.30 (m, 4H), 3.29 (s, 3H), 3.04 (s, 2H), 3.01 - 2.97 (m, 1H), 2.96 - 2.92 (m, 2H), 2.77 - 2.69 (m, 1H), 2.65 - 2.61 (m, 4H), 2.60 - 2.56 (m, 2H), 2.10 - 1.93 (m, 2H). |

### Example 9

### Preparation of N-isopropyl-2-(4-(6-(1-methyl-2-oxo-1,2,3,4-tetrahydroquinolin-6-yl)pyrazin-2-yl)piperazin-1-yl)acetamide (compound 44):

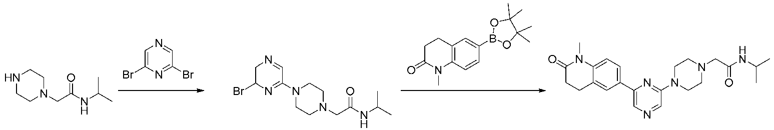

### Step 1: Synthesis of 2-(4-(6-bromopyrazin-2-yl)piperazin-1-yl)-N-isopropylacetamide:

2,6-Dibromopyrazine (320 mg, 1.0 eq), N-isopropyl-2-(piperazin-1-yl)acetamide (260 mg, crude) and caesium carbonate (876.6 mg) were added to dimethyl sulphoxide (6 mL). Under a nitrogen atmosphere, the mixture was stirred overnight at 100°C. The reaction was quenched by the addition of water (20 mL), and extracted with ethyl acetate (10 mL*3). The organic phases were combined, dried over anhydrous sodium sulphate and filtered, and the filtrate was concentrated. The residue was purified by flash chromatography (silica gel, PE : EA = 1 : 1) to afford the product (360 mg). LCMS (ESI) [M+H]⁺ = 341.9.

### Step 2:

With reference to step 6 of example 1, using the sample of preparation example 1 (250 mg, 1.0 eq) and the product obtained in step 1 (297.94 mg) as starting materials, the reaction was stirred overnight at 100°C under a nitrogen atmosphere. The reaction mixture was subjected to layer separation, and the organic phase was concentrated. The residue was purified by flash chromatography (silica gel, DCM : MeOH = 10 : 1) to obtain the crude, which was further separated and purified by reversed-phase chromatography (C18, MeCN : H₂O = 1 : 1) to obtain compound 44 (247.34 mg). LCMS (ESI) [M+H]⁺ = 186.1; ¹H NMR (400 MHz, Chloroform-*d*) δ 8.34 (s, 1H), 8.07 (s, 1H), 7.90 (dd, *J =* 8.5, 2.1 Hz, 1H), 7.81 (d, *J =* 2.0 Hz, 1H),7.07 (d, *J =* 8.5 Hz, 1H), 6.92 (d, *J =* 8.4 Hz, 1H),4.20 - 4.07 (m, 1H), 3.73 (t, *J =* 5.1 Hz, 4H), 3.40 (s, 3H),3.06 (s, 2H), 3.03 - 2.96 (m, 2H), 2.70 (q, *J =* 6.4, 4.9 Hz, 6H), 1.20 (d, *J =* 6.6 Hz, 6H).

### Example 10

### Preparation of 2-(4-(5-(1-methyl-2-oxo-1,2,3,4-tetrahydroquinolin-6-yl)pyridin-3-yl)piperazin-1-yl)-N-(trans-4-(4-methyl-3-oxopiperazin-1-yl)cyclohexyl)acetamide (compound 69):

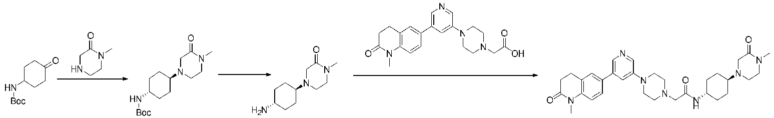

### Step 1: Synthesis of tert-butyl (trans-4-(4-methyl-3-oxopiperazin-1-yl)cyclohexyl)carbamate:

Tert-butyl (4-oxocyclohexyl)carbamate (1.00 g, 1.0 eq) was added to dichloromethane (10 mL). At 0°C, 1-methylpiperazin-2-one (535 mg) and sodium triacetoxyborohydride (3.00 g) were added. The mixture was stirred at 25°C for 16 hours. The reaction solution was quenched by adding ice water (10 mL), and extracted with ethyl acetate (50 mL*3). The organic phases were combined, dried over anhydrous sodium sulphate and filtered, and the filtrate was concentrated. The residue was purified by reversed-phase chromatography (15% ACN in water) to obtain the product (200 mg). LCMS (ESI) [M+H]⁺ = 312.2.

### Step 2: Synthesis of 4-(trans-4-aminocyclohexyl)-1-methylpiperazin-2-one:

The product obtained in step 1 (200 mg, 1.0 eq) was added to dichloromethane (2 mL). At 25°C, a solution of hydrogen chloride in dioxane (4 N in MeOH, 0.5 mL) was added. The reaction mixture was stirred at 25°C for 16 hours. The reaction solution was directly concentrated to afford the product (100.0 mg, crude). The crude was directly used in the next step without further purification. LCMS (ESI) [M+H]⁺ = 212.2.

### Step 3:

With reference to step 3 of example 1, using the product obtained in step 2 (100 mg, crude) and the sample of preparation example 3 (178 mg) as starting materials, the reaction was stirred at 25°C for 16 hours. The reaction solvent was concentrated to dryness, and dissolved in methanol. The residue was purified by reversed-phase chromatography (15%-25% ACN in water) to obtain compound 69 (64.10 mg). LCMS (ESI) [M+H]⁺ = 574.4; ¹H NMR (400 MHz, CD₃OD) δ 8.49 (d, *J* = 1.2 Hz, 1H), 8.38 (d, *J* = 2.8 Hz, 1H), 8.14 (s, 1H), 7.70 (dd, *J =* 8.4, 2.4 Hz, 1H), 7.66 (s, 1H), 7.28 (d, *J =* 8.4 Hz, 1H), 3.91 (d, *J =* 8.4 Hz, 4H), 3.76 (d, *J =* 19.2 Hz, 5H), 3.69 - 3.55 (m, 4H), 3.47 (d, *J* = 3.2 Hz, 4H), 3.39 (s, 3H), 3.33 (d, *J =* 8.8 Hz, 1H), 3.06 - 2.99 (m, 5H), 2.72 - 2.63 (m, 2H), 2.20 - 2.13 (m, 4H), 1.73 - 1.64 (m, 2H), 1.47 - 1.38 (m, 2H).

### Example 11

### Preparation of N-isopropyl-2-methyl-2-(4-(5-(1-methyl-2-oxo-1,2,3,4-tetrahydroquinolin-6-yl)pyridin-3-yl)piperazin-1-yl)propanamide (compound 70):

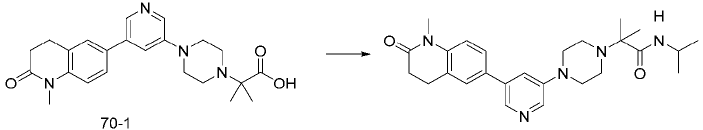

### Step 1: With reference to the preparation method of preparation example 3, 2-methyl-2-(4-(5-(1-methyl-2-oxo-1,2,3,4-tetrahydroquinolin-6-yl)pyridin-3-yl)piperazin-1-yl)propanoic acid (intermediate 70-1) was prepared. LCMS (ESI) [M+H]⁺ = 409.4.

With reference to step 3 of example 1, using the product obtained in step 1 (70 mg, 1.0 eq) and propan-2-amine (15 mg) as starting materials, the reaction was stirred at 25°C for 2 hours. The residue was separated and purified by prep-HPLC (C18, 0.05% NH₃·H₂O in water, MeCN) to obtain compound 70 (29 mg). LCMS (ESI) [M+H]⁺ = 450.4; ¹H NMR (400 MHz, CDCl₃) δ 8.32 (s, 1H), 8.28 (d, *J =* 2.4 Hz, 1H), 7.52 - 7.39 (m, 1H), 7.37 (d, *J* = 2.0 Hz, 1H), 7.31 (t, *J* = 2.0 Hz, 1H), 7.07 - 7.04 (m, 2H), 4.10 - 4.01 (m, 1H), 3.40 (s, 3H), 3.35 - 3.30 (m, 4H), 3.01 - 2.95 (m, 2H), 2.72 - 2.68 (m, 6H), 1.24 (s, 6H), 1.14 (d, *J =* 6.4 Hz, 6H).

### Example 12

### Preparation of N-(3-acetamidocyclobutyl)-2-methyl-2-(4-(5-(1-methyl-2-oxo-1,2,3,4-tetrahydroquinolin-6-yl)pyridin-3-yl)piperazin-1-yl)propanamide (compound 71):

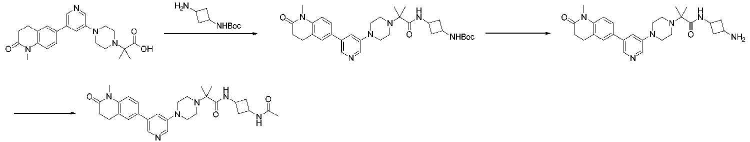

### Step 1: Synthesis of tert-butyl 3-(2-methyl-2-(4-(5-(1-methyl-2-oxo-1,2,3,4-tetrahydroquinolin-6-yl)pyridin-3-yl)piperazin-1-yl)propanamido)cyclobutyl)carbamate:

With reference to step 3 of example 1, using intermediate 70-1 (250 mg, 1.0 eq) and tert-butyl (3-aminocyclobutyl)carbamate (136 mg) as starting materials, the reaction was stirred at room temperature for 2 hours. The residue was separated and purified by flash chromatography (silica gel, PE : EA = 2 : 1) to obtain the product (170 mg). LCMS (ESI) [M+H]⁺ = 577.2.

### Step 2: Synthesis of N-(3-aminocyclobutyl)-2-methyl-2-(4-(5-(1-methyl-2-oxo-1,2,3,4-tetrahydroquinolin-6-yl)pyridin-3-yl)piperazin-1-yl)propanamide:

The product obtained in step 1 (170 mg, 1.0 eq) was dissolved in dichloromethane (2.0 mL). Trifluoroacetic acid (1.0 mL) was added, and the mixture was stirred at 25°C for 30 minutes. The reaction solution was concentrated to obtain the product (120 mg, crude). The crude was directly used in the next step without purification. LCMS (ESI) [M+H]⁺ = 477.4.

### Step 3:

The product obtained in step 2 (120 mg, crude) was dissolved in dichloromethane (2.0 mL). At 0°C, acetic anhydride (77 mg) and triethylamine (75 mg) were added. The mixture was allowed to warm gradually to room temperature and stirred for 2 hours. The reaction solution was filtered, and the filtrate was directly concentrated. The residue was purified by prep-HPLC (C18, 0.5% formic acid in acetonitrile) to obtain compound 71 (49 mg). LCMS (ESI) [M+H]⁺ = 519.4; ¹H NMR (400 MHz, CDCl₃) *δ* 8.33 (d, J = 1.8 Hz, 1H), 8.31 - 8.26 (m, 1H), 7.51 - 7.28 (m, 4H), 7.07 (d, J = 8.4 Hz, 1H), 5.72 (s, 1H), 4.55 - 3.86 (m, 2H), 3.40 (s, 3H), 3.33 (s, 4H), 3.04 - 2.94 (m, 2H), 2.86 - 2.60 (m, 7H), 2.37 - 2.24 (m, 1H), 2.01 - 1.88 (m, 4H), 1.70 (s, 1H), 1.25 (s, 6H).

### Examples 13-14

With reference to step 3 of example 1, using intermediate 70-1 (200 mg, 1.0 eq) and preparation example 3 (100 mg, 1.0 eq) with the starting materials shown in the table below, compounds 72 and 73 were prepared respectively.

| Starting material | Compound | Structural confirmation data |
|---|---|---|
| 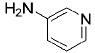 | 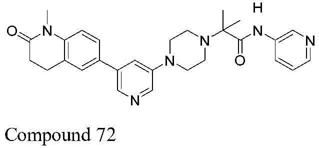 | LCMS (ESI) [M+H]⁺ = 485.2; ¹H NMR (400 MHz, CDCl₃) δ 9.36 (s, 1H), 8.56 (s, 1H), 8.35 - 8.21 (m, 4H), 7.49 - 7.45 (m, 1H), 7.38 - 7.35 (m, 2H), 7.29 - 7.26 (m, 1H), 7.08 (d, J = 8.4 Hz, 1H), 3.43 - 3.40 (m, 7H), 3.01 - 2.97 (m, 2H), 2.83 - 2.77 (m, 4H), 2.73 - 2.69 (m, 2H), 1.38 (s, 6H). |
| 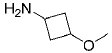 | 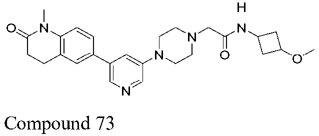 | LCMS (ESI) [M+H]⁺ = 409.2; ¹H NMR (400 MHz, CD₃OD-*d*₄) δ 8.41 (d, *J =* 1.6 Hz, 1H), 8.31 (d, *J =* 2.8 Hz, 1H), 7.93 (s, 1H), 7.71 - 7.61 (m, 2H), 7.29 (d, *J* = 8.4 Hz, 1H), 4.45 - 4.40 (m, 0.3H), 4.10 - 3.95 (m, 1H), 3.82 - 3.64 (m, 6.7H), 3.42 (s, 3H), 3.39 - 3.34 (m, 4H), 3.27-3.25 (m, 3H), 3.09 - 2.96 (m, 2H), 2.78 - 2.64 (m, 3.5H), 2.45 - 2.22 (m, 1H), 1.95 - 1.83 (m, 1.5H). |

### Example 15

### Preparation of 6-(5-(4-(3-(1H-1,2,3-triazol-1-yl)propyl)piperazin-1-yl)pyridin-3-yl)-1-methyl-3,4-dihydroquinolin-2(1H)-one (compound 74):

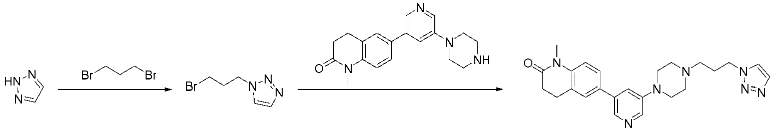

### Step 1: Synthesis of 1-(3-bromopropyl)-1H-1,2,3-triazole:

With reference to step 1 of example 6, using 2H-1,2,3-triazole (1.00 g, 1.0 eq) and 1,3-dibromopropane (2.92 g) as starting materials, the reaction was stirred at 80°C for 16 hours. The residue was purified by flash chromatography (silica gel, PE : EA = 3 : 1) to obtain the product (200 mg). LCMS (ESI) [M+H]⁺ = 190.0, 192.0.

### Step 2:

With reference to step 1 of example 6, using the product obtained in step 1 (100 mg, 1.0 eq) and intermediate 1 (171 mg), the reaction was stirred at 80°C for 16 hours. The residue was purified by prep-HPLC (C18, 0.5% FA in water, MeCN) to obtain compound 74 (26.38 mg). LCMS (ESI) [M+H]⁺ = 432.2; ¹H NMR (400 MHz, DMSO-*d*₆) δ 8.29 (d, J = 2.0 Hz, 1H), 8.26 (d, J = 2.8 Hz, 1H), 8.14 (d, J = 0.8 Hz, 1H), 7.72 (d, J = 0.8 Hz, 1H), 7.64 - 7.60 (m, 2H), 7.51 - 7.48 (m, 1H), 7.17 (d, J = 9.2 Hz, 1H), 4.44 (t, J = 7.2 Hz, 2H), 3.28 (d, J = 5.6 Hz, 7H), 2.97 - 2.91 (m, 2H), 2.58 (dd, J = 8.4, 6.4 Hz, 2H), 2.55 - 2.51 (m, 4H), 2.32 (t, J = 6.8 Hz, 2H), 2.09 - 1.98 (m, 2H).

### Examples 16-18

With reference to the preparation method of preparation example 3, intermediate 75-1 was prepared.

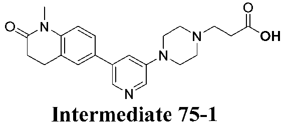

With reference to step 3 of example 1, using intermediate 75-1 (290 mg, 1.0 eq), intermediate 70-1 (150 mg, 1.0 eq) and the sample of preparation example 3 (50 mg, 1.0 eq) with the starting materials shown in the table below, compounds 75, 76 and 78 were prepared respectively.

| Starting material | Compound | Structural confirmation data |
|---|---|---|
| Methylamine hydrochloride | 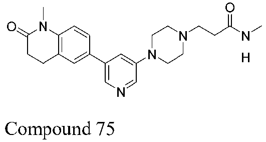 | LCMS (ESI) [M+H]⁺ = 408.2; ¹H NMR (400 MHz, CD₃OD-*d*₄) δ 8.22 (d, J = 1.8 Hz, 1H), 8.19 (d, J = 2.8 Hz, 1H), 7.56 (d, J = 2.8 Hz, 2H), 7.54 - 7.52 (m, 1H), 7.23 (d, J = 8.4 Hz, 1H), 3.39 (s, 3H), 3.37 - 3.33 (m, 4H), 3.04 - 2.97 (m, 2H), 2.78 - 2.72 (m, 5H), 2.72 - 2.63 (m, 6H), 2.44 (t, J = 7.2 Hz, 2H). |
| 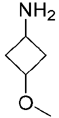 | 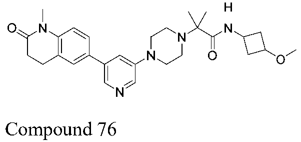 | LCMS (ESI) [M+H]⁺ = 492.4; ¹H NMR (400 MHz, CD₃OD) δ 8.28 (d, J = 16.4 Hz, 2H), 7.72 (s, 1H), 7.65 - 7.58 (m, 2H), 7.26 (d, J = 8.4 Hz, 1H), 4.01 - 3.90 (m, 1H), 3.70 (t, J = 6.8 Hz, 1H), 3.50 (s, 4H), 3.41 (s, 3H), 3.26 (d, J = 2.0 Hz, 3H), 3.06 - 3.00 (m, 2H), 2.88 (s, 4H), 2.75 - 2.64 (m, 4H), 1.97 - 1.82 (m, 2H), 1.33 (s, 6H). |
| 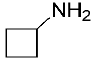 | 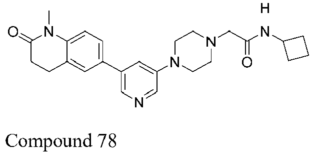 | LCMS (ESI) [M+H]⁺ =434.0; ¹H NMR (400 MHz, DMSO-d₆) δ 8.28 (dd, *J =* 10.7, 2.3 Hz, 2H), 7.94 (d, *J =* 8.2 Hz, 1H), 7.63 (dt, *J* = 4.7, 2.5 Hz, 2H), 7.50 (t, *J =* 2.3 Hz, 1H), 7.17 (d, *J =* 9.1 Hz, 1H), 4.26 (p, *J =* 8.3 Hz, 1H), 3.34 (s, 4H), 3.31 (s, 5H), 2.94 (d, *J=* 10.6 Hz, 3H), 2.59 (q, *J =* 5.9 Hz, 4H), 2.52 (s, 1H), 2.19 - 2.08 (m, 2H), 2.03 - 1.90 (m, 2H), 1.66 - 1.54 (m, 2H). |

### Example 19

### Preparation of 6-(5-(4-(3-(2H-1,2,3-triazol-2-yl)propyl)piperazin-1-yl)pyridin-3-yl)-1-methyl-3,4-dihydroquinolin-2(1H)-one (compound 77):

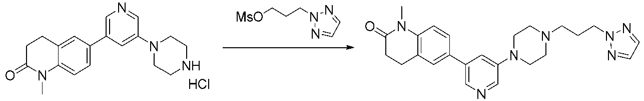

The monohydrochloride of intermediate 1 (50 mg, 1.0 eq) was added to N,N-dimethylformamide (2 mL). Triethylamine (42 mg) and 3-(2H-1,2,3-triazol-2-yl)propyl methanesulphonate (58 mg) were added, and the mixture was stirred at 70°C for 12 hours. The reaction solution was filtered, and the filtrate was concentrated. The residue was separated and purified by prep-HPLC (C18, 10 mmol/L ammonium bicarbonate in acetonitrile) to obtain compound 77 (10 mg). LCMS (ESI) [M+H]⁺ =432.0; ¹H NMR (400 MHz, DMSO-d₆) δ 8.27 (dd, *J* = 12.8, 2.2 Hz, 2H), 7.77 (d, *J* = 0.8 Hz, 2H), 7.69 - 7.59 (m, 2H), 7.49 (t, *J =* 2.4 Hz, 1H), 7.17 (d, *J =* 9.0 Hz, 1H), 4.48 (t, *J =* 6.8 Hz, 2H), 3.28 (d, *J* = 4.9 Hz, 7H), 2.94 (t, *J =* 7.4 Hz, 2H), 2.58 (dd, *J =* 8.6, 6.2 Hz, 6H), 2.33 (t, *J =* 7.0 Hz, 2H), 2.06 (p, *J =* 6.9 Hz, 2H).

### Example 20

### Preparation of 1-methyl-6-(5-(4-((1-methyl-1H-1,2,4-triazol-3-yl)methyl)piperazin-1-yl)pyridin-3-yl)-3,4-dihydroquinolin-2(1H)-one (compound 79):

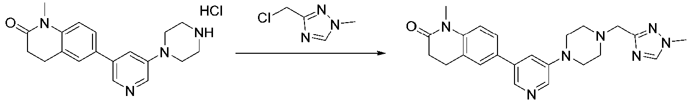

With reference to step 1 of example 5, using the monohydrochloride of intermediate 1 (100 mg, 1.0 eq) and 3-(chloromethyl)-1-methyl-1H-1,2,4-triazole (110 mg) as starting materials, the reaction was stirred at 70°C for 12 hours. The reaction solution was filtered, and the filtrate was concentrated. The residue was purified by prep-HPLC (C18, 10 mmol/L NH₄HCO₃ in water, MeCN) to obtain compound 79 (9.78 mg). LCMS (ESI) [M+H]⁺ =418.0; ¹H NMR (400 MHz, DMSO-d₆) δ 8.38 (s, 1H), 8.26 (dd, *J =* 13.5, 2.3 Hz, 2H), 7.61 (dq, *J* = 4.9, 2.3 Hz, 2H), 7.48 (t, *J =* 2.3 Hz, 1H), 7.17 (d, *J* = 9.1 Hz, 1H), 3.84 (s, 3H), 3.56 (s, 2H), 3.27 (d, *J =* 8.1 Hz, 7H), 2.94 (dd, *J =* 8.6, 6.2 Hz, 2H), 2.65 - 2.54 (m, 6H).

### Example 21

### Preparation of N-(trans-4-acetamidocyclohexyl)-2-(4-(6-(1-methyl-2-oxo-1,2,3,4-tetrahydroquinolin-6-yl)pyrazin-2-yl)piperazin-1-yl)acetamide (compound 80):

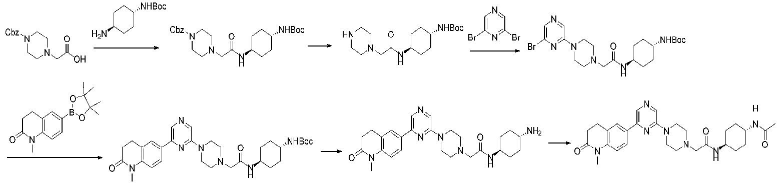

### Step 1: Synthesis of benzyl 4-(2-(((trans-4-((tert-butoxycarbonyl)amino)cyclohexyl)amino)-2-oxoethyl)piperazine-1-carboxylate:

To 2-(4-((benzyloxy)carbonyl)piperazin-1-yl)acetic acid (3 g, 1.0 eq), tert-butyl (trans-4-aminocyclohexyl)carbamate (2.54 g) and N,N'-diisopropylethylamine (4.18 g) were added acetonitrile (60).The reaction solvent was concentrated to dryness, and the residue was purified by flash chromatography (silica gel, PE : EA = 1 : 1) to obtain the product (8 g, crude). The crude was directly used in the next step without further purification. LCMS (ESI) [M+H]⁺ =475.1.

### Step 2: Synthesis of tert-butyl (trans-4-(2-(piperazin-1-yl)acetamido)cyclohexyl)carbamate:

With reference to step 2 of example 2, using the product obtained in step 1 (3 g, crude) with the addition of 5% palladium on carbon (500 mg, wet), the reaction was stirred at room temperature under a hydrogen atmosphere for 12 hours. The reaction solution was filtered through a pad of Celite, and the filtrate was concentrated to obtain the product (2.1 g). LCMS (ESI) [M+H]⁺ =341.

### Step 3: Synthesis of tert-butyl (trans-4-(2-(4-(6-bromopyrazin-2-yl)piperazin-1-yl)acetamido)cyclohexyl)carbamate:

With reference to step 1 of example 9, using tert-butyl (trans-4-(2-(piperazin-1-yl)acetamido)cyclohexyl)carbamate (801.49 mg, 1.0 eq) and 2,6-dibromopyrazine (560 mg) as starting materials, the reaction was stirred overnight at 100°C. The residue was purified by flash chromatography (silica gel, PE : EA = 2 : 3) to obtain the product (380 mg). LCMS (ESI) [M+H]⁺ =496.9.

### Step 4: Synthesis of tert-butyl (trans-4-(2-(4-(6-(1-methyl-2-oxo-1,2,3,4-tetrahydroquinolin-6-yl)pyrazin-2-yl)piperazin-1-yl)acetamido)cyclohexyl)carbamate:

With reference to step 6 of example 1, using the product obtained in step 3 (350 mg, 1.0 eq) and the sample of preparation example 1 (242.26 mg) as starting materials, the reaction was stirred overnight at 100°C under a nitrogen atmosphere. The residue was purified by flash chromatography (silica gel, PE : EA = 1 : 2) to obtain the product (260 mg). LCMS (ESI) [M+H]⁺ =578.1.

### Step 5: Synthesis of N-(trans-4-aminocyclohexyl)-2-(4-(6-(1-methyl-2-oxo-1,2,3,4-tetrahydroquinolin-6-yl)pyrazin-2-yl)piperazin-1-yl)acetamide:

With reference to step 2 of example 12, using the product obtained in step 4 (240 mg, 1.0 eq) as the starting material, the reaction was stirred at room temperature for 12 hours. The reaction solvent was concentrated to dryness, and the residue was purified by flash chromatography (silica gel, DCM : MeOH = 10 : 1) to obtain the product (180 mg). LCMS (ESI) [M+H]⁺ =478.1.

### Step 6:

The product obtained in step 5 (160 mg, 1.0 eq) was dissolved in dichloromethane (5 mL), and triethylamine (101.7 mg) was added. Under ice bath conditions, acetyl chloride (78.89 mg) was added dropwise. The mixture was stirred at room temperature for 3 hours. The reaction solvent was concentrated to dryness, and the residue was purified by flash chromatography (silica gel, DCM : MeOH = 10 : 1) to obtain the crude product. The mixture was slurried with acetonitrile and filtered, and the filter cake was dried to obtain compound 80 (45 mg). LCMS (ESI) [M+H]⁺ =520.1;¹H NMR (400 MHz, DMSO-d₆) δ 8.44 (s, 1H), 8.23 (s, 1H), 8.02 - 7.92 (m, 2H), 7.72 (d, J = 7.8 Hz, 1H), 7.59 (d, J = 8.3 Hz, 1H),7.18 (d, J = 8.5 Hz, 1H), 3.69 (t, J = 4.9 Hz, 4H), 3.46 (s, 2H), 3.29 (s, 3H), 2.96 (dt, J = 8.0, 3.9 Hz, 4H),2.58 (dt, J = 10.5, 5.4 Hz, 6H), 1.87 - 1.65 (m, 7H), 1.39 - 1.13 (m, 4H).

### Example 22

### Preparation of N-isopropyl-N-methyl-2-(4-(5-(1-methyl-2-oxo-1,2,3,4-tetrahydroquinolin-6-yl)pyridin-3-yl)piperazin-1-yl)acetamide (compound 81):

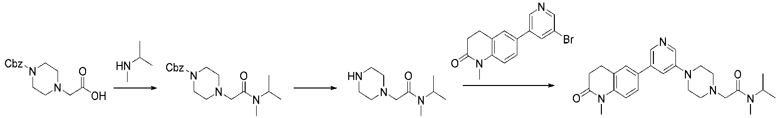

### Step 1: Synthesis of benzyl 4-(2-(isopropyl(methyl)amino)-2-oxoethyl)piperazine-1-carboxylate:

With reference to step 3 of example 1, using 2-(4-((benzyloxy)carbonyl)piperazin-1-yl)acetic acid (500 mg, 1.0 eq) and N-methyl-2-propylamine (170.83 mg) as starting materials, the reaction was stirred at room temperature for 4 hours. The residue was purified by flash chromatography (silica gel, DCM : MeOH = 10 : 1) to obtain the product (160 mg). LCMS (ESI) [M+H]⁺ =334.

With reference sequentially to step 2 and step 3 of example 2, compound 81 was prepared. LCMS (ESI) [M+H]⁺ =436.1; ¹H NMR (400 MHz, Chloroform-d) δ 8.30 (s, 1H), 8.28 (d, *J =* 2.7 Hz, 1H), 7.46 (dd, *J =* 8.4, 2.2 Hz, 1H), 7.37 (s, 1H), 7.30 (d, *J =* 4.7 Hz, 1H), 7.08 (s, 1H), 4.60 (dp, *J =* 215.1, 6.7 Hz, 1H),3.40 (s, 3H), 3.37 - 3.19 (m, 6H), 3.04 - 2.94 (m, 2H), 2.85 (d, *J =* 39.1 Hz, 3H),2.72 (ddd, *J =* 14.7, 9.6, 6.0 Hz, 6H), 1.20 (d, *J* = 6.6 Hz, 3H), 1.11 (d, *J =* 6.8 Hz, 3H).

### Example 23

### Preparation of 6-(5-(4-(2-(3-methoxypyrrolidin-1-yl)-2-oxoethyl)piperazin-1-yl)pyridin-3-yl)-1-methyl-3,4-dihydroquinolin-2(1H)-one (compound 82):

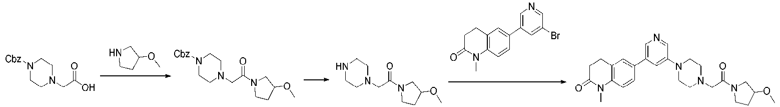

### Step 1: Synthesis of benzyl 4-(2-(3-methoxypyrrolidin-1-yl)-2-oxoethyl)piperazine-1-carboxylate:

With reference to step 3 of example 1, using 2-(4-((benzyloxy)carbonyl)piperazin-1-yl)acetic acid (400 mg, 1.0 eq) and 3-methoxypyrrolidine (174.46 mg) as starting materials, the reaction was stirred at room temperature for 4 hours. The residue was purified by flash chromatography (silica gel, DCM : MeOH = 10 : 1) to obtain the product (220 mg). LCMS (ESI) [M+H]⁺ =362.

With reference sequentially to step 2 and step 3 of example 2, compound 82 was prepared. LCMS (ESI) [M+H]⁺ =464; ¹H NMR (400 MHz, Chloroform-d) δ 8.30 (s, 1H), 8.27 (d, *J =* 2.8 Hz, 1H), 7.46 (dd, *J =* 8.4, 2.2 Hz, 1H), 7.37 (s, 1H), 7.29 (t, *J* = 2.3 Hz, 1H), 7.07 (d, *J =* 8.4 Hz, 1H), 4.00 (dtt, *J =* 26.6, 4.4, 2.3 Hz, 1H), 3.79 - 3.43 (m, 5H), 3.40 (s, 3H), 3.34 (d, *J =* 7.3 Hz, 6H), 3.25 - 3.15 (m, 2H), 3.04 - 2.92 (m, 2H), 2.82 - 2.63 (m, 6H), 2.20 - 1.85 (m, 2H).

### Example 24

### Preparation of 1-methyl-6-(5-(4-((1-methyl-1H-1,2,3-triazol-4-yl)methyl)piperazin-1-yl)pyridin-3-yl)-3,4-dihydroquinolin-2(1H)-one (compound 83):

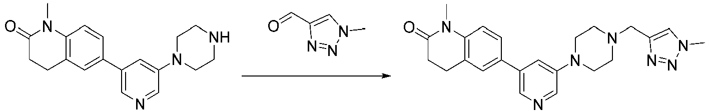

The sample of intermediate 1 (100 mg, 1.0 eq) and 1-methyl-1H-1,2,3-triazole-4-carbaldehyde (34.46 mg) were dissolved in 1,2-dichloroethane (3 mL), and the mixture was stirred at room temperature for 1 hour. Sodium triacetoxyborohydride (65.42 mg) was then added, and the mixture was further stirred for 1 h. The reaction solution was filtered, and the filter cake was washed with tetrahydrofuran. The filtrate was concentrated. The residue was purified by reversed-phase chromatography (0.1% aqueous formic acid solution, acetonitrile) to obtain compound 83 (12.22 mg). LCMS (ESI) [M+H] ⁺ = 417.9; ¹H NMR (400 MHz, DMSO-*d₆*) δ 8.31 - 8.27 (m, 1H), 8.27 - 8.23 (m, 1H), 8.14 (s, 1H), 7.98 (s, 1H), 7.64 - 7.59 (m, 2H), 7.50 - 7.46 (m, 1H), 7.21 - 7.13 (m, 1H), 4.03 (s, 3H), 3.64 (s, 2H), 3.45 - 3.32 (m, 4H), 3.26 (s, 3H), 2.97 - 2.91 (m, 2H), 2.63 - 2.56 (m, 6H).

### Examples 25-34

With reference to step 3 of example 1, using the sample of preparation example 3 (50 mg, 1.0 eq) with the starting materials shown in the table below, compounds 84-93 were prepared respectively.

| Starting material | Compound | Structural confirmation data |
|---|---|---|
| 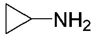 | 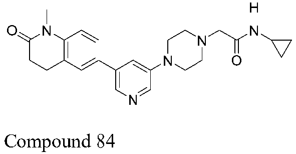 | LCMS (ESI) [M+H] ⁺ = 420.1; ¹H NMR(400 MHz, DMSO-*d₆*) δ 8.32 - 8.28 (m, 1H), 8.28 - 8.24 (m, 1H), 7.82 -7.75 (m, 1H), 7.66 - 7.58 (m, 2H), 7.49 (s, 1H), 7.21 - 7.14 (m, 1H), 3.32 - 3.29 (m, 4H), 3.29 (s, 3H), 2.99 - 2.91 (m, 4H), 2.68 - 2.63 (m, 1H), 2.62 - 2.55 (m, 6H), 0.66 - 0.58 (m, 2H), 0.49 - 0.42 (m, 2H). |
| 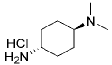 | 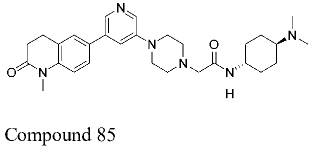 | LCMS (ESI) [M+H]⁺ = 505.4; ¹H NMR (400 MHz, MeOD) δ 8.48 (d, *J=* 1.6 Hz, 1H), 8.37 (d, *J* = 2.8 Hz, 1H), 8.08 (s, 1H), 7.72 - 7.69 (m, 1H), 7.67 (d, *J =* 2.0 Hz, 1H), 7.30 (d, *J =* 8.4 Hz, 1H), 3.85 (s, 2H), 3.83 - 3.69 (m, 5H), 3.47 - 3.38 (m, 7H), 3.08 - 3.02 (m, 2H), 2.88 (s, 6H), 2.72 - 2.66 (m, 2H), 2.16 (d, *J* = 10.8 Hz, 4H), 1.72 - 1.63 (m, 2H), 1.52 - 1.38 (m, 2H). |
| 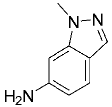 | 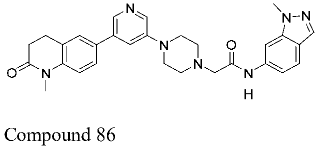 | LCMS (ESI) [M+H] ⁺ = 510.1; ¹H NMR(400 MHz, DMSO-*d₆*) δ 9.97 (s, 1H), 8.33 - 8.26 (m, 2H), 8.11 (s, 1H), 7.97 - 7.93 (m, 1H), 7.69 - 7.65 (m, 1H), 7.65 - 7.61 (m, 2H), 7.53 (s, 1H), 7.28 - 7.22 (m, 1H), 7.20 - 7.14 (m, 1H), 3.97 (s, 3H), 3.41 - 3.37 (m, 4H), 3.29 (s, 3H), 3.27 (s, 2H), 2.98 - 2.92 (m, 2H), 2.79 - 2.71 (m, 4H), 2.61 - 2.5 6 (m, 2H). |
| 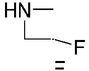 | 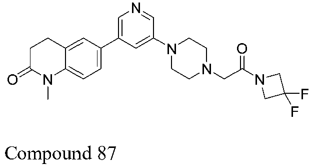 | LCMS (ESI) [M+H] ⁺ = 455.9; ¹H NMR(400 MHz, DMSO-*d₆*) δ 8.29 (d, *J* = 1.7 Hz, 1H), 8.26 (d, *J =* 2.6 Hz, 1H), 7.65 - 7.60 (m, 2H), 7.52 -7.48 (m, 1H), 7.20 -7.14 (m, 1H), 4.69 (t, *J =* 12.4 Hz, 2H), 4.31 (t, *J* = 12.7 Hz, 2H), 3.31 - 3.29 (m, 4H), 3.29 (s, 3H), 3.17 (s, 2H), 2.97 - 2.92 (m, 2H), 2.63 - 2.56 (m, 6H). |
| 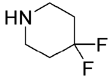 | 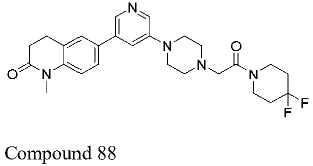 | LCMS (ESI) [M+H] ⁺ = 483.8; ¹H NMR(400 MHz, DMSO-*d₆*) δ 8.32 - 8.24 (m, 2H), 7.65 - 7.59 (m, 2H), 7.53 -7.47 (m, 1H), 7.20 - 7.14 (m, 1H), 3.69 - 3.62 (m, 2H), 3.61 - 3.53 (m, 2H), 3.32 - 3.29 (m, 4H), 3.29 (s, 3H), 3.27 (s, 2H), 2.98 - 2.90 (m, 2H), 2.63 - 2.56 (m, 6H), 2.09 - 1.86 (m, 4H). |
| 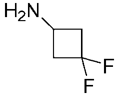 | 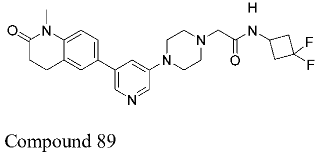 | LCMS (ESI) [M+H] ⁺ = 470.3; ¹H NMR(400 MHz, DMSO-*d₆*) δ 8.30 (d, *J* = 1.7 Hz, 1H), 8.28 - 8.23 (m, 2H), 7.65 - 7.61 (m, 2H), 7.53 - 7.48 (m, 1H), 7.19 - 7.15 (m, 1H), 4.18 - 4.06 (m, 1H), 3.37 - 3.33 (m, 4H), 3.29 (s, 3H), 3.01 (s, 2H), 2.97 - 2.91 (m, 2H), 2.90 - 2.79 (m, 2H), 2.75 - 2.64 (m, 2H), 2.63 - 2.55 (m, 6H). |
| 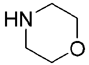 | 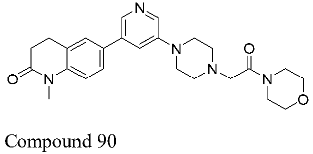 | LCMS (ESI) [M+H] ⁺ = 449.9; ¹H NMR(400 MHz, DMSO-d₆) δ 8.29 (d, *J* = 1.7 Hz, 1H), 8.26 (d, *J =* 2.6 Hz, 1H), 7.64 - 7.60 (m, 2H), 7.51 -7.48 (m, 1H), 7.20 - 7.14 (m, 1H), 3.62 - 3.56 (m, 4H), 3.56 - 3.53 (m, 2H), 3.48 - 3.41 (m, 2H), 3.31 - 3.26 (m, 7H), 3.23 (s, 2H), 2.99 - 2.92 (m, 2H), 2.63 - 2.56 (m, 6H). |
| 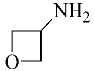 | 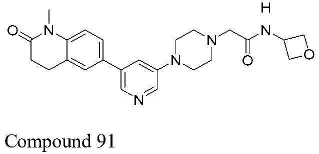 | LCMS (ESI) [M+H]⁺ =436.0; ¹H NMR (400 MHz, DMSO-*d*₆) δ 8.51 (d, J = 6.8 Hz, 1H), 8.28 (dd, J = 10.1, 2.3 Hz, 2H), 7.63 (dq, J = 4.5, 2.3 Hz, 2H), 7.51 (t, J = 2.4 Hz, 1H), 7.17 (d, J = 9.1 Hz, 1H), 4.91 - 4.79 (m, 1H), 4.69 (dd, J = 7.5, 6.2 Hz, 2H), 4.50 (t, J = 6.4 Hz, 2H), 3.34 (s, 5H), 3.29 (s, 3H), 3.02 (s, 2H), 2.95 (t, J = 7.4 Hz, 2H), 2.65 - 2.56 (m, 5H). |
| 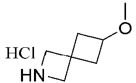 | 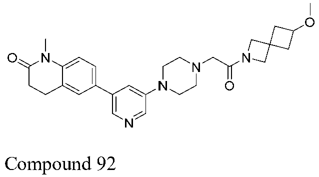 | LCMS (ESI) [M+H]⁺ =490.1; ¹H NMR (400 MHz, DMSO-d₆) δ 8.27 (dd, J *=* 13.6, 2.2 Hz, 2H), 7.62 (dq, J = 4.9, 2.3 Hz, 2H), 7.49 (t, J *=* 2.4 Hz, 1H), 7.17 (d, J = 9.0 Hz, 1H), 4.17 (d, J = 21.8 Hz, 2H), 3.83 (d, J = 21.8 Hz, 2H), 3.73 (td, J = 6.8, 3.5 Hz, 1H), 3.28 (d, J = 4.9 Hz, 7H), 3.10 (s, 3H), 3.01 (d, J = 2.8 Hz, 2H), 2.94 (t, J = 7.4 Hz, 2H), 2.65 - 2.53 (m, 6H), 2.43 (ddt, J = 9.9, 6.7, 3.3 Hz, 2H), 1.99 (ddd, J = 9.9, 7.1, 3.1 Hz, 2H). |
| 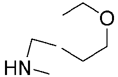 | 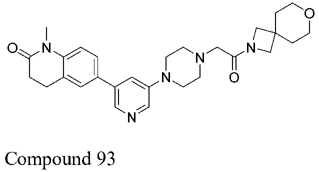 | LCMS (ESI) [M+H]⁺ =490.1; ¹H NMR (400 MHz, DMSO-d₆) δ 8.27 (dd, J *=* 12.4, 2.3 Hz, 2H), 7.62 (dq, J = 5.0, 2.2 Hz, 2H), 7.49 (t, J *=* 2.3 Hz, 1H), 7.17 (d, J = 9.0 Hz, 1H), 3.94 (s, 2H), 3.61 (s, 2H), 3.51 (d, J = 5.5 Hz, 4H), 3.29 (d, J = 5.2 Hz, 7H), 3.05 (s, 2H), 2.94 (dd, J = 8.6, 6.2 Hz, 2H), 2.65 - 2.55 (m, 6H), 1.67 (t, J = 5.3 Hz, 4H). |

### Example 35

### Preparation of N-isopropyl-1-(4-(5-(1-methyl-2-oxo-1,2,3,4-tetrahydroquinolin-6-yl)pyridin-3-yl)piperazin-1-yl)cyclopropane-1-carboxamide (compound 94):

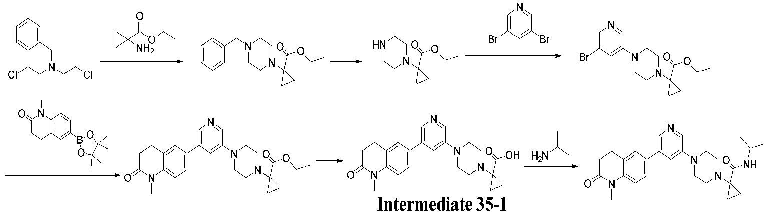

### Step 1: Synthesis of ethyl 1-(4-benzylpiperazin-1-yl)cyclopropane-1-carboxylate:

To N-benzyl-2-chloro-N-(2-chloroethyl)ethan-1-amine (17 g, 1.0 eq) and ethyl 1-aminocyclopropane-1-carboxylate (8.51 g) was added ethylbis(propan-2-yl)amine (150 mL), and the mixture was stirred at 100°C for 16 hours. To the reaction solution was added water (200 mL), and the mixture was extracted with dichloromethane (200 mL*3). The organic phases were combined, dried over anhydrous sodium sulphate and filtered, and the filtrate was concentrated. The residue was purified by flash chromatography (silica gel, PE : EA = 3 : 1) to obtain the product (8 g). LCMS (ESI) [M+H] ⁺ = 289.0; ¹H NMR (400 MHz, CDCl₃) δ 7.34 - 7.28 (m, 4H), 7.26 - 7.21 (m, 1H), 4.18 - 4.08 (m, 2H), 3.49 (s, 2H), 3.08 - 2.86 (m, 4H), 2.48 - 2.21 (m, 4H), 1.28 - 1.24 (m, 5H), 0.91 - 0.87 (m, 2H).

### Step 2: Synthesis of ethyl 1-(piperazin-1-yl)cyclopropane-1-carboxylate:

With reference to step 2 of example 2, using the product obtained in step 1 (5 g, 1.0 eq) as the starting material, the reaction was stirred at 40°C under hydrogen (balloon pressure) for 16 hours. The reaction solution was filtered through a pad of Celite, and the filtrate was concentrated to obtain the product (3 g, crude). LCMS (ESI) [M+H] ⁺ = 199.0; ¹H NMR(400 MHz, DMSO-*d₆*) δ 4.10 - 4.01 (m, 2H), 2.90 - 2.75 (m, 4H), 2.70 - 2.53 (m, 4H), 1.23 - 1.12 (m, 5H), 0.93 - 0.83 (m, 2H).

### Step 3: Synthesis of ethyl 1-(4-(5-bromopyridin-3-yl)piperazin-1-yl)cyclopropane-1-carboxylate:

To a mixture of the product obtained in step 2 (3 g, crude), 3,5-dibromopyridine (4.66 g), sodium tert-butoxide (2.18 g), [5-(diphenylphosphanyl)-9,9-dimethyl-9H-xanthen-4-yl]diphenylphosphane (525.33 mg) and tris(dibenzylideneacetone)dipalladium (277.13 mg) was added toluene (50 mL). Under a nitrogen atmosphere, the mixture was stirred at 100°C for 16 hours. To the reaction solution was added water (100 mL), and the mixture was extracted with ethyl acetate (100 mL*3). The organic phases were combined, dried over anhydrous sodium sulphate and filtered, and the filtrate was concentrated. The residue was purified by flash chromatography (silica gel, PE : EA = 3 : 1) to obtain the product (3.3 g). LCMS (ESI) [M+H] ⁺ = 354.0; ¹H NMR(400 MHz, DMSO-*d₆)* δ 8.34 - 8.22 (m, 1H), 8.09 - 7.98 (m, 1H), 7.55 - 7.48 (m, 1H), 4.06 (q, *J* = 7.1 Hz, 2H), 3.29 - 3.06 (m, 4H), 3.06 - 2.96 (m, 4H), 1.25 - 1.14 (m, 5H), 1.02 - 0.92 (m, 2H).

### Step 4: Synthesis of ethyl 1-(4-(5-(1-methyl-2-oxo-1,2,3,4-tetrahydroquinolin-6-yl)pyridin-3-yl)piperazin-1-yl)cyclopropane-1-carboxylate:

With reference to step 6 of example 1, using the product obtained in step 3 (1 g, 1.0 eq) and the sample of preparation example 1 (1.22 g), the reaction was stirred at 80°C under a nitrogen atmosphere for 2 hours. The residue was purified by flash chromatography (silica gel, EA) to obtain the product (780 mg). LCMS (ESI) [M+H] ⁺ = 435.1; ¹H NMR(400 MHz, DMSO-d₆) δ 8.33 - 8.22 (m, 2H), 7.68 - 7.59 (m, 2H), 7.54 - 7.44 (m, 1H), 7.23 - 7.13 (m, 1H), 4.07 (q, *J* = 7.1 Hz, 2H), 3.29 (s, 3H), 3.28 - 3.12 (m, 4H), 3.11 - 3.02 (m, 4H), 2.98 - 2.90 (m, 2H), 2.63 - 2.56 (m, 2H), 1.25 - 1.20 (m, 2H), 1.18 (t, *J* = 7.1 Hz, 3H), 1.03 - 0.95 (m, 2H).

### Step 5: Synthesis of 1-(4-(5-(1-methyl-2-oxo-1,2,3,4-tetrahydroquinolin-6-yl)pyridin-3-yl)piperazin-1-yl)cyclopropane-1-carboxylic acid (intermediate 35-1):

The product obtained in step 4 (730 mg, 1.0 eq) was dissolved in methanol (5 mL) and water (5 mL), and lithium hydroxide (120.71 mg) was added. The mixture was stirred at 50°C for 16 hours. The reaction mixture was acidified to weakly acidic with dilute hydrochloric acid, and the residue was purified by reversed-phase chromatography (acetonitrile/0.1% formic acid=40%) to obtain the product intermediate 35-1 (500 mg). LCMS (ESI) [M+H] ⁺ = 407.2; ¹H NMR(400 MHz, DMSO-*d₆*) δ 12.30 (s, 1H), 8.32 - 8.21 (m, 2H), 7.67 - 7.59 (m, 2H), 7.52 - 7.45 (m, 1H), 7.21 - 7.14 (m, 1H), 3.29 (s, 3H), 3.28 - 3.12 (m, 4H), 3.12 - 3.01 (m, 4H), 2.99 - 2.92 (m, 2H), 2.63 - 2.55 (m, 2H), 1.25 - 1.14 (m, 2H), 0.99 - 0.88 (m, 2H).

### Step 6:

With reference to step 3 of example 1, using the product intermediate 35-1 obtained in step 5 (50 mg, 1.0 eq) and isopropylamine (7.27 mg) as starting materials, the reaction was stirred at 25°C for 1 hour. The residue was purified by reversed-phase chromatography (0.1% aqueous formic acid solution, acetonitrile) to obtain compound 94 (21.79 mg). LCMS (ESI) [M+H] ⁺= 448.2; ¹H NMR(400 MHz, DMSO-d₆) δ 8.34 - 8.22 (m, 2H), 7.68 (d, *J =* 8.1 Hz, 1H), 7.65 - 7.60 (m, 2H), 7.53 - 7.47 (m, 1H), 7.21 - 7.14 (m, 1H), 3.93 - 3.82 (m, 1H), 3.36 - 3.34 (m, 4H), 3.29 (s, 3H), 2.99 - 2.90 (m, 2H), 2.61 - 2.55 (m, 6H), 1.09 (d, *J =* 6.6 Hz, 6H), 1.01 - 0.91 (m, 4H).

### Example 36

### Preparation of N-(trans-4-acetamidocyclohexyl)-2-(4-(5-(p-tolyl)-1,2,4-triazin-3-yl)piperazin-1-yl)acetamide-2,2-d2 (compound 97):

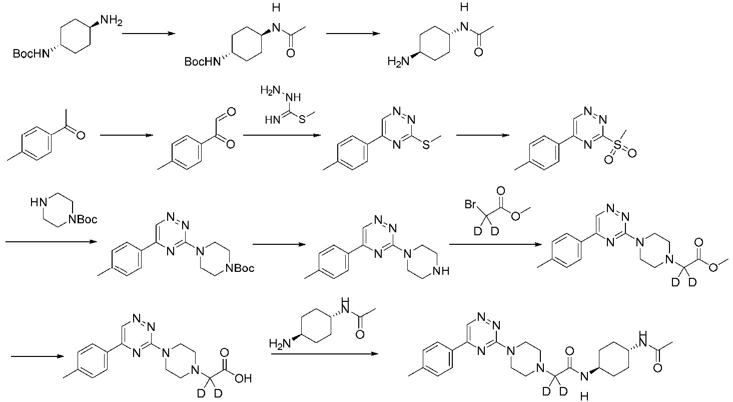

### Step 1: Synthesis of tert-butyl (trans-4-acetamidocyclohexyl)carbamate:

With reference to step 8 of example 1, using tert-butyl ((1R,4R)-4-aminocyclohexyl)carbamate (200 mg, 1.0 eq) and acetic anhydride (0.29 mL) as starting materials, the reaction was stirred at 25°C for 2 hours. The reaction solution was diluted with water (25 mL), and extracted with dichloromethane (10 mL*3). The organic phases were combined, dried over anhydrous sodium sulphate and filtered, and the filtrate was concentrated to obtain the product (200 mg, crude). ¹H NMR (400 MHz, CDCl₃) δ 5.33 - 5.27 (m, 1H), 4.45 - 4.33 (m, 1H), 3.76 - 3.67 (m, 1H), 3.44 - 3.35 (m, 1H), 2.03 - 1.98 (m, 4H), 1.95 (s, 3H), 1.43 (s, 9H), 1.24 - 1.18 (m, 4H).

### Step 2: Synthesis of N-(trans-4-aminocyclohexyl)acetamide:

With reference to step 7 of example 1, using the product obtained in step 1 (200 mg, crude) as the starting material, the reaction was stirred at 25°C for 4 hours. The reaction mixture was concentrated to obtain the product (110 mg, crude). ¹H NMR (400 MHz, DMSO-d₆) δ 8.07 - 7.94 (m, 3H), 7.79 (d, *J* = 7.6 Hz, 1H), 3.45 - 3.40 (m, 1H), 2.98 - 2.89 (m, 1H), 1.97 - 1.91 (m, 2H), 1.83 - 1.78 (m, 2H), 1.77 (s, 3H), 1.41 - 1.32 (m, 2H), 1.23 - 1.16 (m, 2H).

### Step 3: Synthesis of 2-oxo-2-(p-tolyl)acetaldehyde:

Selenium dioxide (33.08 g) was added to a mixed solution of 1,4-dioxane (200 mL) and water (20 mL), and the mixture was stirred at 70°C for 4 hours until all the selenium dioxide was dissolved. Under a nitrogen atmosphere, to the reaction solution was slowly added 1-(4-methylphenyl)ethan-1-one (20 g). The mixture was stirred at 70°C for 16 hours. After the reaction was completed as detected by TLC, the reaction solution was filtered through a pad of Celite, and the filtrate was extracted with ethyl acetate (300 mL). The organic phases were combined, dried over anhydrous sodium sulphate and filtered, and the filtrate was concentrated. To the crude were added petroleum ether and ethyl acetate (50 mL, 5 : 1). The mixture was stirred overnight and filtered, and the filter cake was purified by flash chromatography (silica gel, PE : THF = 3 : 1) to obtain the product (21.8 g). ¹H NMR (400 MHz, DMSO-*d₆*) δ 9.53(s, 1H), 8.02 - 7.93 (m, 2H), 7.41 - 7.30 (m, 2H), 2.41 - 2.36 (m, 3H).

### Step 4: Synthesis of 3-(methylthio)-5-(p-tolyl)-1,2,4-triazine:

The product obtained in step 3 (11 g, 1.0 eq) was dissolved in ethanol (200 mL) and water (40 mL). Methyl hydrazinecarbimidothioate (17.3 g) and sodium bicarbonate (18.71 g) were added, and the mixture was stirred at 100°C for 12 hours. The reaction solution was diluted with water (200 mL), and extracted with ethyl acetate (80 mL*4). The organic phases were combined, dried over anhydrous sodium sulphate and filtered, and the filtrate was concentrated. The residue was purified by flash chromatography (silica gel, PE : THF = 17 : 3 to 1 : 1) to obtain the product (10.5 g). LCMS (ESI) [M+H]⁺ = 218.4; ¹H NMR (400 MHz, DMSO-*d₆*) δ 9.78 (s, 1H), 8.22 (d, *J* = 8.2 Hz, 2H), 7.42 (d, *J =* 8.1 Hz, 2H), 2.67 (s, 3H), 2.41 (s, 3H).

### Step 5: Synthesis of 3-(methylsulphonyl)-5-(p-tolyl)-1,2,4-triazine:

The product obtained in step 4 (3 g, 1.0 eq) was dissolved in dichloromethane (35 mL). At -15°C, 3-chlorobenzene-1-carboperoxoic acid (7.15 g) was added. The mixture was stirred at -15°C for 8 hours. The reaction solution was quenched by adding saturated sodium bicarbonate solution (70 mL), and the mixture was extracted with ethyl acetate (35 mL*3). The organic phases were combined, washed with saturated sodium sulphite solution (45 mL), dried over anhydrous sodium sulphate and filtered, and the filtrate was concentrated. The residue was purified by flash chromatography (silica gel, PE : THF = 2 : 1 to 1 : 1) to obtain the product (2 g). LCMS (ESI) [M+H]⁺ = 250.4; ¹H NMR (400 MHz, DMSO-*d*₆) δ 10.32 (s, 1H), 8.35 (d, *J =* 8.3 Hz, 2H), 7.49 (d, *J =* 8.0 Hz, 2H), 3.58 (s, 3H), 2.44 (s, 3H).

### Step 6: Synthesis of tert-butyl 4-(5-(p-tolyl)-1,2,4-triazin-3-yl)piperazine-1-carboxylate:

The product obtained in step 5 (2 g, 1.0 eq), tert-butyl piperazine-1-carboxylate (1.49 g) and sodium carbonate (850.33 mg) were dissolved in acetonitrile (20 mL), and the mixture was stirred at 25°C for 16 hours. The reaction solution was diluted with water (60 mL), and extracted with ethyl acetate (30 mL*3). The organic phases were combined, dried over anhydrous sodium sulphate and filtered, and the filtrate was concentrated. The residue was purified by flash chromatography (silica gel, PE : THF = 4 : 1) to obtain the product (1.5 g). LCMS (ESI) [M+H]⁺ = 356.5; ¹H NMR (400 MHz, DMSO-*d*₆) δ 9.30 (s, 1H), 8.17 (d, *J =* 8.2 Hz, 2H), 7.39 (d, *J =* 8.1 Hz, 2H), 3.93 - 3.85 (m, 4H), 3.50 - 3.46 (m, 4H), 2.40 (s, 3H), 1.44 (s, 9H).

### Step 7: Synthesis of 3-(piperazin-1-yl)-5-(p-tolyl)-1,2,4-triazine:

With reference to step 7 of example 1, using the product obtained in step 6 (1.5 g, 1.0 eq) as the starting material, the reaction was stirred at 25°C for 16 hours. The reaction solution was directly concentrated to obtain the product (900 mg, crude). LCMS (ESI) [M+H]⁺ = 256.5. The crude was directly used in the next step without further purification.

### Step 8: Synthesis of methyl 2-(4-(5-(p-tolyl)-1,2,4-triazin-3-yl)piperazin-1-yl)acetate-d2:

The product obtained in step 7 (576.57 mg, crude) was dissolved in N,N-dimethylformamide (15 mL). Methyl 2-bromoacetate-d2 (350 mg) and potassium carbonate (937.03 mg) were added, and the mixture was stirred at 70°C for 3 hours. The reaction solution was diluted with water (40 mL), and extracted with ethyl acetate (25 mL*3). The organic phases were combined, dried over anhydrous sodium sulphate and filtered, and the filtrate was concentrated. The residue was purified by flash chromatography (silica gel, PE : THF = 20 : 9) to obtain the product (580 mg). LCMS (ESI) [M+H]⁺ = 330.5; ¹H NMR (400 MHz, DMSO-*d₆*) δ 9.27 (s, 1H), 8.16 (d, *J =* 8.2 Hz, 2H), 7.38 (d, *J =* 8.1 Hz, 2H), 3.93 - 3.86 (m, 4H), 3.63 (s, 3H), 2.67 - 2.62 (m, 4H), 2.40 (s, 3H).

### Step 9: Synthesis of 2-(4-(5-(p-tolyl)-1,2,4-triazin-3-yl)piperazin-1-yl)acetic-2,2-d2 acid:

With reference to step 5 of example 35, using the product obtained in step 8 (580 mg, 1.0 eq) as the starting material, the reaction was stirred at 50°C for 2 hours. The reaction solution was adjusted to pH 5 to 6 with citric acid solution, stirred for 5 minutes, and filtered. The filter cake was dried to obtain the product (460 mg). LCMS (ESI) [M-H]⁻ =314.2; ¹H NMR (400 MHz, DMSO-d₆) δ 9.27 (s, 1H), 8.15 *(d, J=* 8.1 Hz, 2H), 7.38 *(d, J=* 8.0 Hz, 2H), 3.94 - 3.89 (m, 4H), 2.72 - 2.65 (m, 4H), 2.40 (s, 3H).

### Step 10:

With reference to step 3 of example 1, using the product obtained in step 9 (100 mg, 1.0 eq) and N-(trans-4-aminocyclohexyl)acetamide (59.45 mg) as starting materials, the reaction was stirred at 25°C for 3 hours. The reaction solution was diluted with water (20 mL), and extracted with dichloromethane (10 mL*3). The organic phases were combined, dried over anhydrous sodium sulphate and filtered, and the filtrate was concentrated. The residue was purified by flash chromatography (silica gel, DCM : MeOH = 10 : 1) to obtain compound 97 (57.11 mg). LCMS (ESI) [M-H]⁻ =453.9; ¹H NMR (400 MHz, DMSO-d₆) δ 9.28 (s, 1H), 8.16 (d, *J =* 8.2 Hz, 2H), 7.72 (d, *J* = 7.7 Hz, 1H), 7.61 (d, *J =* 8.3 Hz, 1H), 7.38 (d, *J =* 8.1 Hz, 2H), 3.95 - 3.89 (m, 4H), 3.57 - 3.45 (m, 2H), 2.58 - 2.55 (m, 4H), 2.40 (s, 3H), 1.80 - 1.74 (m, 7H), 1.34 - 1.18 (m, 4H).

### Example 37

### Preparation of N-isopropyl-2-(6-(5-(1-methyl-2-oxo-1,2,3,4-tetrahydroquinolin-6-yl)pyridin-3-yl)-2,6-diazaspiro[3.3]heptan-2-yl)acetamide (compound 98):

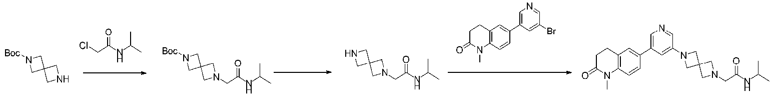

### Step 1: Synthesis of tert-butyl 6-(2-(isopropylamino)-2-oxoethyl)-2,6-diazaspiro[3.3]heptane-2-carboxylate:

With reference to step 1 of example 5, using tert-butyl 2,6-diazaspiro[3.3]heptane-2-carboxylate (50 mg, 1.0 eq) and 2-chloro-N-isopropylacetamide (683.9 mg) as starting materials, the reaction was stirred at 35°C under a nitrogen atmosphere for 3 hours. The residue was purified by flash chromatography (silica gel, DCM : MeOH = 10 : 1) to obtain the product (300 mg). LCMS (ESI) [M+H]⁺ = 298.

### Step 2: Synthesis of N-isopropyl-2-(2,6-diazaspiro[3.3]heptan-2-yl)acetamide:

The product obtained in step 1 (250 mg, crude) was dissolved in dichloromethane (4 mL), and trifluoroacetic acid (1 mL) was added. The mixture was stirred at room temperature for 2 hours. A small amount of sodium carbonate and a small volume of methanol were added to the reaction mixture. After gas evolution ceased, the mixture was filtered, and the filtrate was concentrated to afford the product (120 mg, crude). LCMS (ESI) [M+H]⁺ = 198. The crude was directly used in the next step without further purification.

### Step 3:

With reference to step 3 of example 2, using 6-(5-bromopyridin-3-yl)-1-methyl-3,4-dihydroquinolin-2(1H)-one (100 mg, 1.0 eq) and the product obtained in step 2 (124.4 mg) as starting materials, the reaction was stirred overnight at 100°C under a nitrogen atmosphere. The residue was purified by flash chromatography (silica gel, DCM : MeOH = 10 : 1) to obtain the crude product, which was further purified by reversed-phase chromatography (C18, MeCN : H₂O = 2 : 1) to obtain compound 98 (45 mg). LCMS (ESI) [M+H]⁺ = 434.1; ¹H NMR (400 MHz, Chloroform-d) δ 8.23 (d, *J* = 1.9 Hz, 1H), 7.83 (d, *J* = 2.6 Hz, 1H), 7.45 (dd, *J* = 8.4, 2.1 Hz, 1H), 7.36 (s, 1H),7.07 (d, *J =* 8.5 Hz, 1H), 6.83 (t, *J* = 2.3 Hz, 1H), 6.63 (s, 1H), 4.11 (d, *J =* 6.6 Hz, 1H), 4.07 (s, 4H), 3.51 (s, 4H), 3.40 (s, 3H), 3.11 (s, 2H), 3.02 - 2.94 (m, 2H), 2.74 - 2.65 (m, 2H), 1.17 (d, *J =* 6.5 Hz, 6H).

### Example 38

### Preparation of N-cyclopropyl-2-(4-(6-(1-methyl-2-oxo-1,2,3,4-tetrahydroquinolin-6-yl)pyrazin-2-yl)piperazin-1-yl)acetamide (compound 99):

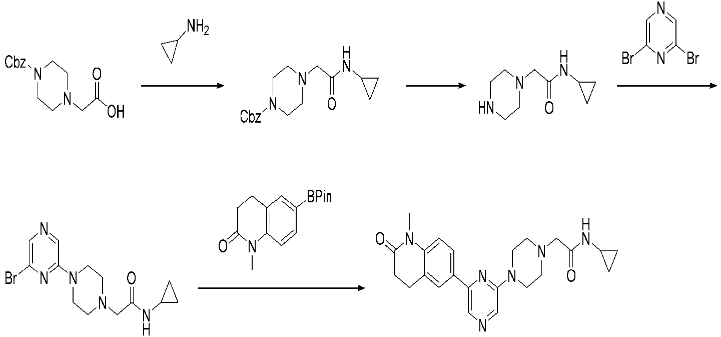

### Step 1: Synthesis of benzyl 4-(2-(cyclopropylamino)-2-oxoethyl)piperazine-1-carboxylate:

With reference to step 1 of example 21, using 2-(4-((benzyloxy)carbonyl)piperazin-1-yl)acetic acid (1 g, 1.0 eq) and cyclopropylamine (246.17 mg) as starting materials, the reaction was stirred at room temperature for 3 hours. The residue was purified by flash chromatography (silica gel, DCM : MeOH = 10 : 1) to obtain the product (500 mg). LCMS (ESI) [M+H]⁺ = 318.

### Step 2: Synthesis of N-cyclopropyl-2-(piperazin-1-yl)acetamide:

With reference to step 2 of example 2, using the product obtained in step 1 (500 mg, 1.0 eq) as the starting material, the reaction was stirred overnight at room temperature under a hydrogen atmosphere. The reaction solution was filtered through a pad of Celite, and the filtrate was concentrated to obtain the product (220 mg, crude). LCMS (ESI) [M+H]⁺ = 184. The product was directly used in the next step without further purification.

### Step 3: Synthesis of 2-(4-(6-bromopyrazin-2-yl)piperazin-1-yl)-N-cyclopropylacetamide:

With reference to step 1 of example 9, using 2,6-dibromopyrazine (220 mg, 1.0 eq) and the product obtained in step 2 (200 mg, crude) as starting materials, the reaction was stirred overnight at 90°C under a nitrogen atmosphere. The residue was purified by flash chromatography (silica gel, PE : EA = 2 : 1) to obtain the product (300 mg). LCMS (ESI) [M+H]⁺ = 339.9.

### Step 4: Synthesis of N-cyclopropyl-2-(4-(6-(1-methyl-2-oxo-1,2,3,4-tetrahydroquinolin-6-yl)pyrazin-2-yl)piperazin-1-yl)acetamide:

The sample of preparation example 1 (310 mg, 1.0 eq), the product obtained in step 3 (367.28 mg), caesium carbonate (703.47 mg) and bis[(cyclopenta-1,3-dien-1-yl)diphenylphosphane]dichloropalladium iron (79.21 mg) were added to 1,4-dioxane (8 mL) and water (2 mL). Under a nitrogen atmosphere, the mixture was stirred overnight at 100°C. The reaction solution was dried over anhydrous sodium sulphate and filtered, and the filtrate was concentrated. The residue was purified by flash chromatography (silica gel, PE : EA = 0 : 1) to obtain the crude, which was further purified by reversed-phase chromatography (C18, MeCN : H₂O = 1 : 1) to obtain compound 99 (248.32 mg). LCMS (ESI) [M+H]⁺ = 421; ¹H NMR (400 MHz, Chloroform-*d*) δ 8.34 (s, 1H), 8.06 (s, 1H), 7.89 (dd, J = 8.4, 2.1 Hz, 1H), 7.81 (d, J = 2.1 Hz, 1H),7.15 (s, 1H), 7.07 (d, J = 8.5 Hz, 1H), 3.71 (t, J = 5.1 Hz, 4H), 3.40 (s, 3H),3.07 (s, 2H), 3.04 - 2.95 (m, 2H), 2.77 (tq, J = 7.4, 3.8 Hz, 1H), 2.73 - 2.63 (m, 6H),0.83 (td, J = 7.0, 5.4 Hz, 2H), 0.58 - 0.48 (m, 2H).

### Example 39

### Preparation of 2-(2,6-dimethyl-4-(5-(1-methyl-2-oxo-1,2,3,4-tetrahydroquinolin-6-yl)pyridin-3-yl)piperazin-1-yl)-N-isopropylacetamide (compound 102):

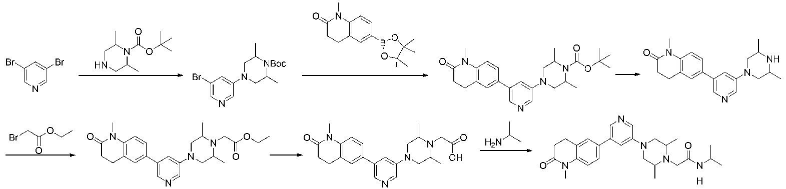

### Step 1: Synthesis of tert-butyl 4-(5-bromopyridin-3-yl)-2,6-dimethylpiperazine-1-carboxylate:

3,5-Dibromopyridine (2,87 g, 1.3 eq), tert-butyl 2,6-dimethylpiperazine-1-carboxylate (2 g), sodium 2-methylpropan-2-olate (1.35 g), (1E,4E)-1,5-diphenylpenta-1,4-dien-3-one dipalladium (83.46 mg) and [5-(diphenylphosphanyl)-9,9-dimethyl-9H-xanthen-4-yl]diphenylphosphane (324.01 mg) were dissolved in toluene (30 mL). Under a nitrogen atmosphere, the mixture was stirred at 100°C for 16 hours. The reaction solution was directly concentrated, and the residue was purified by flash chromatography (silica gel, PE : EA = 2 : 1) to obtain the product (1,65 g). LCMS (ESI) [M+H]⁺ = 372.3; ¹H NMR (400 MHz, DMSO-d₆) δ 8.32 (d, *J =* 2.5 Hz, 1H), 8.03 (d, *J* = 1.8 Hz, 1H), 7.56 (t, *J =* 2.2 Hz, 1H), 4.13 - 4.06 (m, 2H), 3.68 (d, *J =* 12.6 Hz, 2H), 2.92 (dd, *J =* 12.5, 4.4 Hz, 2H), 1.42 (s, 9H), 1.21 (d, *J =* 6.8 Hz, 6H).

### Step 2: Synthesis of tert-butyl 2,6-dimethyl-4-(5-(1-methyl-2-oxo-1,2,3,4-tetrahydroquinolin-6-yl)pyridin-3-yl)piperazine-1-carboxylate:

The product obtained in step 1 (1.65 g, 1.0 eq), the sample of preparation example 1 (2.05 g), dichloropalladium(2+)bis((cyclopentadien-1-yl)diphenylphosphane)iron (327.24 mg) and tripotassium phosphate (2.84 g) were dissolved in 1,4-dioxane (15 mL) and water (3 mL). Under a nitrogen atmosphere, the mixture was stirred at 80°C for 3 hours. The reaction solution was diluted with water (50 mL), and extracted with ethyl acetate (25 mL*3). The organic phases were combined, dried over anhydrous sodium sulphate and filtered, and the filtrate was concentrated. The residue was purified by flash chromatography (silica gel, PE : EA = 5 : 2) to obtain the product (1.6 g). ¹H NMR (400 MHz, DMSO-*d₆*) δ 8.31 - 8.28 (m, 2H), 7.64 - 7.60 (m, 2H), 7.50 - 7.47 (m, 1H), 7.20 - 7.16 (m, 1H), 4.15 - 4.11 (m, 2H), 3.73 - 3.69 (m, 2H), 3.29 (s, 3H), 2.98 - 2.92 (m, 4H), 2.61 - 2.56 (m, 2H), 1.43 (s, 9H), 1.28 (s, 3H), 1.26 (s, 3H).

### Step 3: Synthesis of 6-(5-(3,5-dimethylpiperazin-1-yl)pyridin-3-yl)-1-methyl-3,4-dihydroquinolin-2(1H)-one:

With reference to step 2 of example 12, using the product obtained in step 2 (1.2 g, 1.0 eq) as the starting material, the reaction was stirred at 25°C for 16 hours. The reaction solution was directly concentrated to obtain the product (900 mg). LCMS (ESI) [M+H]⁺ =351.3.

Step 4 to step 6: With reference to step 8 to step 10 of example 36, using the product obtained in step 3 (500 mg, 1.0 eq) and ethyl 2-bromoacetate (0.24 ml) as starting materials, compound 102 (71.18 mg) was prepared. LCMS (ESI) [M+H]⁺ = 450.4; ¹H NMR (400 MHz, DMSO-*d₆*) δ 8.28 - 8.25 (m, 2H), 7.65 - 7.60 (m, 2H), 7.52 - 7.46 (m, 2H), 7.20 - 7.15 (m, 1H), 3.94 - 3.86 (m, 1H), 3.80 - 3.74 (m, 2H), 3.29 (s, 3H), 3.07 (s, 2H), 2.97 - 2.92 (m, 2H), 2.82 - 2.75 (m, 2H), 2.61 - 2.54 (m, 4H), 1.09 - 1.04 (m, 12H).

### Examples 40-49

With reference to step 3 of example 1, using the sample of preparation example 3 (50 mg, 1.0 eq) with the starting materials shown in the table below, compounds 100, 101, 103-107, and 109-110 were prepared respectively. With reference to step 6 of example 35, using intermediate 35-1 (50 mg, 1.0 eq) with the starting materials shown in the table below, compound 108 was prepared.

| Starting material | Compound | Structural confirmation data |
|---|---|---|
| 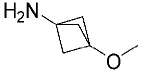 | 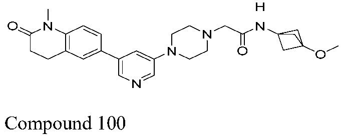 | LCMS (ESI) [M+H] ⁺ = 476.2; ¹H NMR(400 MHz, DMSO-d₆) δ 8.32 (s, 1H), 8.29 (d, *J =* 1.7 Hz, 1H), 8.26 (d, *J* = 2.7 Hz, 1H), 7.66 - 7.60 (m, 2H), 7.52 - 7.47 (m, 1H), 7.21 -7.15 (m, 1H), 3.32 - 3.30 (m, 4H), 3.29 (s, 3H), 3.20 (s, 3H), 2.98 - 2.91 (m, 4H), 2.62 - 2.55 (m, 6H), 2.08 (s, 6H). |
| 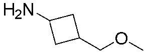 | 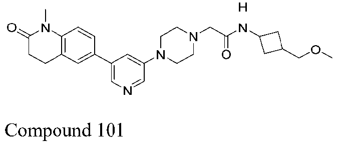 | LCMS (ESI) [M+H] ⁺ = 478.2; ¹H NMR(400 MHz, DMSO-*d₆*) δ 8.29 (d, *J* = 1.8 Hz, 1H), 8.27 (d, *J =* 2.7 Hz, 1H), 7.90 (d, *J =* 8.2 Hz, 1H), 7.65 - 7.62 (m, 2H), 7.52 - 7.49 (m, 1H), 7.20 - 7.16 (m, 1H), 4.20 - 4.08 (m, 1H), 3.38 - 3.32 (m, 5H), 3.29 (s, 3H), 3.28 - 3.25 (m, 2H), 3.22 (s, 3H), 2.97 - 2.93 (m, 4H), 2.62 - 2.57 (m, 6H), 2.28 - 2.18 (m, 2H), 1.80 - 1.59 (m, 2H). |
| 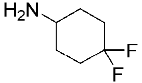 | 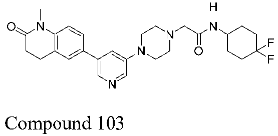 | LCMS (ESI) [M+H]⁺ =498.1; ¹H NMR (400 MHz, DMSO-*d*₆) δ 8.28 (dd, J = 11.1, 2.2 Hz, 2H), 7.75 (d, J = 8.1 Hz, 1H), 7.63 (dd, J *=* 4.5, 2.4 Hz, 2H), 7.50 (d, J = 2.4 Hz, 1H), 7.17 (d, J *=* 9.0 Hz, 1H), 3.81 (d, J = 10.3 Hz, 1H), 3.29 (s, 7H), 3.04 - 2.90 (m, 4H), 2.59 (q, J = 6.7 Hz, 6H), 1.99 (s, 3H), 1.93 - 1.83 (m, 1H), 1.78 (d, J = 13.0 Hz, 2H), 1.63 - 1.50 (m, 2H). |
| 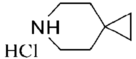 | 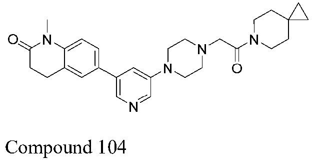 | LCMS (ESI) [M+H]⁺ =474.1; ¹H NMR (400 MHz, DMSO-*d₆*) δ 8.28 (dd, J *=* 11.3, 2.3 Hz, 2H), 7.63 (dq, J *=* 5.5, 2.3 Hz, 2H), 7.50 (t, J = 2.3 Hz, 1H), 7.17 (d, J = 9.1 Hz, 1H), 3.52 (dt, J = 22.9, 5.6 Hz, 4H), 3.29 (d, J = 5.1 Hz, 7H), 3.23 (s, 2H), 2.94 (dd, J = 8.6, 6.1 Hz, 2H), 2.66 - 2.52 (m, 6H), 1.32 (dt, J *=* 42.2, 5.6 Hz, 4H), 0.33 (s, 4H). |
| 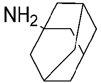 | 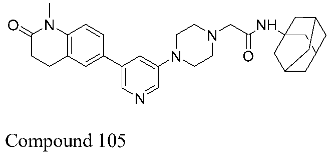 | LCMS (ESI) [M+H]⁺ =514.1; ¹H NMR (400 MHz, DMSO-*d*₆) δ 8.28 (dd, J *=* 11.1, 2.3 Hz, 2H), 7.63 (dq, J = 5.1, 2.3 Hz, 2H), 7.50 (t, J = 2.3 Hz, 1H), 7.17 (d, J = 9.1 Hz, 1H), 7.10 (s, 1H), 3.30 (d, J = 10.3 Hz, 7H), 3.01 - 2.84 (m, 4H), 2.69 - 2.54 (m, 6H), 2.07 - 1.86 (m, 9H), 1.63 (d, J = 3.0 Hz, 6H). |
| 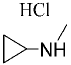 | 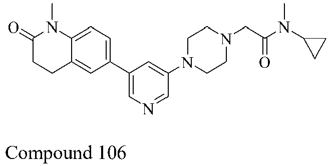 | LCMS (ESI) [M+H]⁺ =434.1; ¹H NMR (400 MHz, DMSO-*d*₆) δ 8.27 (dd, *J* = 10.6, 2.3 Hz, 2H), 7.63 (dq, *J =* 5.2, 2.2 Hz, 2H), 7.49 (t, *J =* 2.3 Hz, 1H), 7.17 (d, *J =* 9.0 Hz, 1H), 3.40 (s, 2H), 3.28 (d, *J* = 4.6 Hz, 7H), 3.00 - 2.55 (m, 12H), 0.78 (d, *J* = 15.5 Hz, 4H). |
| 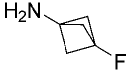 | 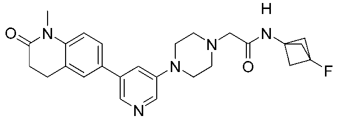 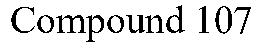 | LCMS (ESI) [M+H] ⁺ =464.1; ¹H NMR (400 MHz, DMSO-*d₆*) δ 8.47 (s, 1H), 8.29 (d, *J =* 1.7 Hz, 1H), 8.26 (d, *J* = 2.6 Hz, 1H), 7.64 - 7.61 (m, 2H), 7.51 - 7.48 (m, 1H), 7.19 - 7.15 (m, 1H), 3.34 - 3.30 (m, 4H), 3.29 (s, 3H), 2.99 (s, 2H), 2.97 - 2.92 (m, 2H), 2.62 - 2.56 (m, 6H), 2.33 (d, *J =* 2.2 Hz, 6H). |
| 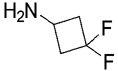 | 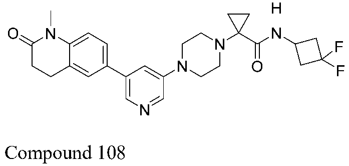 | LCMS (ESI) [M+H] ⁺ = 489.9; ¹H NMR(400 MHz, DMSO-*d₆*) δ 8.45 (s, 1H), 8.29 (d, *J =* 1.7 Hz, 1H), 8.26 (d, *J* = 2.7 Hz, 1H), 7.65 - 7.60 (m, 2H), 7.52 - 7.46 (m, 1H), 7.20 -7.15 (m, 1H), 3.37 - 3.33 (m, 4H), 3.29 (s, 3H), 2.98 - 2.91 (m, 2H), 2.64 - 2.55 (m, 6H), 2.33 (d, *J* = 2.2 Hz, 6H), 1.04 - 0.94 (m, 4H). |
| 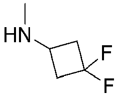 | 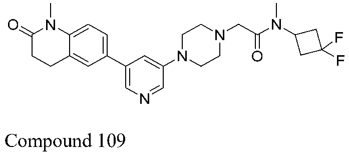 | LCMS (ESI) [M+H] ⁺ = 484.1; ¹H NMR(400 MHz, DMSO-*d₆*) δ 8.31 - 8.23 (m, 2H), 7.66 - 7.60 (m, 2H), 7.52 - 7.47 (m, 1H), 7.21 - 7.14 (m, 1H), 4.78 - 4.57 (m, 1H), 3.31 - 3.27 (m, 7H), 3.27 - 3.22 (m, 2H), 3.03 - 2.91 (m, 4H), 2.90 - 2.75 (m, 5H), 2.65 - 2.55 (m, 6H). |
| 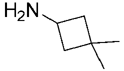 | 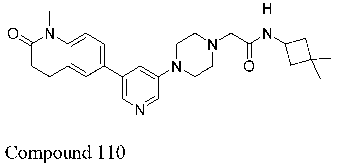 | LCMS (ESI) [M+H] ⁺ = 462.2; ¹H NMR(400 MHz, DMSO-*d₆*) δ 8.33 - 8.23 (m, 2H), 7.89 (d, *J =* 8.1 Hz, 1H), 7.66 - 7.61 (m, 2H), 7.54 - 7.47 (m, 1H), 7.21 - 7.14 (m, 1H), 4.31 - 4.19 (m, 1H), 3.37 - 3.32 (m, 4H), 3.29 (s, 3H), 2.99 - 2.89 (m, 4H), 2.63 - 2.56 (m, 6H), 2.06 - 1.95 (m, 2H), 1.83 - 1.73 (m, 2H), 1.10 (d, *J* = 11.1 Hz, 6H). |

### Example 50

### Preparation of N-(3-fluorobicyclo[1.1.1]pentan-1-yl)-2-(4-(6-(1-methyl-2-oxo-1,2,3,4-tetrahydroquinolin-6-yl)pyrazin-2-yl)piperazin-1-yl)acetamide (compound 111):

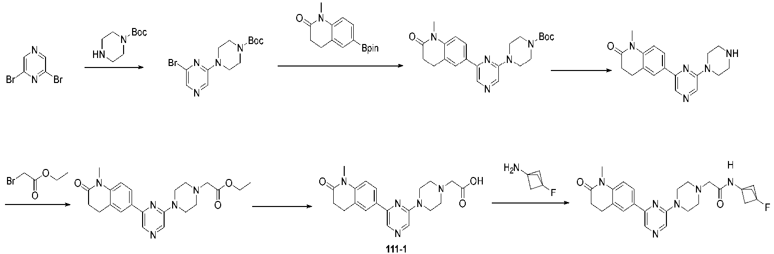

### Step 1: Synthesis of tert-butyl 4-(6-bromopyrazin-2-yl)piperazine-1-carboxylate:

With reference to step 1 of example 9, using 2,6-dibromopyrazine (15 g, 1.0 eq) and tert-butyl piperazine-1-carboxylate (11.74 g) as starting materials, the reaction was stirred at 90°C under a nitrogen atmosphere for 3 hours. The residue was purified by flash chromatography (silica gel, PE : EA = 3 : 1) to obtain the product (19 g). LCMS (ESI) [M+H]⁺ = 342.9.

### Step 2: Synthesis of tert-butyl 4-(6-(1-methyl-2-oxo-1,2,3,4-tetrahydroquinolin-6-yl)pyrazin-2-yl)piperazine-1-carboxylate:

With reference to step 2 of example 9, using the sample of preparation example 1 (15.5 g, 1.0 eq) and the product obtained in step 1 (18.53 g) as starting materials, the reaction was stirred overnight at 100°C under a nitrogen atmosphere . The residue was purified by flash chromatography (silica gel, PE : EA = 1 : 4) to obtain the product (22 g). LCMS (ESI) [M+H]⁺ = 424.

### Step 3: Synthesis of 1-methyl-6-(6-(piperazin-1-yl)pyrazin-2-yl)-3,4-dihydroquinolin-2(1H)-one:

With reference to step 2 of example 12, using the product obtained in step 2 (22 g, 1.0 eq) as the starting material, the reaction was stirred overnight at room temperature. The reaction solution was adjusted to pH 8 with aqueous sodium bicarbonate solution. The reaction solution was subjected to layer separation. The aqueous phase was extracted with dichloromethane (100 mL*3). The organic phases were combined, dried over anhydrous sodium sulphate and filtered, and the filtrate was concentrated to obtain the product (16 g). LCMS (ESI) [M+H]⁺ = 324.

### Step 4: Synthesis of ethyl 2-(4-(6-(1-methyl-2-oxo-1,2,3,4-tetrahydroquinolin-6-yl)pyrazin-2-yl)piperazin-1-yl)acetate:

With reference to step 1 of example 6, using the product obtained in step 3 (10.5 g, 1.0 eq) and ethyl bromoacetate (5.69 g) as starting materials, the reaction was stirred at room temperature for 2 hours. The reaction solvent was concentrated to dryness. To the residue were added water (300 mL) and ethyl acetate (600 mL). The reaction solution was subjected to layer separation, and the organic phase was collected. The solvent was subjected to rotary evaporation to dryness to obtain the product (10.5 g). LCMS (ESI) [M+H]⁺ = 410.

### Step 5: Synthesis of 2-(4-(6-(1-methyl-2-oxo-1,2,3,4-tetrahydroquinolin-6-yl)pyrazin-2-yl)piperazin-1-yl)acetic acid (intermediate 111-1):

The product obtained in step 4 (10.5 g, 1.0 eq) and lithium hydroxide (2.46 g) were added to tetrahydrofuran (100 mL) and water (20 mL). The mixture was stirred overnight at room temperature. The reaction solution was adjusted to acidic pH with dilute hydrochloric acid, and a yellow solid precipitated out. The mixture was filtered, and the filter cake was dried to obtain the product (9 g). LCMS (ESI) [M+H]⁺ = 382.33.

### Step 6:

With reference to step 3 of example 1, using the product obtained in step 5 (50 mg, 1.0 eq) and 3-fluorobicyclo[1.1.1]pentan-1-amine (13.26 mg) as starting materials, the reaction was stirred at room temperature for 1 hour. The reaction solution was purified by reversed-phase chromatography (0.1% aqueous ammonia, acetonitrile) to obtain compound 111 (27.26 mg). LCMS (ESI) [M+H] ⁺ = 465.3, ¹HNMR(400 MHz, DMSO-*d₆*) δ 8.48 (s, 1H), 8.44 (s, 1H), 8.23 (s, 1H), 8.01 - 7.92 (m, 2H), 7.18 (d, *J =* 8.5 Hz, 1H), 3.73 - 3.63 (m, 4H), 3.29 (s, 3H), 2.99 (s, 2H), 2.98 - 2.92 (m, 2H), 2.62 - 2.54 (m, 6H), 2.33 (d, *J* = 2.2 Hz, 6H).

### Example 51

### Preparation of 2-(4-(5-(6-chloro-1-oxo-3,4-dihydroisoquinolin-2(1H)-yl)pyridin-3-yl)piperazin-1-yl)-N-isopropylacetamide (compound 112):

### Step 1: Synthesis of ethyl 2-(4-(5-(6-chloro-1-oxo-3,4-dihydroisoquinolin-2(1H)-yl)pyridin-3-yl)piperazin-1-yl)acetate:

Ethyl 2-(4-(5-bromopyridin-3-yl)piperazin-1-yl)acetate (150 mg, 1.0 eq), 6-chloro-3,4-dihydroisoquinolin-1(2H)-one (124.51 mg), N1,N2-dimethyl-1,2-cyclohexanediamine (65.01 mg), caesium carbonate (297.82 mg) and cuprous iodide (43.52 mg) were dissolved in 1,4-dioxane (10 mL), and the mixture was stirred at 130°C for 16 hours. The reaction solution was diluted with water (40 mL), and extracted with ethyl acetate (25 mL*3). The organic phases were combined, dried over anhydrous sodium sulphate and filtered, and the filtrate was concentrated. The residue was purified by flash chromatography (silica gel, PE: THF = 5 : 3) to obtain the product (130 mg). LCMS (ESI) [M+H]⁺ =429.1.

### Step 2: Synthesis of 2-(4-(5-(6-chloro-1-oxo-3,4-dihydroisoquinolin-2(1H)-yl)pyridin-3-yl)piperazin-1-yl)acetic acid:

With reference to step 5 of example 35, using the product obtained in step 1 (130 mg, 0.30 mmol, 1.0 eq) as the starting material, the reaction was stirred at 50°C for 2 hours. The residue was purified by reversed-phase chromatography (C18, 0.1% NH₄HCO₃ in H₂O/ACN) to obtain the target compound (90 mg). LCMS (ESI) [M+H]⁺ =401.1; ¹H NMR (400 MHz, DMSO-d₆) δ 8.18 (d, *J* = 2.5 Hz, 1H), 8.06 (d, *J =* 1.9 Hz, 1H), 7.93 (d, *J =* 8.3 Hz, 1H), 7.53 - 7.50 (m, 1H), 7.48 - 7.44 (m, 1H), 7.39 - 7.34 (m, 1H), 3.96 (t, *J =* 6.5 Hz, 2H), 3.27 - 3.23 (m, 4H), 3.19 (s, 2H), 3.15 (t, *J =* 6.4 Hz, 2H), 2.72 - 2.66 (m, 4H).

### Step 3: Synthesis of 2-(4-(5-(6-chloro-1-oxo-3,4-dihydroisoquinolin-2(1H)-yl)pyridin-3-yl)piperazin-1-yl)-N-isopropylacetamide:

With reference to step 3 of example 1, using the product obtained in step 2 (40 mg, 1.0 eq) and propan-2-amine (5.9 mg) as starting materials, the reaction was stirred at room temperature for 2 hours. The residue was purified by reversed-phase chromatography (C18, 0.1% NH₄HCO₃ in H₂O/ACN) to obtain compound 112 (37.98 mg). LCMS (ESI) [M+H]⁺ =441.9; ¹H NMR (400 MHz, DMSO-d₆) δ 8.19 (d, *J =* 2.6 Hz, 1H), 8.06 (d, *J =* 1.9 Hz, 1H), 7.93 (d, *J=* 8.4 Hz, 1H), 7.55 - 7.50 (m, 2H), 7.48 - 7.44 (m, 1H), 7.39 - 7.36 (m, 1H), 3.96 (t, *J*= 6.5 Hz, 2H), 3.93 - 3.87 (m, 1H), 3.29 - 3.24 (m, 4H), 3.15 (t, *J =* 6.4 Hz, 2H), 2.95 (s, 2H), 2.61 - 2.57 (m, 4H), 1.07 (d, *J =* 6.6 Hz, 6H).

### Examples 52-57

With reference to step 3 of example 1, using the sample of preparation example 3 (50 mg, 1.0 eq) or intermediate 70-1 (50 mg, 1.0 eq) with the starting materials shown in the table below, compounds 113-118 were prepared respectively.

| Starting material | Compound | Structural confirmation data |
|---|---|---|
| 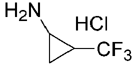 | 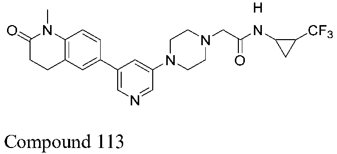 | LCMS (ESI) [M+H] ⁺ =488.1; ¹H NMR (400 MHz, DMSO-d₆) δ 8.32 - 8.25 (m, 2H), 8.09 (d, *J* = 4.6 Hz, 1H), 7.65 - 7.61 (m, 2H), 7.51 - 7.48 (m, 1H), 7.20 - 7.15 (m, 1H), 3.35 - 3.33 (m, 2H), 3.32 - 3.30 (m, 2H), 3.29 (s, 3H), 3.10 - 3.04 (m, 1H), 3.00 (d, *J =* 1.8 Hz, 2H), 2.97 - 2.92 (m, 2H), 2.62 - 2.56 (m, 6H), 2.06 - 1.96 (m, 1H), 1.22 - 1.11 (m, 2H). |
| 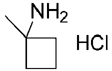 | 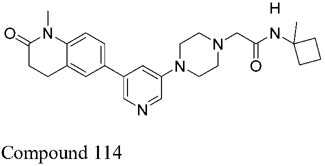 | LCMS (ESI) [M+H] ⁺ =488.1; ¹H NMR (400 MHz, DMSO-*d₆*) δ 8.32 - 8.24 (m, 2H), 8.18 (s, 1H), 7.68 (s, 1H), 7.65 - 7.60 (m, 2H), 7.53 - 7.48 (m, 1H), 7.20 - 7.14 (m, 1H), 3.35 - 3.30 (m, 4H), 3.29 (s, 3H), 2.97 - 2.91 (m, 4H), 2.65 - 2.56 (m, 6H), 2.30 - 2.21 (m, 2H), 1.95 - 1.87 (m, 2H), 1.80 - 1.71 (m, 2H), 1.38 (s, 3H). |
| 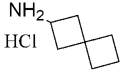 | 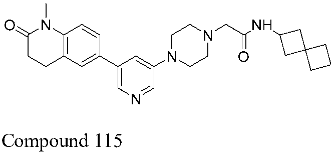 | LCMS (ESI) [M+H]⁺ =474.1; ¹H NMR (400 MHz, DMSO-*d*₆) δ 8.28 (dd, J = 11.3, 2.3 Hz, 2H), 7.87 (d, J = 8.0 Hz, 1H), 7.63 (dq, J = 4.7, 2.3 Hz, 2H), 7.50 (dd, J = 2.8, 1.9 Hz, 1H), 7.17 (d, J = 9.1 Hz, 1H), 4.17 - 4.02 (m, 1H), 3.34 (s, 2H), 3.29 (s, 5H), 2.99 - 2.90 (m, 4H), 2.58 (dt, J = 6.9, 4.2 Hz, 6H), 2.29 - 2.20 (m, 2H), 2.03 - 1.97 (m, 2H), 1.95 - 1.85 (m, 4H), 1.83 - 1.71 (m, 2H). |
| 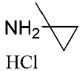 | 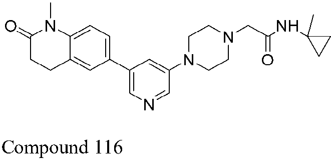 | LCMS (ESI) [M+H]⁺ =434.1; ¹H NMR (400 MHz, DMSO-d₆) δ 8.27 (dd, J = 12.6, 2.3 Hz, 2H), 7.92 (s, 1H), 7.63 (dq, J = 4.8, 2.3 Hz, 2H), 7.49 (dd, J = 2.8, 1.9 Hz, 1H), 7.17 (d, J *=* 9.0 Hz, 1H), 3.30 (d, J = 11.4 Hz, 7H), 2.98 - 2.88 (m, 4H), 2.62 - 2.54 (m, 6H), 1.28 (s, 3H), 0.69 - 0.48 (m, 4H). |
| 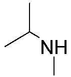 | 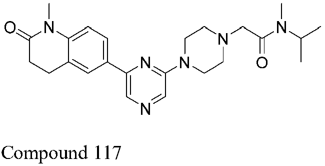 | LCMS (ESI) [M+H]⁺ =437.1; ¹H NMR (400 MHz, DMSO-*d*₆) δ 8.44 (d, J = 2.8 Hz, 1H), 8.23 (d, J = 1.1 Hz, 1H), 8.03 - 7.92 (m, 2H), 7.18 (d, J = 8.5 Hz, 1H), 4.35 (p, J = 6.6 Hz, 1H), 3.72 - 3.58 (m, 4H), 3.29 (s, 3H), 3.24 (s, 1H), 3.19 (s, 1H), 2.95 (d, J = 7.8 Hz, 2H), 2.86 (s, 1H), 2.69 - 2.52 (m, 8H), 1.15 (d, J *=* 6.6 Hz, 3H), 1.03 (d, J = 6.8 Hz, 3H). |
| 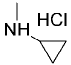 | 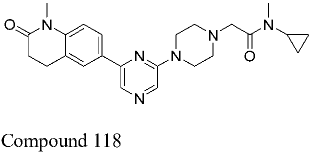 | LCMS (ESI) [M+H]⁺ =435.1; ¹H NMR (400 MHz, DMSO-d₆) δ 8.43 (s, 1H), 8.23 (s, 1H), 8.03 - 7.89 (m, 2H), 7.19 (d, J *=* 8.5 Hz, 1H), 3.65 (t, J = 5.0 Hz, 4H), 3.42 (s, 2H), 3.29 (s, 3H), 3.02 - 2.77 (m, 6H), 2.72 - 2.55 (m, 6H), 0.87 - 0.51 (m, 4H). |

### Example 58

### Preparation of 2-(4-(5-(6-chloro-1,1-dimethyl-3-oxoisoindolin-2-yl)pyridin-3-yl)piperazin-1-yl)-N-isopropylacetamide (compound 119):

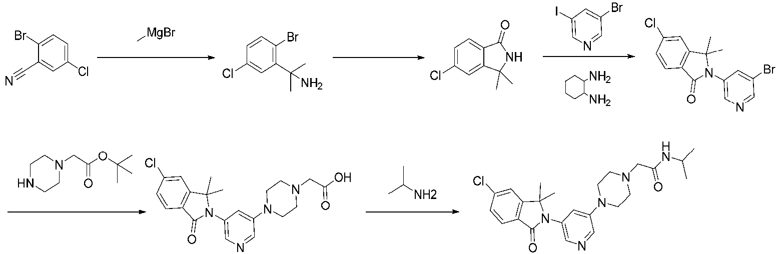

### Step 1: Synthesis of 2-(2-bromo-5-chlorophenyl)propan-2-amine:

2-Bromo-5-chlorobenzonitrile (10 g, 1.0 eq) was dissolved in tetrahydrofuran (200 mL). At 0°C, methylmagnesium bromide (77 mL) was added dropwise under a nitrogen atmosphere. After the mixture was stirred for 2 hours, tetraisopropyl titanate (13.13 g) was added, and the mixture was stirred at room temperature for 16 hours. The reaction solution was adjusted to pH 3 to 4 with 3 M hydrochloric acid, and extracted with ethyl acetate (400 mL). The aqueous phase was adjusted to pH 9 to 10 with sodium hydroxide solution (1 M), and extracted with ethyl acetate (300 mL*3). The organic phases were combined, dried over anhydrous sodium sulphate and filtered, and the filtrate was concentrated to obtain the product (3 g). LCMS (ESI) [M+H] ⁺ = 248.0; ¹H NMR(400 MHz, CDCl₃) δ 7.53 (d, *J =* 2.6 Hz, 1H), 7.45 - 7.42 (m, 1H), 7.01 - 6.96 (m, 1H), 1.85 - 1.76 (m, 2H), 1.57 (s, 6H).

### Step 2: Synthesis of 5-chloro-3,3-dimethylisoindolin-1-one:

The product obtained in step 1 (2.5 g, 1.0 eq) was dissolved in N,N-dimethylformamide (20 mL), and 1,1'-bis(diphenylphosphino)ferrocene dichloropalladium (II) (369 mg) and ethylbis(propan-2-yl)amine (3.9 mL) were added. The mixture was stirred at 130°C under carbon monoxide atmosphere for 16 hours. To the reaction solution was added water (60 mL), and the mixture was extracted with ethyl acetate (60 mL*4). The organic phases were combined, dried over anhydrous sodium sulphate and filtered, and the filtrate was concentrated. The residue was purified by flash chromatography (silica gel, PE : EA = 1 : 1) to obtain the product (600 mg). LCMS (ESI) [M+ACN+H] ⁺ = 237.1; ¹H NMR(400 MHz, CDCl₃) δ 7.73 (s, 1H), 7.45 - 7.40 (m, 1H), 7.40 - 7.37 (m, 1H), 7.29 - 7.24 (m, 1H), 1.56 (s, 6H).

### Step 3: Synthesis of 2-(5-bromopyridin-3-yl)-5-chloro-3,3-dimethylisoindolin-1-one:

The product obtained in step 2 (100 mg, 1.0 eq) and 3-bromo-5-iodopyridine (217.65 mg) were dissolved in dioxane (1 mL). Tripotassium phosphate (216.99 mg), cyclohexane-1,2-diamine (35.02 mg) and cuprous iodide (58.41 mg) were added. Under a nitrogen atmosphere, the mixture was stirred at 120°C for 3 hours. To the reaction solution was added water (20 mL), and the mixture was extracted with ethyl acetate (20 mL*3). The organic phases were combined, dried over anhydrous sodium sulphate and filtered, and the filtrate was concentrated. The residue was purified by flash chromatography (silica gel, PE : EA = 4 : 1) to obtain the product (90 mg). LCMS (ESI) [M+H] ⁺ = 350.8; ¹H NMR (400 MHz, DMSO-*d₆*) δ 8.82 (d, *J* = 2.1 Hz, 1H), 8.59 (d, *J* = 2.0 Hz, 1H), 8.24 - 8.19 (m, 1H), 8.02 - 8.00 (m, 1H), 7.80 - 7.77 (m, 1H), 7.63 - 7.59 (m, 1H), 1.52 (s, 6H).

### Step 4: Synthesis of 2-(4-(5-(6-chloro-1,1-dimethyl-3-oxoisoindolin-2-yl)pyridin-3-yl)piperazin-1-yl)acetic acid:

To a mixture of the product obtained in step 3 (90 mg), tert-butyl 2-(piperazin-1-yl)acetate (76.89 mg), sodium tert-butoxide (99.95 mg), triethylamine (105.24 mg), [1-(2-diphenylphosphinonaphthalen-1-yl)naphthalen-2-yl]-diphenylphosphine (32.38 mg) and tris(1,5-diphenylpenta-1,4-dien-3-one)dipalladium (23.81 mg) was added toluene (2 mL). Under a nitrogen atmosphere, the mixture was stirred at 110°C for 16 hours. The reaction solution was directly concentrated, and the residue was purified by reversed-phase chromatography (acetonitrile/0.1% aqueous formic acid solution =50%) to obtain the product (30 mg). LCMS (ESI) [M+H] ⁺ = 415.0.

### Step 5: Synthesis of 2-(4-(5-(6-chloro-1,1-dimethyl-3-oxoisoindolin-2-yl)pyridin-3-yl)piperazin-1-yl)-N-isopropylacetamide:

With reference to step 3 of example 1, using the product obtained in step 4 (30 mg, 1.0 eq) and propan-2-amine (4.27 mg) as starting materials, the reaction was stirred at room temperature for 1 hour. The residue was purified by reversed-phase chromatography (0.1% aqueous ammonia, acetonitrile) to obtain compound 119 (8.45 mg). LCMS (ESI) [M+H] ⁺ = 455.8; ¹H NMR(400 MHz, DMSO-*d₆*) δ 8.37 (d, *J =* 2.6 Hz, 1H), 7.98 (d, *J =* 1.6 Hz, 1H), 7.91 (d, *J =* 1.7 Hz, 1H), 7.75 (d, *J =* 8.1 Hz, 1H), 7.61 - 7.57 (m, 1H), 7.52 (d, *J =* 8.1 Hz, 1H), 7.30 - 7.23 (m, 1H), 3.97 - 3.84 (m, 1H), 3.31 - 3.26 (m, 4H), 2.95 (s, 2H), 2.61 - 2.55 (m, 4H), 1.49 (s, 6H), 1.07 (d, *J* = 6.6 Hz, 6H).

### Examples 59-68

With reference to step 3 of example 1, using the sample of preparation example 3 (50 mg, 1.0 eq) or intermediate 111-1 (100 mg, 1.0 eq) with the starting materials shown in the table below, compounds 120-129 were prepared respectively.

| Starting material | Compound | Structural confirmation data |
|---|---|---|
| 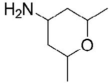 | 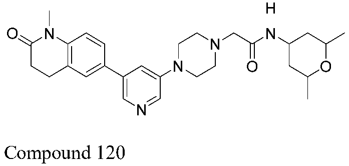 | LCMS (ESI) [M+H] ⁺ = 491.9; ¹H NMR(400 MHz, DMSO-*d₆*) δ 8.33 - 8.23 (m, 2H), 7.83 - 7.59 (m, 3H), 7.54 - 7.47 (m, 1H), 7.21 - 7.14 (m, 1H), 4.08 - 3.79 (m, 1H), 3.66 - 3.44 (m, 2H), 3.35 - 3.31 (m, 4H), 3.29 (s, 3H), 3.07 - 2.97 (m, 2H), 2.97 - 2.91 (m, 2H), 2.68 - 2.56 (m, 6H), 1.76 - 1.58 (m, 2H), 1.39 - 1.11 (m, 1H), 1.11 - 1.02 (m, 7H). |
| 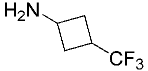 | 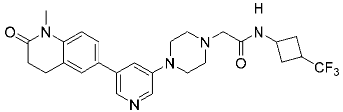 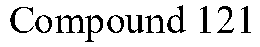 | LCMS (ESI) [M+H] ⁺ = 501.8; ¹H NMR(400 MHz, DMSO-*d₆*) δ 8.31 (s, 1H), 8.30 - 8.25 (m, 2H), 8.23 - 8.15 (m, 1H), 7.66 - 7.60 (m, 2H), 7.50 (s, 1H), 7.21 - 7.13 (m, 1H), 4.36 - 4.19 (m, 1H), 3.37 - 3.31 (m, 4H), 3.29 (s, 3H), 3.00 - 2.92 (m, 4H), 2.92 - 2.80 (m, 1H), 2.65 - 2.56 (m, 6H), 2.41 - 2.05 (m, 4H). |
| 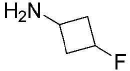 | 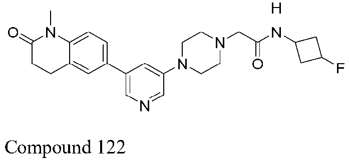 | LCMS (ESI) [M+H] ⁺ = 451.8; ¹H NMR(400 MHz, DMSO-*d₆*) δ 8.32 - 8.26 (m, 2H), 8.14 - 8.04 (m, 1H), 7.65 - 7.60 (m, 2H), 7.54 - 7.48 (m, 1H), 7.22 - 7.14 (m, 1H), 5.33 - 4.69 (m, 1H), 4.46 - 3.79 (m, 1H), 3.31 - 3.27 (m, 7H), 3.01 - 2.92 (m, 4H), 2.63 - 2.57 (m, 6H), 2.48 - 2.32 (m, 3H), 2.32 - 2.10 (m, 1H). |
| 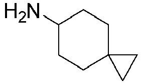 | 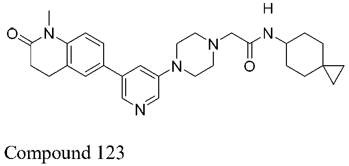 | LCMS (ESI) (M+H)⁺ =488.2; ¹H NMR (400 MHz, DMSO-*d*₆) δ 8.28 (dd, *J* = 10.5, 2.2 Hz, 2H), 7.65 - 7.61 (m, 2H), 7.59 (d, *J =* 8.1 Hz, 1H), 7.50 (d, *J* = 2.2 Hz, 1H), 7.20 - 7.15 (m, 1H), 3.73 - 3.61 (m, 1H), 3.36 - 3.32 (m, 4H), 3.29 (s, 3H), 3.00 - 2.91 (m, 4H), 2.64 - 2.56 (m, 6H), 1.75 - 1.61 (m, 4H), 1.40 (dt, *J* = 12.0, 10.4 Hz, 2H), 0.96 (d, *J* = 12.3 Hz, 2H), 0.31 - 0.16 (m, 4H). |
| 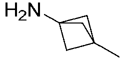 | 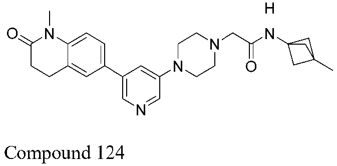 | LCMS (ESI) (M+H)⁺ =460.2.¹H NMR (400 MHz, MeOD) δ 8.20 (dd, *J =* 11.0, 2.0 Hz, 2H), 7.60 - 7.51 (m, 3H), 7.23 (d, *J* = 8.4 Hz, 1H), 3.39 (s, 3H), 3.39 - 3.35 (m, 4H), 3.02 (s, 2H), 3.02 - 2.97 (m, 2H), 2.74 - 2.68 (m, 4H), 2.68 - 2.62 (m, 2H), 1.95 (s, 6H), 1.22 (s, 3H). |
| 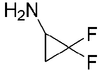 | 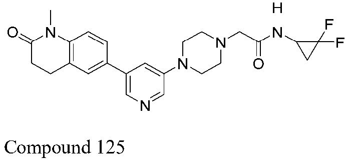 | LCMS (ESI) (M+H) ⁺ =456.1; ¹H NMR (400 MHz, DMSO-*d₆*) δ 8.30 - 8.24 (m, 2H), 8.23 - 8.16 (m, 1H), 7.65 - 7.61 (m, 2H), 7.52 - 7.48 (m, 1H), 7.20 - 7.15 (m, 1H), 3.35 - 3.33 (m, 4H), 3.29 (s, 3H), 3.06 (s, 2H), 2.98 - 2.92 (m, 2H), 2.64 - 2.56 (m, 7H), 1.92 - 1.83 (m, 1H), 1.67 - 1.58 (m, 1H). |
| 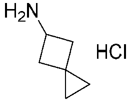 | 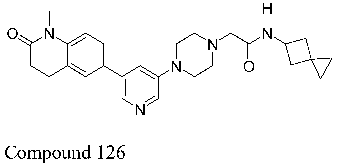 | LCMS (ESI) (M+H) ⁺ =460.1; ¹H NMR (400 MHz, DMSO-*d₆*) δ 8.30 - 8.25 (m, 2H), 8.05 (d, *J* = 7.8 Hz, 1H), 7.65 - 7.61 (m, 2H), 7.52 - 7.49 (m, 1H), 7.20 - 7.15 (m, 1H), 4.46 - 4.37 (m, 1H), 3.36 - 3.33 (m, 4H), 3.29 (s, 3H), 2.99 - 2.92 (m, 4H), 2.63 - 2.57 (m, 6H), 2.28 - 2.23 (m, 2H), 2.20 - 2.14 (m, 2H), 0.48 - 0.43 (m, 2H), 0.40 - 0.35 (m, 2H). |
| 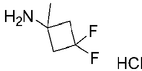 | 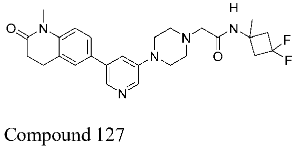 | LCMS (ESI) (M+H)⁺ =483.8; ¹H NMR (400 MHz, MeOD-*d*₄₎ δ 8.21 (d, *J=* 8.9 Hz, 2H), 7.58 (dd, *J* = 8.4, 2.1 Hz, 2H), 7.54 (s, 1H), 7.23 (d, *J =* 8.4 Hz, 1H), 3.41 - 3.37 (m, 7H), 3.07 (s, 2H), 3.03 - 2.98 (m, 2H), 2.96 - 2.85 (m, 2H), 2.74 - 2.70 (m, 4H), 2.68 - 2.60 (m, 4H), 1.53 (s, 3H). |
| 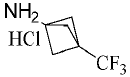 | 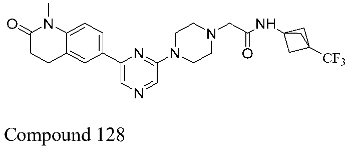 | LCMS (ESI) [M+H]⁺ =515.0; ¹H NMR (400 MHz, DMSO-d₆) δ 8.56 (s, 1H), 8.44 (s, 1H), 8.24 (s, 1H), 8.04 - 7.90 (m, 2H), 7.18 (d, *J* = 8.5 Hz, 1H), 3.69 (t, *J =* 4.9 Hz, 4H), 3.29 (s, 3H), 2.97 (d, *J* = 10.3 Hz, 4H), 2.65 - 2.52 (m, 6H), 2.25 (s, 6H). |
| 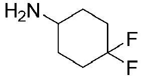 | 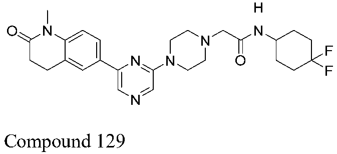 | LCMS (ESI) [M+H]⁺ = 499.1; ¹H NMR (400 MHz, Chloroform-d) δ 8.35 (s, 1H), 8.07 (s, 1H), 7.89 (dd, *J* = 8.5, 2.1 Hz, 1H), 7.81 (s, 1H), 7.07 (d, *J* = 8.5 Hz, 2H), 4.01 - 3.88 (m, 1H), 3.72 (t, *J =* 5.0 Hz, 4H), 3.40 (s, 3H), 3.09 (s, 2H), 3.04 - 2.94 (m, 2H), 2.73 - 2.65 (m, 4H), 2.16 - 1.80 (m, 6H), 1.28 (d, *J* = 18.7 Hz, 4H). |

### Example 69

### Preparation of 2-(4-(5-(7-fluoro-1-methyl-2-oxo-1,2,3,4-tetrahydroquinolin-6-yl)pyridin-3-yl)piperazin-1-yl)-N-isopropylacetamide (compound 130):

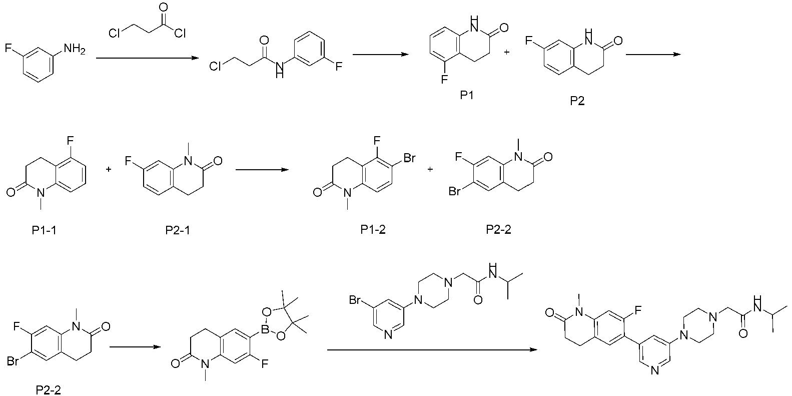

### Step 1: Synthesis of 3-chloro-N-(3-fluorophenyl)propanamide:

Under a nitrogen atmosphere, pyridine (14.24 g) and 3-fluoroaniline (10 g, 1.0 eq) were dissolved in acetone (240 mL) at 0°C. 3-Chloropropionyl chloride (12.57 g) was slowly added to the system. After the dropwise addition was completed, the mixture was stirred at 50°C for 16 hours. The reaction solution was cooled to room temperature. The reaction solution was concentrated, diluted with water (100 mL), adjusted to pH 7 with 1 M hydrochloric acid, and extracted with ethyl acetate (80 mL*3). The organic phases were combined, dried over anhydrous sodium sulphate and filtered, and the filtrate was concentrated to obtain the product (15 g). LCMS (ESI) [M+H] ⁺ = 202.0, ¹H NMR (400 MHz, CDCl₃) δ 7.56 - 7.40 (m, 2H), 7.30 - 7.24 (m, 1H), 7.19 - 7.11 (m, 1H), 6.83 (td, *J* = 8.2, 1.9 Hz, 1H), 3.88 (t, *J =* 6.4 Hz, 2H), 2.82 (t, *J =* 6.4 Hz, 2H).

### Step 2: Synthesis of 5-fluoro-3,4-dihydroquinolin-2(1H)-one (P1) and 7-fluoro-3,4-dihydroquinolin-2(1H)-one (P2):

Aluminium trichloride (3306 mg) was added to the product obtained in step 1 (5 g, 1.0 eq), and the mixture was stirred at 125°C for 5 hours. The reaction solution was cooled to room temperature. The reaction was quenched by adding ice water (50 mL) slowly at 0°C, and extracted with dichloromethane (30 mL*3). The organic phases were combined, dried over anhydrous sodium sulphate and filtered, and the filtrate was concentrated to obtain a mixture of intermediate compounds P1 and P2 (4.0 g). LCMS (ESI) (M+H) ⁺ = 166.1.

### Step 3: Synthesis of 5-fluoro-1-methyl-3,4-dihydroquinolin-2(1H)-one (P1-1) and 7-fluoro-1-methyl-3,4-dihydroquinolin-2(1H)-one (P2-1):

At 0°C, a solution of a mixture of intermediate compound P1 (1.83 g) and intermediate compound P2 (9.17 g, 1.0 eq) in dimethylformamide (200 mL) was added to a solution of sodium hydride (2.55 g) in dimethylformamide (200 mL). The mixture was stirred at 0°C for 10 minutes. Iodomethane (10.25 g) was slowly added to the system, and the mixture was stirred at room temperature for 1 hour. The reaction solution was quenched by adding saturated ammonium chloride solution (300 mL), diluted with water (300 mL), and extracted with ethyl acetate (300 mL*3). The organic phases were combined, dried over anhydrous sodium sulphate and filtered, and the filtrate was concentrated. The residue was purified by flash chromatography (silica gel, PE : EA = 87 : 13) to obtain a mixture of products P1-1 and P2-1 (12.19 g, crude). NMR showed a ratio of P1-1/P2-1=1/5. LCMS (ESI) [M+H] ⁺ =180.0.

### Step 4: Synthesis of 6-bromo-5-fluoro-1-methyl-3,4-dihydroquinolin-2(1H)-one (P1-2) and 6-bromo-7-fluoro-1-methyl-3,4-dihydroquinolin-2(1H)-one (P2-2):

1-Bromopyrrolidine-2,5-dione (15.27 g) was added to a solution of a mixture of P1-1 (1.71 g, crude) and P2-1 (10.25 g, crude) in dimethylformamide (200 mL), and the mixture was stirred at 25°C for 16 hours. Water (500 mL) was added to the reaction solution, and the mixture was extracted with ethyl acetate (300 mL*3). The organic phases were combined, dried over anhydrous sodium sulphate and filtered, and the filtrate was concentrated. The residue was purified by flash chromatography (silica gel, PE : EA = 4 : 1) to obtain a mixture of the products P1-2 and P2-2 (15.66 g). NMR showed a ratio of P1-2/P2-2=1/7. 1 g of the mixture was subjected to SFC (supercritical fluid chromatography) to obtain P1-2 (90 mg) and P2-2 (860 mg). Compound P1-2: LCMS (ESI) [M+H] ⁺ =259.9. ¹H NMR (400 MHz, CDCl₃) δ 7.44 - 7.38 (m, 1H), 6.68 (dd, *J =* 8.8, 0.9 Hz, 1H), 3.34 (s, 3H), 3.00 - 2.94 (m, 2H), 2.69 - 2.62 (m, 2H). Compound P2-2: LCMS (ESI) [M+H] ⁺ =259.9. ¹H NMR (400 MHz, CDCl₃) δ 7.32 (d, *J* = 7.3 Hz, 1H), 6.76 (d, *J =* 10.2 Hz, 1H), 3.31 (s, 3H), 2.90 - 2.85 (m, 2H), 2.65 (dd, *J =* 8.5, 6.2 Hz, 2H).

### Step 5: Synthesis of 7-fluoro-1-methyl-6-(4,4,5,5-tetramethyl-1,3,2-dioxaborolan-2-yl)-3,4-dihydroquinolin-2(1H)-one:

Bis[(cyclopenta-1,3-dien-1-yl)diphenylphosphane]dichloropalladium iron (114 mg) was added to a solution of compound P2-2 (400 mg, 1.0 eq), 4,4,5,5-tetramethyl-2-(4,4,5,5-tetramethyl-1,3,2-dioxaborolan-2-yl)-1,3,2-dioxaborolane (787 mg) and potassium acetate (456 mg) in 1,4-dioxane (10 mL). Under a nitrogen atmosphere, the mixture was stirred at 90°C for 16 hours. To the reaction solution was added water (20 mL), and the mixture was extracted with ethyl acetate (20 mL*3). The organic phases were combined, dried over anhydrous sodium sulphate and filtered, and the filtrate was concentrated. The residue was purified by reversed-phase chromatography (C18, 0.1% aqueous ammonia solution/acetonitrile =80%/20%) to obtain the product (90 mg). LCMS (ESI) [M+H] ⁺ =305.1; LCMS (ESI) (boric acid) [M+H] ⁺ =224.1.

### Step 6: Synthesis of 2-(4-(5-(7-fluoro-1-methyl-2-oxo-1,2,3,4-tetrahydroquinolin-6-yl)pyridin-3-yl)piperazin-1-yl)-N-isopropylacetamide:

Under a nitrogen atmosphere, bis[(cyclopenta-1,3-dien-1-yl)diphenylphosphane]dichloropalladium iron (15 mg) was added to a solution of 2-(4-(5-bromopyridin-3-yl)piperazin-1-yl)-N-isopropylacetamide (70 mg, 1.0 eq), the product obtained in step 5 (75 mg) and potassium phosphate (131 mg) in 1,4-dioxane (5 mL) and water (1 mL), and the mixture was stirred at 80°C for 1 hour. Water (10 mL) was added to the reaction solution, and the mixture was extracted with ethyl acetate (10 mL*3). The organic phases were combined, dried over anhydrous sodium sulphate and filtered, and the filtrate was concentrated. The residue was purified by prep-HPLC (C18, 0.1% NH₃.H₂O in H₂O/ACN) to obtain compound 130 (47.03 mg). LCMS (ESI) [M+H] ⁺ =439.9; ¹H NMR (400 MHz, DMSO-*d₆*) δ 8.30 (d, *J* = 2.7 Hz, 1H), 8.15 (t, *J =* 1.8 Hz, 1H), 7.50 (dd, *J =* 17.8, 8.3 Hz, 2H), 7.42 - 7.39 (m, 1H), 7.11 (d, *J =* 12.8 Hz, 1H), 3.95 - 3.86 (m, 1H), 3.32 - 3.28 (m, 4H), 3.27 (s, 3H), 2.96 (s, 2H), 2.91 (t, *J =* 7.4 Hz, 2H), 2.62 - 2.56 (m, 6H), 1.08 (d, *J =* 6.6 Hz, 6H).

### Examples 70-73

With reference to step 3 of example 1, using the sample of preparation example 3 (50 mg, 1.0 eq) or intermediate 111-1 (100 mg, 1.0 eq) with the starting materials shown in the table below, compounds 131-134 were prepared respectively.

| Starting material | Compound | Structural confirmation data |
|---|---|---|
| 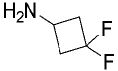 | 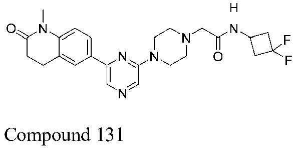 | LCMS (ESI) [M+H]⁺ = 471; ¹H NMR (400 MHz, Chloroform-*d*) δ 8.35 (s, 1H), 8.08 (s, 1H), 7.90 (dd, *J =* 8.5, 2.1 Hz, 1H), 7.81 (d, *J* = 2.1 Hz, 1H), 7.37 (d, *J =* 7.3 Hz, 1H), 7.07 (d, *J =* 8.5 Hz, 1H), 4.34 (s, 1H), 3.85 - 3.68 (m, 4H), 3.40 (s, 3H), 3.10 (s, 2H), 3.09 - 2.96 (m, 4H), 2.71 (t, *J =* 5.4 Hz, 6H), 2.58 - 2.41 (m, 2H). |
| 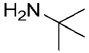 | 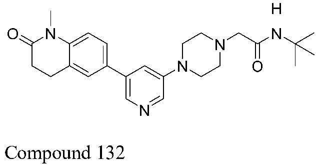 | LCMS (ESI) [M+H] ⁺ = 436.7; ¹H NMR(400 MHz, DMSO-*d₆*) δ 8.30 (d, *J =* 1.7 Hz, 1H), 8.27 (d*, J = 2.7* Hz, 1H), 7.67 - 7.60 (m, 2H), 7.54 - 7.48 (m, 1H), 7.23 (s, 1H), 7.20 - 7.14 (m, 1H), 3.32 - 3.30 (m, 4H), 3.29 (s, 3H), 2.99 - 2.90 (m, 4H), 2.65 - 2.56 (m, 6H), 1.29 (s, 9H). |
| 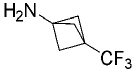 | 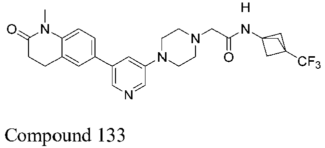 | LCMS (ESI) [M+H] ⁺ = 514.0; ¹H NMR(400 MHz, DMSO-*d₆*) δ 8.54 (s, 1H), 8.29 (d, *J =* 1.7 Hz, 1H), 8.26 (d, *J* = 2.6 Hz, 1H), 7.66 - 7.60 (m, 2H), 7.53 - 7.47 (m, 1H), 7.21 - 7.14 (m, 1H), 3.36 - 3.32 (m, 4H), 3.29 (s, 3H), 2.98 (s, 2H), 2.97 - 2.91 (m, 2H), 2.64 - 2.56 (m, 6H), 2.25 (s, 6H). |
| 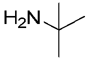 | 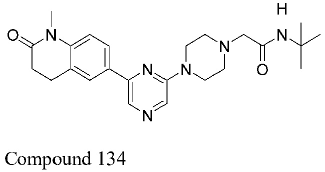 | LCMS (ESI) [M+H]⁺ =437.4; ¹H NMR (400 MHz, DMSO-*d₆*) δ 8.44 (s, 1H), 8.24 (s, 1H), 8.00 - 7.94 (m, 2H), 7.24 (s, 1H), 7.18 (d, J = 8.5 Hz, 1H), 3.70 - 3.65 (m, 4H), 3.29 (s, 3H), 2.98 - 2.93 (m, 2H), 2.92 (s, 2H), 2.61 - 2.57 (m, 6H), 1.29 (s, 9H). |

### Example 74

### Preparation of 2-(4-(5-(5-fluoro-1-methyl-2-oxo-1,2,3,4-tetrahydroquinolin-6-yl)pyridin-3-yl)piperazin-1-yl)-N-isopropylacetamide (compound 135):

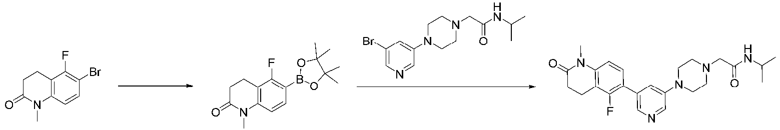

### Step 1: Synthesis of 5-fluoro-1-methyl-6-(4,4,5,5-tetramethyl-1,3,2-dioxaborolan-2-yl)-3,4-dihydroquinolin-2(1H)-one:

6-Bromo-5-fluoro-1-methyl-3,4-dihydroquinolin-2(1H)-one (60 mg, 1.0 eq), bis(pinacolato)diboron (118.07 mg), potassium acetate (68.45 mg), and 1,1'-bis(diphenylphosphino)ferrocene dichloropalladium (17.06 mg) were dissolved in 1,4-dioxane (3 mL). Under a nitrogen atmosphere, the mixture was stirred at 90°C for 16 hours. The reaction solution was cooled. Water (10 mL) was added to the reaction solution, and the mixture was extracted with ethyl acetate (15 mL*3). The organic phase was dried over anhydrous sodium sulphate and filtered, and the filtrate was concentrated. The residue was purified by flash chromatography (silica gel, PE : EA = 7 : 1) to obtain the target compound crude, which was further purified by reversed-phase chromatography (0.1% aqueous ammonia solution/acetonitrile) to obtain the product (40 mg). LCMS (ESI) (M+H)⁺ =224.1 (Molecular weight of boric acid).

### Step 2:

With reference to step 6 of example 69, using the product obtained in step 1 (30 mg, 1.0 eq) and 2-(4-(5-bromopyridin-3-yl)piperazin-1-yl)-N-isopropylacetamide (33.55 mg) as starting materials, the reaction was stirred at 80°C under a nitrogen atmosphere for 2 hours. The residue was purified by prep-HPLC (0.1% NH₃·H₂O in water/ACN) to obtain compound 135 (6.34 mg). LCMS (ESI) (M+H)⁺ =440.3; ¹H NMR (400 MHz, MeOD-*d*₄) δ 8.23 (d, *J* = 2.4 Hz, 1H), 8.13 (s, 1H), 7.51 (s, 1H), 7.44 (t, *J =* 8.5 Hz, 1H), 7.07 (d, *J =* 8.5 Hz, 1H), 4.10 - 3.96 (m, 1H), 3.39 (s, 3H), 3.38 - 3.34 (m, 4H), 3.07 (s, 2H), 3.05 - 2.99 (m, 2H), 2.73 - 2.65 (m, 6H), 1.17 (d, *J* = 6.6 Hz, 6H).

### Example 75

### Preparation of N-isopropyl-2-(4-(5-(6-methoxy-1,1-dimethyl-3-oxoisoindolin-2-yl)pyridin-3-yl)piperazin-1-yl)acetamide (compound 136):

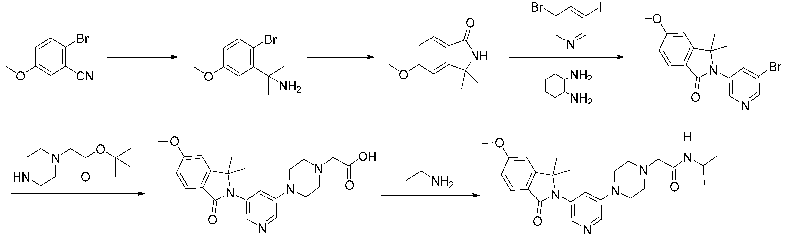

### Step 1: Synthesis of 2-(2-bromo-5-methoxyphenyl)propan-2-amine:

With reference to step 1 of example 58, using 2-bromo-5-methoxybenzonitrile (5 g, 1.0 eq) as the starting material, the reaction was stirred at room temperature for 16 hours. With reference to step 1 of example 58, the product (2 g, crude) was obtained. LCMS (ESI) [M+H] ⁺ = 244.0; ¹H NMR(400 MHz, CDCl₃) δ 7.48 (d, *J =* 8.7 Hz, 1H), 7.16 (d, *J* = 3.0 Hz, 1H), 6.62 (dd, *J =* 8.7, 3.0 Hz, 1H), 3.79 (s, 3H), 2.07 - 1.92 (m, 2H), 1.64 (s, 6H).

With reference to step 2 to step 5 of example 58, using the product obtained in step 1 (2 g, crude) as the starting material, compound 136 (8.63 mg) was prepared. LCMS (ESI) [M+H] ⁺ = 452.1; ¹H NMR(400 MHz, DMSO-*d₆*) δ 8.34 (d, *J =* 2.5 Hz, 1H), 7.89 (d, *J =* 1.5 Hz, 1H), 7.64 (d, *J =* 8.4 Hz, 1H), 7.52 (d, *J =* 8.1 Hz, 1H), 7.33 (d, *J =* 2.1 Hz, 1H), 7.29 - 7.22 (m, 1H), 7.06 (dd, *J* = 8.4, 2.2 Hz, 1H), 3.95 - 3.86 (m, 4H), 3.30 - 3.27 (m, 4H), 2.95 (s, 2H), 2.63 - 2.54 (m, 4H), 1.47 (s, 6H), 1.07 (d, *J =* 6.6 Hz, 6H).

Product of step 2 (5-methoxy-3,3-dimethylisoindolin-1-one): LCMS (ESI) [M+H] ⁺ = 192.1; ¹H NMR(400 MHz, CDCl₃) δ 7.73 (d, *J =* 8.4 Hz, 1H), 6.96 (M, 1H), 6.89 (s, 1H), 6.85 (d, *J =* 2.1 Hz, 1H), 3.89 (s, 3H), 1.54 (s, 6H).

Product of step 3 (2-(5-bromopyridin-3-yl)-5-methoxy-3,3-dimethylisoindolin-1-one): LCMS (ESI) [M+H] ⁺ = 348.9; ¹H NMR(400 MHz, CDCl₃) δ 8.70 (d, *J =* 2.1 Hz, 1H), 8.52 (d, *J =* 2.1 Hz, 1H), 7.86 - 7.82 (m, 2H), 7.04 - 7.00 (m, 1H), 6.92 (d, *J =* 2.1 Hz, 1H), 3.92 (s, 3H), 1.55 (s, 6H).

Product of step 4 (2-(4-(5-(6-methoxy-1,1-dimethyl-3-oxoisoindolin-2-yl)pyridin-3-yl)piperazin-1-yl)acetic acid): LCMS (ESI) [M+H] ⁺ = 411.2; ¹H NMR(400 MHz, DMSO-*d₆*) δ 8.34 (d, *J =* 2.6 Hz, 1H), 7.96 - 7.86 (m, 1H), 7.70 - 7.62 (m, 1H), 7.34 (d, *J* = 2.1 Hz, 1H), 7.29 - 7.23 (m, 1H), 7.06 (dd, *J =* 8.4, 2.2 Hz, 1H), 3.89 (s, 3H), 3.35 - 3.24 (m, 4H), 3.17 (s, 2H), 2.84 - 2.61 (m, 4H), 1.47 (s, 6H).

### Example 76

### Preparation of N-isopropyl-2-(4-(5-(1-methyl-2-oxo-1,2,3,4-tetrahydroquinolin-6-yl)-1,2,4-triazin-3-yl)piperazin-1-yl)acetamide (compound 137):

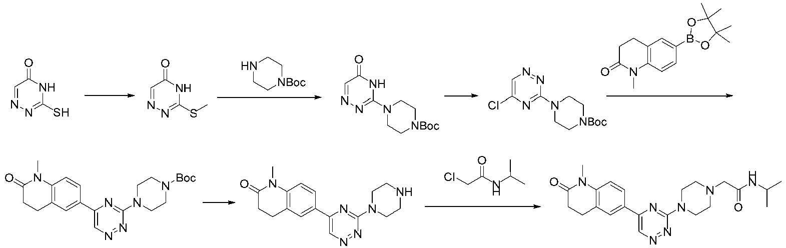

### Step 1: Synthesis of 3-(methylthio)-1,2,4-triazin-5(4H)-one:

In an ice bath, sodium hydride (4 g, 1.29 eq) was added to water (100 mL, 100.0%) , and the mixture was stirred until the solution was clear. 3-Mercapto-1,2,4-triazin-5(4H)-one (10 g) and iodomethane (7.23 mL) were added, and the mixture was stirred at room temperature for 12 hours. The reaction solution was filtered, and the filter cake was dried to obtain the product (4.7 g). LCMS (ESI) [M+H]⁺ = 144.04.

### Step 2: Synthesis of tert-butyl 4-(5-oxo-4,5-dihydro-1,2,4-triazin-3-yl)piperazine-1-carboxylate:

The product obtained in step 1 (4.6 g, 1.0 eq) and tert-butyl piperazine-1-carboxylate (6.58 g) were added to N-methylpyrrolidone (40 mL), and the mixture was stirred at 150°C for 2 hours. The reaction solution was poured into methyl tert-butyl ether, and the mixture was filtered to obtain the product (8.26 g). LCMS (ESI) [M+H]⁺ = 282.17.

### Step 3: Synthesis of tert-butyl 4-(5-chloro-1,2,4-triazin-3-yl)piperazine-1-carboxylate:

Triphenylphosphine (10.07 g) was dissolved in 1,4-dioxane (60 mL). 1-Chloropyrrolidine-2,5-dione (5.04 g) was added, and the mixture was stirred at 25°C for 30 minutes. The product obtained in step 2 (3.6 g, 1.0 eq) was then added, and the mixture was stirred at 125°C for 1 hour. The reaction solution was cooled, and N,N-diisopropylethylamine (4 mL) was added. The mixture was filtered, and the filtrate was concentrated. The residue was purified by flash chromatography (silica gel, PE : EA = 6 : 1) to obtain the product (700 mg). LCMS (ESI) [M+H]⁺ = 300.4.

### Step 4: Synthesis of tert-butyl 4-(5-(1-methyl-2-oxo-1,2,3,4-tetrahydroquinolin-6-yl)-1,2,4-triazin-3-yl)piperazine-1-carboxylate:

With reference to step 2 of example 39, using the product obtained in step 3 (700 mg, 1.0 eq) and the sample of preparation example 1 (1.07 g) as starting materials, the reaction was stirred at 80°C under a nitrogen atmosphere for 1 hour. The residue was purified by flash chromatography (silica gel, PE : THF = 1 : 1) to obtain the product (850 mg). LCMS (ESI) [M+H]⁺ =425.2; ¹H NMR (400 MHz, DMSO-*d₆*) δ 9.31 (s, 1H), 8.21 - 8.15 (m, 2H), 7.25 (d, *J =* 8.5 Hz, 1H), 3.92 - 3.87 (m, 4H), 3.51 - 3.47 (m, 4H), 3.31 (s, 3H), 3.02 - 2.96 (m, 2H), 2.64 - 2.60 (m, 2H), 1.44 (s, 9H).

### Step 5: Synthesis of 1-methyl-6-(3-(piperazin-1-yl)-1,2,4-triazin-5-yl)-3,4-dihydroquinolin-2(1H)-one:

With reference to step 3 of example 50, using the product obtained in step 4 (1.6 g, 1.0 eq) as the starting material, the reaction was stirred at room temperature for 3 hours. After the work-up, the product (1.4 g, crude) was obtained. LCMS (ESI) [M+H]⁺ = 325. The product was directly used in the next step without further purification.

### Step 6:

With reference to step 1 of example 5, using the product obtained in step 5 (80 mg, crude) and 2-chloro-N-(propan-2-yl)acetamide (43.47 mg) as starting materials, the reaction was stirred at 35°C under a nitrogen atmosphere for 3 hours. The residue was purified by prep-HPLC (C18, 10 mmol/L NH₄HCO₃ in water, MeCN) to obtain compound 137 (45.18 mg). LCMS (ESI) [M+H]⁺ = 424.41; ¹H NMR (400 MHz, DMSO-*d*₆) δ 9.29 (s, 1H), 8.22 - 8.13 (m, 2H), 7.57 (d, *J =* 8.1 Hz, 1H), 7.25 (d, *J =* 8.5 Hz, 1H), 3.97 - 3.85 (m, 5H), 3.31 (s, 3H), 2.98 (d, *J* = 7.1 Hz, 4H), 2.65 - 2.54 (m, 6H), 1.09 (d, *J =* 6.6 Hz, 6H).

### Example 77

### Preparation of 2-(4-(5-(1-methyl-2-oxo-1,2,3,4-tetrahydroquinolin-6-yl)pyridin-3-yl)piperazin-1-yl)-N-(3-morpholinocyclobutyl)acetamide (compound 138):

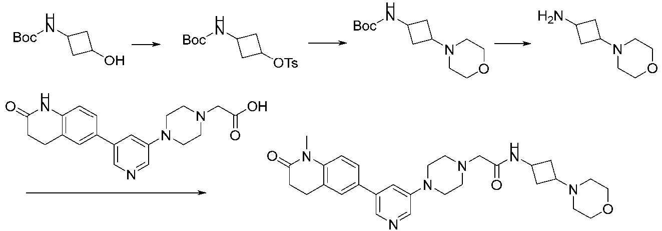

### Step 1: Synthesis of 3-((tert-butoxycarbonyl)amino)cyclobutyl 4-methylbenzenesulphonate:

Tert-butyl (3-hydroxycyclobutyl)carbamate (900 mg, 1.0 eq) was dissolved in dichloromethane (20 mL). N,N-dimethylpyridin-4-amine (1.17 g) and triethylamine (972.77 mg) were added. After the mixture was stirred evenly, 4-methylbenzene-1-sulphonyl chloride (916.39 mg) was added dropwise. The mixture was stirred at 45°C for 2 hours. The reaction solution was extracted with dichloromethane (10 mL*3). The organic phases were combined, dried over anhydrous sodium sulphate and filtered, and the filtrate was concentrated. The residue was purified by flash chromatography (silica gel, PE : EA = 1 : 4) to obtain the product (1.1 g). LCMS (ESI) [M+H-Boc]⁺ = 242.14.

### Step 2: Synthesis of tert-butyl (3-morpholinocyclobutyl)carbamate:

The product obtained in step 1 (500 mg, 1.0 eq) and morpholine (1.28 g) were dissolved in N,N-dimethylpyridin-4-amine (17.89 mg), and the mixture was stirred overnight at 100°C. The reaction solution was diluted with water (50 mL), and extracted with ethyl acetate (30 mL*2). The organic phases were combined, dried over anhydrous sodium sulphate and filtered, and the filtrate was concentrated. The residue was purified by flash chromatography (silica gel, PE : EA = 1 : 1) to obtain the product (300 mg).

### Step 3: Synthesis of 3-morpholinocyclobutan-1-amine:

With reference to step 2 of example 37, using the product obtained in step 2 (300 mg, 1.0 eq) as the starting material, the reaction was stirred at room temperature for 3 hours. After the workup and separation, the product (150 mg) was obtained. LCMS (ESI) [M+H]⁺ = 157.1. The product was directly used in the next step without further purification.

### Step 4:

With reference to step 3 of example 1, using the sample of preparation example 3 (100 mg, 1.0 eq) and the product obtained in step 3 (100 mg, crude) as starting materials, the reaction was stirred at room temperature for 2 hours. The residue was purified by prep-HPLC (C18, 10 mmol/L NH₄HCO₃ in water, MeCN) to obtain compound 138 (25.17 mg). LCMS (ESI) [M+H]⁺ = 519.1; ¹H NMR (400 MHz, DMSO-*d*₆) δ 8.28 (dd, *J* = 10.4*,* 2.2 Hz, 2H), 8.00 (dd, *J =* 34.6, 7.9 Hz, 1H), 7.63 (dq, *J* = 4.8*,* 2.3 Hz, 2H), 7.50 (d, *J* = 2.4 Hz, 1H), 7.17 (d, *J =* 9.0 Hz, 1H), 4.19 (q, *J* = 7.0 Hz, 1H), 3.56 (dt, *J =* 10.8, 4.5 Hz, 4H), 3.29 (s, 3H), 2.95 (dd, *J* = 11.7, 8.4 Hz, 4H), 2.75 (q, *J =* 7.2, 6.2 Hz, 1H), 2.59 (dt, *J =* 9.4, 6.5 Hz, 6H), 2.43 - 2.12 (m, 7H), 1.98 (dt, *J =* 12.7, 7.2 Hz, 1H), 1.77 (q, *J =* 10.2, 8.3 Hz, 1H).

### Example 78

### Preparation of 2-(4-(5-(6'-chloro-3'-oxospiro[cyclopropane-1,1'-isoindolin]-2'-yl)pyridin-3-yl)piperazin-1-yl)-N-isopropylacetamide (compound 139):

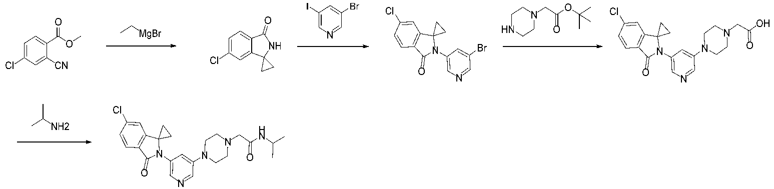

### Step 1: Synthesis of 6'-chlorospiro[cyclopropane-1,1'-isoindolin]-3'-one:

Methyl 4-chloro-2-cyanobenzoate (600 mg, 1.0 eq) was dissolved in diethyl ether (10 mL). Tetraisopropyl titanate (959.02 mg) was added. Under a nitrogen atmosphere, ethylmagnesium bromide (2.04 mL, 3 M) was added dropwise to the reaction solution at 0°C. After the dropwise addition was completed, the mixture was stirred at room temperature for 3 hours. The reaction solution was quenched by adding hydrochloric acid (1 M, 10 mL), and extracted with ethyl acetate (30 mL*3). The organic phases were combined, dried over anhydrous sodium sulphate and filtered, and the filtrate was concentrated. The residue was purified by flash chromatography (silica gel, PE : EA = 1 : 1) to obtain the product (140 mg). LCMS (ESI) [M+H] ⁺ = 194.1; ¹HNMR(400 MHz, DMSO-*d₆*) δ 8.80 (s, 1H), 7.71 - 7.62 (m, 1H), 7.55 - 7.40 (m, 2H), 1.60 - 1.36 (m, 4H).

### Step 2: Synthesis of 2'-(5-bromopyridin-3-yl)-6'-chlorospiro[cyclopropane-1,1'-isoindolin]-3'-one:

With reference to step 3 of example 58, using the product obtained in step 1 (140 mg, 1.0 eq) and 3-bromo-5-iodopyridine (307.89 mg) as starting materials, the reaction was stirred at 120°C under a nitrogen atmosphere for 3 hours. The residue was purified by flash chromatography (silica gel, PE : EA = 2 : 1) to obtain the product (120 mg). LCMS (ESI) [M+H] ⁺ = 350.9; ¹H NMR(400 MHz, DMSO-*d₆*) δ 8.82 (d, *J* = 2.1 Hz, 1H), 8.55 (d, *J =* 2.0 Hz, 1H), 8.24 - 8.19 (m, 1H), 7.85 - 7.82 (m, 1H), 7.64 - 7.63 (m, 1H), 7.60 - 7.57 (m, 1H), 1.58 (d, *J* = 2.1 Hz, 2H), 1.37 - 1.32 (m, 2H).

With reference to step 4 and step 5 of example 58, using the product obtained in step 2 as the starting material (120 mg, 1.0 eq), compound 139 (26.46 mg) was obtained. LCMS (ESI) [M+H] ⁺ = 454.0; ¹HNMR(400 MHz, DMSO-*d₆*) δ 8.37 (d, *J =* 2.7 Hz, 1H), 7.85 (d, *J =* 1.8 Hz, 1H), 7.83 - 7.78 (m, 1H), 7.64 - 7.60 (m, 1H), 7.60 - 7.55 (m, 1H), 7.52 (d, *J =* 8.2 Hz, 1H), 7.28 - 7.23 (m, 1H), 3.95 - 3.85 (m, 1H), 3.31 - 3.26 (m, 4H), 2.94 (s, 2H), 2.60 - 2.54 (m, 4H), 1.60 - 1.49 (m, 2H), 1.34 - 1.25 (m, 2H), 1.07 (d, *J =* 6.6 Hz, 6H).

Product of step 3 (2-(4-(5-(6'-chloro-3'-oxospiro[cyclopropane-1,1'-isoindolin]-2'-yl)pyridin-3-yl)piperazin-1-yl)acetic acid): LCMS (ESI) [M+H] ⁺ = 413.1.

### Examples 79-85

With reference to step 3 of example 1, using the sample of preparation example 3 (50 mg, 1.0 eq) with the starting materials shown in the table below, compounds 140-145 and 148 were prepared respectively.

| Starting material | Compound | Structural confirmation data |
|---|---|---|
| 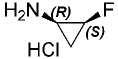 | 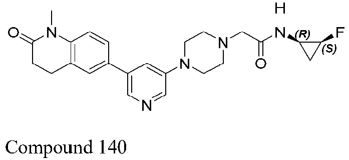 | LCMS (ESI) [M+H] ⁺ = 438.1; ¹HNMR(400 MHz, DMSO-*d₆*) δ 8.29 (d, *J* = 1.7 Hz, 1H), 8.26 (d*, J =* 2.7 Hz, 1H), 7.86 (d, *J =* 4.2 Hz, 1H), 7.65 - 7.60 (m, 2H), 7.52 - 7.48 (m, 1H), 7.20 - 7.14 (m, 1H), 4.84 - 4.60 (m, 1H), 3.32 - 3.30 (m, 4H), 3.29 (s, 3H), 3.10 - 2.98 (m, 2H), 2.98 - 2.91 (m, 2H), 2.72 - 2.66 (m, 1H), 2.64 - 2.55 (m, 6H), 1.07 - 0.94 (m, 2H). |
| 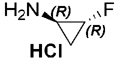 | 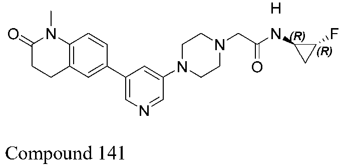 | LCMS (ESI) [M+H]⁺ =438.4; ¹H NMR (400 MHz, DMSO-*d₆*) δ 8.29 (d, *J =* 1.6 Hz, 1H), 8.26 (d, *J =* 2.6 Hz, 1H), 7.87 (s, 1H), 7.64 - 7.61 (m, 2H), 7.51- 7.48 (m, 1H), 7.19 - 7.15 (m, 1H), 4.80 - 4.62 (m, 1H), 3.31 - 3.29 (m, 4H), 3.29 (s, 3H), 3.06 - 3.00 (m, 1H), 2.97 (s, 2H), 2.96 - 2.92 (m, 2H), 2.60 - 2.56 (m, 6H), 1.33 - 1.23 (m, 1H), 1.02 - 0.94 (m, 1H). |
| 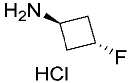 | 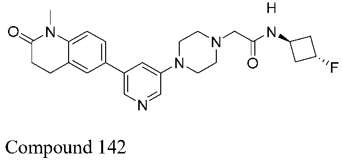 | LCMS(ESI)[M+H]⁺ = 452.0; ¹HNMR(400 MHz, DMSO-*d₆*) δ 8.29 (d, *J =* 1.6 Hz, 1H), 8.27 (d*, J =* 2.6 Hz, 1H), 8.11 (d*, J =* 7.4 Hz, 1H), 7.65 - 7.61 (m, 2H), 7.52 - 7.49 (m, 1H), 7.20 - 7.15 (m, 1H), 5.33 - 5.11 (m, 1H), 4.48 - 4.37 (m, 1H), 3.38 - 3.33 (m, 4H), 3.29 (s, 3H), 2.98 (s, 2H), 2.97 - 2.92 (m, 2H), 2.62 - 2.56 (m, 6H), 2.46 - 2.32 (m, 4H). |
| 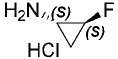 | 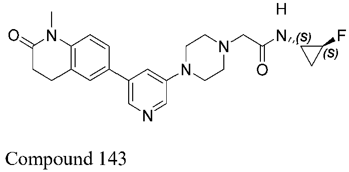 | LCMS (ESI) [M+H] ⁺ = 438.1; ¹HNMR(400 MHz, DMSO-*d₆*) δ 8.29 (d, *J* = 1.5 Hz, 1H), 8.26 (d, *J =* 2.6 Hz, 1H), 7.92 - 7.82 (m, 1H), 7.67 - 7.56 (m, 2H), 7.55 - 7.46 (m, 1H), 7.26 - 7.10 (m, 1H), 4.86 - 4.59 (m, 1H), 3.32 - 3.30 (m, 4H), 3.29 (s, 3H), 3.08 - 3.00 (m, 1H), 3.00 - 2.92 (m, 4H), 2.61 - 2.54 (m, 6H), 1.40 - 1.21 (m, 1H), 1.03 - 0.90 (m, 1H). |
| 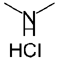 | 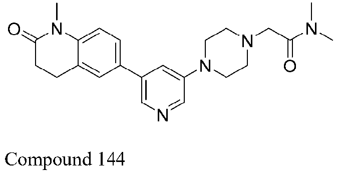 | LCMS (ESI) [M+H] ⁺ = 408.1; ¹H NMR(400 MHz, DMSO-*d₆*) δ 8.29 (d, *J* = 1.7 Hz, 1H), 8.26 (d*, J =* 2.7 Hz, 1H), 7.66 - 7.61 (m, 2H), 7.52 - 7.47 (m, 1H), 7.21 - 7.14 (m, 1H), 3.31 - 3.26 (m, 7H), 3.21 (s, 2H), 3.04 (s, 3H), 2.97 - 2.91 (m, 2H), 2.82 (s, 3H), 2.64 - 2.56 (m, 6H). |
| 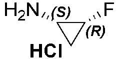 | 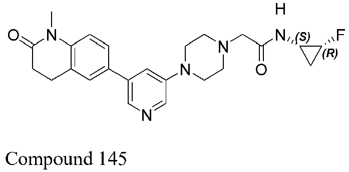 | LCMS (ESI) [M+H] ⁺ = 438.3; ¹H NMR(400 MHz, DMSO-*d₆*) δ 8.29 (d, *J* = 1.6 Hz, 1H), 8.26 (d, *J =* 2.6 Hz, 1H), 7.86 (d, *J =* 4.2 Hz, 1H), 7.66 - 7.60 (m, 2H), 7.52 - 7.48 (m, 1H), 7.21 - 7.13 (m, 1H), 4.83 - 4.60 (m, 1H), 3.32 - 3.29 (m, 4H), 3.29 (s, 3H), 3.11 - 2.99 (m, 2H), 2.98 - 2.91 (m, 2H), 2.71 - 2.67 (m, 1H), 2.64 - 2.55 (m, 6H), 1.09 - 0.96 (m, 2H). |
| 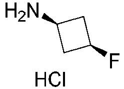 | 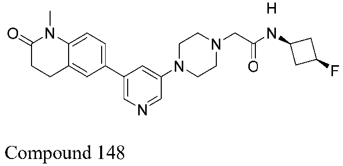 | LCMS (ESI) [M+H]⁺ = 452.1; ¹H NMR(400 MHz, DMSO-*d₆*) δ 8.36 - 8.23 (m, 2H), 8.10 (d, *J* = 8.0 Hz, 1H), 7.70 - 7.59 (m, 2H), 7.55 - 7.46 (m, 1H), 7.26 - 7.10 (m, 1H), 4.93 - 4.69 (m, 1H), 3.94 - 3.72 (m, 1H), 3.37 - 3.33 (m, 4H), 3.29 (s, 3H), 2.98 (s, 2H), 2.97 - 2.89 (m, 2H), 2.69 - 2.62 (m, 2H), 2.62 - 2.56 (m, 6H), 2.32 - 2.10 (m, 2H). |

### Example 86

### Preparation of N-(trans-4-acetamidocyclohexyl)-2-(4-(5-(1-methyl-2-oxo-1,2,3,4-tetrahydroquinolin-6-yl)-1,2,4-triazin-3-yl)piperazin-1-yl)acetamide (compound 146):

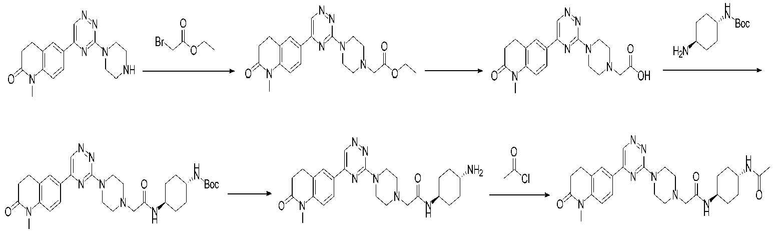

### Step 1: Synthesis of ethyl 2-(4-(5-(1-methyl-2-oxo-1,2,3,4-tetrahydroquinolin-6-yl)-1,2,4-triazin-3-yl)piperazin-1-yl)acetate:

With reference to step 4 of example 50, using 1-methyl-6-(3-(piperazin-1-yl)-1,2,4-triazin-5-yl)-3,4-dihydroquinolin-2(1H)-one (1.1 g, 1.0 eq) and ethyl 2-bromoacetate (594.63 mg) as starting materials, the reaction was stirred at room temperature for 2 hours. The reaction solution was directly concentrated, and the residue was purified by flash chromatography (silica gel, PE : EA = 3 : 1) to obtain the product (1.1 g). LCMS (ESI) [M+H]⁺ = 411.1.

### Step 2: Synthesis of 2-(4-(5-(1-methyl-2-oxo-1,2,3,4-tetrahydroquinolin-6-yl)-1,2,4-triazin-3-yl)piperazin-1-yl)acetic acid:

With reference to step 5 of example 50, using the product obtained in step 1 (1 g, 1.0 eq), the reaction was stirred at room temperature for 3 hours. The reaction solution was adjusted to acidic pH with 1 M hydrochloric acid, and a solid precipitated out. The reaction solution was filtered and dried to obtain the product (1 g, crude). LCMS (ESI) [M+H]⁺ = 383. The product was directly used in the next step without further purification.

### Step 3: Synthesis of tert-butyl (trans-4-(2-(4-(5-(1-methyl-2-oxo-1,2,3,4-tetrahydroquinolin-6-yl)-1,2,4-triazin-3-yl)piperazin-1-yl)acetamido)cyclohexyl)carbamate:

With reference to step 3 of example 1, using the product obtained in step 2 (200 mg, crude) and tert-butyl (trans-4-aminocyclohexyl)carbamate (224.15 mg) as starting materials, the reaction was stirred at room temperature for 2 hours. The residue was purified by flash chromatography (silica gel, PE : EA = 2 : 1) to obtain the product (300 mg). LCMS (ESI) [M+H]⁺ = 579.1.

### Step 4: Synthesis of N-((1R,4R)-4-aminocyclohexyl)-2-(4-(5-(1-methyl-2-oxo-1,2,3,4-tetrahydroquinolin-6-yl)-1,2,4-triazin-3-yl)piperazin-1-yl)acetamide:

With reference to step 5 of example 76, using the product obtained in step 3 (200 mg, 1.0 eq), the reaction was stirred at room temperature for 2 hours. The product (150 mg, crude) was obtained. LCMS (ESI) [M+H]⁺ = 479.1. The product was directly used in the next step without further purification.

### Step 5:

With reference to step 6 of example 21, using the product obtained in step 4 (150 mg, crude) and acetyl chloride (29.52 mg) as starting materials, the reaction was stirred at room temperature for 2 hours. The residue was purified by prep-HPLC (C18, 10 mmol/L NH₄HCO₃ in water, MeCN) to obtain compound 146 (12.12 mg). LCMS (ESI) [M+H]⁺ =521.1;¹H NMR (400 MHz, DMSO-*d*₆) δ 9.28 (s, 1H), 8.22 - 8.13 (m, 2H), 7.72 (d, *J =* 7.8 Hz, 1H), 7.61 (d, *J =* 8.2 Hz, 1H), 7.25 (d, *J =* 8.6 Hz, 1H), 3.93 (t, *J =* 5.0 Hz, 4H), 3.46 (tt, *J =* 7.6*,* 3.8 Hz, 2H), 3.31 (s, 3H), 2.98 (d, *J =* 3.2 Hz, 4H), 2.59 (dq, *J =* 16.0, 5.5 Hz, 6H), 1.78 (d, *J =* 5.7 Hz, 4H), 1.76 (s, 3H), 1.38 - 1.14 (m, 4H).

### Example 87

### Preparation of N-isopropyl-2-(4-(5-(1-methyl-2-oxo-1,2,3,4-tetrahydroquinolin-6-yl)pyridin-3-yl)piperazin-1-yl)acetamide-2,2-d₂ (compound 147):

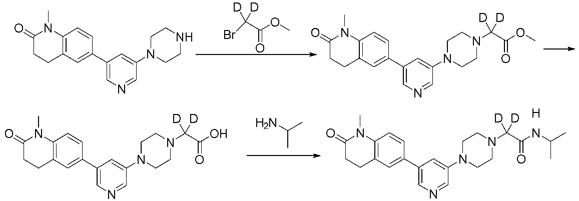

### Step 1: Synthesis of methyl 2-(4-(5-(1-methyl-2-oxo-1,2,3,4-tetrahydroquinolin-6-yl)pyridin-3-yl)piperazin-1-yl)acetate-d₂:

With reference to step 8 of example 36, using intermediate 1 (700 mg, crude) and methyl 2-bromoacetate-d₂ (336.52 mg) as starting materials, the reaction was stirred at 25°C for 2 hours. The residue was purified by prep-HPLC (C18, 0.1% NH₄HCO₃ in H₂O/ACN) to obtain the product (390 mg). LCMS (ESI) [M+H]⁺ =397.5, 397.4; ¹H NMR (400 MHz, DMSO-*d₆*) δ 8.30 - 8.25 (m, 2H), 7.65 - 7.60 (m, 2H), 7.49 (s, 1H), 7.19 - 7.15 (m, 1H), 3.64 (s, 3H), 3.30 - 3.27 (m, 7H), 2.97 - 2.91 (m, 2H), 2.71 - 2.66 (m, 4H), 2.61 - 2.56 (m, 2H).

### Step 2: Synthesis of 2-(4-(5-(1-methyl-2-oxo-1,2,3,4-tetrahydroquinolin-6-yl)pyridin-3-yl)piperazin-1-yl)acetic-2,2-d₂ acid:

With reference to step 9 of example 36, using the product obtained in step 1 (390 mg, 1.0 eq) as the starting material, the mixture was stirred at 50°C for 2 hours. The reaction solution was directly concentrated, and the residue was adjusted to pH = 6 to 7 by dropwise adding dilute hydrochloric acid (1 M). After a solid precipitated out, the mixture was filtered, and the filter cake was dried to obtain the product (300 mg). LCMS (ESI) [M+H]⁺ =383.4.

### Step 3:

With reference to step 3 of example 1, using the product obtained in step 2 (50 mg, 1.0 eq) and isopropylamine (7.73 mg) as starting materials, the reaction was stirred at 25°C for 2 hours. The residue was purified by prep-HPLC (C18, 0.1% NH₃H₂O in H₂O/ACN) to obtain compound 147 (4.69 mg). LCMS (ESI) [M+H]⁺ =424.4; ¹H NMR (400 MHz, MeOD-*d₄*) δ 8.24 - 8.18 (m, 2H), 7.59 - 7.56 (m, 2H), 7.54 (s, 1H), 7.23 (d, *J =* 8.4 Hz, 1H), 4.07 - 3.99 (m, 1H), 3.40 - 3.36 (m, 7H), 3.03 - 2.98 (m, 2H), 2.74 - 2.69 (m, 4H), 2.68 - 2.63 (m, 2H), 1.17 (d, *J =* 6.6 Hz, 6H).

### Example 88

### Preparation of N-(1-(2-(4-(5-(1-methyl-2-oxo-1,2,3,4-tetrahydroquinolin-6-yl)pyridin-3-yl)piperazin-1-yl)acetyl)azetidin-3-yl)acetamide (compound 149):

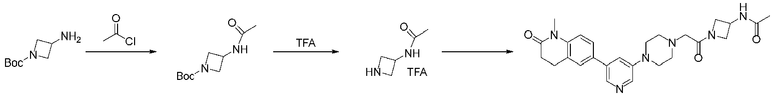

### Step 1: Synthesis of tert-butyl 3-acetamidoazetidine-1-carboxylate:

Tert-butyl 3-aminoazetidine-1-carboxylate (500 mg, 1 eq) was dissolved in dichloromethane (10 mL). Acetyl chloride (273.49 mg) was added at 0°C, and the mixture was stirred at room temperature for 2 hours. The reaction solution was quenched by adding water (20 mL), and extracted with dichloromethane (20 mL*3). The organic phases were combined, dried over anhydrous sodium sulphate and filtered, and the filtrate was concentrated to obtain the product (260 mg, crude). LCMS (ESI) [M+H] ⁺ = 215.2; ¹H NMR(400 MHz, CDCl₃) δ 6.13-5.92 (m, 1H), 4.70-4.51 (m, 1H), 4.37-4.17 (m, 2H), 3.81-3.62 (m, 2H), 2.01 (s, 3H), 1.44 (s, 9H).

### Step 2: Synthesis of N-(azetidin-3-yl)acetamide trifluoroacetate:

The product of step 1 (260 mg, crude, 1 eq) was dissolved in dichloromethane (2 mL), and trifluoroacetic acid (1 mL) was added. The mixture was stirred at room temperature for 16 hours. The reaction solution was directly concentrated to obtain the product (110 mg, crude). LCMS (ESI) [M+H] ⁺ = 115.1.

### Step 3: Synthesis of compound 149:

The product of preparation example 3 (50 mg, 1 eq) was dissolved in N,N-dimethylformamide (1 mL). N,N-diisopropylethylamine (0.07 mL), HATU (74.96 mg) and the product of step 2 (29.99 mg) were added, and the mixture was stirred at room temperature for 1 hour. The residue was purified by reversed-phase chromatography (0.1% aqueous ammonia, acetonitrile) to obtain the target compound (8.16 mg, yield 13%). LCMS (ESI) [M+H] ⁺ = 477.3; ¹H NMR(400 MHz, DMSO-*d₆*) δ 8.50 (d, *J =* 6.4 Hz, 1H), 8.29 (d, *J =* 1.7 Hz, 1H), 8.26 (d, *J =* 2.7 Hz, 1H), 7.66-7.60 (m, 2H), 7.52-7.47 (m, 1H), 7.20-7.15 (m, 1H), 4.50-4.37 (m, 2H), 4.18-4.05 (m, 1H), 4.05-3.95 (m, 1H), 3.74-3.64 (m, 1H), 3.30-3.27 (m, 7H), 3.11-2.99 (m, 2H), 2.98-2.91 (m, 2H), 2.62-2.56 (m, 6H), 1.82 (s, 3H).

### Example 89

### Preparation of N-(3-acetamidocyclobutyl)-2-(4-(5-(1-methyl-2-oxo-1,2,3,4-tetrahydroquinolin-6-yl)pyridin-3-yl)piperazin-1-yl)acetamide (compound 150):

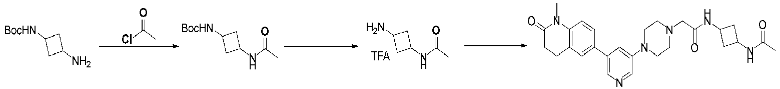

### Step 1: Synthesis of tert-butyl (3-acetamidocyclobutyl)carbamate:

With reference to step 1 of example 88, using tert-butyl (3-aminocyclobutyl)carbamate (200 mg, 1 eq) as the starting material, the product (115 mg, crude) was obtained. LCMS (ESI) [M+H-56]⁺ =173.6.

### Step 2: Synthesis of N-(3-aminocyclobutyl)acetamide trifluoroacetate:

With reference to step 2 of example 88, using tert-butyl (3-acetamidocyclobutyl)carbamate (115 mg, 1 eq) as the starting material, the product (60 mg, crude) was obtained. LCMS (ESI) [M+H]⁺ =129.6.

### Step 3: Synthesis of compound 150:

With reference to step 3 of example 88, the product of preparation example 3 (80 mg, 1 eq) was dissolved in N,N-dimethylformamide (2 mL). Triethylamine (0.09 mL), HATU (95.95 mg) and the product of step 2 (58.57 mg) were added, and the reaction was stirred at room temperature for 2 hours. The reaction solution was filtered, and the filtrate was concentrated. The residue was purified by reversed-phase chromatography (C18, 0.1% NH₃H₂O in H₂O/ACN) to obtain the target compound (41.45 mg). LCMS[ESI][M+H)⁺=491.3;¹H NMR (400 MHz, MeOD*-d₄*) δ 8.26-8.17 (m, 2H), 7.60-7.55 (m, 2H), 7.53 (s, 1H), 7.22 (d, *J =* 8.4 Hz, 1H), 4.42-4.27 (m, 1H), 4.11 -3.91 (m, 1H), 3.41-3.37 (m, 7H), 3.10-3.06 (m, 2H), 3.03-2.97 (m, 2H), 2.75 -2.63 (m, 7H), 2.38-2.30 (m, 2H), 1.99-1.93 (m, 1H), 1.93-1.88 (m, 3H).

### Example 90

### Preparation of N-methyl-2-(4-(5-(1-methyl-2-oxo-1,2,3,4-tetrahydroquinolin-6-yl)pyridin-3-yl)piperazin-1-yl)-N-(3-(N-methylacetamido)cyclobutyl)acetamide (compound 151):

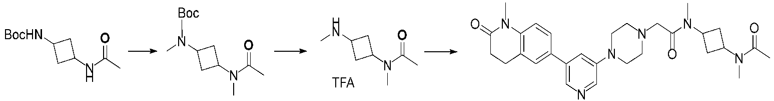

### Step 1: Synthesis of tert-butyl (methyl(3-(N-methylacetamido)cyclobutyl)carbamate:

Tert-butyl (3-acetamidocyclobutyl)carbamate (300 mg, 1 eq) was dissolved in N.N-dimethylformamide (6 mL). Under a nitrogen atmosphere and in an ice bath, sodium hydride (90.63 mg) was added, and the mixture was stirred for 30 minutes. Iodomethane (0.25 mL) was then added, and the mixture was further stirred at room temperature for 2 hours. The reaction solution was quenched by adding water (45 mL), and extracted with ethyl acetate (25 mL*3). The organic phases were combined, dried over anhydrous sodium sulphate and filtered, and the filtrate was concentrated. The residue was purified by flash chromatography (silica gel, petroleum ether : tetrahydrofuran = 3 : 1) to obtain the product (80 mg). LCMS (ESI) [M+H]⁺ =257.5.

### Step 2: Synthesis of N-methyl-N-(3-(methylamino)cyclobutyl)acetamide trifluoroacetate:

With reference to step 2 of example 88, using the product of step 1 of example 90 (110 mg, 1 eq) as the starting material, the product (60 mg) was obtained. LCMS (ESI) [M+H]⁺ =157.6.

### Step 3: Synthesis of compound 151:

The product of preparation example 3 (80 mg, 1 eq) and HATU (95.95 mg) were dissolved in N,N-dimethylformamide (2 mL). In an ice bath, triethylamine (0.09 ml) and the product of step 2 (56.83 mg) were sequentially added. The mixture was stirred at room temperature for 2 hours. The reaction solution was filtered, and the filtrate was purified by reversed-phase chromatography (C18, 0.1% NH₃H₂O in H₂O/ACN) to obtain the target compound (32.27 mg). LCMS (ESI) [M+H]⁺ =519.3; ¹H NMR (400 MHz, DMSO-*d₆*) δ 8.27 (dd, *J =* 12.2, 2.0 Hz, 2H), 7.64-7.60 (m, 2H), 7.51-7.47 (m, 1H), 7.19-7.15 (m, 1H), 5.00-4.04 (m, 2H), 3.30-3.27 (m, 7H), 3.26-3.21 (m, 2H), 3.02-2.91 (m, 5H), 2.90-2.80 (m, 3H), 2.64-2.55 (m, 6H), 2.45-2.17 (m, 4H), 1.99 (d, *J =* 10.9 Hz, 3H).

### Example 91

### Preparation of 2-(4-(5-(8-fluoro-1-methyl-2-oxo-1,2,3,4-tetrahydroquinolin-6-yl)pyridin-3-yl)piperazin-1-yl)-N-isopropylacetamide (compound 152):

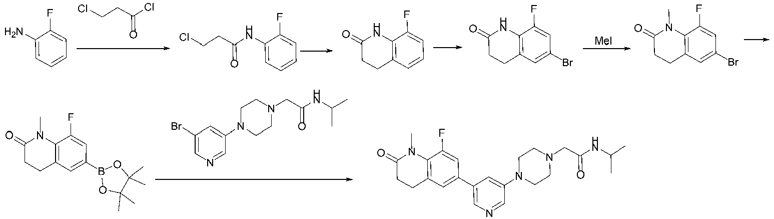

### Step 1: Synthesis of 3-chloro-N-(2-fluorophenyl)propanamide:

2-Fluoroaniline (5 g, 1 eq) and pyridine (3.92 g) were added to 1,2-dichloroethane (100 mL). Under ice bath conditions, 3-chloropropionyl chloride (6.28 g) was added dropwise. The mixture was stirred at room temperature for 2 hours. The reaction solution was washed with water and 2 N hydrochloric acid solution. The organic phases were combined, dried over anhydrous sodium sulphate and filtered, and the filtrate was concentrated to obtain the product (8.8 g). LCMS (ESI) [M+H]⁺ = 201.8.

### Step 2: Synthesis of 8-fluoro-3,4-dihydroquinolin-2(1H)-one:

The product of step 1 (4 g, 1 eq) and aluminium trichloride (13.23 g) were added to a reaction flask, and the reaction was heated to 150°C and stirred for 1.5 hours. After the reaction temperature was cooled to below 100°C, the reaction was quenched by adding water dropwise slowly. After the system was cooled to room temperature, the pH was adjusted to neutral with aqueous sodium hydroxide solution. The mixture was extracted with dichloromethane. The organic phases were combined, dried over anhydrous sodium sulphate and filtered, and the filtrate was concentrated. The residue was purified by flash chromatography (silica gel, petroleum ether : ethyl acetate = 4 : 1) to obtain the product (2.1 g). LCMS (ESI) [M+H]⁺ = 166.0.

### Step 3: Synthesis of 6-bromo-8-fluoro-3,4-dihydroquinolin-2(1H)-one:

The product of step 2 (2 g, 1 eq) was dissolved in DMF (40 mL). Under ice bath conditions, N-bromosuccinimide (2.37 g) was added in batches. The reaction was stirred overnight at room temperature. The reaction solution was poured into water (200 mL) and filtered, and the filter cake was dried to obtain the product (2.2 g). LCMS (ESI) [M+H]⁺ = 243.7.

### Step 4: Synthesis of 6-bromo-8-fluoro-1-methyl-3,4-dihydroquinolin-2(1H)-one:

The product of step 3 (2.8 g, 1 eq) was dissolved in DMF (60 mL). Under ice bath conditions, potassium tert-butoxide (2.57 g) was added. At this temperature, the reaction was stirred for 0.5 hours, and then iodomethane (2.44 g) was added. The reaction was stirred at room temperature for 2 hours. The reaction solution was quenched by pouring into water (300 mL), and extracted with ethyl acetate (150 mL*3). The organic phases were combined, dried over anhydrous sodium sulphate and filtered, and the filtrate was concentrated. The residue was purified by flash chromatography (silica gel, petroleum ether : ethyl acetate = 3 : 1) to obtain the product (1.8 g). LCMS (ESI) [M+H]⁺ = 257.9.

### Step 5: Synthesis of 8-fluoro-1-methyl-6-(4,4,5,5-tetramethyl-1,3,2-dioxaborolan-2-yl)-3,4-dihydroquinolin-2(1H)-one:

The product of step 4 (500 mg, 1 eq), bis(pinacolato)diboron (737.94 mg), potassium acetate (570.38 mg) and 1,1-bis(diphenylphosphino)ferrocene dichloropalladium (142.14 mg) were added to 1,4-dioxane (20 mL). Under a nitrogen atmosphere, the reaction was stirred at 100°C for 2 hours. The reaction solution was directly concentrated, and the residue was purified by flash chromatography (silica gel, petroleum ether : ethyl acetate = 3 : 1) to obtain the product (400 mg). LCMS (ESI) [M+H]⁺ = 305.9.

### Step 6: Synthesis of compound 152:

The product of step 5 (100 mg, 1 eq), the product of preparation example 4 (111.83 mg), caesium carbonate (213.55 mg) and 1,1-bis(diphenylphosphino)ferrocene dichloropalladium (24.04 mg) were added to 1,4-dioxane (5 mL) and water (1 mL). Under a nitrogen atmosphere, the reaction was heated to 100°C and stirred for 5 hours. The reaction solution was directly concentrated, and the residue was purified by flash chromatography (silica gel, ethyl acetate) to obtain the target compound crude. The crude was dissolved in acetonitrile and filtered, and the filtrate was concentrated. The residue was purified by prep-HPLC (C18, 10 mmol/L NH₄HCO₃ in water, MeCN) to obtain the target compound (69.86 mg). LCMS (ESI) [M+H]⁺ = 440.1; ¹H NMR (400 MHz, DMSO-*d*₆) δ 8.33 (d, *J =* 1.8 Hz, 1H), 8.29 (d, *J =* 2.7 Hz, 1H), 7.61 (dd, *J =* 14.4, 2.0 Hz, 1H), 7.57-7.47 (m, 3H), 3.91 (dp, *J =* 8.0, 6.5 Hz, 1H), 3.35 (d, *J =* 2.7 Hz, 3H), 3.33 (s, 4H), 2.95 (d, *J =* 4.5 Hz, 4H), 2.64-2.53 (m, 6H), 1.08 (d, *J =* 6.6 Hz, 6H).

### Example 92

### Preparation of 2-(4-(5-(6-cyano-1,1-dimethyl-3-oxoisoindolin-2-yl)pyridin-3-yl)piperazin-1-yl)-N-isopropylacetamide (compound 153):

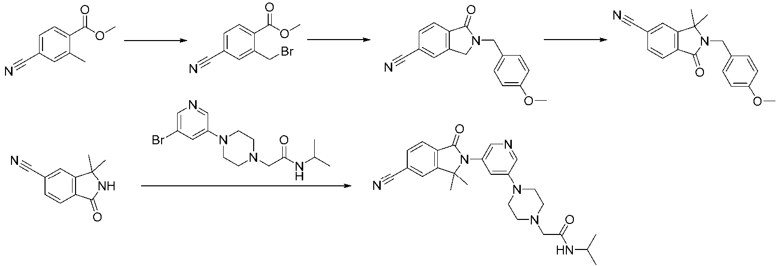

### Step 1: Synthesis of methyl 2-(bromomethyl)-4-cyanobenzoate:

Methyl 4-cyano-2-methylbenzoate (200 mg, 1 eq) was dissolved in carbon tetrachloride (8 mL). N-bromosuccinimide (243.84 mg) and azobisisobutyronitrile (93.74 mg) were added, and the reaction was heated to 80°C and stirred overnight. The reaction solvent was directly subjected to rotary evaporation to dryness, and the residue was purified by flash chromatography (silica gel, petroleum ether : ethyl acetate = 10 : 1) to obtain the product (100 mg). ¹H NMR (400 MHz, Chloroform-*d*) δ 8.05 (d, J = 8.1 Hz, 1H), 7.78 (d, J = 1.7 Hz, 1H), 7.66 (dd, J = 8.1, 1.7 Hz, 1H), 4.92 (s, 2H), 3.98 (s, 3H).

### Step 2: Synthesis of 2-(4-methoxybenzyl)-1-oxoisoindoline-5-carbonitrile:

The product of step 1 (5.5 g, 1 eq) was dissolved in tetrahydrofuran (60 mL). Under ice bath conditions, 1-(4-methoxyphenyl)methylamine (7.42 g) was added. The reaction was stirred at room temperature for 3 hours. The reaction solution was filtered, and the filtrate was subjected to rotary evaporation to dryness. The residue was dissolved in ethyl acetate, and then washed with water and saturated aqueous sodium chloride solution. The organic phase was dried over anhydrous sodium sulphate and filtered, and the filtrate was concentrated. The residue was purified by flash chromatography (silica gel, petroleum ether : ethyl acetate = 3 : 1) to obtain the product (3.3 g). LCMS (ESI) [M+H]⁺ = 278.9.

### Step 3: Synthesis of 2-(4-methoxybenzyl)-3,3-dimethyl-1-oxoisoindoline-5-carbonitrile:

The product of step 2 (3.3 g, 1 eq) was dissolved in tetrahydrofuran (60 mL). 60% sodium hydride (2.27 g) was added, and the reaction was stirred at room temperature for 1 hour. Iodomethane (10.1 g) was then added, and the reaction was heated to 70°C and stirred for 2 hours. The reaction was cooled to room temperature, and quenched by adding saturated ammonium chloride solution. The mixture was extracted with ethyl acetate (30 mL*3). The organic phases were combined, dried over anhydrous sodium sulphate and filtered, and the filtrate was concentrated. The residue was purified by flash chromatography (silica gel, petroleum ether : ethyl acetate = 2 : 1) to obtain the product (4 g, crude). LCMS (ESI) [M+H]⁺ = 307.

### Step 4: Synthesis of 3,3-dimethyl-1-oxoisoindoline-5-carbonitrile:

The product of step 3 (4 g, 1 eq) was dissolved in acetonitrile (80 mL). Under ice bath conditions, an aqueous solution (30 mL) of ceric ammonium nitrate (8.62 g) was added. The reaction was further stirred for 1 hour. The reaction solution was poured into water (200 mL), and extracted with ethyl acetate (80 mL*3). The organic phases were combined, dried over anhydrous sodium sulphate and filtered, and the filtrate was concentrated. The residue was purified by flash chromatography (silica gel, petroleum ether : ethyl acetate = 3 : 1) to obtain the product (1.3 g). LCMS (ESI) [M+H]⁺ = 187.

### Step 5: Synthesis of compound 153:

The product of step 4 (100 mg, 1 eq), the product of preparation example 4 (74.89 mg), potassium phosphate (227.99 mg), cuprous iodide (30.68 mg) and cyclohexane-1,2-diamine (36.79 mg) were added to 1,4-dioxane (5 mL). The reaction was heated to reflux for 5 hours. The reaction solution was subjected to rotary evaporation to dryness, and the residue was purified by flash chromatography (silica gel, petroleum ether : ethyl acetate = 0 : 1) to obtain the target compound crude. The crude was dissolved in acetonitrile and filtered, and the filtrate was purified by prep-HPLC (C18, 10 mmol/L NH₄HCO₃ in water, MeCN) to obtain the target compound (18.06 mg). LCMS (ESI) [M+H]⁺ = 447.0; ¹H NMR (400 MHz, DMSO-*d*₆) δ 8.42 (t, *J =* 1.1 Hz, 1H), 8.38 (d, *J =* 2.7 Hz, 1H), 8.01 (dd, *J =* 7.8*,* 1.3 Hz, 1H), 7.95-7.89 (m, 2H), 7.52 (d, *J* = 8.1 Hz, 1H), 7.30 (t, *J =* 2.3 Hz, 1H), 3.90 (dp, *J =* 8.0, 6.5 Hz, 1H), 3.30 (d, *J* = 5.3 Hz, 4H), 2.95 (s, 2H), 2.59 (t, *J =* 5.1 Hz, 4H), 1.51 (s, 6H), 1.07 (d, *J =* 6.6 Hz, 6H).

### Example 93

### Preparation of N-(3-acetamidobicyclo[1.1.1]pentan-1-yl)-2-(4-(5-(1-methyl-2-oxo-1,2,3,4-tetrahydroquinolin-6-yl)pyridin-3-yl)piperazin-1-yl)acetamide (compound 154):

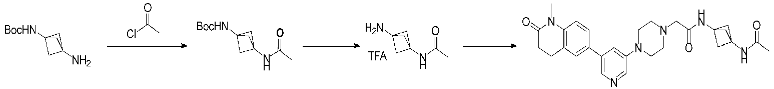

### Step 1: Synthesis of tert-butyl (3-aminobicyclo[1.1.1]pentan-1-yl)carbamate:

With reference to step 1 of example 88, using tert-butyl (3-aminobicyclo[1.1.1]pentan-1-yl)carbamate (530 mg, 1 eq) as the starting material, the product (290 mg) was obtained. LCMS (ESI) [M+H-56]⁺ =185.5.

### Step 2: Synthesis of N-(3-aminobicyclo[1.1.1]pentan-1-yl)acetamide trifluoroacetate:

With reference to step 2 of example 88, using the product of step 1 of example 93 (290 mg) as the starting material, the product (150 mg) was obtained. LCMS (ESI) [M+H]⁺ =141.6.

### Step 3: Synthesis of compound 154:

With reference to step 3 of example 90, using the product of preparation example 3 (60 mg, 1 eq) and the product of step 2 (40.09 mg) as starting materials, the reaction was stirred at room temperature for 2 hours. The residue was purified by flash chromatography (C18, 0.1% NH₃H₂O in H₂O/ACN) to obtain the target compound (37.38 mg). LCMS (ESI) [M+H]⁺ =503.3; ¹H NMR (400 MHz, DMSO-*d₆*) δ 8.40 (s, 1H), 8.30-8.25 (m, 3H), 7.65-7.60 (m, 2H), 7.52-7.48 (m, 1H), 7.19-7.15 (m, 1H), 3.37-3.34 (m, 2H), 3.31-3.30 (m, 2H), 3.29 (s, 3H), 2.97-2.91 (m, 4H), 2.62-2.55 (m, 6H), 2.18 (s, 6H), 1.75 (s, 3H).

### Example 94

### Preparation of N-isopropyl-2-(4-(5-(1-(methyl-d₃)-2-oxo-1,2,3,4-tetrahydroquinolin-6-yl)pyridin-3-yl)piperazin-1-yl)acetamide-2,2-d₂ (compound 155):

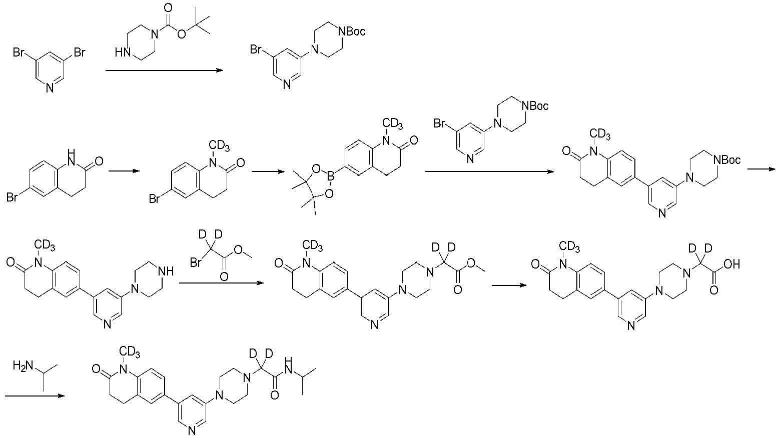

### Step 1: Synthesis of tert-butyl 4-(5-bromopyridin-3-yl)piperazine-1-carboxylate:

To a mixture of tert-butyl piperazine-1-carboxylate (30 g, 1 eq), 3,5-dibromopyridine (49.6 g), sodium tert-butoxide (23.22 g), 4,5-bis(diphenylphosphino)-9,9-dimethylxanthene (XANT PHOS, 5.59 g) and tris(dibenzylideneacetone)dipalladium (2.95 g) was added toluene (400 mL). The mixture was stirred at 100°C under a nitrogen atmosphere for 16 hours. To the reaction solution was added water (500 mL), and the mixture was extracted with ethyl acetate (500 mL*3). The organic phases were combined, dried over anhydrous sodium sulphate and filtered, and the filtrate was concentrated. The residue was purified by flash chromatography (silica gel, petroleum ether : ethyl acetate = 4 : 1) to afford the product (42 g). LCMS (ESI) [M+H]⁺ = 341.9; ¹H NMR(400 MHz, CDCl₃) δ 8.21 (d, *J =* 2.6 Hz, 1H), 8.15 (d, *J =* 1.8 Hz, 1H), 7.32-7.29 (m, 1H), 3.61-3.57 (m, 4H), 3.21-3.16 (m, 4H), 1.49 (s, 9H).

### Step 2: Synthesis of 6-bromo-1-(methyl-d₃)-3,4-dihydroquinolin-2(1H)-one:

6-Bromo-3,4-dihydroquinolin-2(1H)-one (4 g, 1 eq) was dissolved in N,N-dimethylformamide (40 mL). Sodium hydride (813.55 mg) was slowly added under ice-bath cooling, and the mixture was stirred at 0°C for 30 minutes. Deuterated iodomethane (3.01 g) was then added dropwise. After the dropwise addition was completed, the mixture was further stirred at room temperature for 3 hours. The reaction solution was quenched by adding water (120 mL), and extracted with ethyl acetate (70 mL*3). The combined organic phases were dried over anhydrous sodium sulphate and filtered. The filtrate was concentrated and purified by flash chromatography (silica gel, petroleum ether : ethyl acetate = 2 : 1) to afford the product (3.5 g). LCMS[ESI] [M+H]⁺=245.4 (isotope peak); ¹H NMR (400 MHz, CDCl₃) δ 7.36 (dd, *J =* 8.6, 2.3 Hz, 1H), 7.31-7.28 (m, 1H), 6.83 (d, *J =* 8.6 Hz, 1H), 2.91-2.86 (m, 2H), 2.66-2.62 (m, 2H).

### Step 3: Synthesis of 1-(methyl-d₃)-6-(4,4,5,5-tetramethyl-1,3,2-dioxaborolan-2-yl)-3,4-dihydroquinolin-2(1H)-one:

The product of step 2 (4 g, 1 eq), bis(pinacolato)diboron (8.36 g), [1,1'-bis(diphenylphosphino)ferrocene]dichloropalladium (1.21 g) and potassium acetate (4.84 g) were dissolved in 1,4-dioxane (60 mL). The mixture was stirred at 90°C under a nitrogen atmosphere for 16 hours. The reaction solution was filtered, and the filtrate was concentrated. The residue was purified by flash chromatography (silica gel, petroleum ether : ethyl acetate = 2 : 1) to obtain the product (3.5 g). LCMS[ESI] [M+H]⁺=291.3 (isotope peak).

### Step 4: Synthesis of tert-butyl 4-(5-(1-(methyl-d₃)-2-oxo-1,2,3,4-tetrahydroquinolin-6-yl)pyridin-3-yl)piperazine-1-carboxylate:

The product of step 1 (1.5 g, 1 eq), the product of step 3 (1.91 g), potassium carbonate (1.82 g) and [1,1'-bis(diphenylphosphino)ferrocene]dichloropalladium (321.37 mg) were dissolved in 1,4-dioxane (30 mL) and water (6 mL). The mixture was stirred at 80°C under a nitrogen atmosphere for 2 hours. The reaction solution was diluted with water (70 mL), and extracted with ethyl acetate (45 mL*3). The combined organic phases were dried over anhydrous sodium sulphate and filtered. The filtrate was concentrated and purified by flash chromatography (silica gel, petroleum ether : tetrahydrofuran = 2 : 1) to afford the product (1 g). LCMS (ESI) [M+H]⁺ =426.5; ¹H NMR (400 MHz, CDCl₃) δ 8.33 (d, *J* = 1.6 Hz, 1H), 8.28 (d, *J =* 2.6 Hz, 1H), 7.46 (dd, *J* = 8.4, 2.0 Hz, 1H), 7.38-7.36 (m, 1H), 7.31 (t, *J* = 2.1 Hz, 1H), 7.06 (d, *J =* 8.4 Hz, 1H), 3.65-3.61 (m, 4H), 3.27-3.22 (m, 4H), 3.01-2.96 (m, 2H), 2.73-2.68 (m, 2H), 1.49 (s, 9H).

### Step 5: Synthesis of 1-(methyl-d₃)-6-(5-(piperazin-1-yl)pyridin-3-yl)-3,4-dihydroquinolin-2(1H)-one:

The product of step 4 (1 g, 1 eq) was dissolved in dichloromethane (10 mL), and trifluoroacetic acid (3 mL) was added. The mixture was stirred at room temperature for 16 hours. The reaction solution was concentrated to obtain the product (750 mg). LCMS (ESI) [M+H]⁺ =326.5.

### Step 6: Synthesis of methyl 2-(4-(5-(1-(methyl-d₃)-2-oxo-1,2,3,4-tetrahydroquinolin-6-yl)pyridin-3-yl)piperazin-1-yl)acetate-d₂:

The product of step 5 (750 mg, 1 eq) was dissolved in N,N-dimethylformamide (10 mL). Methyl 2-bromoacetate-d₂ (0.43 mL) and potassium carbonate (955.6 mg) were added. The mixture was stirred at room temperature for 2 hours. The reaction solution was diluted with water (55 mL), and extracted with ethyl acetate (35 mL*3). The combined organic phases were dried over anhydrous sodium sulphate and filtered. The filtrate was concentrated and purified by flash chromatography (silica gel, petroleum ether : tetrahydrofuran = 1 : 4) to afford the product (660 mg). ¹H NMR (400 MHz, DMSO-*d₆*) δ 8.27 (dd, *J =* 11.8, 2.2 Hz, 2H), 7.65-7.59 (m, 2H), 7.51-7.47 (m, 1H), 7.19-7.14 (m, 1H), 3.64 (s, 3H), 3.31-3.27 (m, 4H), 2.98-2.91 (m, 2H), 2.70-2.65 (m, 4H), 2.61-2.56 (m, 2H).

### Step 7: Synthesis of 2-(4-(5-(1-(methyl-d₃)-2-oxo-1,2,3,4-tetrahydroquinolin-6-yl)pyridin-3-yl)piperazin-1-yl)acetic-2,2-d₂ acid:

The product of step 6 (660 mg, 1 eq) was dissolved in methanol (8 mL) and water (8 mL). Lithium hydroxide (197.83 mg) was added. The mixture was stirred at 50°C for 2 hours. The reaction solution was adjusted to pH 5 to 6 by dropwise adding dilute hydrochloric acid (3 M). After a solid precipitated out, the mixture was filtered, and the filter cake was dried to obtain the product (600 mg). LCMS (ESI) [M+H]⁺ =386.5.

### Step 8: Synthesis of compound 155:

The product of step 7 (300 mg, 1 eq) and HATU (355.11 mg) were dissolved in N,N-dimethylformamide (5 mL). In an ice bath, triethylamine (236.26 mg) and isopropylamine (55.2 mg) were sequentially added. The mixture was stirred at room temperature for 2 hours. The reaction solution was filtered, and the filtrate was concentrated. The residue was purified by flash chromatography (C18, 0.1% NH₄HCO₃ in H₂O/ACN) to obtain the target compound (114.5 mg). LCMS (ESI) [M+H]⁺ =427.5; ¹H NMR (400 MHz, DMSO-*d₆*) δ 8.30-8.25 (m, 2H), 7.65-7.60 (m, 2H), 7.56-7.48 (m, 2H), 7.18-7.14 (m, 1H), 3.95-3.85 (m, 1H), 3.35-3.33 (m, 2H), 3.31-3.29 (m, 2H), 2.97-2.92 (m, 2H), 2.63-2.56 (m, 6H), 1.08 (d, *J =* 6.6 Hz, 6H).

### Example 95

### Preparation of N-isopropyl-2-(3-((5-(1-methyl-2-oxo-1,2,3,4-tetrahydroquinolin-6-yl)pyridin-3-yl)amino)azetidin-1-yl)acetamide (compound 156):

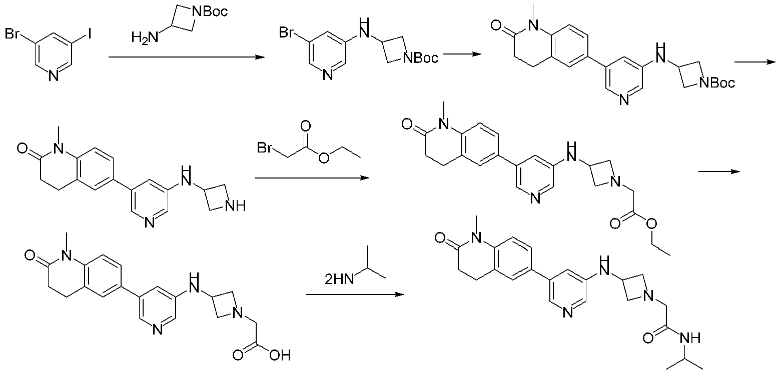

### Step 1: Synthesis of tert-butyl 3-((5-bromopyridin-3-yl)amino)azetidine-1-carboxylate:

3-Bromo-5-iodopyridine (5 g, 1 eq) was dissolved in N,N-dimethylformamide (50 mL). Tert-butyl 3-aminoazetidine-1-carboxylate (3.94 g), cuprous bromide (505.3 mg), potassium phosphate (7.48 g) and [1,1'-binaphthalene]-2,2'-diol (1.01 g) were added, and the mixture was stirred at 85°C for 3 hours. To the reaction solution was added water (100 mL), and the mixture was extracted with ethyl acetate (100 mL*3). The organic phases were combined, dried over anhydrous sodium sulphate and filtered, and the filtrate was concentrated. The residue was purified by flash chromatography (silica gel, petroleum ether : ethyl acetate = 1 : 1) to obtain the target product (2 g). LCMS (ESI) [M+H] ⁺ = 330.1 (isotope peak); ¹H NMR (400 MHz, CDCl₃) δ 8.07 (d, *J =* 1.7 Hz, 1H), 7.88 (d, *J =* 2.3 Hz, 1H), 6.91 (t, *J =* 2.2 Hz, 1H), 4.36-4.29 (m, 2H), 4.29-4.23 (m, 1H), 4.21-4.13 (m, 1H), 3.78-3.70 (m, 2H), 1.45 (s, 9H).

### Step 2: Synthesis of tert-butyl 3-((5-(1-methyl-2-oxo-1,2,3,4-tetrahydroquinolin-6-yl)pyridin-3-yl)amino)azetidine-1-carboxylate:

To a mixture of tert-butyl 3-((5-bromopyridin-3-yl)amino)azetidine-1-carboxylate (1.5 g, 4.57 mmol, 1 eq), the product of preparation example 1 (1.71 g), potassium carbonate (1.9 g) and [1,1'-bis(diphenylphosphino)ferrocene]dichloropalladium (335.34 mg) were added dioxane (20 mL) and water (4 mL). Under a nitrogen atmosphere, the mixture was stirred at 80°C for 3 hours. To the reaction solution was added water (60 mL), and the mixture was extracted with ethyl acetate (60 mL*3). The organic phases were combined, dried over anhydrous sodium sulphate and filtered, and the filtrate was concentrated. The residue was purified by flash chromatography (silica gel, petroleum ether : tetrahydrofuran =1 : 4) to obtain the product (1.7 g). LCMS (ESI) [M+H] ⁺ = 409.4; ¹H NMR (400 MHz, CDCl₃) δ 8.24 (d, *J =* 1.5 Hz, 1H), 7.94 (d, *J =* 2.6 Hz, 1H), 7.47-7.41 (m, 1H), 7.39-7.32 (m, 1H), 7.07 (d, *J =* 8.4 Hz, 1H), 6.95-6.88 (m, 1H), 4.37-4.20 (m, 4H), 3.88-3.72 (m, 2H), 3.40 (s, 3H), 3.03-2.93 (m, 2H), 2.75-2.65 (m, 2H), 1.45 (s, 9H).

### Step 3: Synthesis of 6-(5-(azetidin-3-ylamino)pyridin-3-yl)-1-methyl-3,4-dihydroquinolin-2(1H)-one:

With reference to step 2 of example 88, using the product of step 2 (1.7 g) as the starting material, the separation and purification were carried out by reversed-phase chromatography (acetonitrile/0.1% aqueous ammonia solution system, 25%) to obtain the product (1.2 g). LCMS (ESI) [M+H] ⁺ = 309.2.

### Step 4: Synthesis of ethyl 2-(3-(((5-(1-methyl-2-oxo-1,2,3,4-tetrahydroquinolin-6-yl)pyridin-3-yl)amino)azetidin-1-yl)acetate:

With reference to step 6 of example 94, using the product of step 5 (800 mg, 1 eq) as the starting material, the separation and purification were carried out by reversed-phase chromatography (acetonitrile/0.1% trifluoroacetic acid aqueous solution system) to obtain the product (600 mg). LCMS (ESI) [M+H] ⁺ = 395.2.

### Step 5: Synthesis of 2-(3-((5-(1-methyl-2-oxo-1,2,3,4-tetrahydroquinolin-6-yl)pyridin-3-yl)amino)azetidin-1-yl)acetic acid:

With reference to step 7 of example 94, using the product of step 4 (200 mg, 1 eq) as the starting material, the separation and purification were carried out by reversed-phase chromatography (acetonitrile/pure water system) to obtain the product (100 mg). LCMS (ESI) [M+H] ⁺ = 367.2; ¹H NMR (400 MHz, DMSO-*d₆*) δ 8.12 (s, 1H), 7.89 (d, *J* = 2.3 Hz, 1H), 7.62-7.50 (m, 2H), 7.17 (d, *J =* 8.5 Hz, 1H), 7.12-7.01 (m, 1H), 6.53 (d, J = 7.1 Hz, 1H), 4.37-4.24 (m, 1H), 4.09-4.02 (m, 2H), 3.37-3.35 (m, 4H), 3.28 (s, 3H), 2.98-2.92 (m, 2H), 2.62-2.55 (m, 2H).

### Step 6: Synthesis of compound 156:

With reference to step 3 of example 88, using the product of step 5 (50 mg, 1 eq) and propan-2-amine (8.07 mg) as starting materials, the separation and purification were carried out by reversed-phase chromatography (0.1% aqueous ammonia solution, acetonitrile) to obtain the target compound (21.27 mg). LCMS (ESI) [M+H] ⁺ = 408.0; ¹H NMR (400 MHz, DMSO-*d₆*) δ 8.09 (d, *J =* 1.6 Hz, 1H), 7.88 (d, *J =* 2.5 Hz, 1H), 7.58-7.50 (m, 2H), 7.39 (d, *J =* 8.1 Hz, 1H), 7.21-7.13 (m, 1H), 7.07-6.99 (m, 1H), 6.41 (d, *J* = 7.0 Hz, 1H), 4.18-4.09 (m, 1H), 3.91-3.82 (m, 1H), 3.80-3.73 (m, 2H), 3.28 (s, 3H), 3.02 (s, 2H), 3.00-2.91 (m, 4H), 2.61-2.55 (m, 2H), 1.06 (d, *J =* 6.6 Hz, 6H).

### Example 96

### Preparation of N-methyl-2-(4-(4-(5-(1-methyl-2-oxo-1,2,3,4-tetrahydroquinolin-6-yl)pyridin-3-yl)piperazin-1-yl)methyl)-1H-1,2,3-triazol-1-yl)acetamide (compound 157):

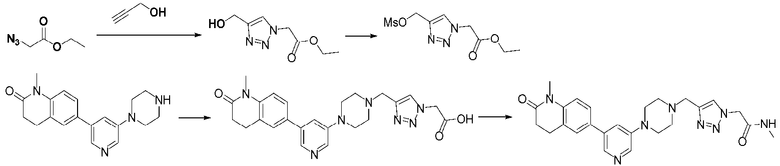

### Step 1: Synthesis of ethyl 2-(4-(hydroxymethyl)-1H-1,2,3-triazol-1-yl)acetate:

Sodium ascorbate (460 mg) was added to a solution of ethyl 2-azidoacetate (3 g, 1 eq), propan-2-yn-1-ol (1.95 g) and copper sulphate pentahydrate (58 mg) in tert-butanol (30 mL) and water (30 mL). The mixture was stirred at room temperature for 16 hours. Water (50 mL) was added, and the mixture was extracted with ethyl acetate (50 mL*3). The organic phases were combined, dried over anhydrous sodium sulphate and filtered, and the filtrate was concentrated to obtain the product (1.4 g). LCMS (ESI) [M+H]⁺ =186.1; ¹H NMR (400 MHz, CDCl₃) δ 7.69 (s, 1H), 5.16 (s, 2H), 4.80 (s, 2H), 4.26 (dd, *J =* 8.3, 5.9 Hz, 2H), 1.31 (t, *J =* 7.1 Hz, 3H).

### Step 2: Synthesis of ethyl 2-(4-(((methylsulphonyl)oxy)methyl)-1H-1,2,3-triazol-1-yl)acetate:

At 0°C, methanesulphonyl chloride (1.73 g) was added to a solution of ethyl 2-(4-(hydroxymethyl)-1H-1,2,3-triazol-1-yl)acetate (1.4 g, 1 eq) and triethylamine (2.3 g) in dichloromethane (20 mL). The mixture was stirred at room temperature for 1 hour. The reaction solution was concentrated to obtain the product (1.78 g, crude). LCMS (ESI) [M+H]⁺ =264.1.

### Step 3: Synthesis of 2-(4-((4-(5-(1-methyl-2-oxo-1,2,3,4-tetrahydroquinolin-6-yl)pyridin-3-yl)piperazin-1-yl)methyl)-1H-1,2,3-triazol-1-yl)acetic acid:

Potassium carbonate (578 mg) was added to a solution of intermediate 1 (0.9 g, 1 eq) and the product of step 2 (1.47 g) in acetonitrile (10 mL). The mixture was stirred at 80°C for 16 hours. The reaction solution was filtered, and the filtrate was concentrated. The residue was purified by reversed-phase chromatography (C18, 0.1% NH₄HCO₃ in water/MeCN=80%/20%) to obtain the product (200 mg). LCMS (ESI) [M+H]⁺ =462.3.

### Step 4: Synthesis of compound 157:

With reference to step 3 of example 88, using the product of step 3 (160 mg, 1 eq) and methylamine hydrochloride (47 mg) as starting materials, the reaction was stirred at room temperature for 2 hours. To the reaction solution was added water (10 mL), and the mixture was extracted with ethyl acetate (10 mL*3). The organic phases were combined, dried over anhydrous sodium sulphate and filtered, and the filtrate was concentrated. The residue was purified by prep-HPLC (C18, 0.1% NH₃.H₂O in water/MeCN) to obtain the target compound (16.23 mg). LCMS (ESI) [M+H]⁺ =475.2; ¹H NMR (400 MHz, DMSO-*d₆*) δ 8.30-8.19 (m, 3H), 7.97 (s, 1H), 7.65-7.59 (m, 2H), 7.49 (s, 1H), 7.19-7.14 (m, 1H), 5.05 (s, 2H), 3.64 (s, 2H), 3.30-3.25 (m, 7H), 2.97-2.91 (m, 2H), 2.64 (d, *J* = 4.6 Hz, 3H), 2.61-2.55 (m, 6H).

### Example 97

### Preparation of 3-(4-(5-(1-methyl-4,5-dihydro-[1,2,4]triazolo[4,3-a]quinolin-7-yl)pyridin-3-yl)piperazin-1-yl)propan-1-ol (compound 158):

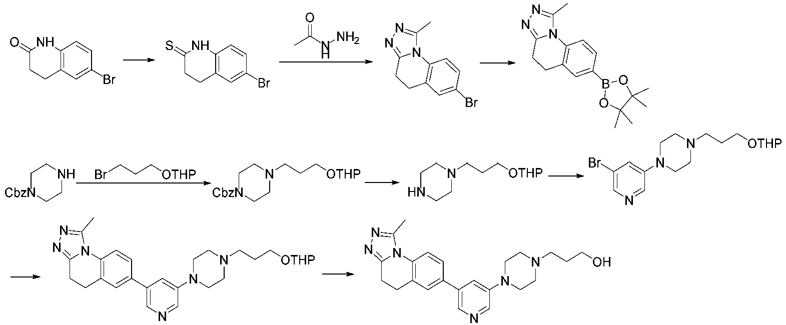

### Step 1: Synthesis of 6-bromo-3,4-dihydroquinolin-2(1H)-thione:

6-Bromo-3,4-dihydroquinolin-2(1H)-one (5 g, 1 eq) and Lawesson's reagent (13.42 g) were dissolved in toluene (100 mL), and the mixture was refluxed at 120°C for 3 hours. The reaction solution was filtered, and washed with dichloromethane. The filter cake was purified by flash chromatography (silica gel, petroleum ether : tetrahydrofuran = 1 : 1) to obtain the product (3.5 g). LCMS (ESI) [M+H]⁺ =244.3.

### Step 2: Synthesis of 7-bromo-1-methyl-4,5-dihydro-[1,2,4]triazolo[4,3-a]quinoline:

The product of step 1 (3.5 g, 1 eq) and acetylhydrazine (1.5 g) were dissolved in cyclohexanol (60 mL). The mixture was stirred at 170°C for 6 hours. The reaction solution was concentrated, and the residue was purified by flash chromatography (silica gel, petroleum ether : tetrahydrofuran = 1 : 1) to obtain the product (1.5 g). LCMS (ESI) [M+H]⁺ =264.3; ¹H NMR (400 MHz, DMSO-*d₆*) δ 7.74-7.71 (m, 1H), 7.61-7.59 (m, 2H), 3.00-2.96 (m, 4H), 2.65 (s, 3H).

### Step 3: Synthesis of 1-methyl-7-(4,4,5,5-tetramethyl-1,3,2-dioxaborolan-2-yl)-4,5-dihydro-[1,2,4]triazolo[4,3-a]quinoline:

The product of step 2 (300 mg, 1 eq), bis(pinacolato)diboron (432.66 mg), 1,1'-bis(diphenylphosphino)ferrocene dichloropalladium (83.34 mg) and potassium acetate (334.42 mg) were dissolved in 1,4-dioxane (10 mL). Under a nitrogen atmosphere, the mixture was stirred at 90°C for 16 hours. The reaction solution was filtered, and the filtrate was concentrated. The residue was purified by flash chromatography (silica gel, petroleum ether : tetrahydrofuran = 1 : 2) to obtain the product (330 mg). LCMS (ESI) [M+H]⁺ =312.3.

### Step 4: Synthesis of benzyl 4-(3-((tetrahydro-2H-pyran-2-yl)oxy)propyl)piperazine-1-carboxylate:

Benzyl piperazine-1-carboxylate (6 g, 1 eq) and potassium carbonate (11.29 g) were dissolved in acetone (60 mL). In an ice bath, a solution of 2-(3-bromopropoxy)tetrahydro-2H-pyran (6.08 g) in acetone (20 mL) was added dropwise. The mixture was stirred at room temperature for 16 hours. The reaction solution was diluted with water (150 mL), and extracted with dichloromethane (80 mL*3). The organic phases were combined, dried over anhydrous sodium sulphate and filtered, and the filtrate was concentrated. The residue was purified by flash chromatography (silica gel, petroleum ether : ethyl acetate = 1 : 1) to obtain the product (5 g). LCMS (ESI) [M+H]⁺ =363.5.

### Step 5: Synthesis of 1-(3-((tetrahydro-2H-pyran-2-yl)oxy)propyl)piperazine:

The product of step 4 (4.6 g, 1 eq) was dissolved in methanol (80 mL). Palladium on carbon (13.51 g) was added. The system was purged with hydrogen balloon three times, and the mixture was stirred at room temperature for 16 hours. The reaction solution was filtered, and the filtrate was concentrated to obtain the product (2.4 g). LCMS (ESI) [M+H]⁺ =229.5; ¹H NMR (400 MHz, CDCl₃) δ 4.60-4.55 (m, 1H), 3.89-3.74 (m, 2H), 3.52-3.40 (m, 2H), 2.92 (t, *J* = 4.9 Hz, 4H), 2.51-2.40 (m, 6H), 1.83-1.68 (m, 4H), 1.60-1.49 (m, 4H).

### Step 6: Synthesis of 1-(5-bromopyridin-3-yl)-4-(3-((tetrahydro-2H-pyran-2-yl)oxy)propyl)piperazine:

3,5-Dibromopyridine (2.18 g), the product of step 5 (2.1 g, 1 eq), sodium tert-butoxide (2.65 g), tris(dibenzylideneacetone)dipalladium (842.21 mg) and 2,2'-bis(diphenylphosphino)-1,1'-binaphthyl (BINAP) (1.15 g) were dissolved in toluene (50 mL). Under a nitrogen atmosphere, the mixture was stirred at 100°C for 16 hours. The reaction solution was filtered and washed with tetrahydrofuran, and the filtrate was concentrated. The residue was purified by flash chromatography (silica gel, petroleum ether : tetrahydrofuran = 2 : 1) to obtain the product (620 mg). LCMS (ESI) [M+H]⁺ =386.1; ¹H NMR (400 MHz, DMSO-*d₆*) δ 8.30-8.27 (m, 1H), 8.05-8.00 (m, 1H), 7.54-7.50 (m, 1H), 4.56-4.52 (m, 1H), 3.77-3.64 (m, 2H), 3.44-3.36 (m, 2H), 3.25-3.21 (m, 4H), 2.49-2.45 (m, 4H), 2.40-2.36 (m, 2H), 1.75-1.68 (m, 3H), 1.63-1.57 (m, 1H), 1.50-1.41 (m, 4H).

### Step 7: Synthesis of 1-methyl-7-(5-(4-(3-((tetrahydro-2H-pyran-2-yl)oxy)propyl)piperazin-1-yl)pyridin-3-yl)-4,5-dihydro-[1,2,4]triazolo[4,3-a]quinoline:

The product of step 6 (300 mg, 1 eq), the product of step 3 (364.38 mg), potassium phosphate (497.11 mg) and 1,1'-bis(diphenylphosphino)ferrocene dichloropalladium (50.88 mg) were dissolved in 1,4-dioxane (5 mL) and water (1 mL). Under a nitrogen atmosphere, the mixture was stirred at 80°C for 4 hours. The reaction solution was diluted with water (50 mL), and extracted with ethyl acetate (25 mL*3). The organic phases were combined, dried over anhydrous sodium sulphate and filtered, and the filtrate was concentrated. The residue was purified by flash chromatography (silica gel, dichloromethane : methanol = 10 : 1) to obtain the product (150 mg). LCMS (ESI) [M+H]⁺ =489.4; ¹H NMR (400 MHz, DMSO-*d₆*) δ 8.35-8.29 (m, 2H), 7.88-7.85 (m, 1H), 7.78-7.72 (m, 2H), 7.57-7.53 (m, 1H), 4.57-4.53 (m, 1H), 3.79-3.65 (m, 2H), 3.45-3.38 (m, 2H), 3.30-3.27 (m, 4H), 3.07-3.02 (m, 4H), 2.71 (s, 3H), 2.56-2.53 (m, 4H), 2.43-2.39 (m, 2H), 1.76-1.64 (m, 4H), 1.50-1.43 (m, 4H).

### Step 8: Synthesis of 3-(4-(5-(1-methyl-4,5-dihydro-[1,2,4]triazolo[4,3-a]quinolin-7-yl)pyridin-3-yl)piperazin-1-yl)propan-1-ol:

To the product of step 7 (150 mg, 1 eq) was added hydrogen chloride-dioxane solution (10 mL, 4 M), and the mixture was stirred at room temperature for 3 hours. The reaction solution was adjusted to pH 6 to 7 with aqueous sodium hydroxide solution (3 M) and filtered, and the filtrate was concentrated. The residue was purified by flash chromatography (C18, 0.1% NH₃H₂O in H₂O/ACN) to obtain the target compound (43.66 mg). LCMS (ESI) [M+H]⁺ =405.3; ¹H NMR (400 MHz, DMSO-*d₆*) δ 8.34-8.30 (m, 2H), 7.87 (s, 1H), 7.78-7.72 (m, 2H), 7.56-7.53 (m, 1H), 4.47 (s, 1H), 3.47-3.45 (m, 2H), 3.31-3.28 (m, 4H), 3.04 (s, 4H), 2.70 (s, 3H), 2.55 --2.52 (m, 4H), 2.42-2.37 (m, 2H), 1.66-1.59 (m, 2H).

### Example 98

### Preparation of N-isopropyl-2-(4-(5-(1-(methyl-d₃)-2-oxo-1,2,3,4-tetrahydroquinolin-6-yl)pyridin-3-yl)piperazin-1-yl)acetamide (compound 159):

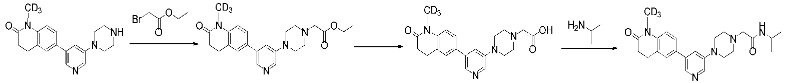

### Step 1: Synthesis of ethyl 2-(4-(5-(1-(methyl-d₃)-2-oxo-1,2,3,4-tetrahydroquinolin-6-yl)pyridin-3-yl)piperazin-1-yl)acetate:

With reference to step 6 of example 94, using 1-(methyl-d₃)-6-(5-(piperazin-1-yl)pyridin-3-yl)-3,4-dihydroquinolin-2(1H)-one (2 g, 1 eq) and ethyl 2-bromoacetate (923.73 mg). The target product (1 g) was obtained through separation and purification via reversed-phase chromatography. LCMS (ESI) [M+H]⁺ = 412.3; ¹H NMR (400 MHz, DMSO-*d₆*) δ 8.33-8.22 (m, 2H), 7.66-7.58 (m, 2H), 7.53-7.46 (m, 1H), 7.19-7.13 (m, 1H), 4.17-4.05 (m, 2H), 3.32-3.25 (m, 6H), 3.00-2.89 (m, 2H), 2.72-2.66 (m, 4H), 2.62-2.55 (m, 2H), 1.21 (t, *J* = 7.1 Hz, 3H).

### Step 2: Synthesis of 2-(4-(5-(1-(methyl-d₃)-2-oxo-1,2,3,4-tetrahydroquinolin-6-yl)pyridin-3-yl)piperazin-1-yl)acetic acid:

With reference to step 7 of example 94, using the product of step 1 (1 g, 1 eq) as the starting material. The target product (900 mg, yield 96%) was obtained through separation and purification via reversed-phase chromatography (acetonitrile/0.1% aqueous formic acid solution =25%). LCMS (ESI) [M+H]⁺ = 384.2; ¹H NMR (400 MHz, DMSO-*d₆*) δ 8.29 (d, *J =* 1.7 Hz, 1H), 8.26 (d*, J =* 2.7 Hz, 1H), 7.67-7.59 (m, 2H), 7.55-7.46 (m, 1H), 7.20-7.12 (m, 1H), 3.34-3.30 (m, 4H), 3.23 (s, 2H), 2.97-2.91 (m, 2H), 2.78-2.71 (m, 4H), 2.61-2.55 (m, 2H).

### Step 3: Synthesis of compound 159:

With reference to step 3 of example 88, using the product of step 2 (800 mg, 1 eq) and isopropylamine (123.32 mg) as starting materials. The target compound (393.15 mg) was obtained through separation and purification via reversed-phase chromatography (C18; acetonitrile/0.1% aqueous ammonia solution system). LCMS (ESI) [M+H]⁺ = 425.4; ¹H NMR (400 MHz, DMSO-*d₆*) δ 8.29 (d, *J =* 1.7 Hz, 1H), 8.27 (d, *J =* 2.7 Hz, 1H), 7.67-7.60 (m, 2H), 7.58-7.46 (m, 2H), 7.21-7.13 (m, 1H), 3.91 (tt, *J =* 13.2, 6.6 Hz, 1H), 3.33-3.29 (m, 4H), 3.00-2.88 (m, 4H), 2.66-2.54 (m, 6H), 1.08 (d, *J =* 6.6 Hz, 6H).

### Example 99

### Preparation of 1-isopropyl-3-(2-(4-(5-(1-methyl-2-oxo-1,2,3,4-tetrahydroquinolin-6-yl)pyridin-3-yl)piperazin-1-yl)ethyl)urea (compound 160):

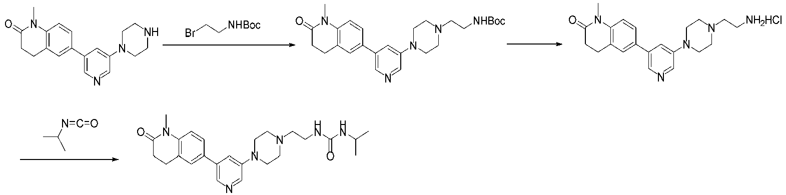

### Step 1: Synthesis of tert-butyl 2-(4-(5-(1-methyl-2-oxo-1,2,3,4-tetrahydroquinolin-6-yl)pyridin-3-yl)piperazin-1-yl)ethyl)carbamate:

Intermediate 1 (200 mg, 1 eq) was dissolved in acetonitrile (3 mL). Potassium carbonate (171.38 mg) and tert-butyl (2-bromoethyl)carbamate (166.73 mg) were added, and the mixture was stirred at 50°C for 16 hours. The reaction solution was filtered, and the filtrate was concentrated. The residue was purified by flash chromatography (silica gel, petroleum ether : tetrahydrofuran = 1 : 4) to obtain the product (200 mg). LCMS (ESI) [M+H]⁺ = 466.4; ¹H NMR (400 MHz, CDCl₃) δ 8.30 (d, *J =* 1.8 Hz, 1H), 8.27 (d, *J =* 2.7 Hz, 1H), 7.46 (dd, *J =* 8.4, 2.1 Hz, 1H), 7.40-7.35 (m, 1H), 7.34-7.29 (m, 1H), 7.07 (d, *J =* 8.4 Hz, 1H), 4.98 (s, 1H), 3.40 (s, 3H), 3.36-3.24 (m, 6H), 3.05-2.91 (m, 2H), 2.75-2.68 (m, 2H), 2.68-2.62 (m, 4H), 2.59-2.48 (m, 2H), 1.46 (s, 9H).

### Step 2: Synthesis of 6-(5-(4-(2-aminoethyl)piperazin-1-yl)pyridin-3-yl)-1-methyl-3,4-dihydroquinolin-2(1H)-one hydrochloride:

The product of step 1 (200 mg, 1 eq) was dissolved in dioxane (3 mL). A solution of hydrogen chloride in dioxane (3 mL, 4 M) was added, and the mixture was stirred at room temperature for 2 hours. The reaction solution was concentrated to obtain the product (150 mg, crude). LCMS (ESI) [M+H] ⁺ = 366.3.

### Step 3: Synthesis of 1-isopropyl-3-(2-(4-(5-(1-methyl-2-oxo-1,2,3,4-tetrahydroquinolin-6-yl)pyridin-3-yl)piperazin-1-yl)ethyl)urea:

The product of step 2 (150 mg, 1 eq) was dissolved in acetonitrile (3 mL). Triethylamine (75.53 mg) and isopropyl isocyanate (0.05 mL) were added, and the mixture was stirred at room temperature for 1 hour. The reaction solution was purified by reversed-phase chromatography (0.1% aqueous ammonia solution/acetonitrile) to obtain the target compound (121.74 mg). LCMS (ESI) [M+H]⁺ = 451.0; ¹H NMR (400 MHz, DMSO-*d₆*) δ 8.29 (d, *J* = 1.8 Hz, 1H), 8.26 (d, *J =* 2.7 Hz, 1H), 7.65-7.58 (m, 2H), 7.52-7.47 (m, 1H), 7.20-7.13 (m, 1H), 5.85 (d, *J* = 7.6 Hz, 1H), 5.63 (t, *J =* 5.4 Hz, 1H), 3.71-3.59 (m, 1H), 3.32-3.24 (m, 7H), 3.19-3.10 (m, 2H), 2.98-2.90 (m, 2H), 2.62-2.53 (m, 6H), 2.38 (t, *J =* 6.4 Hz, 2H), 1.02 (d, *J =* 6.5 Hz, 6H).

### Example 100

### Preparation of 2-(4-(5-(1-methyl-2-oxo-1,2,3,4-tetrahydroquinolin-6-yl)pyridin-3-yl)piperazin-1-yl)-N-(spiro[2.2]pentan-1-yl)acetamide (compound 161):

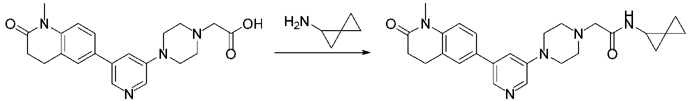

With reference to step 3 of example 88, using the product of preparation example 3 (50 mg, 1 eq) and spiro[2.2]pentan-1-amine (10.93 mg) as starting materials, the separation and purification were carried out by reversed-phase chromatography (0.1% aqueous ammonia solution, acetonitrile) to obtain the target compound (13.15 mg). LCMS (ESI) [M+H]⁺ = 446.2; ¹H NMR (400 MHz, DMSO-*d₆*) δ 8.29 (d, *J =* 1.7 Hz, 1H), 8.26 (d, *J* = 2.6 Hz, 1H), 7.77 (d, *J =* 4.1 Hz, 1H), 7.66-7.60 (m, 2H), 7.53-7.48 (m, 1H), 7.20-7.15 (m, 1H), 3.32-3.29 (m, 4H), 3.29 (s, 3H), 2.99 (s, 2H), 2.99-2.90 (m, 3H), 2.65-2.54 (m, 6H), 1.21-1.15 (m, 1H), 0.94-0.85 (m, 2H), 0.85-0.69 (m, 3H).

### Example 101

### Preparation of 6-(5-(4-(1-ethyl-1H-pyrazole-4-carbonyl)piperazin-1-yl)pyridin-3-yl)-1-methyl-3,4-dihydroquinolin-2(1H)-one (compound 162):

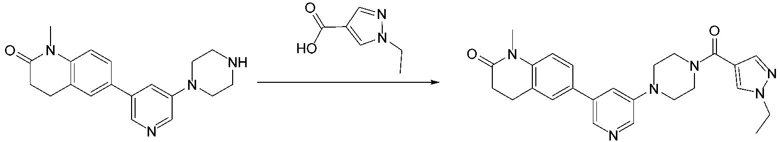

With reference to step 3 of example 90, using 1-ethyl-1H-pyrazole-4-carboxylic acid (100 mg, 1 eq) and intermediate 1 (230.06 mg) as starting materials, the separation and purification were carried out by flash chromatography (C18, 0.1% NH₃H₂O in H₂O/ACN) to obtain the target compound (100.93 mg). LCMS (ESI) [M+H]⁺ =445.2; ¹H NMR (400 MHz, DMSO-*d₆*) δ 8.35-8.26 (m, 2H), 8.15 (s, 1H), 7.72 (s, 1H), 7.66-7.61 (m, 2H), 7.56-7.52 (m, 1H), 7.20-7.16 (m, 1H), 4.16 (q, J = 7.3 Hz, 2H), 3.83-3.73 (m, 4H), 3.37-3.33 (m, 4H), 3.29 (s, 3H), 2.97-2.92 (m, 2H), 2.62-2.56 (m, 2H), 1.39 (t, *J =* 7.3 Hz, 3H).

### Example 102

### Preparation of N-isopropyl-2-(4-(5-(2-oxo-1,2,3,4-tetrahydroquinolin-6-yl)pyridin-3-yl)piperazin-1-yl)acetamide (compound 163):

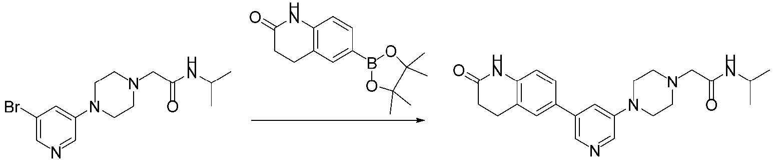

To a mixture of the product of preparation example 4 (200 mg, 1 eq), 6-(4,4,5,5-tetramethyl-1,3,2-dioxaborolan-2-yl)-3,4-dihydroquinolin-2(1H)-one (160.08 mg), potassium carbonate (243.01 mg) and 1,1'-bis(diphenylphosphino)ferrocene dichloropalladium (43 mg) were added dioxane (5 mL) and water (1 mL), and the mixture was stirred at 80°C for 3 hours. To the reaction solution was added water (20 mL), and the mixture was extracted with ethyl acetate (20 mL*3). The organic phases were combined, dried over anhydrous sodium sulphate and filtered, and the filtrate was concentrated. The residue was purified by reversed-phase chromatography (0.1% aqueous ammonia solution/acetonitrile system) to obtain the target compound (112.72 mg). LCMS (ESI) [M+H]⁺ = 408.0; ¹H NMR (400 MHz, DMSO-*d₆*) δ 10.17 (s, 1H), 8.24 (d, *J =* 3.4 Hz, 2H), 7.59-7.55 (m, 1H), 7.55-7.48 (m, 2H), 7.48-7.44 (m, 1H), 6.94 (d, *J =* 8.2 Hz, 1H), 3.98-3.84 (m, 1H), 3.31-3.26 (m, 4H), 2.98-2.91 (m, 4H), 2.63-2.57 (m, 4H), 2.49-2.46 (m, 2H), 1.08 (d, *J =* 6.6 Hz, 6H).

### Example 103

### Preparation of N-isopropyl-2-(4-(5-(1-methyl-2-oxoindolin-5-yl)pyridin-3-yl)piperazin-1-yl)acetamide (compound 164):

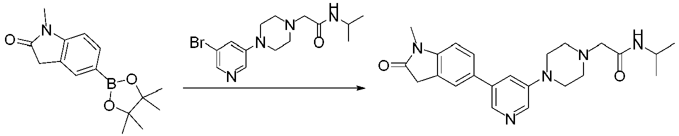

With reference to example 102, using the product of preparation example 4 (50 mg, 1 eq) and 1-methyl-5-(4,4,5,5-tetramethyl-1,3,2-dioxaborolan-2-yl)indol-2-one (40.02 mg) as starting materials, the separation and purification were carried out by reversed-phase chromatography (0.1% aqueous ammonia solution/acetonitrile system) to obtain the target compound (38.6 mg). LCMS (ESI) [M+H]⁺ = 408.0; ¹H NMR (400 MHz, DMSO-*d₆*) δ 8.31-8.22 (m, 2H), 7.68-7.62 (m, 2H), 7.52 (d, *J =* 8.1 Hz, 1H), 7.49-7.45 (m, 1H), 7.10-7.05 (m, 1H), 3.96-3.85 (m, 1H), 3.61 (s, 2H), 3.32-3.29 (m, 4H), 3.16 (s, 3H), 2.96 (s, 2H), 2.64-2.56 (m, 4H), 1.08 (d, *J =* 6.6 Hz, 6H).

### Example 104

### Preparation of 1-methyl-6-(5-(4-(2-oxopyrrolidin-3-yl)piperazin-1-yl)pyridin-3-yl)-3,4-dihydroquinolin-2(1H)-one (compound 165):

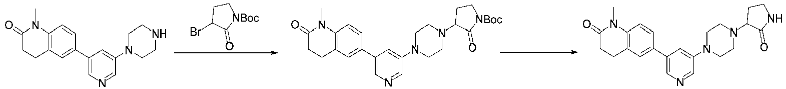

### Step 1: Synthesis of tert-butyl 3-(4-(5-(1-methyl-2-oxo-1,2,3,4-tetrahydroquinolin-6-yl)pyridin-3-yl)piperazin-1-yl)-2-oxopyrrolidine-1-carboxylate:

Intermediate 1 (60 mg, 1 eq) and potassium carbonate (77.16 mg) were dissolved in acetonitrile (5 mL). Tert-butyl 3-bromo-2-oxopyrrolidine-1-carboxylate (49.15 mg) was added. The mixture was stirred at 40°C for 16 hours. The reaction solution was concentrated, and the residue was purified by reversed-phase chromatography (C18, 0.1% TFA in H₂O/ACN) to obtain the product (50 mg). LCMS (ESI) [M+H]⁺ =506.3.

### Step 2: Synthesis of compound 165:

The product of step 1 (50 mg, 1 eq) was dissolved in dichloromethane (3 mL). A solution of hydrogen chloride in dioxane (3 mL, 4 M) was added. The mixture was stirred at room temperature for 2 hours. The reaction solution was concentrated, and the residue was dissolved in a small amount of tetrahydrofuran (1 mL). The pH was adjusted to neutral with aqueous sodium hydroxide solution (3 M), and the mixture was filtered. The filtrate was concentrated, and the residue was purified by reversed-phase chromatography (C18, 0.1 % NH₃H₂O in H₂O/ACN) to obtain the target compound (21.09 mg). LCMS (ESI) [M+H]⁺ =406.2; ¹H NMR (400 MHz, DMSO-*d₆*) δ 8.29-8.24 (m, 2H), 7.74 (s, 1H), 7.64-7.60 (m, 2H), 7.50-7.46 (m, 1H), 7.20-7.15 (m, 1H), 3.30-3.25 (m, 8H), 3.19-3.08 (m, 2H), 3.00-2.91 (m, 4H), 2.64-2.56 (m, 4H), 2.17-2.08 (m, 1H), 2.03-1.92 (m, 1H).

### Example 105

### Preparation of 1-methyl-6-(5-(4-((1-methyl-2-oxopyrrolidin-3-yl)methyl)piperazin-1-yl)pyridin-3-yl)-3,4-dihydroquinolin-2(1H)-one formate (compound 166):

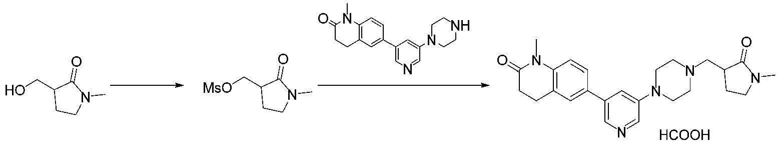

### Step 1: Synthesis of (1-methyl-2-oxopyrrolidin-3-yl)methyl methanesulphonate:

With reference to step 2 of example 96, 3-(hydroxymethyl)-1-methylpyrrolidin-2-one (150 mg, 1 eq) was used as the starting material. After the reaction was completed, water (20 mL) was added to the reaction solution, and the mixture was extracted with dichloromethane (20 mL*3). The organic phases were combined, dried over anhydrous sodium sulphate and filtered, and the filtrate was concentrated to obtain the product (200 mg, crude). LCMS (ESI) [M+H]⁺ = 208.2.

### Step 2: Synthesis of compound 166:

With reference to step 3 of example 96, using intermediate 1 (200 mg, 1 eq) and the product of step 1 (192.74 mg) as starting materials, the separation and purification were carried out by reversed-phase chromatography (0.1% aqueous ammonia solution/acetonitrile system), followed by the separation and purification by prep-HPLC (0.1% formic acid solution/acetonitrile system) to obtain the target compound (44.82 mg). LCMS (ESI) [M+H]⁺ = 434.3; ¹H NMR (400 MHz, DMSO-*d₆*) δ 8.34-8.23 (m, 2H), 8.18 (s, 1H), 7.67-7.57 (m, 2H), 7.54-7.45 (m, 1H), 7.21-7.14 (m, 1H), 3.33-3.28 (m, 5H), 3.28-3.22 (m, 4H), 2.98-2.91 (m, 2H), 2.73 (s, 3H), 2.68-2.54 (m, 6H), 2.50-2.35 (m, 3H), 2.22-2.09 (m, 1H), 1.89-1.73 (m, 1H).

### Example 106

### Preparation of N-isopropyl-2-(4-(5-(1-methyl-2-oxo-1,4-dihydro-2H-benzo[d][1,3]oxazin-6-yl)pyridin-3-yl)piperazin-1-yl)acetamide (example 167):

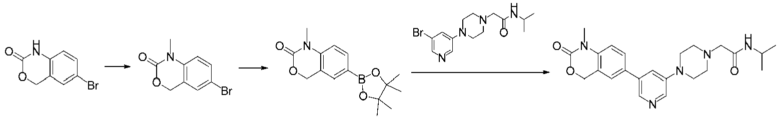

### Step 1: Synthesis of 6-bromo-1-methyl-1,4-dihydro-2H-benzo[d][1,3]oxazin-2-one:

With reference to step 1 of preparation example 1, using 6-bromo-1,4-dihydro-2H-benzo[d][1,3]oxazin-2-one (200 mg, 1 eq) as the starting material, the separation and purification were carried out by flash chromatography (silica gel, petroleum ether : ethyl acetate = 3 : 1) to obtain the product (200 mg). LCMS (ESI) [M+H]⁺ = 244.1 (isotope peak); ¹H NMR (400 MHz, DMSO-*d₆*) δ 7.55 (dd, *J =* 8.6, 2.3 Hz, 1H), 7.52-7.48 (m, 1H), 7.05 (d, *J=* 8.6 Hz, 1H), 5.24 (s, 2H), 3.25 (s, 3H).

### Step 2: Synthesis of 1-methyl-6-(4,4,5,5-tetramethyl-1,3,2-dioxaborolan-2-yl)-1,4-dihydro-2H-benzo[d][1,3]oxazin-2-one:

With reference to step 2 of preparation example 1, using the product obtained in step 1 (200 mg, 1 eq) as the starting material, the target compound (200 mg, crude) was obtained. LCMS (ESI) [M+H]⁺ = 290.1.

### Step 3: Synthesis of compound 167:

With reference to example 102, using the product of preparation example 4 (150 mg, 1 eq) and the product of step 2 (152.51 mg, 1.2 eq) as starting materials, the separation and purification were carried out by flash chromatography (silica gel, petroleum ether : tetrahydrofuran = 1 : 4), followed by the separation and purification by reversed-phase chromatography (0.1% aqueous ammonia solution/acetonitrile system) to obtain the target compound (111.58 mg). LCMS (ESI) [M+H]⁺ = 424.3; ¹H NMR (400 MHz, DMSO-*d₆*) δ 8.33-8.25 (m, 2H), 7.80-7.72 (m, 1H), 7.71-7.64 (m, 1H), 7.58-7.46 (m, 2H), 7.18 (d, *J =* 8.5 Hz, 1H), 5.31 (s, 2H), 3.98-3.84 (m, 1H), 3.34-3.28 (m, 7H), 2.96 (s, 2H), 2.69-2.56 (m, 4H), 1.08 (d, *J =* 6.6 Hz, 6H).

### Example 107

### Preparation of N-isopropyl-2-(4-(5-(1'-methyl-2'-oxospiro[cyclopropane-1,3'-indolin]-5'-yl)pyridin-3-yl)piperazin-1-yl)acetamide (compound 168):

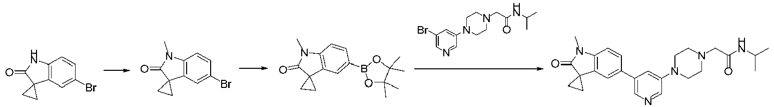

### Step 1: Synthesis of 5'-bromo-1'-methylspiro[cyclopropane-1,3'-indolin]-2'-one:

With reference to step 1 of preparation example 1, using 5'-bromospiro[cyclopropane-1,3'-indolin]-2'-one (400 mg, 1 eq) as the starting material, the separation and purification were carried out by flash chromatography (silica gel, petroleum ether : tetrahydrofuran = 1 : 1) to obtain the product (400 mg). LCMS (ESI) [M+H]⁺= 254.0 (isotope peak); ¹H NMR (400 MHz, DMSO-*d₆*) δ 7.43 (dd, *J =* 8.3, 2.0 Hz, 1H), 7.28 (d, *J =* 2.0 Hz, 1H), 7.03 (d, *J = 8.3* Hz, 1H), 3.19 (s, 3H), 1.72-1.66 (m, 2H), 1.54-1.50 (m, 2H).

### Step 2: Synthesis of 1'-methyl-5'-(4,4,5,5-tetramethyl-1,3,2-dioxaborolan-2-yl)spiro[cyclopropane-1,3'-indolin]-2'-one:

With reference to step 2 of preparation example 1, using the product of step 1 (200 mg, 1 eq) as the starting material, the separation and purification were carried out by flash chromatography (silica gel, petroleum ether : tetrahydrofuran = 5 : 1) to obtain the product (200 mg). LCMS (ESI) [M+H]⁺ = 300.1.

### Step 3: Synthesis of compound 168:

With reference to example 102, the product of preparation example 4 (70 mg, 1 eq), the product of step 2 (92.05 mg), [1,1'-bis(di-tert-butylphosphino)ferrocene]dichloropalladium (13.37 mg) and potassium phosphate (130.63 mg) were dissolved in 1,4-dioxane (2.5 mL) and water (0.5 mL). Under a nitrogen atmosphere, the reaction was stirred at 80°C for 2 hours. The reaction solution was diluted with water (30 mL), and extracted with ethyl acetate (15 mL *3). The organic phases were combined, dried over anhydrous sodium sulphate and filtered, and the filtrate was concentrated. The residue was purified by reversed-phase chromatography (C18, 0.1% NH₃H₂O in H₂O/ACN) to obtain the target compound (40.12 mg). LCMS (ESI) [M+H]⁺ =434.2; ¹H NMR (400 MHz, DMSO-*d₆*) δ 8.29 (d, *J =* 1.7 Hz, 1H), 8.24 (d, *J =* 2.7 Hz, 1H), 7.62 (dd, *J =* 8.1, 1.8 Hz, 1H), 7.55-7.48 (m, 2H), 7.42 (d, *J =* 1.6 Hz, 1H), 7.15 (d, *J =* 8.2 Hz, 1H), 3.94-3.87 (m, 1H), 3.31-3.28 (m, 4H), 3.25 (s, 3H), 2.96 (s, 2H), 2.63-2.59 (m, 4H), 1.75 (z, *J =* 7.7, 3.8 Hz, 2H), 1.57-1.52 (m, 2H), 1.08 (d, *J =* 6.6 Hz, 6H).

### Example 108

### Preparation of N-isopropyl-2-(4-(5-(1,3,3-trimethyl-2-oxoindolin-5-yl)pyridin-3-yl)piperazin-1-yl)acetamide (compound 169):

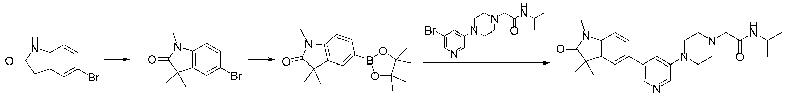

### Step 1: Synthesis of 5-bromo-1,3,3-trimethylindolin-2-one:

5-Bromoindolin-2-one (500 mg, 2.36 mmol, 1 eq) was dissolved in tetrahydrofuran (10 mL). With reference to step 1 of preparation example 1, the separation and purification were carried out by flash chromatography (silica gel, petroleum ether : ethyl acetate = 3 : 1) to obtain the product (200 mg). LCMS (ESI) [M+H+41]⁺ = 295.2; ¹H NMR (400 MHz, CDCl₃) δ 7.42-7.35 (m, 1H), 7.31 (d, *J =* 2.0 Hz, 1H), 6.72 (d, *J =* 8.2 Hz, 1H), 3.20 (s, 3H), 1.37 (s, 6H).

### Step 2: Synthesis of 1,3,3-trimethyl-5-(4,4,5,5-tetramethyl-1,3,2-dioxaborolan-2-yl)indol-2-one:

With reference to step 2 of preparation example 1, using 5-bromo-1,3,3-trimethylindolin-2-one (260 mg, 1 eq), the product (250 mg, crude) was obtained. LCMS (ESI) [M+H]⁺ = 302.4.

### Step 3: Synthesis of compound 169:

With reference to example 102, using the product of preparation example 4 (200 mg, 1 eq) and the product of step 2 (211.83 mg) as starting materials, the separation and purification were carried out by flash chromatography (silica gel, ethyl acetate), followed by the separation and purification by reversed-phase chromatography (0.1% aqueous ammonia solution/acetonitrile system) to obtain the target compound (95.07 mg). LCMS (ESI) [M+H]⁺ = 436.3; ¹H NMR (400 MHz, DMSO-*d₆*) δ 8.30 (d, *J =* 1.6 Hz, 1H), 8.26 (d, *J =* 2.6 Hz, 1H), 7.77 (d, *J =* 1.6 Hz, 1H), 7.64 (dd, *J =* 8.1, 1.7 Hz, 1H), 7.60-7.43 (m, 2H), 7.10 (d, *J =* 8.1 Hz, 1H), 3.99-3.82 (m, 1H), 3.37-3.33 (m, 4H), 3.18 (s, 3H), 2.97 (s, 2H), 2.68-2.55 (m, 4H), 1.33 (s, 6H), 1.08 (d, *J =* 6.6 Hz, 6H).

### Example 109

### Preparation of N-isopropyl-2-(4-(5-(1-methyl-4,5-dihydro-[1,2,4]triazolo[4,3-a]quinolin-7-yl)pyridin-3-yl)piperazin-1-yl)acetamide (compound 170):

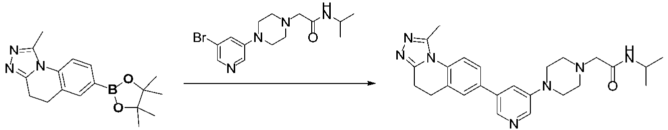

With reference to example 102, using the reaction of step 3 of example 97 (60 mg, 1 eq) and the product of preparation example 4 (52.64 mg) as starting materials, the separation and purification were carried out by reversed-phase chromatography (C18, 0.1% NH₃H₂O in H₂O/ACN) to obtain the target compound (18 mg). LCMS (ESI) [M+H]⁺ =446.2; ¹H NMR (400 MHz, DMSO-*d₆*) δ 8.35-8.31 (m, 2H), 7.88-7.86 (m, 1H), 7.78-7.72 (m, 2H), 7.57-7.55 (m, 1H), 7.55-7.50 (m, 1H), 3.94-3.86 (m, 1H), 3.36-3.35 (m, 2H), 3.30-3.28 (m, 2H), 3.04 (s, 4H), 2.96 (s, 2H), 2.71 (s, 3H), 2.63-2.59 (m, 4H), 1.08 (d, *J =* 6.6 Hz, 6H).

### Example 110

### Preparation of 1-methyl-6-(5-(4-(1-propionylazetidin-3-yl)piperazin-1-yl)pyridin-3-yl)-3,4-dihydroquinolin-2(1H)-one (compound 171):

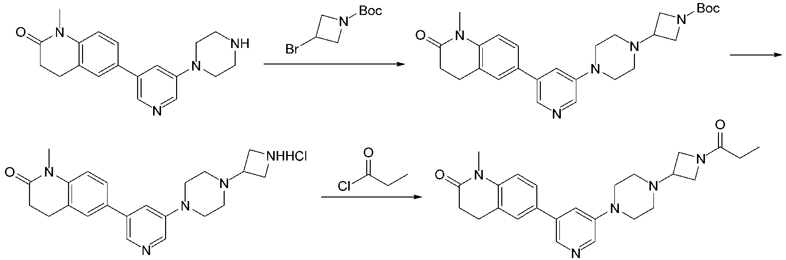

### Step 1: Synthesis of tert-butyl 3-(4-(5-(1-methyl-2-oxo-1,2,3,4-tetrahydroquinolin-6-yl)pyridin-3-yl)piperazin-1-yl)azetidine-1-carboxylate:

Intermediate 1 (400 mg, 1 eq) was dissolved in acetonitrile (5 mL). Potassium carbonate (342.95 mg), potassium iodide (41.19 mg) and tert-butyl 3-bromoazetidine-1-carboxylate (585.88 mg) were added, and the mixture was stirred at 100°C for 48 hours. The reaction solution was filtered, and the filtrate was concentrated. The residue was purified by flash chromatography (silica gel, ethyl acetate) to obtain the product (170 mg). LCMS (ESI) [M+H]⁺ = 478.3; ¹H NMR (400 MHz, DMSO-*d₆*) δ 8.29 (d*, J =* 1.7 Hz, 1H), 8.26 (d*, J =* 2.7 Hz, 1H), 7.67-7.58 (m, 2H), 7.54-7.45 (m, 1H), 7.20-7.14 (m, 1H), 3.97-3.79 (m, 2H), 3.78-3.64 (m, 2H), 3.32-3.27 (m, 7H), 3.15-3.04 (m, 1H), 2.99-2.87 (m, 2H), 2.62-2.55 (m, 2H), 2.49-2.42 (m, 4H), 1.39 (s, 9H).

### Step 2: Synthesis of 6-(5-(4-(azetidin-3-yl)piperazin-1-yl)pyridin-3-yl)-1-methyl-3,4-dihydroquinolin-2(1H)-one hydrochloride:

With reference to step 2 of example 99, using the product of step 1 as the starting material (150 mg), the product (120 mg, crude) was obtained. LCMS (ESI) [M+H]⁺ = 378.2.

### Step 3: Synthesis of compound 171:

The product of step 2 (120 mg, 1 eq) was dissolved in dichloromethane (3 mL). At 0°C, triethylamine (58.55 mg) and propionyl chloride (40.23 mg) were added. The mixture was stirred at room temperature for 1 hour. The reaction solution was concentrated, and the residue was purified by reversed-phase chromatography (0.1% aqueous ammonia solution/acetonitrile system) to obtain the target compound (69.09 mg). LCMS (ESI) [M+H]⁺ = 434.2; ¹H NMR (400 MHz, DMSO-*d₆*) δ 8.30 (d, *J =* 1.7 Hz, 1H), 8.27 (d, *J =* 2.7 Hz, 1H), 7.66-7.60 (m, 2H), 7.53-7.47 (m, 1H), 7.20-7.14 (m, 1H), 4.14 (t, *J =* 7.9 Hz, 1H), 4.03-3.93 (m, 1H), 3.91-3.83 (m, 1H), 3.74-3.65 (m, 1H), 3.32-3.26 (m, 7H), 3.20-3.10 (m, 1H), 2.98-2.90 (m, 2H), 2.62-2.56 (m, 2H), 2.50-2.44 (m, 4H), 2.06 (q, *J =* 7.5 Hz, 2H), 0.96 (t, *J =* 7.5 Hz, 3H).

### Example 111

### Preparation of N-isopropyl-2-(4-(5-(3-methyl-5-oxo-4,5,6,7-tetrahydropyrazolo[1,5-a]pyrimidin-2-yl)pyridin-3-yl)piperazin-1-yl)acetamide (compound 173):

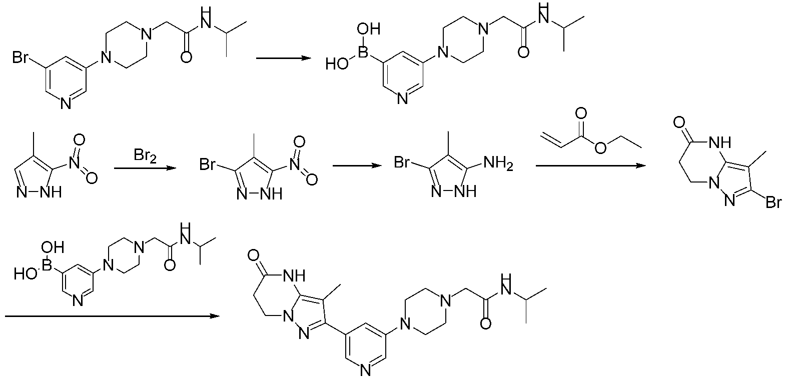

### Step 1: Synthesis of (5-(4-(2-(isopropylamino)-2-oxoethyl)piperazin-1-yl)pyridin-3-yl)boronic acid:

The product of preparation example 4 (100 mg, 1 eq) was dissolved in dioxane (3 mL). Bis(pinacolato)diboron (148.83 mg), potassium acetate (86.28 mg) and 1,1'-bis(diphenylphosphino)ferrocene dichloropalladium (21.5 mg) were added. Under a nitrogen atmosphere, the mixture was stirred at 100°C for 16 hours. The reaction solution was concentrated to obtain the product (85 mg, crude). LCMS (ESI) [M+H] ⁺ = 307.1.

### Step 2: Synthesis of 3-bromo-4-methyl-5-nitro-1H-pyrazole:

4-Methyl-5-nitro-1H-pyrazole (4.5 g, 1 eq) was dissolved in N,N-dimethylformamide (50 mL). At 0°C, liquid bromine (3.63 mL) was added dropwise. After the dropwise addition was completed, the mixture was stirred at 40°C for 16 hours. The reaction solution was cooled to room temperature, quenched by adding saturated aqueous sodium thiosulphate solution, and extracted with ethyl acetate (100 mL*3). The organic phases were washed with saturated aqueous sodium chloride solution (100 mL*3). The organic phases were combined, dried over anhydrous sodium sulphate and filtered, and the filtrate was concentrated. The residue was purified by flash chromatography (silica gel, petroleum ether : ethyl acetate = 2 : 1) to obtain the product (4.5 g). ¹H NMR (400 MHz, DMSO-*d₆*) δ 14.73 (s, 1H), 2.22 (s, 3H).

### Step 3: Synthesis of 3-bromo-4-methyl-1H-pyrazol-5-amine:

The product of step 2 (1.5 g, 1 eq) was dissolved in ethanol (20 mL) and water (4 mL). Iron powder (2.03 g) and ammonium chloride (778.98 mg) were added, and the mixture was stirred at 90°C for 1 hour. The reaction solution was filtered through a pad of Celite, and washed with ethyl acetate. The filtrate was dried over anhydrous sodium sulphate and filtered. The filtrate was concentrated. The residue was purified by flash chromatography (silica gel, petroleum ether : ethyl acetate = 1 : 1) to obtain the product (1.2 g). LCMS (ESI) [M+H]⁺ =176.1; ¹H NMR (400 MHz, DMSO-*d₆*) δ 11.60 (s, 1H), 5.01 (s, 2H), 1.74 (s, 3H).

### Step 4: Synthesis of 2-bromo-3-methyl-6,7-dihydropyrazolo[1,5-a]pyrimidin-5(4H)-one:

The product of step 3 (1 g, 1 eq) was dissolved in pyridine (10 mL) and water (1 mL). Ethyl acrylate (0.85 mL) was added, and the mixture was stirred at 130°C in a sealed tube for 16 hours. The reaction solution was added to a solution of petroleum ether and ethyl acetate (v/v 5 : 1, 80 mL). A solid precipitated out and was then filtered, and the filter cake was collected to obtain the product (500 mg). LCMS (ESI) [M+H] ⁺ =231.9 (isotope peak); ¹H NMR (400 MHz, DMSO-*d₆*) δ 10.84 (s, 1H), 4.18 (t, *J =* 7.0 Hz, 2H), 2.77 (t, *J =* 7.0 Hz, 2H), 1.84 (s, 3H).

### Step 5: Synthesis of compound 173:

To a mixture of the product of step 4 (40 mg, 1 eq), (5-(4-(2-(isopropylamino)-2-oxoethyl)piperazin-1-yl)pyridin-3-yl)boronic acid (79.85 mg), potassium carbonate (72.09 mg) and 1,1'-bis(diphenylphosphino)ferrocene dichloropalladium (12.76 mg) were added dioxane (2.5 mL) and water (0.5 mL). Under a nitrogen atmosphere, the mixture was stirred at 80°C for 3 hours. To the reaction solution was added water (20 mL), and the mixture was extracted with ethyl acetate (20 mL*3). The organic phases were combined, dried over anhydrous sodium sulphate and filtered, and the filtrate was concentrated. The residue was purified by reversed-phase chromatography (0.1% NH₃H₂O in H₂O/ACN) to obtain the target compound (36.19 mg). LCMS (ESI) [M+H]⁺ = 412.2; ¹H NMR (400 MHz, DMSO-*d₆*) δ 10.75 (s, 1H), 8.27 (d, *J =* 2.7 Hz, 1H), 8.22 (d, *J =* 1.0 Hz, 1H), 7.52 (d, *J =* 8.1 Hz, 1H), 7.45-7.38 (m, 1H), 4.26 (t, *J =* 7.0 Hz, 2H), 3.96-3.83 (m, 1H), 3.30-3.21 (m, 4H), 2.95 (s, 2H), 2.80 (t, *J =* 7.0 Hz, 2H), 2.63-2.57 (m, 4H), 2.07 (s, 3H), 1.08 (d, *J =* 6.6 Hz, 6H).

### Example 112

### Preparation of 1-methyl-6-(5-(4-(methylsulphonyl)piperazin-1-yl)pyridin-3-yl)-3,4-dihydroquinolin-2(1H)-one (compound 175):

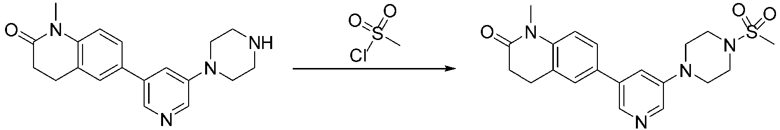

Intermediate 1 (100 mg, 1 eq) was dissolved in dichloromethane (5 mL). In an ice bath, triethylamine (94.16 mg) and methanesulphonyl chloride (71.06 mg) were sequentially added. The mixture was stirred at room temperature for 2 hours. The reaction solution was quenched by adding water (30 mL), and extracted with ethyl acetate (15 mL*3). The organic phases were combined, dried over anhydrous sodium sulphate and filtered, and the filtrate was concentrated. The residue was purified by reversed-phase chromatography (C18, 0.1% NH₃H₂O in H₂O/ACN) to obtain the target compound (61.59 mg). LCMS (ESI) [M+H]⁺ = 401.0; ¹H NMR (400 MHz, DMSO-*d₆*) δ 8.36-8.29 (m, 2H), 7.66-7.62 (m, 2H), 7.58-7.54 (m, 1H), 7.20-7.16 (m, 1H), 3.44-3.40 (m, 4H), 3.29-3.25 (m, 7H), 2.95-2.93 (m, 4H), 2.61-2.57 (m, 3H).

### Example 113

### Preparation of N-(cis-3-methoxycyclobutyl)-2-(6-(5-(1-methyl-2-oxo-1,2,3,4-tetrahydroquinolin-6-yl)pyridin-3-yl)-2,6-diazaspiro[3.3]heptan-2-yl)acetamide (compound 187):

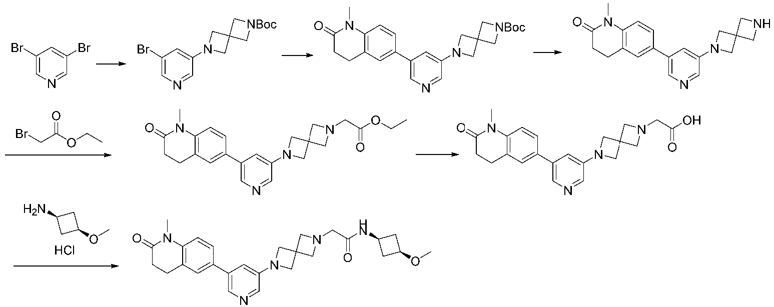

### Step 1: Synthesis of tert-butyl 6-(5-bromopyridin-3-yl)-2,6-diazaspiro[3.3]heptane-2-carboxylate:

With reference to step 3 of preparation example 4, using 3,5-dibromopyridine (5 g, 1 eq) and tert-butyl 2,6-diazaspiro[3.3]heptane-2-carboxylate hemioxalate (5.24 g), the reaction was stirred at 100°C under a nitrogen atmosphere for 16 hours. To the reaction solution was added water (100 mL), and the mixture was extracted with ethyl acetate (100 mL*3). The organic phases were combined, dried over anhydrous sodium sulphate and filtered, and the filtrate was concentrated. The residue was purified by flash chromatography (silica gel, petroleum ether : ethyl acetate = 2 : 1) to obtain the product (3.2 g). LCMS (ESI) [M+H]⁺ = 356.0 (isotope peak); ¹H NMR (400 MHz, CDCl₃) δ 8.06 (d, *J =* 1.7 Hz, 1H), 7.75 (d, *J =* 2.4 Hz, 1H), 6.85 (t, *J =* 2.1 Hz, 1H), 4.11 (s, 4H), 4.04 (s, 4H), 1.45 (s, 9H).

With reference to steps 4-8 of example 94, the following intermediates and target compounds were prepared respectively:

| | | | |
|---|---|---|---|
| Product of step 2 | Tert-butyl 6-(5-(1-methyl-2-oxo-1,2,3,4-tetrahydroquinolin-6-yl)pyridin-3-yl)-2,6-diazaspiro[3.3]heptane-2-carboxylate | Using product of step 1 (3.2 g, 1 eq) and product of preparation example 1 (3.89 g) as starting materials | LCMS (ESI) [M+H]⁺ = 435.1; ¹H NMR (400 MHz, CDCl₃) δ 8.24 (d, *J* = 1.4 Hz, 1H), 7.83 (d, *J =* 2.5 Hz, 1H), 7.45 (dd, *J =* 8.4, 1.7 Hz, 1H), 7.38-7.33 (m, 1H), 7.06 (d, *J =* 8.4 Hz, 1H), 6.87-6.80 (m, 1H), 4.13 (s, 4H), 4.09 (s, 4H), 3.40 (s, 3H), 3.03-2.94 (m, 2H), 2.75-2.66 (m, 2H), 1.46 (s, 9H) |
| Product of step 3 | Synthesis of 6-(5-(2,6-diazaspiro[3.3]heptan-2-yl)pyridin-3-yl)-1-methyl-3,4-dihydroquinolin-2(1H)-one | Using product of step 2 (3 g, 1 eq) as starting material | LCMS (ESI) [M+H]⁺ = 335.1; ¹H NMR (400 MHz, DMSO-*d₆*) δ 8.20 (d, *J =* 1.6 Hz, 1H), 7.77 (d, *J =* 2.5 Hz, 1H), 7.62-7.55 (m, 2H), 7.17 (d, *J =* 9.1 Hz, 1H), 7.03-6.95 (m, 1H), 4.00 (s, 4H), 3.62 (s, 3H), 3.28 (s, 4H), 2.99-2.88 (m, 2H), 2.62-2.55 (m, 2H) |
| Product of step 4 | Ethyl 2-(6-(5-(1-methyl-2-oxo-1,2,3,4-tetrahydroquinolin-6-yl)pyridin-3-yl)-2,6-diazaspiro[3.3]heptan-2-yl)acetate | Using product of step 3 (800 mg, 1 eq) and ethyl bromoacetate (0.24 ml) as starting materials | LCMS (ESI) [M+H] ⁺ = 421.3 |
| Product of step 5 | 2-(6-(5-(1-methyl-2-oxo-1,2,3,4-tetrahydroquinolin-6-yl)pyridin-3-yl)-2,6-diazaspiro[3.3]heptan-2-yl)acetic acid | Using product of step 4 (2 g, 1 eq) as starting material | LCMS (ESI) [M+H]⁺ = 393.3; ¹H NMR (400 MHz, DMSO-*d₆*) δ 8.21 (d, *J =* 1.7 Hz, 1H), 7.78 (d, *J =* 2.5 Hz, 1H), 7.63-7.52 (m, 2H), 7.22-7.12 (m, 1H), 7.04-6.96 (m, 1H), 4.05 (s, 4H), 3.84 (s, 4H), 3.31-3.25 (m, 5H), 2.97-2.90 (m, 2H), 2.61-2.55 (m, 2H) |
| Product of step 6 | Target compound | Using product of step 5 (50 mg, 1 eq) and cis-3-methoxycyclobutanamine hydrochloride (17.53 mg) as starting materials | LCMS (ESI) [M+H] ⁺ = 476.3; ¹H NMR (400 MHz, MeOD) δ 8.12 (d, *J* = 1.8 Hz, 1H), 7.72 (d, *J =* 2.6 Hz, 1H), 7.55 (dd, *J =* 8.4, 2.1 Hz, 1H), 7.52-7.49 (m, 1H), 7.22 (d, *J =* 8.4 Hz, 1H), 7.09-7.04 (m, 1H), 4.07 (s, 4H), 3.94-3.87 (m, 1H), 3.68-3.63 (m, 1H), 3.56 (s, 4H), 3.39 (s, 3H), 3.23 (s, 3H), 3.13 (s, 2H), 3.03-2.97 (m, 2H), 2.70-2.63 (m, 4H), 1.88-1.78 (m, 2H) |

### Example 114

### Preparation of N-(trans-3-methoxycyclobutyl)-2-(6-(5-(1-methyl-2-oxo-1,2,3,4-tetrahydroquinolin-6-yl)pyridin-3-yl)-2,6-diazaspiro[3.3]heptan-2-yl)acetamide (compound 188):

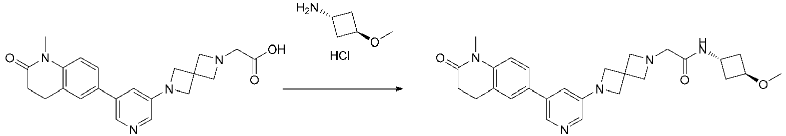

With reference to step 8 of example 94, using the product of step 5 of example 113 (50 mg, 1 eq) and trans-3-methoxycyclobutanamine hydrochloride (17.53 mg) as starting materials, the separation and purification were carried out by reversed-phase chromatography (acetonitrile/0.1% aqueous ammonia solution system) to obtain the target compound (4.83 mg). LCMS (ESI) [M+H]⁺ = 476.3; ¹H NMR (400 MHz, MeOD) δ 8.13 (d, *J =* 1.5 Hz, 1H), 7.72 (d, *J* = 2.4 Hz, 1H), 7.55 (dd, *J =* 8.4, 2.1 Hz, 1H), 7.53-7.49 (m, 1H), 7.22 (d, *J =* 8.4 Hz, 1H), 7.09-7.05 (m, 1H), 4.37-4.29 (m, 1H), 4.09 (s, 4H), 4.06-3.98 (m, 1H), 3.67 (s, 4H), 3.39 (s, 3H), 3.24 (s, 2H), 3.23 (s, 3H), 3.03-2.97 (m, 2H), 2.69-2.63 (m, 2H), 2.36-2.28 (m, 2H), 2.25-2.17 (m, 2H).

### Example 115 (compound 177)

### Preparation of N-(3-acetamidocyclobutyl)-2-(6-(5-(1-methyl-2-oxo-1,2,3,4-tetrahydroquinolin-6-yl)pyridin-3-yl)-2,6-diazaspiro[3.3]heptan-2-yl)acetamide:

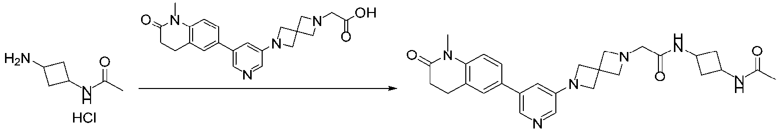

With reference to step 3 of example 88, using 2-(6-(5-(1-methyl-2-oxo-1,2,3,4-tetrahydroquinolin-6-yl)pyridin-3-yl)-2,6-diazaspiro[3.3]heptan-2-yl)acetic acid (70 mg, 1 eq) and N-(3-aminocyclobutyl)acetamide hydrochloride (32.3 mg) as starting materials, the separation and purification were carried out by reversed-phase chromatography (0.1% NH₃H₂O in H₂O/ACN), followed by the separation and purification by flash chromatography (silica gel, dichloromethane : methanol = 10 : 1) to obtain the target compound (8.56 mg). LCMS (ESI) [M+H] ⁺ = 503.4; ¹H NMR (400 MHz, MeOD) δ 8.13 (d, *J =* 1.7 Hz, 1H), 7.72 (d, *J =* 2.5 Hz, 1H), 7.55 (dd, *J =* 8.4, 2.1 Hz, 1H), 7.52-7.49 (m, 1H), 7.22 (d, *J =* 8.4 Hz, 1H), 7.08-7.05 (m, 1H), 4.39-4.23 (m, 2H), 4.08 (s, 4H), 3.67-3.64 (m, 4H), 3.38 (s, 3H), 3.25-3.21 (m, 2H), 3.02-2.97 (m, 2H), 2.68-2.64 (m, 2H), 2.41-2.18 (m, 4H), 1.94-1.91 (m, 3H).

### Example 116 (compound 178)

### Preparation of 2-(6-(5-(1-methyl-2-oxo-1,2,3,4-tetrahydroquinolin-6-yl)pyridin-3-yl)-2,6-diazaspiro[3.3]heptan-2-yl)-N-(oxetan-3-yl)acetamide:

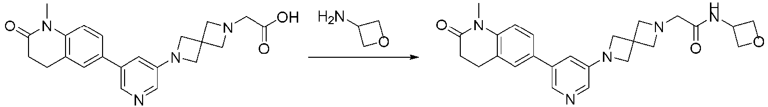

With reference to step 3 of example 88, using 2-(6-(5-(1-methyl-2-oxo-1,2,3,4-tetrahydroquinolin-6-yl)pyridin-3-yl)-2,6-diazaspiro[3.3]heptan-2-yl)acetic acid (100 mg, 1 eq) and 3-aminooxetane (20.49 mg) as starting materials, the separation and purification were carried out by reversed-phase chromatography (0.1% NH₃H₂O in H₂O/ACN), followed by the separation and purification by flash chromatography (silica gel, dichloromethane : methanol = 10 : 1) to obtain the target compound (6.76 mg). LCMS (ESI) [M+H] ⁺ = 448.3; ¹H NMR (400 MHz, MeOD) δ 8.12 (d, *J =* 1.8 Hz, 1H), 7.72 (d, *J* = 2.6 Hz, 1H), 7.55 (dd, *J* = 8.4, 2.1 Hz, 1H), 7.52-7.48 (m, 1H), 7.21 (d, *J =* 8.4 Hz, 1H), 7.09-7.03 (m, 1H), 4.98-4.90 (m, 1H), 4.88-4.86 (m, 1H), 4.85-4.82 (m, 1H), 4.58 (t, *J =* 6.5 Hz, 2H), 4.08 (s, 4H), 3.61 (s, 4H), 3.38 (s, 3H), 3.23 (s, 2H), 3.03-2.96 (m, 2H), 2.71-2.62 (m, 2H).

### Example 117 (compound 172)

### Preparation of 2-(4-(5-(1-cyclopropyl-2-oxo-1,2,3,4-tetrahydroquinolin-6-yl)pyridin-3-yl)piperazin-1-yl)-N-isopropylacetamide:

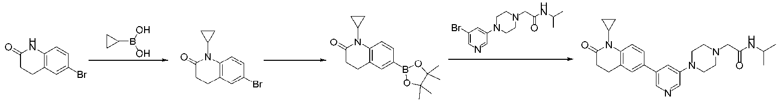

### Step 1: Synthesis of 6-bromo-1-cyclopropyl-3,4-dihydroquinolin-2(1H)-one:

6-Bromo-3,4-dihydroquinolin-2(1H)-one (2 g, 1 eq) was dissolved in dichloromethane (30 mL). Cyclopropylboronic acid (1.52 g), cupric acetate monohydrate (3.18 g) and triethylamine (2.69 g) were added. The mixture was stirred at room temperature under an oxygen atmosphere for 24 hours. The reaction solution was filtered, and the filtrate was concentrated. The residue was purified by flash chromatography (silica gel, petroleum ether : ethyl acetate = 3 : 1) to obtain the product (400 mg). LCMS (ESI) [M+H] ⁺ = 268.0 (isotope peak); ¹H NMR (400 MHz, CDCl₃) δ 7.36 (dd, *J =* 8.6, 2.3 Hz, 1H), 7.28-7.26 (m, 1H), 7.17 (d, *J =* 8.6 Hz, 1H), 2.82-2.76 (m, 2H), 2.75-2.69 (m, 1H), 2.64-2.56 (m, 2H), 1.18-1.10 (m, 2H), 0.66-0.62 (m, 2H).

### Step 2: Synthesis of 1-cyclopropyl-6-(4,4,5,5-tetramethyl-1,3,2-dioxaborolan-2-yl)-3,4-dihydroquinolin-2(1H)-one:

With reference to step 2 of preparation example 1, using the product of step 1 (150 mg, 1 eq) as the starting material, the reaction solution was filtered, and the filtrate was concentrated to obtain the product (170 mg, crude). LCMS (ESI) [M+H] ⁺ = 314.2.

### Step 3: Synthesis of compound 172:

With reference to example 102, using the product of preparation example 4 (120 mg, 1 eq) and the product of step 2 (143.18 mg) as starting materials, the separation and purification were carried out by reversed-phase chromatography (0.1% NH₃H₂O in H₂O/ACN) to obtain the target compound (46.87 mg). LCMS (ESI) [M+H] ⁺ = 448.3; ¹H NMR (400 MHz, DMSO-*d₆*) δ 8.29 (d, *J =* 1.6 Hz, 1H), 8.26 (d, *J =* 2.6 Hz, 1H), 7.63 (dd, *J =* 8.4, 2.1 Hz, 1H), 7.61-7.57 (m, 1H), 7.56-7.47 (m, 2H), 7.38 (d, *J =* 8.4 Hz, 1H), 3.95-3.86 (m, 1H), 3.33-3.30 (m, 4H), 2.96 (s, 2H), 2.85 (t, *J =* 7.1 Hz, 2H), 2.78-2.71 (m, 1H), 2.65-2.57 (m, 4H), 2.56-2.51 (m, 2H), 1.13-1.05 (m, 8H), 0.58-0.48 (m, 2H).

### Example 118 (compound 186)

### Preparation of N-isopropyl-2-(4-(5-(1-isopropyl-2-oxo-1,2,3,4-tetrahydroquinolin-6-yl)pyridin-3-yl)piperazin-1-yl)acetamide:

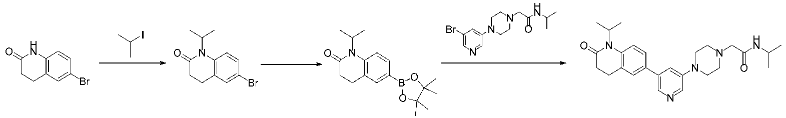

### Step 1: Synthesis of 6-bromo-1-isopropyl-3,4-dihydroquinolin-2(1H)-one:

With reference to step 1 of preparation example 1, using 6-bromo-3,4-dihydroquinolin-2(1H)-one (200 mg, 1 eq) and isopropyl iodide (451.16 mg) as starting materials, the separation and purification were carried out by flash chromatography (silica gel, petroleum ether : ethyl acetate = 3 : 1) to obtain the product (120 mg). LCMS (ESI) [M+H] ⁺ = 268.0; ¹H NMR (400 MHz, CDCl₃) δ 7.32 (dd, *J =* 8.6, 2.3 Hz, 1H), 7.30-7.27 (m, 1H), 6.99 (d, *J* = 8.6 Hz, 1H), 4.71-4.60 (m, 1H), 2.83-2.74 (m, 2H), 2.58-2.51 (m, 2H), 1.50 (d, *J =* 7.0 Hz, 6H).

### Step 2: Synthesis of 1-isopropyl-6-(4,4,5,5-tetramethyl-1,3,2-dioxaborolan-2-yl)-3,4-dihydroquinolin-2(1H)-one:

With reference to step 2 of preparation example 1, using the product of step 1 (120 mg, 1 eq) as the starting material, the product (130 mg, crude) was obtained. LCMS (ESI) [M+H] ⁺ = 316.1.

### Step 3: Synthesis of compound 186:

With reference to example 102, using preparation example 4 (80 mg, 1 eq) and the product of step 2 (96.06 mg) as starting materials, the separation and purification were carried out by reversed-phase chromatography (0.1% NH₃H₂O in H₂O/ACN) to obtain the target compound (30.43 mg). LCMS (ESI) [M+H] ⁺ = 450.3; ¹H NMR (400 MHz, DMSO-*d₆*) δ 8.38-8.15 (m, 2H), 7.64-7.60 (m, 1H), 7.58 (dd, *J =* 8.4, 2.1 Hz, 1H), 7.56-7.51 (m, 1H), 7.50-7.47 (m, 1H), 7.28 (d, *J =* 8.5 Hz, 1H), 4.68-4.58 (m, 1H), 3.95-3.86 (m, 1H), 3.32-3.28 (m, 4H), 2.96 (s, 2H), 2.89-2.83 (m, 2H), 2.63-2.57 (m, 4H), 2.49-2.45 (m, 2H), 1.46 (d, *J =* 6.9 Hz, 6H), 1.08 (d, *J =* 6.6 Hz, 6H).

### Example 119 (compound 182)

### Preparation of 2-(4-(5-(5-fluoro-4-methyl-3-oxo-3,4-dihydro-2H-benzo[b][1,4]oxazin-7-yl)pyridin-3-yl)piperazin-1-yl)-N-isopropylacetamide:

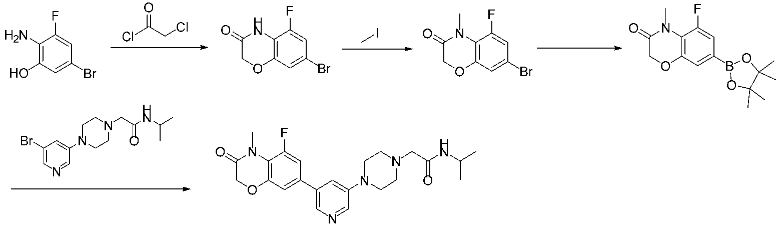

### Step 1: Synthesis of 7-bromo-5-fluoro-2H-benzo[b][1,4]oxazin-3(4H)-one:

2-Amino-5-bromo-3-fluorophenol (500 mg, 1 eq) was dissolved in N,N-dimethylformamide (10 mL). Chloroacetyl chloride (274.11 mg) and potassium carbonate (670.89 mg) were added. The mixture was stirred at 80°C for 2 hours. To the reaction solution was added water (40 mL), and the mixture was extracted with ethyl acetate (40 mL*3). The organic phase was washed with saturated aqueous sodium chloride solution (50 mL*3). The organic phases were combined, dried over anhydrous sodium sulphate and filtered, and the filtrate was concentrated. The residue was purified by flash chromatography (silica gel, petroleum ether : ethyl acetate = 2 : 1) to obtain the product (400 mg). LCMS (ESI) [M+H] ⁺ = 246.0; ¹H NMR (400 MHz, DMSO-*d₆*) δ 11.02 (s, 1H), 7.22 (dd, *J* = 9.8*,* 1.9 Hz, 1H), 7.17-6.96 (m, 1H), 4.65 (s, 2H).

### Step 2: Synthesis of 7-bromo-5-fluoro-4-methyl-2H-benzo[b][1,4]oxazin-3(4H)-one:

With reference to step 1 of preparation example 1, using the product of step 1 (400 mg, 1 eq) and iodomethane (0.15 ml) as starting materials, the separation and purification were carried out by flash chromatography (silica gel, petroleum ether : ethyl acetate = 2 : 1) to obtain the product (300 mg). LCMS (ESI) [M+H] ⁺ = 260.0; ¹H NMR (400 MHz, CDCl₃₎ δ 7.09-6.90 (m, 2H), 4.55 (s, 2H), 3.46 (d, *J=* 6.0 Hz, 3H).

### Step 3: Synthesis of 5-fluoro-4-methyl-7-(4,4,5,5-tetramethyl-1,3,2-dioxaborolan-2-yl)-2H-benzo[b][1,4]oxazin-3(4H)-one:

With reference to step 2 of preparation example 1, using the product of step 2 (150 mg, 1 eq) as the starting material, the product (170 mg, crude) was obtained. LCMS (ESI) [M+H] ⁺ = 308.1.

### Step 4: Synthesis of compound 182:

With reference to example 102, using the product of preparation example 1 (80 mg, 1 eq) and the product of step 3 (144 mg) as starting materials, the separation and purification were carried out by reversed-phase chromatography (0.1% NH₃H₂O in H₂O/ACN) to obtain the target compound (67.97 mg). LCMS (ESI) [M+H] ⁺ = 442.2; ¹H NMR (400 MHz, DMSO-*d₆*) δ 8.32 (d, *J =* 1.8 Hz, 1H), 8.30 (d, *J =* 2.7 Hz, 1H), 7.57-7.51 (m, 2H), 7.49 (dd, *J =* 14.1, 2.0 Hz, 1H), 7.39-7.35 (m, 1H), 4.70 (s, 2H), 3.96-3.86 (m, 1H), 3.40 (d, *J =* 6.0 Hz, 3H), 3.35-3.32 (m, 4H), 2.96 (s, 2H), 2.64-2.54 (m, 4H), 1.08 (d, *J =* 6.6 Hz, 6H).

**With reference to the preparation methods described in the above examples, the following compounds were prepared, with their structures and characterisation data as follows:**

| No. | Structure | LCMS (ESI) [M+H] ⁺ | No. | Structure | LCMS (ESI) [M+H] ⁺ |
|---|---|---|---|---|---|
| 2 | 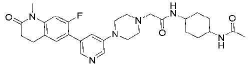 | 537.3 | 7 | 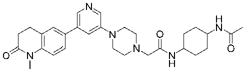 | 519.3 |
| 8 | 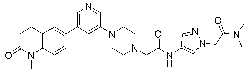 | 531.3 | 10 | 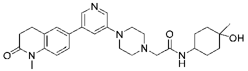 | 492.3 |
| 11 | 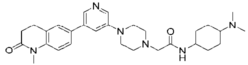 | 505.3 | 12 | 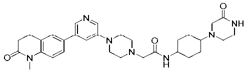 | 560.3 |
| 13 | 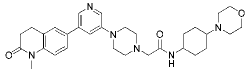 | 547.3 | 14 | 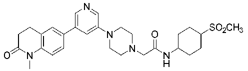 | 540.3 |
| 16 | 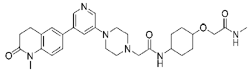 | 549.3 | 17 | 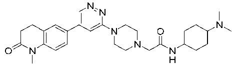 | 506.3 |
| 18 | 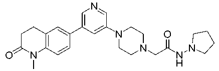 | 449.3 | 19 | 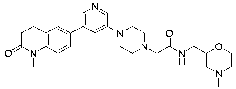 | 493.3 |
| 20 | 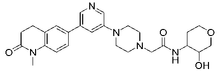 | 480.2 | 21 | 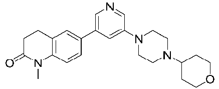 | 407.2 |
| 22 | 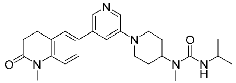 | 436.3 | 23 | 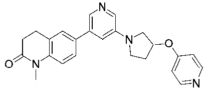 | 401.2 |
| 24 | 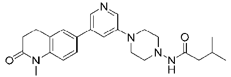 | 422.2 | 25 | 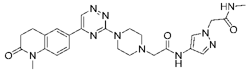 | 519.2 |
| 26 | 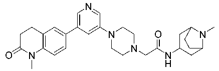 | 503.3 | 27 | 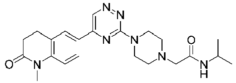 | 424.2 |
| 29 | 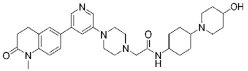 | 561.4 | 30 | 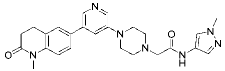 | 460.2 |
| 31 | 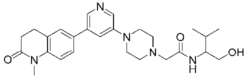 | 466.3 | 32 | 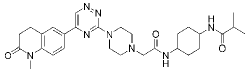 | 549.3 |
| 33 | 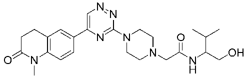 | 468.3 | 34 | 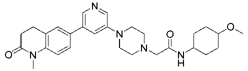 | 492.3 |
| 35 | 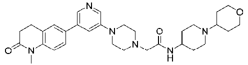 | 547.3 | 36 | 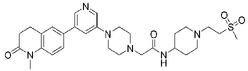 | 569.3 |
| 37 | 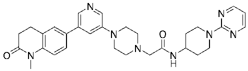 | 541.3 | 38 | 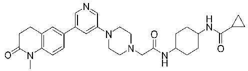 | 545.3 |
| 39 | 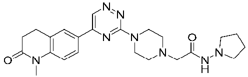 | 451.2 | 40 | 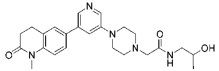 | 438.2 |
| 41 | 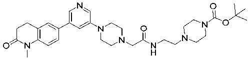 | 592.4 | 42 | 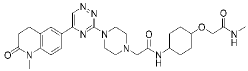 | 551.3 |
| 43 | 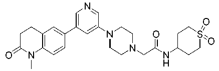 | 512.2 | 45 | 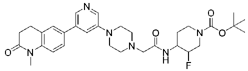 | 581.3 |
| 46 | 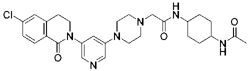 | 539.2 | 47 | 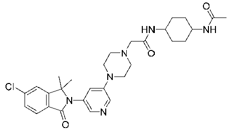 | 553.3 |
| 48 | 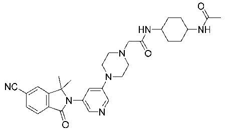 | 544.3 | 49 | 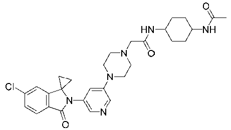 | 551.2 |
| 50 | 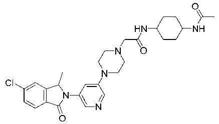 | 539.2 | 51 | 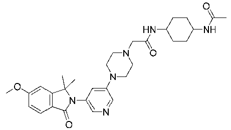 | 549.3 |
| 52 | 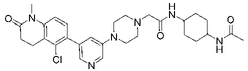 | 553.3 | 53 | 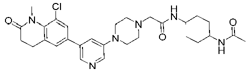 | 553.3 |
| 54 | 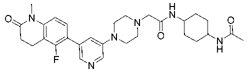 | 537.3 | 55 | 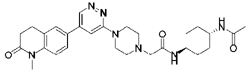 | 520.3 |
| 56 | 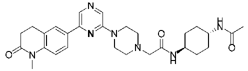 | 520.3 | 57 | 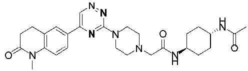 | 521.3 |
| 58 | 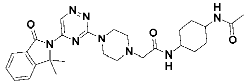 | 521.3 | 59 | 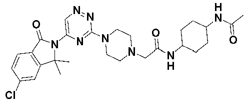 | 555.2 |
| 60 | 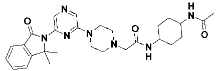 | 520.3 | 61 | 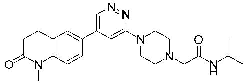 | 423.2 |
| 62 | 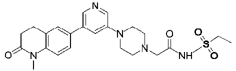 | 472.2 | 63 | 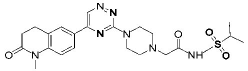 | 488.2 |
| 64 | 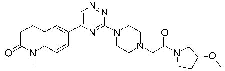 | 466.2 | 65 | 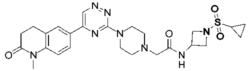 | 541.2 |
| 66 | 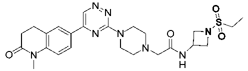 | 529.2 | 67 | 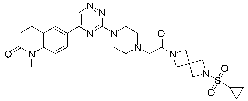 | 567.2 |
| 68 | 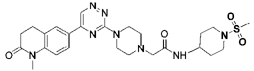 | 543.2 | 95 | 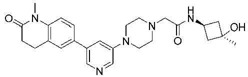 | 464.2 |
| 96 | 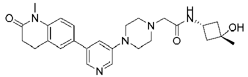 | 464.3 | | | |
| 17 4 | 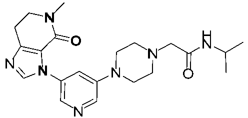 | 412.2 | 17 6 | 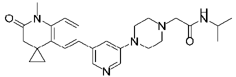 | 448.3 |
| 17 9 | 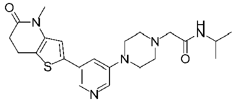 | 428.2 | 18 0 | 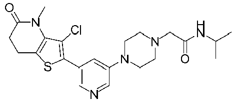 | 462.2 |
| 18 1 | 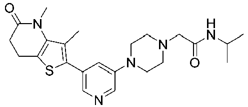 | 442.2 | 18 3 | 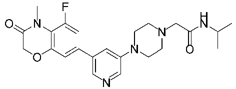 | 442.2 |
| 18 4 | 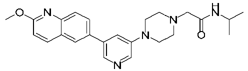 | 420.2 | 18 5 | 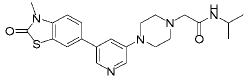 | 426.2 |
| 18 9 | 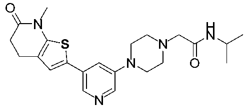 | 428.2 | 19 0 | 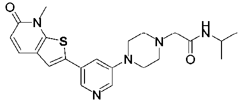 | 426.2 |
| 19 1 | 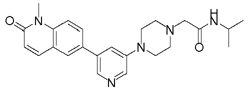 | 420.2 | 19 2 | 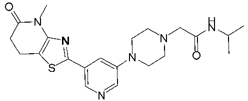 | 429.2 |
| 19 3 | 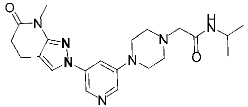 | 412.2 | 19 4 | 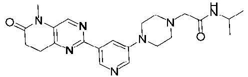 | 424.2 |
| 19 5 | 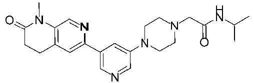 | 423.2 | 19 6 | 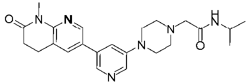 | 423.2 |

### Biological examples

### Experimental example 1: Assay of human aldosterone synthase (CYP11B2) inhibition

**Experimental reagents and instruments:** Aldosterone kit (Cisbio, Cat#64ALDPEG); Envision plate reader (PerkinElmer).

**Experimental principle and method:** In humans, aldosterone synthase (CYP11B2) catalyses the conversion of 11-deoxycorticosterone (11-DOC) to aldosterone through a series of metabolic processes. In this experiment, a G402 cell line stably overexpressing-Human CYP11B2 was established in vitro (according to Valentin-Goyco J, et al. Selectivity of osilodrostat as an inhibitor of human steroidogenic cytochromes P450. J Steroid Biochem Mol Biol. 2023, 231:106316). The cells were incubated with 11-deoxycorticosterone (11-DOC) as the substrate, the test compound was added simultaneously. After co-incubation, the supernatant was collected, and aldosterone levels in the supernatant were then measured by HTRF assay. The inhibitory effect of the compound on aldosterone synthase (CYP11B2) was calculated based on aldosterone concentration.

**Experimental steps:** 11-Deoxycorticosterone (11-DOC) was added to the wells of a plate pre-seeded with the cells (10,000 cells/well) 24h prior to assay initiation. Then, the compound diluted to different concentrations was added to the corresponding wells and mixed thoroughly. Control wells were set up simultaneously. The final concentration of DMSO was 0.1%. The plate was placed in a 37°C/5% CO₂ humidified incubator and incubated for 24 h. Post-incubation, 10 µL supernatant was added to a 384-well plate. 5 µL of Acceptor working solution and 5 µL of Donor Antibody were added to each well, followed by centrifugation at 1000 rpm for 1 minute. The plate was incubated at room temperature for 60-120 minutes. Finally, the fluorescence signal ratio at 665 nm/615 nm was measured using an Envision plate reader (PerkinElmer).

**Experimental data processing method:** Test data were processed and analysed using GraphPad Prism 5 software. First, the mean reaction signal values of the high-signal control wells and low-signal control wells were calculated separately. Then, the reaction inhibition rate for each compound well was calculated according to the following formula: Inhibition rate (%) = 100 - (Mean of high-signal control group - Signal value of individual well) / (Mean of high-signal control group - Mean of low-signal control group). Subsequently, the concentration and corresponding inhibition rate data were imported into Prism 5 software. The inhibition rate-concentration curve was fitted using the "log(inhibitor) vs. response - Variable slope" method in the software, and the IC₅₀ value of the compound was calculated.

The compounds of the present application exhibit potent CYP11B2 inhibitory activity. The activity data of exemplary compounds are shown in the table below.

**Table 1 Human aldosterone synthase (CYP11B2) inhibitory activity assay**

| Compound No. | IC₅₀ (nM) | Compound No. | IC₅₀ (nM) | Compound No. | IC₅₀ (nM) |
|---|---|---|---|---|---|
| Compound 1 | A | Compound 100 | A+ | Compound 131 | A+ |
| Compound 3 | A++ | Compound 101 | A+ | Compound 132 | A+ |
| Compound 5 | A++ | Compound 103 | A+ | Compound 133 | A++ |
| Compound 6 | A+ | Compound 104 | A+ | Compound 134 | A+ |
| Compound 28 | A | Compound 105 | A | Compound 135 | A+ |
| Compound 44 | A++ | Compound 106 | A++ | Compound 137 | A+ |
| Compound 72 | A+ | Compound 107 | A+ | Compound 138 | A |
| Compound 73 | A+ | Compound 109 | A+ | Compound 140 | A |
| Compound 74 | A | Compound 110 | A++ | Compound 141 | A+ |
| Compound 76 | A | Compound 111 | A++ | Compound 142 | A+ |
| Compound 77 | A | Compound 112 | A | Compound 147 | A+ |
| Compound 78 | A++ | Compound 113 | A++ | Compound 148 | A+ |
| Compound 79 | A+ | Compound 114 | A+ | Compound 151 | A |
| Compound 80 | A+ | Compound 115 | A++ | Compound 152 | A+ |
| Compound 81 | A++ | Compound 116 | A+ | Compound 153 | A |
| Compound 82 | A+ | Compound 117 | A++ | Compound 155 | A++ |
| Compound 83 | A+ | Compound 118 | A++ | Compound 159 | A++ |
| Compound 84 | A++ | Compound 119 | A+ | Compound 160 | A |
| Compound 87 | A++ | Compound 121 | A++ | Compound 161 | A++ |
| Compound 88 | A+ | Compound 122 | A++ | Compound 162 | A+ |
| Compound 89 | A++ | Compound 123 | A++ | Compound 164 | A+ |
| Compound 90 | A+ | Compound 124 | A++ | Compound 165 | A |
| Compound 91 | A | Compound 125 | A++ | Compound 166 | A |
| Compound 92 | A+ | Compound 126 | A++ | Compound 167 | A+ |
| Compound 93 | A | Compound 127 | A+ | Compound 172 | A |
| Compound 95 | A | Compound 128 | A++ | Compound 175 | A+ |
| Compound 97 | A | Compound 129 | A+ | Compound 183 | A+ |
| Compound 99 | A++ | Compound 130 | A+ | Compound 184 | A++ |
| | | | | Compound 185 | A++ |

IC₅₀ values for CYP11B2 enzyme inhibition: A++≤ 3 nM, 3 nM < A+ ≤ 10 nM, 10 nM < A ≤ 20 nM.

### Experimental example 2: Assay of human aldosterone synthase (CYP11B1) inhibition

### Experimental reagents and instruments: Cortisol kit (Cisbio, Cat#62ALDPEG); Envision plate reader (PerkinElmer)

**Experimental principle and method:** In humans, 11β-hydroxylase (CYP11B1) is the key enzyme responsible for the biosynthesis of cortisol. In this experiment, a G402-derived stable cell line overexpressing human CYP11B1 was established in vitro. The cells were incubated with using 11-deoxycortisol as the substrate, and the test compound was added simultaneously. After co-incubation, the supernatant was collected, and cortisol levels in the supernatant were then measured by HTRF assay, and the inhibitory effect of the compound on 11β-hydroxylase (CYP11B1) was calculated.

**Experimental steps:** 11-Deoxycortisol was added to the wells of a plate pre-seeded with the cells (10,000 cells/well) 24h prior to assay initiation. Then, the compound diluted to different concentrations was added to the corresponding wells and mixed thoroughly. Control wells were set up simultaneously. The final concentration of DMSO was 0.1%. The plate was placed in a 37°C/5% CO₂ humidified incubator and incubated for 24 h. Post-incubation, 10 µL supernatant was added to a 384-well plate. 5 µL of Acceptor working solution and 5 µL of Donor Antibody were added to each well, followed by centrifugation at 1000 rpm for 1 minute. The plate was incubated at room temperature for 60-120 minutes. Finally, the fluorescence signal ratio at 665 nm/615 nm was measured using an Envision plate reader (PerkinElmer).

**Experimental data processing method:** Test data were processed and analysed using GraphPad Prism 5 software. First, the mean reaction signal values of the high-signal control wells and low-signal control wells were calculated separately. Then, the reaction inhibition rate for each compound well was calculated according to the following formula: Inhibition rate (%) = 100 - (Mean of high-signal control group - Signal value of individual well) / (Mean of high-signal control group - Mean of low-signal control group). Subsequently, the concentration and corresponding inhibition rate data were imported into Prism 5 software. The inhibition rate-concentration curve was fitted using the "log(inhibitor) vs. response - Variable slope" method in the software, and the IC₅₀ value of the compound was calculated.

The compounds of the present application exhibit favourable selectivity for CYP11B2/CYP11B1. The selectivity data of exemplary compounds are shown in the table below.

**Table 2 Selectivity results of CYP11B2/CYP11B1**

| **Compound** | **Ratio of CYP11B1 IC₅₀ to CYP11B2 IC₅₀** | **Compound** | **Ratio of CYP11B1 IC₅₀ to CYP11B2 IC₅₀** |
|---|---|---|---|
| Compound 1 | B | Compound 114 | B |
| Compound 6 | B | Compound 115 | A |
| Compound 28 | A | Compound 116 | B |
| Compound 44 | C | Compound 117 | B |
| Compound 73 | B | Compound 121 | B |
| Compound 76 | C | Compound 122 | B |
| Compound 80 | A | Compound 123 | B |
| Compound 81 | C | Compound 124 | B |
| Compound 84 | B | Compound 125 | C |
| Compound 88 | B | Compound 126 | B |
| Compound 89 | B | Compound 127 | B |
| Compound 91 | C | Compound 128 | A |
| Compound 92 | C | Compound 133 | C |
| Compound 99 | C | Compound 152 | B |
| Compound 100 | C | Compound 155 | B |
| Compound 101 | B | Compound 159 | B |
| Compound 103 | B | Compound 160 | C |
| Compound 105 | B | Compound 161 | C |
| Compound 107 | C | Compound 163 | B |
| Compound 110 | A | Compound 162 | C |
| Compound 111 | B | Compound 164 | C |
| Compound 113 | B | Compound 172 | B |
| Reference compound 1 | D | | |

Ratio of CYP11B1 IC₅₀ to CYP11B2 IC₅₀: A > 500; 500 ≥ B > 200; 200 ≥ C > 100; D ≤ 100

**Reference compound 1 has the following structure:**

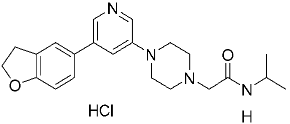

### Experimental example 3: Cassette pharmacokinetic study in cynomolgus monkeys

Test animals: six healthy, adult male non-naive cynomolgus monkeys, aged 40-65 months.

Formulation preparation: 5% DMSO + 10% Solutol + 85% Saline (1/2/17, V/V/V). A pre-weighed quantity of the test compound was weighed and transferred to a centrifuge tube, followed by the addition of a predetermined volume of DMSO. The mixture was vortexed until a clear solution was obtained. Solutol was then added, followed by vortexing to mix thoroughly. Subsequently, physiological saline was incorporated, and the mixture was vortexed thoroughly to obtain a clear solution. The two prepared formulations were mixed thoroughly at equal volumes to obtain a final concentration of 0.1 mg/mL for the intravenous administration group and a 0.2 mg/mL for the oral (PO) administration group. The formulations were prepared fresh immediately prior to dosing.

Administration: For the intravenous (i.v.) administration group and the oral (p.o.) administration group, three male cynomolgus monkeys were assigned to each group. The animals were fasted overnight prior to administration, and food was restored 4 hours after administration. Water was freely available throughout the study. The intravenous group received a single dose of 0.2 mg/kg at an administration volume of 2 mL/kg. The oral group received a single oral gavage dose of 1 mg/kg at an administration volume of 5 mL/kg.

Sample collection: For the intravenous group, plasma samples were collected at 5 min, 15 min, 30 min, 1 h, 2 h, 4 h, and 8 h post-dose. For the oral group, plasma samples were collected at 15 min, 30 min, 1 h, 2 h, 4 h, 8 h, and 24 h post-dose. At each specified time point, 500 µL of blood sample was collected into an anticoagulant tube containing EDTA-K2 as an anticoagulant. Plasma was separated by centrifugation within 30 min. Prior to centrifugation, whole blood samples were placed on wet ice. All collected plasma samples were stored on dry ice or in an ultra-low temperature freezer until analysis and detection.

Sample processing: Dilution of test samples: An aliquot of 3.0 µL of the test plasma sample was transferred into an EP tube containing 27 µL of blank plasma and vortexed thoroughly to ensure homogeneity. Extraction procedure for standard curve, quality control, blank, and test samples: Aliquots of 7.0 µL of blank samples, standard samples, quality control samples, and diluted test samples were transferred into a 96-well plate. Then, 70.0 µL of internal standard working solution was added to each wells. The 96-well plate was placed on a shaker and mixed thoroughly at 500 rpm for 10 min at room temperature. After mixing, the plate was centrifuged at 4000 rpm for 10 min at 4°C. An aliquot of 50.0 µL of the supernatant was transferred into a 96-well plate containing 50.0 µL of ultrapure water. The 96-well plate was again mixed thoroughly at 500 rpm for 10 min at room temperature, completing the extraction of standard curve, quality control, blank, and test samples. Plasma samples were analysed and detected by LC-MS/MS to determine the concentration of the test compound (LC-MS/MS instrument: Triple Quad 6500).

Data analysis: Raw bioanalytical data were acquired and calculated using AB Sciex mass spectrometer software Analyst 1.7.2. The main pharmacokinetic parameters were calculated via non-compartmental model using WinNonlin^{®}8.2. Data processing and graphing were performed using Microsoft Office Excel (Microsoft, USA).

### Experimental results:

The compounds of the present application exhibit lower clearance and favourable oral absorption properties. The results for exemplary compounds are shown in the table below.

| **Compound** | **Intravenous: 0.2 mg/kg** | | **Oral: 1 mg/kg** | | |
|---|---|---|---|---|---|
| | CL_pred (mL/min/kg) | AUClast (hr*ng/mL) | Cₘₐₓ (ng/mL) | Tₘₐₓ (hr) | AUCₗₐₛₜ (hr*ng/mL) |
| Compound 6 | 1.20±0.183 | 2717±434 | 2343±815 | 2.17±1.76 | 13342±4098 |

### Experimental example 4: Concurrent Pharmacokinetic study in cynomolgus monkeys

Experimental animals: healthy, adult male non-naive cynomolgus monkeys, aged 40-65 months.

Formulation preparation: 5% DMSO + 10% Solutol + 85% Saline (1/2/17, V/V/V). A pre-weighed quantity of the test compound was weighed and transferred to a centrifuge tube, followed by the addition of a predetermined volume of DMSO. The mixture was vortexed until a clear solution was obtained. Solutol was then added, followed by vortexing to mix thoroughly. Subsequently, physiological saline was incorporated, and the mixture was vortexed thoroughly to obtain a clear solution.

Animal model: After a 7-day acclimation period, animals received a single intramuscular injection of ACTH (ACTH challenge model). Plasma samples were collected before administration and 1 h post-injection for PD biomarker measurement. Animals exhibiting a ≥2-fold increase in the PD biomarker relative to baseline were enrolled in the subsequent PD efficacy assessment.

Grouping and administration:
(1) administration route: oral (p.o.) administration; administration dose: 1 mg/kg; administration volume: 5 mL/kg; administration frequency: single dose on the day.
(2) ACTH injection method: intramuscular injection; injection time: 1 h after oral (p.o.) administration; dose: 14.5 ug/kg; injection volume: 0.1 ml/kg.

Sample collection and processing: Blood samples were collected before p.o. administration and at 15 min, 30 min, 1 h, 2 h, 4 h, 6 h, 8 h, and 24 h after p.o. administration. Approximately 0.3 mL of blood was collected from the limb veins into EDTA-K2 tubes, gently mixed by shaking, and placed in an ice-water bath. Plasma was separated by centrifugation at 2,000 × g for 10 minutes at 4°C within 30 minutes of collection. The plasma samples were stored in a refrigerator at -80°C until analysis, and the separated plasma was frozen in a refrigerator until further analysis. During sample processing, all operations were performed in an ice-water bath.

Data analysis method: Pharmacokinetic parameters after oral administration to cynomolgus monkeys were calculated using Phoenix WinNonlin software with a non-compartmental model. Individual plasma drug concentration-time data and curves for each subject animal, as well as the mean values, standard deviations and corresponding curves for each dose group were presented. Data processing and graphing were performed using Microsoft Office Excel (Microsoft, USA).

### Experimental results:

The compounds of the present disclosure exhibit favourable oral absorption properties. The results for exemplary compounds are shown in the table below.

| **Compound** | **Oral: 1 mg/kg** | | |
|---|---|---|---|
| | **Cₘₐₓ (ng/mL)** | **Tₘₐₓ (hr)** | **AUCₗₐₛₜ (hr*ng/mL)** |
| Compound 155 | 1250±98.99 | 2.00 | 4848.33±77.23 |

The embodiments of the present disclosure are illustrated as above. However, the present disclosure is not limited to the above-described embodiments. Any modifications, equivalent substitutions, improvements, etc. made within the spirit and principles of the present disclosure shall be included within the scope of protection of the present disclosure.

## Claims

1. A compound, or a stereoisomer, tautomer, isotopic derivative or pharmaceutically acceptable salt thereof, **characterized in that**, the compound is as represented by formula (I), 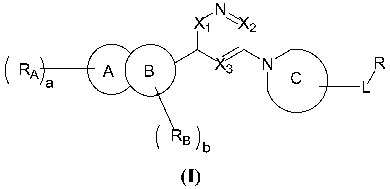 wherein,
ring A is 5- to 6-membered heterocyclyl, 5- to 6-membered heteroaryl or phenyl;
ring B is phenyl, 5- to 6-membered heterocyclyl or 5- to 6-membered heteroaryl;
each R_{A} at each occurrence is independently selected from deuterium, halogen, oxo, -CN, -NH₂, or the following optionally substituted groups: C₁-₄ alkyl, C₁-₄ deuterioalkyl, C₃-₄ cycloalkyl, C₁-₄ alkoxy, C₁-₄ alkylthio, C₁-₄ haloalkyl and C₁-₄ haloalkoxy; or, when two R_{A} groups are attached to the same carbon atom of the 5- to 6-membered heterocyclyl, and the two R_{A} groups are each independently selected from C₁-₄ alkyl, C₁-₄ alkoxy and C₁-₄ alkylthio, the two R_{A} groups together with the carbon atom to which they are attached form C₃-₆ cycloalkyl or 3- to 7-membered heterocyclyl; wherein, the optionally substituted means being unsubstituted or substituted with one or more substituents independently selected from deuterium, halogen, C₁₋₃ alkyl, C₁₋₃ alkoxy, hydroxy, and oxo;
each R_{B} at each occurrence is independently selected from deuterium, halogen, oxo, -CN, -NH₂, or the following optionally substituted groups: C₁-₄ alkyl, C₃-₄ cycloalkyl, C₁-₄ alkoxy, C₁-₄ alkylthio, C₁-₄ haloalkyl and C₁-₄ haloalkoxy; or, when two R_{B} groups are attached to the same carbon atom of the 5- to 6-membered heterocyclyl, and the two R_{B} groups are each independently selected from C₁-₄ alkyl, C₁-₄ alkoxy and C₁-₄ alkylthio, the two R_{B} groups together with the carbon atom to which they are attached form C₃-₆ cycloalkyl or 3-to 7-membered heterocyclyl; wherein, the optionally substituted means being unsubstituted or substituted with one or more substituents independently selected from deuterium, halogen, C₁₋₃ alkyl, C₁₋₃ alkoxy, hydroxy, and oxo;
ring C is optionally substituted 4- to 8-membered heterocyclyl; wherein the optionally substituted means being unsubstituted or substituted with one or more substituents, and the substituents are each independently selected from deuterium, halogen, oxo, -CN, -NH₂, -OH, C₁-₄ alkyl, C₁-₄ alkoxy, C₁-₄ alkylthio, C₁-₄ haloalkyl and C₁-₄ haloalkoxy; the heterocyclyl contains one N atom, and optionally, further contains 1, 2 or 3 heteroatoms, wherein the heteroatoms are independently selected from O, N and S;
X₁ is selected from N and CR_{X1};
X₂ is selected from N and CR_{X2};
X₃ is selected from N and CR_{X3};
provided that X₁, X₂ and X₃ are not N simultaneously;
R_{X1}, R_{X2}, and R_{X3} are each independently selected from hydrogen, deuterium, halogen, -OH, -CN, -NH₂, C₁-₃ alkyl, C₁-₃ alkoxy, C₁-₃ alkylthio, C₁-₃ haloalkyl and C₁-₃ haloalkoxy;
L is a bond, -O-, -S-, or the following optionally substituted group: -C₀₋₄ alkylene-N(R₁)-, -C₀₋₄ alkylene-C(O)-, -C₀₋₄ alkylene-N(R₁)-C(O)-, -C₀₋₄ alkylene-N(R₁)-C(O)-N(R₁)-, -C₀₋₄ alkylene-N(R₁)-S(O)₂-, -C₀₋₄ alkylene-C(O)-N(R₁)-S(O)₂-, -C₀₋₄ alkylene-C(O)-N(R₁)-, -C₀₋₄ alkylene-S(O)₂-N(R₁)-, -C₁₋₄ alkylene-, -C₃-₆ cycloalkyl-C₀₋₄ alkylene-C(O)-N(R₁)-, or 3- to 10-membered heterocyclyl-C₀₋₄ alkylene-C(O)-N(R₁)-; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R₁ is selected from hydrogen, deuterium, or the following optionally substituted groups: C₁-₃ alkyl and C₃-₆ cycloalkyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R is selected from -N(R₂)R₃ and -S(O)₂R₄, or is the following group optionally substituted with one or more R_{w1}: C₁-₆ alkyl, C₁-₆ alkoxy, C₁-₆ alkylthio, C₃-₁₀ cycloalkyl, 3- to 10-membered heterocyclyl, 5- to 12-membered heteroaryl, or C₆₋₁₂ aryl; wherein, R_{w1} is selected from deuterium, halogen, oxo, -CN, -OH, -N(R₂)R₃, -N(R₅)-C(O)-R₆, -S(O)₂R₄, -C(O)OR₂, -C(O)-N(R₂)R₃, or the following groups optionally substituted with one or more R_{w2}: C₁-₄ alkyl, C₁-₄ alkoxy, C₁-₄ alkylthio, C₃-₁₀ cycloalkyl, 3- to 10-membered heterocyclyl, C₆₋₁₀ aryl, and 5- to 12-membered heteroaryl; wherein, R_{w2} is selected from deuterium, halogen, oxo, -CN, -OH, -N(R₂)R₃, -S(O)₂R₄, -C(O)N(R₇)R₈, -C(O)OR₉, or the following groups optionally substituted with one or more R_{w3}: C₁-₄ alkyl, C₁-₄ alkoxy, C₁-₄ alkylthio, C₆₋₁₀ aryl, and 5- to 12-membered heteroaryl; wherein, R_{w3} is selected from deuterium, halogen, oxo, -CN, -OH, -N(R₂)R₃, C₁-₄ alkyl, C₁-₄ alkoxy, C₁-₄ alkylthio, C₃-₆ cycloalkyl, 3- to 6-membered heterocyclyl, 5- to 6-membered heteroaryl, phenyl, C₁-₄ haloalkyl and C₁-₄ haloalkoxy;
R₂ and R₃ are each independently selected from hydrogen, deuterium, or the following optionally substituted groups: C₁-₆ alkyl, C₃-₆ cycloalkyl and 4- to 6-membered heterocyclyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R₄ is selected from hydrogen, deuterium, or the following optionally substituted groups: C₁-₃ alkyl and C₃-₆ cycloalkyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R₅ is selected from hydrogen, deuterium, or the following optionally substituted groups: C₁-₃ alkyl and C₃-₆ cycloalkyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R₆ is selected from hydrogen, deuterium, or the following optionally substituted groups: C₁-₃ alkyl, C₃-₆ cycloalkyl and C₄-₆ heterocyclyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R₇ and R₈ are each independently selected from hydrogen, deuterium, or the following optionally substituted groups: C₁-₃ alkyl, C₃-₆ cycloalkyl and C₄-₆ heterocyclyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
R₉ is selected from hydrogen, deuterium, or the following optionally substituted groups: C₁-₆ alkyl and C₃-₆ cycloalkyl; wherein the optionally substituted means being unsubstituted or substituted with one or more substituents, and the substituents are each independently selected from deuterium, halogen, oxo, -CN, -NH₂, C₁-₄ alkyl, C₁-₄ alkoxy and C₁-₄ alkylthio;
a is selected from 0, 1, 2, 3 and 4;
b is selected from 0, 1, 2, 3 and 4;
unless otherwise specified, the heteroatom(s) in the above-described heterocyclyl or heteroaryl are independently selected from O, N and S, and the number of heteroatoms is 1, 2, 3 or 4;
provided that a combination of definitions of the above-described variables results in a stable chemical structure.

2. The compound, or the stereoisomer, tautomer, isotopic derivative or pharmaceutically acceptable salt thereof according to claim 1, **characterized in that**, when a bicyclic system formed by ring A and ring B is 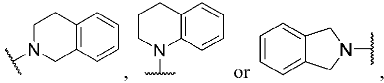 b is not 0, and 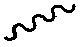 represents the point of attachment to 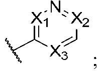
and/or, ring A and ring B are not simultaneously a 6-membered aromatic ring system, i.e., when ring A is phenyl or pyridyl, ring B is not phenyl or pyridyl;
and/or, the compound represented by formula (I) is not:

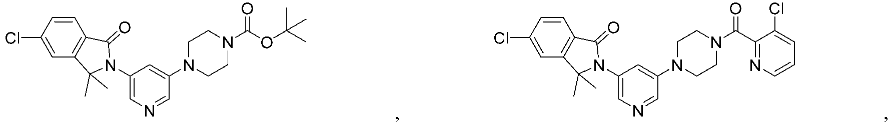

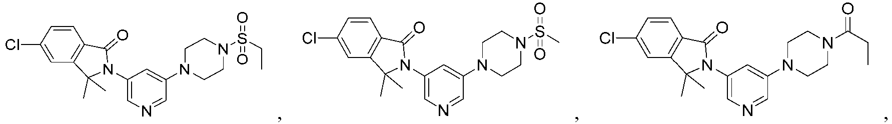

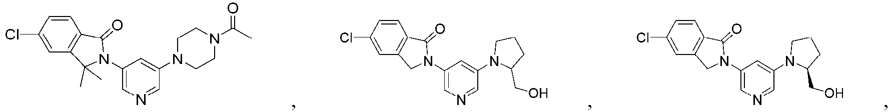

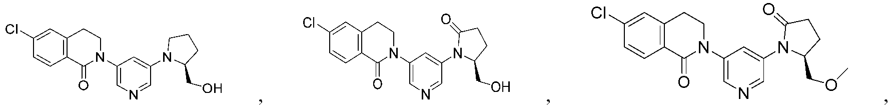

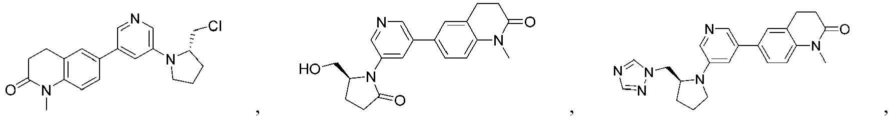

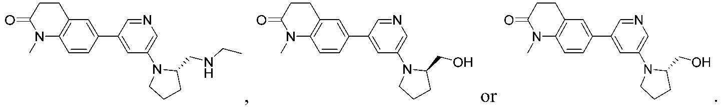

3. The compound, or the stereoisomer, tautomer, isotopic derivative or pharmaceutically acceptable salt thereof according to any one of claims 1-2, **characterized in that**, the bicyclic system formed by ring A and ring B is selected from: 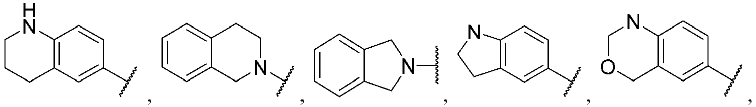 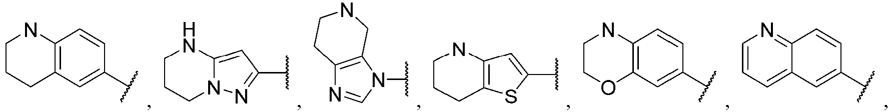 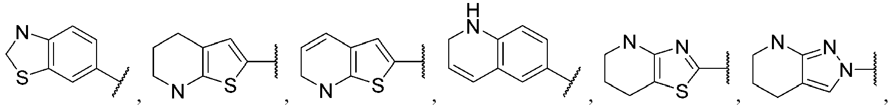 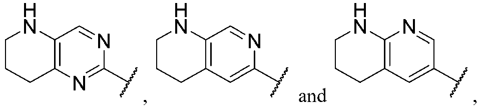 wherein, 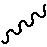 represents the point of attachment to ring

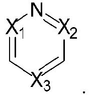

4. The compound, or the stereoisomer, tautomer, isotopic derivative or pharmaceutically acceptable salt thereof according to any one of claims 1-3, **characterized in that**, 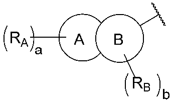 is selected from:

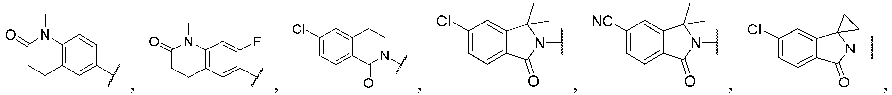

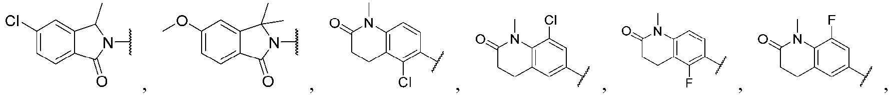

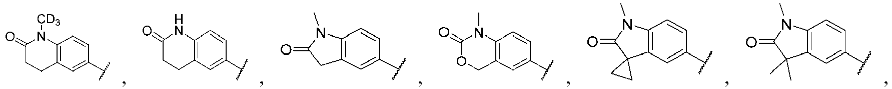

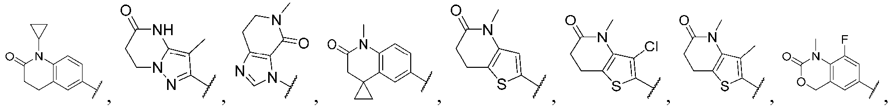

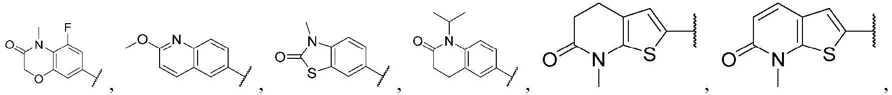

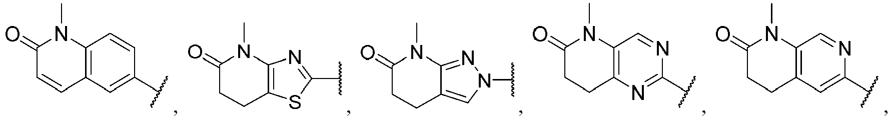

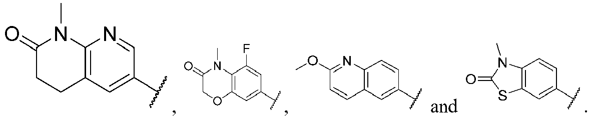

5. The compound, or the stereoisomer, tautomer, isotopic derivative or pharmaceutically acceptable salt thereof according to any one of claims 1-4, **characterized in that**, ring C is optionally substituted 5- to 8-membered heterocyclyl; preferably, ring C is selected from the following optionally substituted groups: 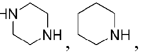 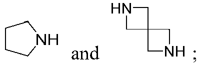 preferably, ring C is selected from the following optionally substituted groups: 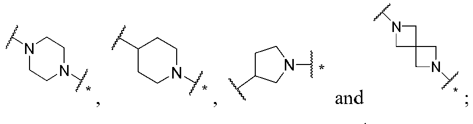 preferably, ring C is selected from the following optionally substituted groups: 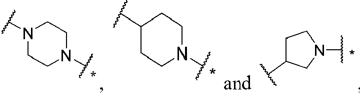 wherein, 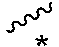 represents the point of attachment to ring 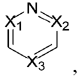 and 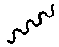 represents the point of attachment to L;
wherein, the optionally substituted means being unsubstituted or substituted with one or more substituents which are each independently selected from deuterium, halogen, oxo, -CN, -NH₂, -OH, C₁-₄ alkyl, C₁-₄ alkoxy, C₁-₄ alkylthio, C₁-₄ haloalkyl and C₁-₄ haloalkoxy.

6. The compound, or the stereoisomer, tautomer, isotopic derivative or pharmaceutically acceptable salt thereof according to any one of claims 1-5, **characterized in that**, structural fragment 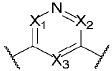 is 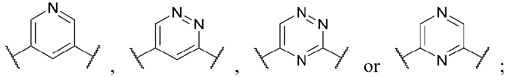 preferably, 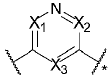 is 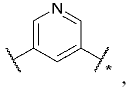 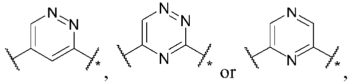 wherein, 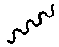 represents the point of attachment to ring B, and 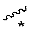 represents the point of attachment to ring C.

7. The compound, or the stereoisomer, tautomer, isotopic derivative or pharmaceutically acceptable salt thereof according to any one of claims 1-6, **characterized in that**, L is a bond, -O-, or the following optionally substituted group: -C₀₋₄ alkylene-N(R₁)-, -C₀₋₄ alkylene-C(O)-, -C₀₋₄ alkylene-N(R₁)-C(O)-, -C₀₋₄ alkylene-N(R₁)-C(O)-N(R₁)-, -C₀₋₄ alkylene-C(O)-N(R₁)-S(O)₂-, -C₀₋₄ alkylene-C(O)-N(R₁)-, -C₁₋₄ alkylene-, or -C₃-₆ cycloalkyl-C₀₋₄ alkylene-C(O)-N(R₁)-; wherein, the optionally substituted means being unsubstituted or substituted with one or more (such as 2, 3, 4, 5, 6, 7 or 8) substituents, and the substituents are each independently selected from deuterium, halogen, oxo, -CN, -NH₂, C₁-₄ alkyl, C₁-₄ alkoxy and C₁-₄ alkylthio;
or, L is a bond, -O-, 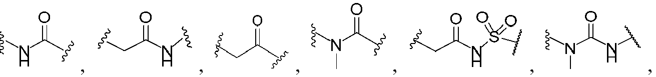 methylene, ethylene, propylene, 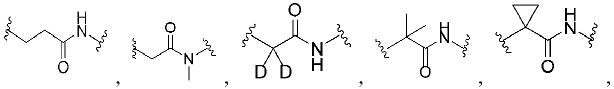 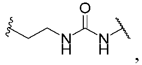 -C(O)- or -S(O)₂-; preferably, the left side of the groups is linked to ring C.

8. The compound, or the stereoisomer, tautomer, isotopic derivative or pharmaceutically acceptable salt thereof according to any one of claims 1-7, **characterized in that**, R is -N(R₂)R₃, -S(O)₂R₄, or the following group optionally substituted with one or more R_{w1}: C₁-₆ alkyl, C₁-₆ alkoxy, C₃-₁₀ cycloalkyl, 3- to 10-membered heterocyclyl, 5- to 10-membered heteroaryl, or 6- to 12-membered aryl; wherein, R_{w1} is selected from deuterium, halogen, oxo, -CN, -OH, -NR₂R₃, -N(R₅)-C(O)-R₆, -S(O)₂R₄, -C(O)OR₂, or the following groups optionally substituted with one or more R_{w2}: C₁-₄ alkyl, C₁-₄ alkoxy, C₃-₁₀ cycloalkyl, 3- to 10-membered heterocyclyl, C₆₋₁₀ aryl, and 5- to 12-membered heteroaryl; wherein, R_{w2} is selected from deuterium, halogen, oxo, -CN, -OH, -NR₂R₃, -S(O)₂R₄, -C(O)NR₇R₈, -C(O)OR₉, or the following groups optionally substituted with one or more R_{w3}: C₁-₄ alkyl, C₁-₄ alkoxy, C₁-₄ alkylthio, C₆₋₁₀ aryl, and 5- to 12-membered heteroaryl; R_{w3} is selected from deuterium, halogen, oxo, -CN, -OH, -NR₂R₃, C₁-₄ alkyl, C₁-₄ alkoxy, C₁-₄ alkylthio, C₁-₄ haloalkyl and C₁-₄ haloalkoxy;
or, R is

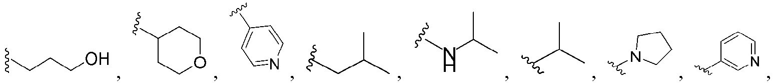

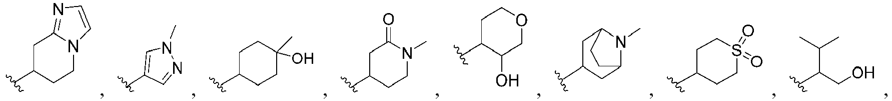

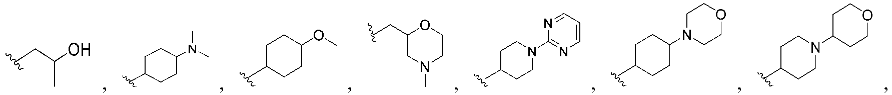

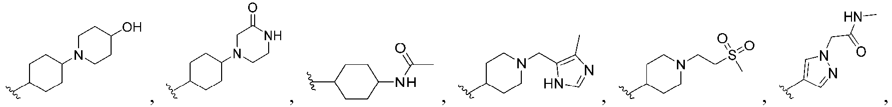

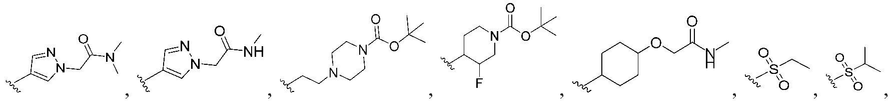

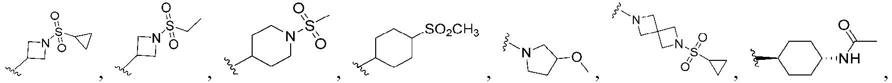

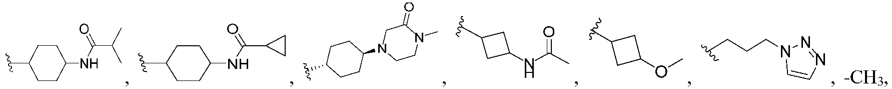

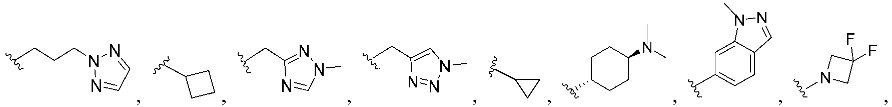

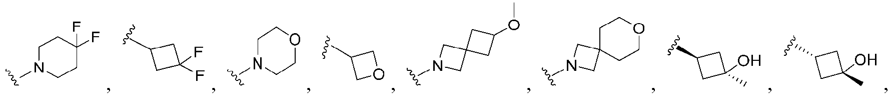

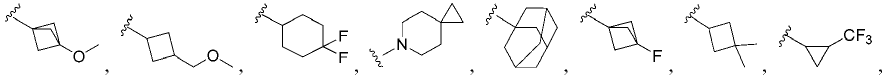

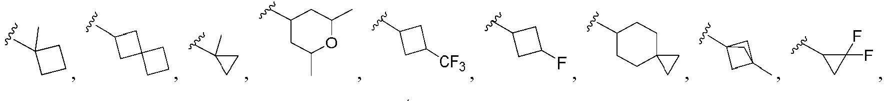

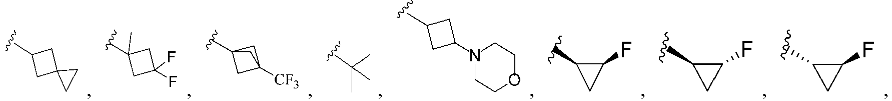

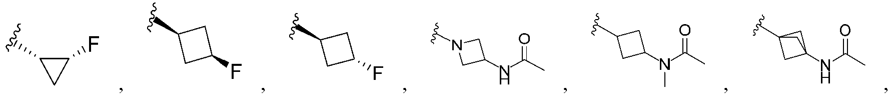

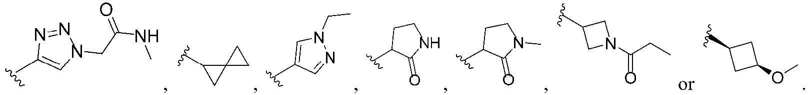

9. The compound, or the stereoisomer, tautomer, isotopic derivative or pharmaceutically acceptable salt thereof according to any one of claims 1-8, **characterized in that**, the compound is a compound represented by formula (II-1) or formula (II-2): 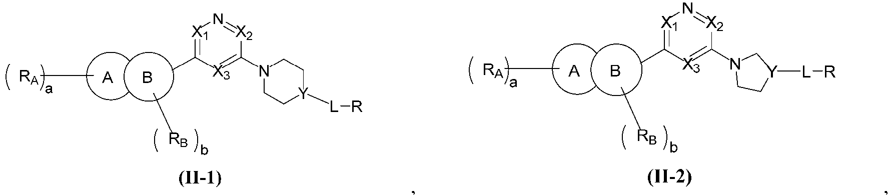 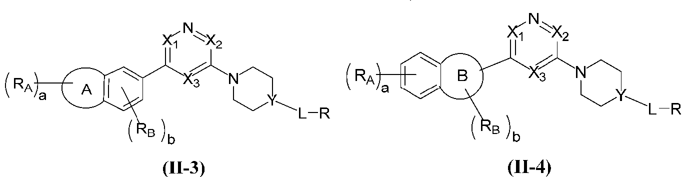
wherein, ring A, ring B, R_{A}, R_{B}, X₁, X₂, X₃, L, R, a, and b are as defined in claims 1-8;
Y is N or CR_{Y};
R_{Y} is selected from hydrogen, deuterium, halogen, -OH, -CN, -NH₂, C₁-₃ alkyl, C₁-₃ alkoxy, C₁-₃ haloalkyl and C₁-₃ haloalkoxy.

10. The compound, or the stereoisomer, tautomer, isotopic derivative or pharmaceutically acceptable salt thereof according to any one of claims 1-9, **characterized in that**, the compound is a compound represented by formula (III-1), formula (III-2), formula (III-3) or formula (III-4): 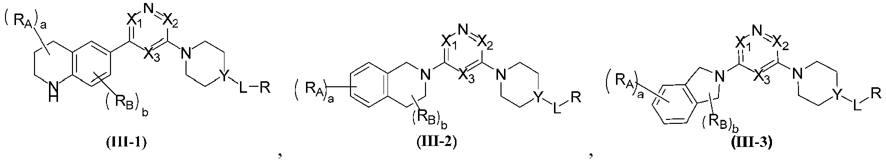 or 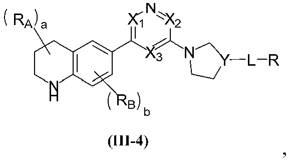 wherein, R_{A}, R_{B}, X₁, X₂, X₃, L, R, a, b, and Y are as defined in claims 1-9.

11. The compound, or the stereoisomer, tautomer, isotopic derivative or pharmaceutically acceptable salt thereof according to any one of claims 1-10, **characterized in that**, the compound is a compound represented by formula (V): 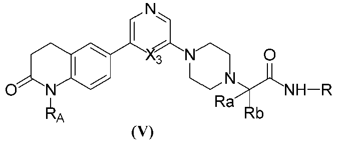
wherein, X₃ and R are as defined in claims 1-10;
Rₐ and R_{b} are each independently selected from hydrogen, deuterium and C₁-₄ alkyl.

12. The compound, or the stereoisomer, tautomer, isotopic derivative or pharmaceutically acceptable salt thereof according to claim 1, **characterized in that**, 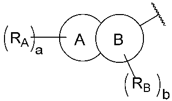 is selected from: 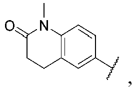 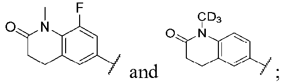 ring C is 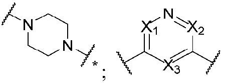 is 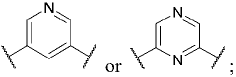 L is 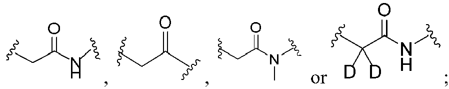 R is

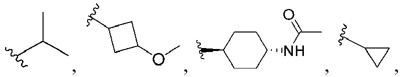

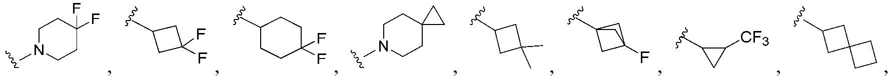

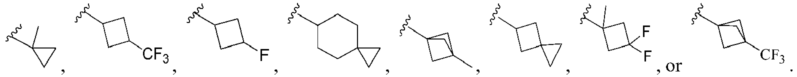

13. A compound, or a stereoisomer, tautomer, isotopic derivative or pharmaceutically acceptable salt thereof, **characterized in that** the compound is selected from:
| No. | Structure | No. | Structure |
|---|---|---|---|
| 1 | 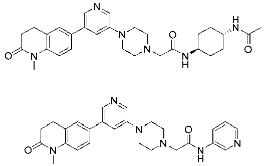 | 2 | 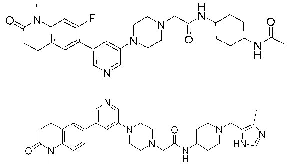 |
| 3 | 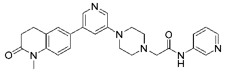 | 4 | 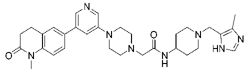 |
| 5 | 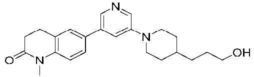 | 6 | 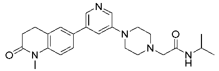 |
| 7 | 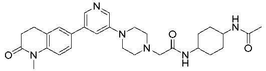 | 8 | 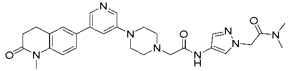 |
| 9 | 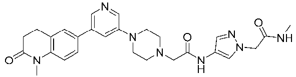 | 10 | 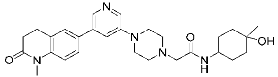 |
| 11 | 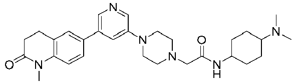 | 12 | 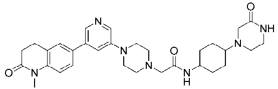 |
| 13 | 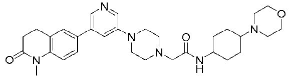 | 14 | 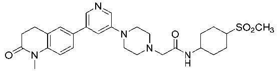 |
| 15 | 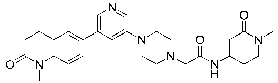 | 16 | 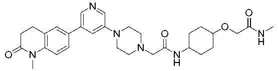 |
| 17 | 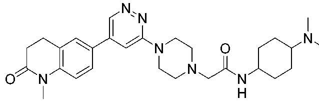 | 18 | 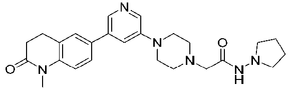 |
| 19 | 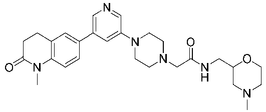 | 20 | 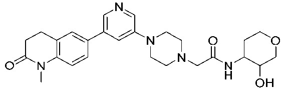 |
| 21 | 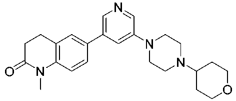 | 22 | 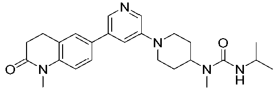 |
| 23 | 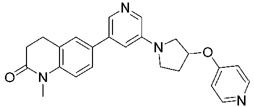 | 24 | 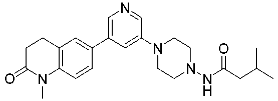 |
| 25 | 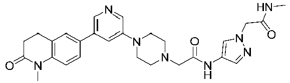 | 26 | 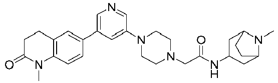 |
| 27 | 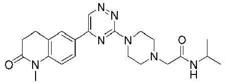 | 28 | 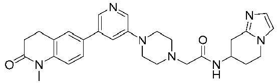 |
| 29 | 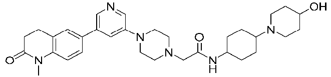 | 30 | 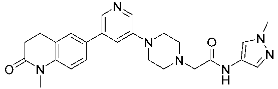 |
| 31 | 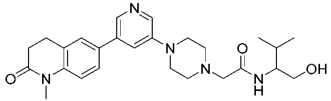 | 32 | 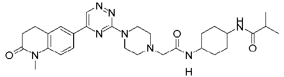 |
| 33 | 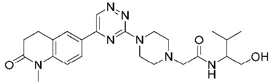 | 34 | 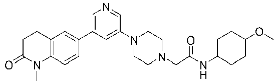 |
| 35 | 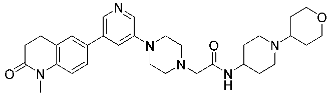 | 36 | 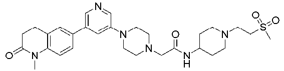 |
| 37 | 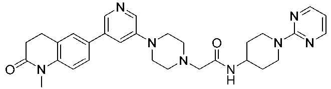 | 38 | 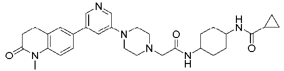 |
| 39 | 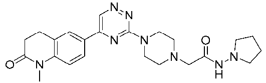 | 40 | 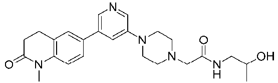 |
| 41 | 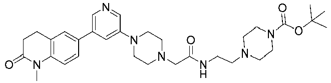 | 42 | 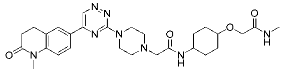 |
| 43 | 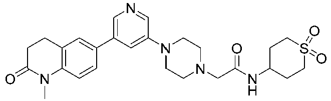 | 44 | 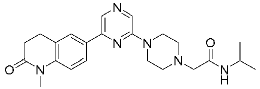 |
| 45 | 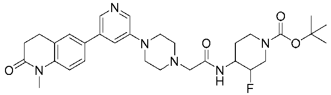 | 46 | 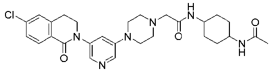 |
| 47 | 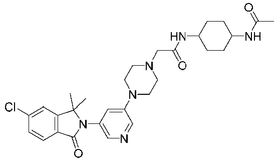 | 48 | 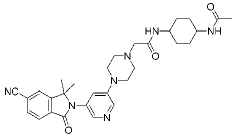 |
| 49 | 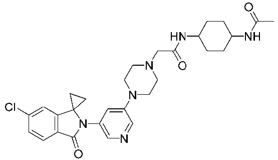 | 50 | 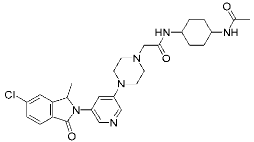 |
| 51 | 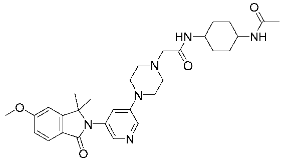 | 52 | 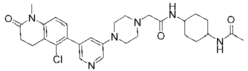 |
| 53 | 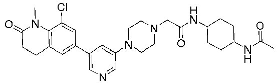 | 54 | 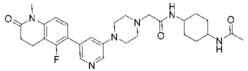 |
| 55 | 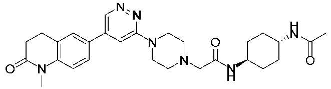 | 56 | 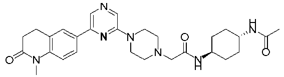 |
| 57 | 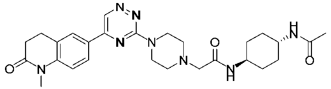 | 58 | 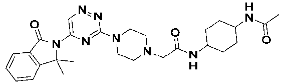 |
| 59 | 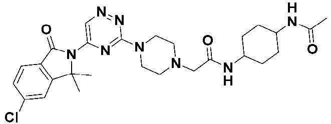 | 60 | 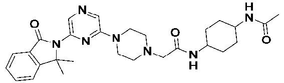 |
| 61 | 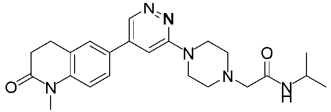 | 62 | 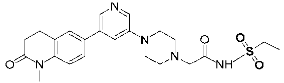 |
| 63 | 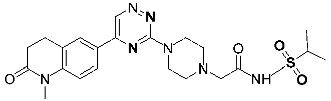 | 64 | 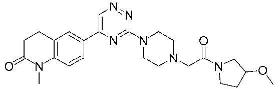 |
| 65 | 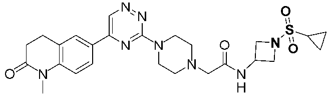 | 66 | 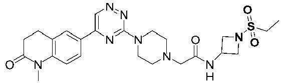 |
| 67 | 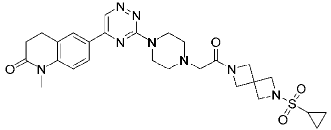 | 68 | 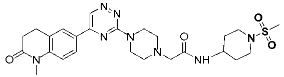 |
| 69 | 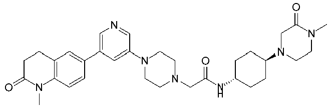 | 70 | 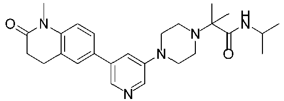 |
| 71 | 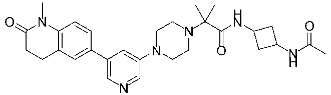 | 72 | 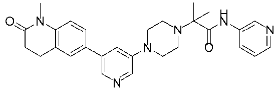 |
| 73 | 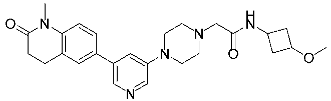 | 74 | 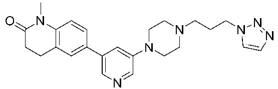 |
| 75 | 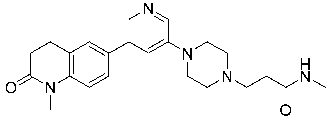 | 76 | 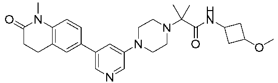 |
| 77 | 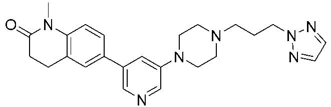 | 78 | 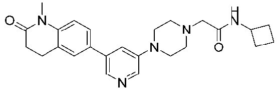 |
| 79 | 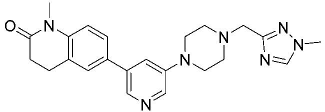 | 80 | 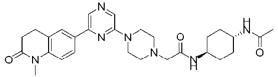 |
| 81 | 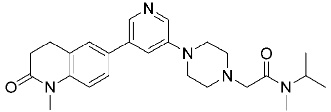 | 82 | 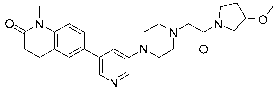 |
| 83 | 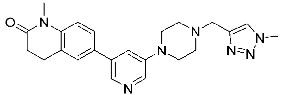 | 84 | 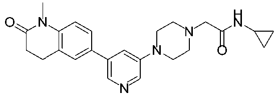 |
| 85 | 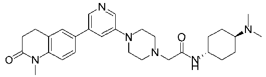 | 86 | 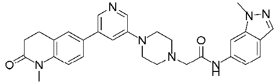 |
| 87 | 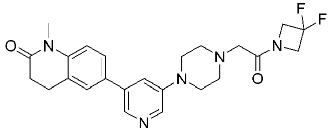 | 88 | 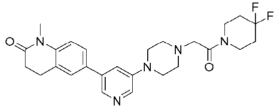 |
| 89 | 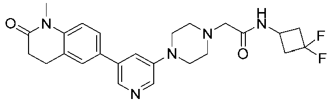 | 90 | 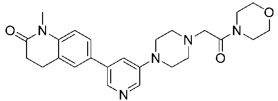 |
| 91 | 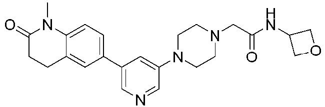 | 92 | 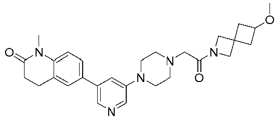 |
| 93 | 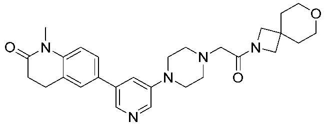 | 94 | 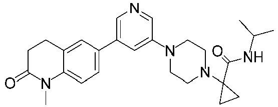 |
| 95 | 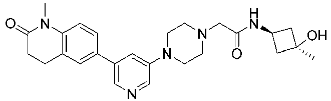 | 96 | 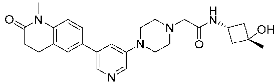 |
| 97 | 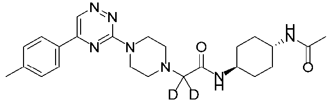 | 98 | 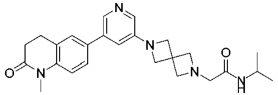 |
| 99 | 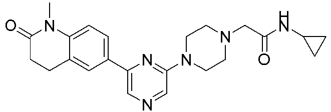 | 100 | 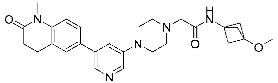 |
| 101 | 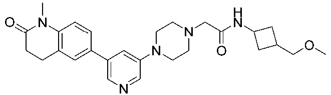 | 102 | 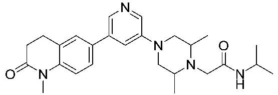 |
| 103 | 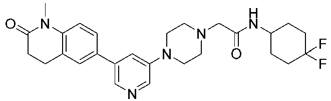 | 104 | 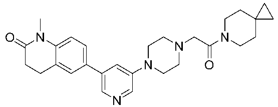 |
| 105 | 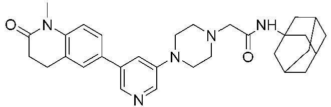 | 106 | 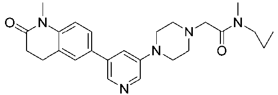 |
| 107 | 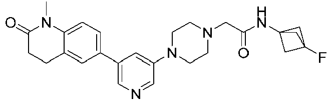 | 108 | 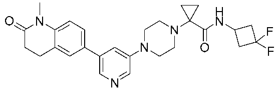 |
| 109 | 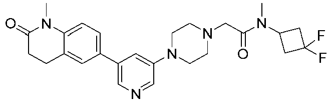 | 110 | 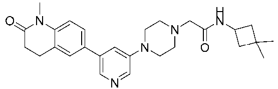 |
| 111 | 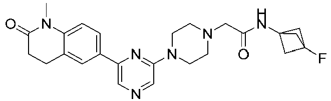 | 112 | 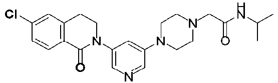 |
| 113 | 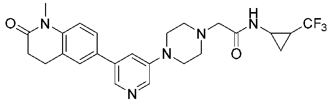 | 114 | 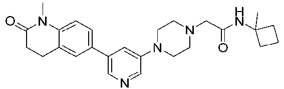 |
| 115 | 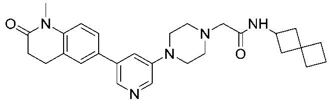 | 116 | 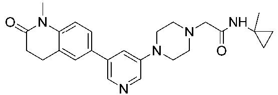 |
| 117 | 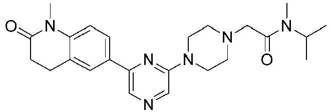 | 118 | 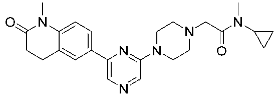 |
| 119 | 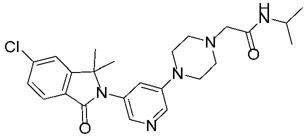 | 120 | 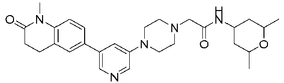 |
| 121 | 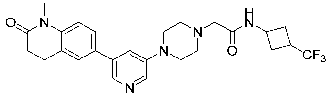 | 122 | 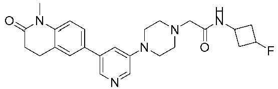 |
| 123 | 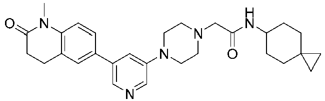 | 124 | 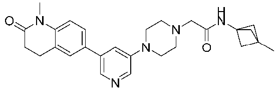 |
| 125 | 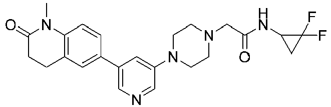 | 126 | 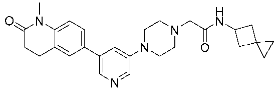 |
| 127 | 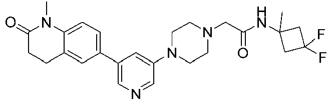 | 128 | 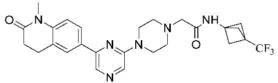 |
| 129 | 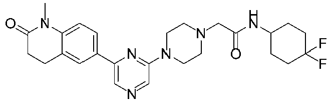 | 130 | 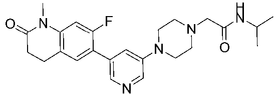 |
| 131 | 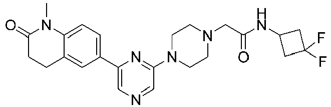 | 132 | 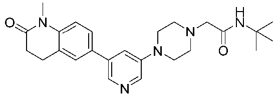 |
| 133 | 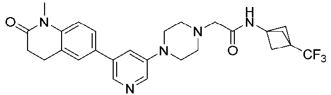 | 134 | 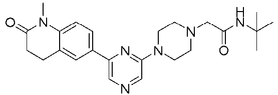 |
| 135 | 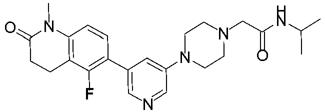 | 136 | 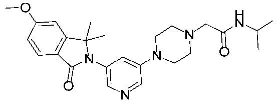 |
| 137 | 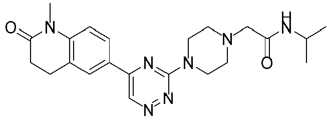 | 138 | 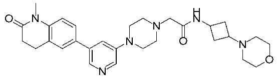 |
| 139 | 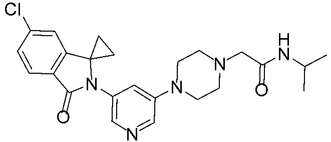 | 140 | 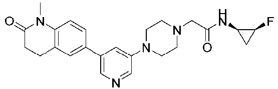 |
| 141 | 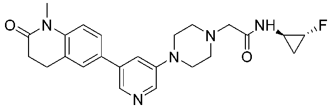 | 142 | 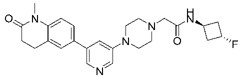 |
| 143 | 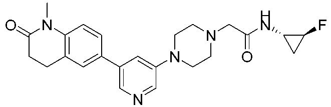 | 144 | 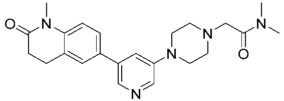 |
| 145 | 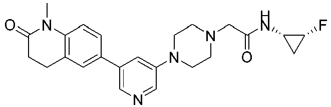 | 146 | 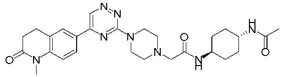 |
| 147 | 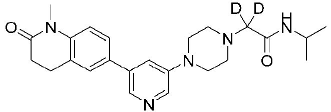 | 148 | 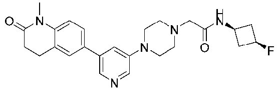 |
| 149 | 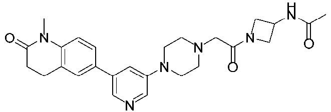 | 150 | 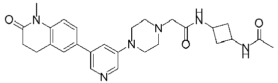 |
| 151 | 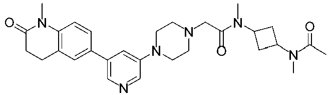 | 152 | 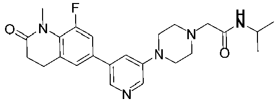 |
| 153 | 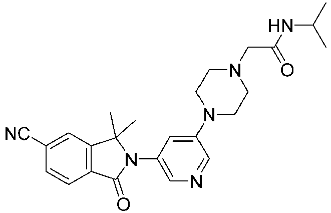 | 154 | 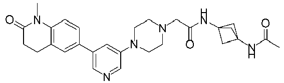 |
| 155 | 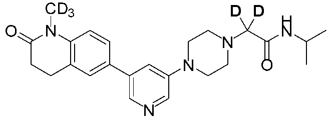 | 156 | 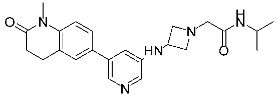 |
| 157 | 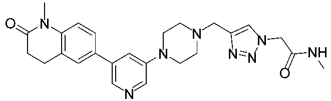 | 158 | 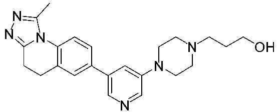 |
| 159 | 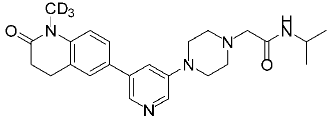 | 160 | 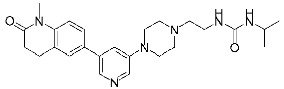 |
| 161 | 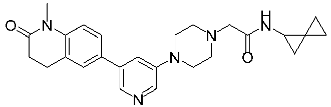 | 162 | 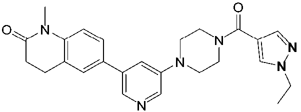 |
| 163 | 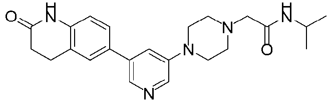 | 164 | 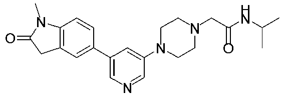 |
| 165 | 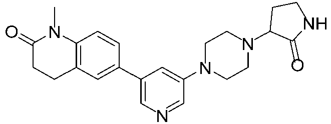 | 166 | 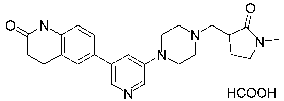 |
| 167 | 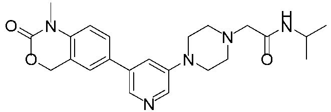 | 168 | 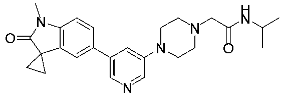 |
| 169 | 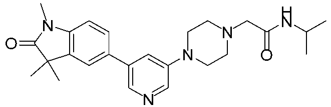 | 170 | 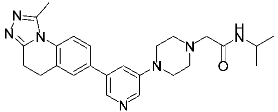 |
| 171 | 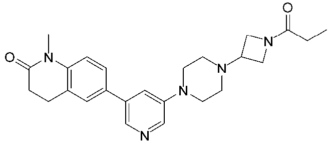 | 172 | 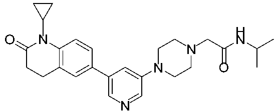 |
| 173 | 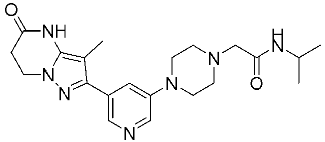 | 174 | 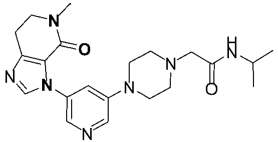 |
| 175 | 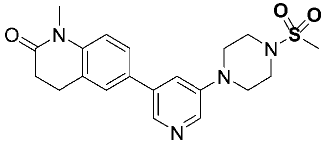 | 176 | 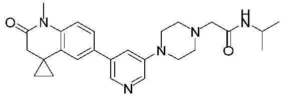 |
| 177 | 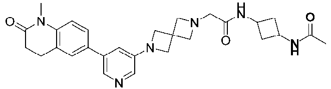 | 178 | 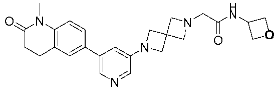 |
| 179 | 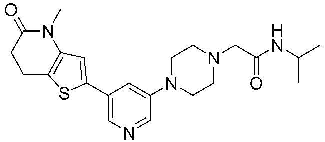 | 180 | 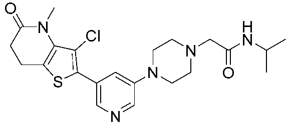 |
| 181 | 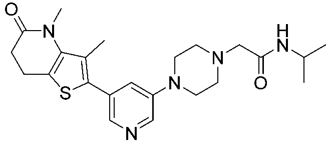 | 182 | 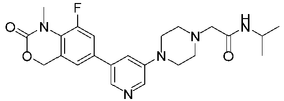 |
| 183 | 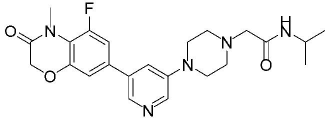 | 184 | 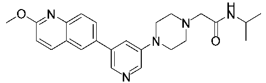 |
| 185 | 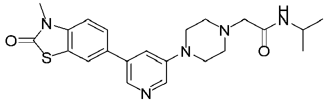 | 186 | 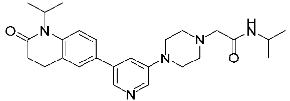 |
| 187 | 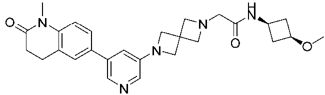 | 188 | 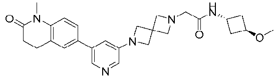 |
| 189 | 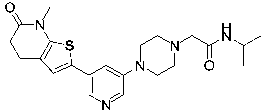 | 190 | 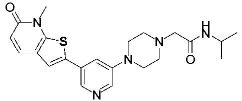 |
| 191 | 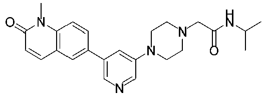 | 192 | 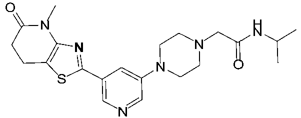 |
| 193 | 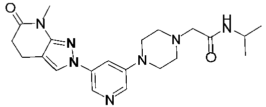 | 194 | 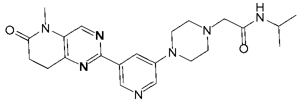 |
| 195 | 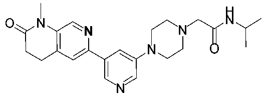 | 196 | 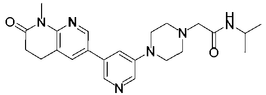 |

14. A pharmaceutical composition, **characterized by** comprising the compound, or the tautomer, stereoisomer, isotopic derivative or pharmaceutically acceptable salt thereof according to any one of claims 1-13.

15. Use of the compound, or the tautomer, stereoisomer, isotopic derivative or pharmaceutically acceptable salt thereof according to any one of claims 1-13, or the pharmaceutical composition according to claim 14 as a medicament or in the manufacture of a medicament; preferably, the medicament is used as a CYP11B2 inhibitor; the medicament is used for the prevention and/or treatment of a CYP11B2-mediated disease; further preferably, the medicament is used for the treatment and/or prevention of a disease or condition caused by aldosterone excess; further preferably, the medicament is used for the treatment and/or prevention of a cardio-cerebrovascular disease or a kidney disease; further preferably, the medicament is used for the treatment and/or prevention of hypertension, hyperaldosteronism, Cushing's disease, heart failure or chronic kidney disease; further preferably, the medicament is used for the treatment and/or prevention of refractory hypertension, hypertension due to primary aldosteronism, primary hyperaldosteronism, Cushing's disease, heart failure or chronic kidney disease.
